(19) Europäisches Patentamt
European Patent Office
Office européen des brevets

(11) Veröffentlichungsnummer: **0 525 516 B1**

(12) **EUROPÄISCHE PATENTSCHRIFT**

(45) Veröffentlichungstag der Patentschrift: **27.09.95**

(21) Anmeldenummer: **92112086.1**

(22) Anmeldetag: **15.07.92**

(51) Int. Cl.[6]: **C07C 69/734**, A01N 37/36, C07C 69/736, C07C 233/11, C07C 251/38, C07C 327/22, A61K 31/21, C07D 277/68, A01N 43/78, C07C 205/56, C07C 69/738, C07C 67/343

(54) **Beta-substituierte Zimtsäurederivate.**

(30) Priorität: **27.07.91 DE 4124989**

(43) Veröffentlichungstag der Anmeldung:
**03.02.93 Patentblatt 93/05**

(45) Bekanntmachung des Hinweises auf die Patenterteilung:
**27.09.95 Patentblatt 95/39**

(84) Benannte Vertragsstaaten:
**AT BE CH DE DK ES FR GB GR IT LI NL PT SE**

(56) Entgegenhaltungen:
**EP-A- 0 009 386**
**EP-A- 0 224 823**
**EP-A- 0 251 082**
**EP-A- 0 342 459**
**DE-A- 1 542 872**
**US-A- 4 296 120**
**EP-A- 0 120 321**
**EP-A- 0 235 722**
**EP-A- 0 260 832**
**EP-A- 0 407 891**
**DE-A- 2 108 234**

(73) Patentinhaber: **BASF Aktiengesellschaft**
**Carl-Bosch-Strasse 38**
**D-67063 Ludwigshafen (DE)**

(72) Erfinder: **Sauter, Hubert, Dr.**
**Neckarpromenade 20,**
**W-6800 Mannheim 1 (DE)**
Erfinder: **Bayer Herbert, Dr.**
**Schwedlerstrasse 122**
**W-6700 Ludwigshafen (DE)**
Erfinder: **Oberdorf, Klaus, Dr.**
**Bienenstrasse 3**
**W-6900 Heidelberg (DE)**
Erfinder: **Wingert, Horst, Dr.**
**D 3,4,**
**W-6800 Mannheim 1 (DE)**
Erfinder: **von Deyn, Wolfgang, Dr.**
**Luederitzstrasse 4**
**W-6730 Neustadt (DE)**
Erfinder: **Grammenos, Wassilios, Dr.**
**Riedsaumstrasse 11**
**W-6700 Ludwigshafen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 525 516 B1

CHEMICAL ABSTRACTS, vol. 117, no. 15, 12. Oktober 1992, Columbus, Ohio, US; abstract no. 150724, Seite 799 ;Spalte 2 ; & JP-A-4 091 065

CHEMICAL ABSTRACTS, vol. 107, no. 23, 7. Dezember 1987, Columbus, Ohio, US; abstract no. 217432, A FRIMER ET AL 'Reaction of coumarins with superoxide anion radical.' Seite 571 ;Spalte 1 ;

JOURNAL OF ORGANIC CHEMISTRY Bd. 56, Nr. 4, 15. Februar 1991, WASHINGTON D.C., U.S. Seiten 1364 - 1373 DESHONG P. ET AL 'A nitrone-based cycloaddition approach to the synthesis of the glycosyl system of nogalomycin,menogaril, and their congeners.'

JOURNAL OF THE CHEMICAL SOCIETY,PERKINS TRANSACTIONS I Nr. 5, 1984, LONDON,G.B. Seiten 1053 - 1059 STAUNTON J ET AL 'Biomimetic synthesis of polyketide aromatics from reaction of an orsellinate anion with pyrones and a pyrylium salt.'

JOURNAL OF THE AMERICAN CHEMICAL SOCIETY Bd. 106, Nr. 10, 1984, WASHINGTON D.C. ,U.S. Seiten 2870 - 2874 CABARET D. ET AL 'Atropisomeric alkenyl cobaloximes. Stereochemical study of the vinyl halide substitution by transition-metal complexes.'

JOURNAL OF ORGANIC CHEMISTRY Bd. 51, Nr. 9, 1986, WASHINGTON D.C.,U.S. Seiten 1402 - 1406 EISENBRAUN E.J. ET AL 'Steric effects in the synthesis of 1,7- dialkylindans'

CHEMISCHE BERICHTE Bd. 103, Nr. 7, 1970, WEINHEIM, DE Seiten 2320 - 2325 RAHMAN A. ET AL 'Notiz über die Totalsynthese von 1-Methyl-2.3-dihydro-1H-cyclopenta(1)phenanthren durch Succinoylierung und Acetylierung von Naphthalin'

TETRAHEDRON LETTERS Nr. 44, 1973, OXFORD,G.B. VISWANATHA ET AL 'Unusual products of oxidation with Cr(VI) of methyl 5-methyl-7 -methoxy-8-isopropyl-3,4-dihydro-2-naphthoate'

CHEMICAL ABSTRACTS, vol. 115, no. 3, 22. Juli 1991, Columbus, Ohio, US; abstract no. 28568, Seite 701 ;Spalte 2 ;

Erfinder: **Koenig, Hartmann, Dr.**
**Albert-Einstein-Allee 16,**
**W-6703 Limburgerhof (DE)**
Erfinder: **Rang, Harald, Dr.**
**Maximilianstrasse 30**
**W-6700 Ludwigshafen (DE)**
Erfinder: **Roehl, Franz, Dr.**
**Sebastian-Kneipp-Strasse 14**
**W-6707 Schifferstadt (DE)**
Erfinder: **Lorenz, Gisela, Dr.**
**Erlenweg 13**
**W-6730 Neustadt (DE)**
Erfinder: **Ammermann, Eberhard, Dr.**
**Sachsenstrasse 3**
**W-6700 Ludwigshafen (DE)**

JOURNAL OF MEDICINAL CHEMISTRY Bd. 20, Nr. 6, 1977, WASHINGTON D.C. Seiten 781 - 791 PARKER R A ET AL '5-(TETRADECYLOXY)-2-FURANCARBOXYLIC ACID AND RELATED HYPOLIPIDEMIC FATTY ACID-LIKE ARYLOXYCARBOXYLIC ACIDS'

ACTA CHEMICA SCADINAVICA Bd. B40, Nr. 4, 1986, COPENHAGEN,DK Seiten 303 - 309 STORFLOR H ET AL 'POTENTIALLY AROMATIC THIOPHENIUM YLIDES 4. FORMATION AND CYCLIZATION OF METHYL AND METHOXYCARBONYL SUBSTITUTED 2-(2- THIENYL)BENZOYLCARBENES'

**Beschreibung**

Die Vorliegende Erfindung betrifft neue β-substituierte Zimtsäurederivate, die die mitochondriale Atmung von Pilzen, Insekten und Spinnmilben hemmen und die antimykotische, insektizide und akarizide Wirkung sowie insbesondere fungizide Wirkung gegen pflanzenpathogene Pilze haben.

Die vorliegende Erfindung betrifft weiterhin Verfahren zur Herstellung solcher Verbindungen und Zwischenprodukte zu ihrer Herstellung sowie Verfahren und Mittel zur Anwendung als Fungizide, Antimykotika, Insektizide und Akarizide, die diese β-substituierten Zimtsäurederivate benützen bzw. enthalten.

Es ist bekannt, Zimtsäureamide, z.B. das β-Phenylzimtsäuremorpholid als Fungizid zu verwenden (DE 3 306 996). Seine fungizide Wirkung ist jedoch unbefriedigend.

Gegenstand der vorliegenden Erfindung sind β-substituierte Zimtsäurederivate der allgemeinen Formel 1,

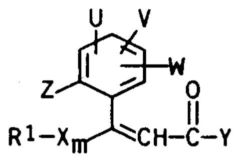

1

in der

$R^1$ gegebenenfalls durch Halogen (F, Cl, Br, J), $C_1$-$C_6$-Alkoxy, $C_1$-$C_6$-Alkylthio oder $C_3$-$C_6$-Cycloalkyl substituiertes $C_1$-$C_8$-Alkyl, $C_2$-$C_8$-Alkenyl, $C_2$-$C_8$-Alkinyl, $C_3$-$C_8$-Cycloalkyl oder $C_3$-$C_8$-Cycloalkenyl bedeutet, und in der $R^1$ zusätzlich auch Chlor oder Brom bedeuten kann, wenn m = 0 ist.

-X- für die Gruppierungen
-O-, -S-,

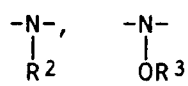

steht,

m 0 oder 1 bedeutet,

-Y für die Gruppen $-OR^4$, $-O-N=CR^5R^6$, $-NR^7R^8$, $-N(OR^9)R^{10}$ oder $-SR^{11}$ steht,

wobei die Substituenten $R^2$ bis $R^{11}$ unabhängig voneinander gleich oder verschieden sind und gegebenenfalls durch Halogen (F, Cl, Br, J), durch $C_1$-$C_6$-Alkoxy, durch $C_1$-$C_6$-Alkylthio oder durch $C_3$-$C_6$-Cycloalkyl substituiertes $C_1$-$C_8$-Alkyl, $C_2$-$C_8$-Alkenyl, $C_2$-$C_8$-Alkinyl, $C_3$-$C_8$-Cycloalkyl oder $C_3$-$C_8$-Cycloalkenyl bedeuten, und die Reste $R^2$, $R^3$ und $R^5$ bis $R^{11}$ zusätzlich Wasserstoff bedeuten, wenn $R^4$ nicht Ethyl oder tert.-Butyl bedeutet, und wenn m = 0 ist,

Halogen (F, Cl, Br, J), Nitro, Cyano, gegebenenfalls substituiertes Alkyl, gegebenenfalls substituiertes Cycloalkyl, gegebenenfalls substituiertes Aralkyl, gegebenenfalls substituiertes Aryloxyalkyl, gegebenenfalls substituiertes Arylthioalkyl, gegebenenfalls substituiertes Heteroarylalkyl, gegebenenfalls substituiertes Heteroaryloxyalkyl, gegebenenfalls substituiertes Heteroarylthioalkyl, gegebenenfalls substituiertes Alkenyl, gegebenenfalls substituiertes Aralkenyl, gegebenenfalls substituiertes Aryloxyalkenyl, gegebenenfalls substituiertes Arylthioalkenyl, gegebenenfalls substituiertes Heteroarylalkenyl, gegebenenfalls substituiertes Heteroaryloxyalkenyl, gegebenenfalls substituiertes Heteroarylthioalkenyl, gegebenenfalls substituiertes Alkinyl, gegebenenfalls substituiertes Arylalkinyl, gegebenenfalls substituiertes Heteroarylalkinyl, gegebenenfalls substituiertes Aryl, gegebenenfalls substituiertes Heteroaryl, gegebenenfalls substituiertes Arylazo, gegebenenfalls substituiertes Acylamino, $-OR^{12}$, $-SR^{13}$, $-SOR^{14}$, $-SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$, $-CH_2-OCOR^{39}$ oder $-NR^{37}R^{38}$ bedeutet,

wobei die Gruppierungen $R^{12}$ bis $R^{28}$, sowie $R^{38}$ und $R^{39}$ gleich oder verschieden sind und Wasserstoff, gegebenenfalls substituiertes Alkyl, gegebenenfalls substituiertes Alkenyl, gegebenenfalls substituiertes Alkinyl gegebenenfalls substituiertes Cycloalkyl, gegebenenfalls substituiertes Cycloalkylalkyl, gegebenenfalls substituiertes Aryl, gegebenenfalls substituiertes Heteroaryl, gegebenenfalls substituiertes Aralkyl, gegebenenfalls substituiertes Heteroarylalkyl, gegebenenfalls substituiertes Aryloxyalkyl, gegebenenfalls

substituiertes Arylthioalkyl, gegebenenfalls substituiertes Heteroaryloxyalkyl oder gegebenenfalls substituiertes Heteroarylthioalkyl bedeuten und $R^{37}$ Wasserstoff oder $C_1$-$C_4$-Alkyl bedeutet und $R^{19}$ nicht Wasserstoff und $R^{12}$ nicht gegebenenfalls substituiertes Alkyl bedeutet, wenn m = 0 ist und in der

U,V,W gleich oder verschieden sind und Wasserstoff bedeuten oder eine der für Z genannten Bedeutungen haben,

oder in der

zwei der Gruppierungen Z, U, V oder W in benachbarten Positionen des Phenylrings zusammen mit den Kohlenstoffatomen, deren Substituenten sie sind, einen gegebenenfalls substituierten, an den Phenylring ankondensierten fünf- oder sechsgliedrigen, aromatischen oder aliphatischen Ring bilden, der ggf. ein bis drei Heteroatome (N, S, O) enthalten kann,

ausgenommen die Verbindungen

- 3-Methoxy-3-[2-methoxyphenyl]acrylsäure methylester,
- 3-Methoxy-3-[(1-hydroxy-3-methyl-6,8-dimethoxy)-naphth-2-yl]-acrylsäure methylester,
- 3-Chlor-3-[2-methyl-naphth-1-yl]-acrylsäure methyl-ester,
- 3-Methyl-3-[2-methyl-naphth-1-yl]-acrylsaure methylester,
- 3-Methyl-3-[(2-isopropylcarbonyl-4-methoxycarbonyl)-phenyl]-acrylsäure methylester,

sowie Verbindungen, in denen Z für $NO_2$, $NH_2$ oder N-Pyrrollidon steht, wenn $R^1X_m$ Methyl bedeutet, U, V und W für Wasserstoff stehen und Y Alkylamino oder Cycloalkylamino bedeutet.

Die Verbindungen 3-Methoxy-3-[2-methoxyphenyl]acrylsäure methylester, 3-Methoxy-3-[(1-hydroxy-3-methyl-6,8-dimethoxy)naphth-2-yl]-acrylsäure methylester, 3-Chlor-3-[2-methyl-naphth-1-yl]-acrylsäuremethyl-ester, 3-Methyl-3-[2-methyl-naphth-1-yl]-acrylsäure methylester und 3-Methyl-3-[(2-isopropylcarbonyl-4-methoxycarbonyl)phenyl]-acrylsäure methylester sind bekannt [Chem. Abstr. 107: 217432e; J. Chem. Soc. PT 1984, 1053; J. Am. Chem. Soc. 106, 2870 (1984); Tetrahedron Lett. 44, 4339 (1973)]. Eine biologische Aktivität dieser Verbindungen ist den betreffenden Referaten jedoch nicht zu entnehmen.

Außerdem werden Verbindungen, in denen Z für $NO_2$, $NH_2$ oder N-Pyrrollidon steht, wenn $R^1X_m$ Methyl bedeutet, U, V und W für Wasserstoff stehen und Y Alkylamino oder Cycloalkylamino bedeutet, als Zwischenprodukte und als Pharmaka zur Behandlung von Krankheiten der Atemwege beschrieben.

Für die in den Verbindungen der Formel I angegebenen Gruppierungen $R^1$ bis $R^{11}$ stehen beispielsweise die folgenden Reste: Methyl, Ethyl, n-Propyl, Isopropyl, tert.-Butyl, n-Octyl, 2-Ethylhexyl, 2-Chlorethyl, 3-Brompropyl, 2-Methoxyethyl, 2-Isopropoxyethyl, 1-Methoxy-prop-2-yl, 2-Ethylthio-ethyl, Cyclopropylmethyl, Cyclohexylmethyl, Allyl, 2-Chlorallyl, Propargyl, 4-Ethoxy-pent-2-in-1-yl, 1-Methylcyclopropyl, Cyclohex-2-en-1-yl.

Bevorzugt sind Reste $R^1$ bis $R^{11}$ mit bis zu 4 Kohlenstoffatomen, besonders bevorzugt Ethyl und insbesondere Methyl. Für die Reste $R^5$ bis $R^{10}$ ist darüber hinaus auch Wasserstoff bevorzugt.

Für die in den Verbindungen der Formel 1 angegebenen Gruppierungen Z und $R^{12}$ bis $R^{28}$, $R^{38}$ und $R^{39}$ bedeutet "Alkyl" als eigenständige Gruppe oder als Teil einer Gruppe (wie z.B. in Alkoxy, Alkylthio, Heteroarylalkyl, Aryloxyalkyl) geradkettiges oder verzweigtes Alkyl mit 1 bis 10 Kohlenstoffatomen, bevorzugt mit 1 bis 6 und besonders bevorzugt mit 1 bis 4 Kohlenstoffatomen, z.B. Methyl, Ethyl, n-Propyl, Isopropyl, n-Butyl, sec-Butyl, Isobutyl, tert.-Butyl, n-Decyl. Cycloalkyl bedeutet z.B. Cycloalkyl mit 3 bis 8 C-Atomen, z.B. Cyclopropyl, Cyclohexyl oder Cyclooctyl.

"Aryl" als Bestandteil innerhalb der Gruppierung Z bzw. $R^{12}$ bis $R^{28}$, $R^{38}$ und $R^{39}$, also z.B. auch als Bestandteil der Gruppierung Arylalkyl, Aryloxyalkyl, bedeutet beispielsweise $\alpha$-Naphthyl, $\beta$-Naphthyl, Phenanthrenyl und vorzugsweise Phenyl.

"Heteroaryl" als Bestandteil innerhalb der Gruppierung Z bzw. $R^{12}$ bis $R^{28}$, $R^{38}$ und $R^{39}$, also z.B. auch als Bestandteil der Gruppierung Heteroarylalkyl, Heteroaryloxyalkyl, Heteroarylalkenyl, bedeutet beispielsweise Furyl, Thienyl, Pyrrolyl, Isoxazolyl, Isothiazolyl, Oxazolyl, Thiazolyl, 1,2,4-Oxadiazolyl, 1,2,4-Thiadiazolyl, 1,3,4-Oxadiazolyl, 1,3,4-Thiadiazolyl, Imidazolyl, Pyrazolyl, 1,2,4-Triazolyl, Pyridinyl, Pyrimidinyl, Pyridazinyl, Pyrazinyl, 1,3,5-Triazinyl, 1,2,4-Triazinyl, Benzoxazolyl, Benzothiazolyl, Chinolyl oder Isochinolyl.

Demzufolge bedeutet Z bzw. $R^{12}$ bis $R^{28}$, $R^{38}$ und $R^{39}$ Aralkyl, beispielsweise $\alpha$-Naphthylmethyl, 2-($\beta$-Naphthyl)-ethyl und vorzugsweise Phenylalkyl (insbesondere Benzyl, 1-Phenylethyl, 2-Phenylethyl, 3-Phenylpropyl, 4-Phenylbutyl und 6-Phenyl-hexyl). Aryloxyalkyl bedeutet demzufolge beispielsweise 4-Phenoxybutyl, 3-Phenoxypropyl, 2-($\beta$-Naphtoxy)ethyl, und insbesondere 1-Phenoxyethyl, 2-Phenoxyethyl und Phenoxymethyl.

Arylthioalkyl bedeutet beispielsweise Phenylthiomethyl. Heteroarylalkyl bedeutet beispielsweise Heteroarylmethyl oder 2-Heteroarylethyl (insbesondere 2- oder 3- oder 4-Pyridinylethyl, 2- oder 3- oder 5- oder 6-Pyrazinylethyl, 2- oder 4- oder 5-Thiazolylethyl, 2- oder 4- oder 5-Oxazolylethyl, 3- oder 4- oder 5-Isoxazolylethyl).

Heteroaryloxyalkyl bedeutet beispielsweise bevorzugt Heteroaryloxymethyl und 2-(Heteroaryloxy)-ethyl, beispielsweise 2-Pyridinyloxymethyl oder 2-(2'-Pyridinyloxy)-ethyl.

Heteroarylthioalkyl bedeutet beispielsweise bevorzugt Heteroarylthiomethyl oder 2-(Heteroarylthio)-ethyl, z.B. Thiazol-2-yl-thiomethyl.

Für die in den Verbindungen der Formel 1 angegebenen Gruppierungen Z und $R^{12}$ bis $R^{28}$, $R^{38}$ und $R^{39}$ bedeutet Alkenyl bzw. Alkinyl als eingeständige Gruppe oder als Teil einer Gruppe (wie z.B. in Alkenyloxy, Heteroarylalkenyl, Arylalkinyl) geradkettiges oder verzweigtes Alkenyl bzw. Alkinyl mit 1 bis 10 Kohlenstoffatomen, bevorzugt mit 1 bis 6 Kohlenstoffatomen und besonders bevorzugt mit 1 bis 4 Kohlenstoffatomen, z.B. Ethenyl, Ethinyl, Allyl, Propargyl, 3-Methyl-pent-2-en-1-yl aber auch Alkenylreste mit mehreren Doppelbindungen wie z.B. Buta-1,3-dien-1-yl, 4-Methyl-penta-1,3-dien-1-yl. (Da die Alkenylgruppe ihrerseits gegebenenfalls wiederum mit Alkenyl substituiert sein kann, können z.B. als Z oder als $R^{12}$ bis $R^{28}$, oder als $R^{38}$ und $R^{39}$ auch größere Polyenylreste auftreten z.B. der Rest 4,8-Dimethyl-nona-1,3,7-trien-1-yl).

Aralkenyl bedeutet beispielsweise 2-($\alpha$-Naphthyl)-ethen-1-yl oder besonders bevorzugt 2-Phenyl-ethen-1-yl. Aryloxyalkenyl bedeutet beispielsweise 2-Phenoxyethen-1-yl. Arylthio-alkenyl bedeutet beispielsweise 2-Phenylthioethen-1-yl. Heteroarylalkenyl bedeutet beispielsweise 2-(3'-Isoxazolyl)-ethen-1-yl, 2-(2'-Thiazolyl)-ethen-1-yl, 2-(2'-Pyridyl)-ethen-1-yl. Aryloxy-alkenyl bedeutet beispielsweise 2-(2'-Pyridyloxy)-ethen-1-yl.

Arylthioalkenyl bedeutet beispielsweise 2-(2'-Thiazolylthio)-ethen-1-yl. Arylalkinyl bedeutet beispielsweise bevorzugt Phenylethinyl. Heteroarylalkinyl bedeutet beispielsweise (2-Pyrimidinyl)-ethinyl.

Als Substituenten für die gegebenenfalls substituierte Acylaminogruppe sind neben Wasserstoff insbesondere gegebenenfalls substituiertes Alkyl und Aryl zu nennen.

Als Substituenten, von denen einer oder mehrere (gleich oder verschieden) gegebenenfalls in den für Z sowie für $R^{12}$ bis $R^{28}$, $R^{38}$ und $R^{39}$ genannten Gruppierungen - und dort bevorzugt innerhalb der Aryl- bzw. Heteroarylreste - anwesend sein können, seien insbesondere die folgenden genannt:

Halogen, Hydroxy, $C_1$-$C_6$-Alkyl (besonders Methyl, Ethyl, Isopropyl und t-Butyl), $C_2$-$C_6$-Alkenyl (besonders Allyl und Methallyl), $C_2$-$C_6$-Alkinyl (besonders Propargyl), $C_1$-$C_6$-Alkoxy (besonders Methoxy, Ethoxy, Isopropoxy, t-Butoxy), $C_3$-$C_6$-Alkenyloxy (besonders Allyloxy, Methallyloxy, jedoch nicht bevorzugt Alkenyloxysubstituenten mit Enoletherstruktur), $C_3$-$C_6$-Alkinyloxy (besonders Propargyloxy, jedoch nicht bevorzugt Alkinyloxysubstituenten mit Inoletherstruktur), Halogen-$C_1$-$C_6$-alkyl (besonders Trifluormethyl, Trichlormethyl, Chlormethyl und Brommethyl), Halogen-$C_1$-$C_6$-alkoxy (besonders Trifluormethoxy), $C_1$-$C_6$-Alkylthio (besonders Methylthio), Hydroxy-$C_1$-$C_6$-alkyl (besonders Hydroxymethyl), $C_1$-$C_4$-Alkoxy-$C_1$-$C_6$-alkyl (besonders 2-Methoxyethyl, 2-Methoxypropyl, 3-Methoxypropyl, 2-Ethoxyethyl), $C_3$-$C_6$-Cycloalkyl, $C_3$-$C_6$-Cycloalkyl-$C_1$-$C_6$-alkyl (besonders Cyclopropylmethyl, Cyclohexylmethyl), Oximinoalkylgruppen der Formel $R^{40}$-O-N=C($R^{41}$)-, in welcher $R^{40}$ Wasserstoff, gegebenenfalls substituiertes $C_1$-$C_{10}$-Alkyl, gegebenenfalls substituiertes $C_2$-$C_{10}$-Alkenyl, gegebenenfalls substituiertes $C_2$-$C_{10}$-Alkinyl, gegebenenfalls substituiertes Aralkyl (insbesondere gegebenenfalls mit Halogen, $C_1$-$C_4$-Alkyl, Trifluormethyl und/oder $C_1$-$C_4$-Alkoxy substituiertes Benzyl) und $R^{41}$ Wasserstoff oder $C_1$-$C_{10}$-Alkyl bedeutet, Formyl, $C_2$-$C_{11}$-Alkanoyl (insbesondere Acetyl, Propionyl, Isobutyryl und Pivaloyl), Iminoalkylgruppen der Formel $R^{42}$-N=C($R^{43}$)- in welcher $R^{42}$ $C_1$-$C_{10}$-Alkyl (insbesondere Isopropyl, tert.-Butyl) oder gegebenenfalls substituiertes Phenyl oder gegebenenfalls substituiertes Benzyl und $R^{43}$ Wasserstoff oder $C_1$-$C_{10}$-Alkyl bedeutet, Cyano, Thiocyanato, $C_1$-$C_8$-Acyloxy (insbesondere Acetyloxy und gegebenenfalls substituiertes Benzoyloxy), Nitro, gegebenenfalls substituiertes Aryl (insbesondere gegebenenfalls substituiertes Phenyl); gegebenenfalls substituiertes Heteroaryl (insbesondere gegebenenfalls substituiertes Pyridinyl, Pyrimidinyl, Thiazolyl, Oxazolyl und Isoxazolyl), gegebenenfalls substituiertes Aryloxy (insbesondere gegebenenfalls substituiertes Phenoxy), gegebenenfalls substituiertes Heteroaryloxy (insbesondere gegebenenfalls substituiertes Pyridinyloxy oder Pyrimidinyloxy), gegebenenfalls substituiertes Arylthio (insbesondere gegebenenfalls substituiertes Phenylthio), gegebenenfalls substituiertes Heteroarylthio (insbesondere gegebenenfalls substituiertes Pyridinylthio, Pyrimidinylthio oder Thiazolylthio), gegebenenfalls substituiertes Aryl-$C_1$-$C_4$-alkyl (insbesondere gegebenenfalls substituiertes Benzyl), gegebenenfalls substituiertes Heteroaryl-$C_1$-$C_4$-alkyl (insbesondere gegebenenfalls substituiertes Pyridinyl-, Pyrimidinyl-, Thiazolyl-, Oxazolyl- oder Isoxazolyl-substituiertes $C_1$-$C_4$-Alkyl), gegebenenfalls substituiertes Aryl-$C_1$-$C_4$-alkoxy (insbesondere gegebenenfalls substituiertes Benzyloxy), gegebenenfalls substituiertes Heteroaryl-$C_1$-$C_4$-alkoxy (insbesondere gegebenenfalls substituiertes Pyridinylmethyloxy, Pyrimidinylmethyloxy, Thiazolylmethyloxy, Oxazolylmethyloxy oder Isoxazolylmethyloxy), gegebenenfalls substituiertes Aryloxy-$C_1$-$C_4$-alkyl (insbesondere gegebenenfalls substituiertes Phenoxymethyl, 1-Phenoxyethyl oder 2-Phenoxyethyl), gegebenfalls substituiertes Arylthio-$C_1$-$C_4$-alkyl (insbesondere gegebenenfalls substituiertes Phenylthiomethyl), gegebenenfalls substituiertes Heteroaryloxy-$C_1$-$C_4$-alkyl (insbesondere Pyridinyloxymethyl oder Pyrimidinyloxymethyl), gegebenenfalls substituiertes Heteroarylthio-$C_1$-$C_4$-alkyl (insbesondere Pyridinylthiomethyl, Pyrimidinylthiomethyl oder Thiazolylthiomethyl), $-NR^{44}R^{45}$, $-NR^{44}COR^{45}$, $-NHCONR^{44}R^{45}$, $-CONR^{44}R^{45}$, $-CH_2ON=CR^{44}R^{45}$, $-COOR^{44}$, $-OSO_2R^{44}$, $-SO_2R^{44}$, $-SO_2NR^{44}R^{45}$,

$-NR^{44}SO_2R^{45}$, $-N=CR^{44}R^{45}$, worin $R^{44}$ und $R^{45}$ unabhängig voneinander Wasserstoff, $C_1$-$C_6$-Alkyl, $C_3$-$C_6$-Cycloalkyl, $C_3$-$C_6$-Cycloalkyl-$C_1$-$C_4$-alkyl, gegebenenfalls substituiertes Aryl (insbesondere gegebenenfalls substituiertes Phenyl) oder gegebenenfalls substituiertes Aryl-$C_1$-$C_4$-alkyl (insbesondere gegebenenfalls substituiertes Benzyl) bedeuten und worin die gegebenenfalls substituierten Aryl bzw. Heteroarylgruppen gegebenenfalls solche Substituenten tragen können, wie die oben für die Aryl- bzw. Heteroarylreste innerhalb der Gruppierungen Z, $R^{12}$ bis $R^{28}$, $R^{38}$ bzw. $R^{39}$ genannten Substituenten.

Innerhalb dieses für die Gruppierungen Z, $R^{12}$ bis $R^{28}$, $R^{38}$ bzw. $R^{39}$ genannten Rahmens sind diejenigen Gruppierungen Z, $R^{12}$ bis $R^{28}$, $R^{38}$ bzw. $R^{39}$ bevorzugt, in denen die Anzahl der darin vorhandenen Kohlenstoffatome - unabhängig von Art und Zahl der möglichen Heteroatome - 28 nicht übersteigt. Verbindungen der Formel 1, die Reste Z, $R^{12}$ bis $R^{28}$, $R^{38}$ bzw. $R^{39}$ mit höherer Kohlenstoffanzahl haben, können zwar ebenfalls noch biologische, z.B. fungizide Aktivität zeigen; ihrer Wirkung bzw. ihrer praktischen Anwendung stehen jedoch bisweilen zu hohe Lipophilie und/oder zu geringe Löslichkeit und/oder besonders schwierige Herstellbarkeit im Wege.

Weiterhin sind solche Verbindungen der Formel 1 besonders wirksam und deshalb hinsichtlich der Wirkung bevorzugt, in denen die Gesamtzahl der in den Gruppierungen Z, $R^{12}$ bis $R^{28}$, $R^{38}$ bzw. $R^{39}$ vorhandenen Kohlenstoffatome - unabhängig von Art und Zahl der möglichen Heteroatome - mehr als 4 beträgt. Verbindungen der Formel 1, in denen die Gruppierungen Z, $R^{12}$ bis $R^{28}$, $R^{38}$ bzw. $R^{39}$ eine geringere Zahl von Kohlenstoffatomen aufweisen, können allerdings ebenfalls noch biologische, z.B fungizide Aktivität aufweisen. Von besonderem Interesse sind solche erfindungsgemäßen Verbindungen der Formel 1 jedoch nicht nur wegen ihrer biologischen Wirkung sondern vielmehr auch wegen ihrer häufig besonders guten Verwendbarkeit als Zwischenprodukte zur Synthese von anderen, noch wirksameren Verbindungen der Formel 1.

Zu den als Zwischenprodukte besonders bevorzugten gehören beispielsweise folgende Verbindungen der Formel 1, in der Z folgende Bedeutungen hat:
Cl, Br, Cyano, -SH, $-NH_2$, $-CH_3$, $-C_2H_5$, $-CH_2Cl$, $-CH_2Br$, $CH_2OCH_3$, $-CH_2OH$, -Acetyl, $-CH=CH_2$, $-C{\equiv}CH$, $-O-CH_2-CH=CH_2$ und in der Z auch CHO, OH oder COOH bedeuten kann, falls m = 1 ist.

Aus derartigen Verbindungen der Formel 1 lassen sich durch synthetischen Aufbau der Seitenkette Z andere, noch wirksamere Verbindungen der Formel 1 herstellen, in denen z.B. die Zahl der in Z anwesenden Kohlenstoffatome im oben genannten, bevorzugten Bereich von mehr als 4 bis zu 28 C-Atomen liegt.

Die erfindungsgemäßen, β-substituierten Zimtsäurederivate können als E/Z-Isomere vorliegen. Beide Isomere werden von der Erfindung umfaßt, wobei grundsätzlich diejenigen Isomeren bevorzugt sind, in denen die Gruppe $R^1$-$X_m$- relativ zur Gruppe COY trans-ständig ist. Dies sind nach der Cahn-Ingold-Prelog-Nomenklatur (siehe z.B. J. March, "Advanced Organic Chemistry", 3rd edition, Wiley-Intenscience, S. 109 ff) für den Fall m = 1 die E-Isomeren, für den Fall m = 0 die Z-Isomeren.

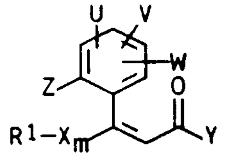

"trans"

E-Isomer (wenn m=1)

Z-Isomer (wenn m=0)

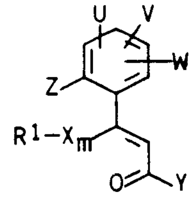

"cis"

Z-Isomer (wenn m=1)

E-Isomer (wenn m=0)

In den Beispielen für die einzelnen Verbindungen (siehe Tabellen 1, 1a und 1b) werden der Einfachheit halber die Isomeren im o.a. Sinne als "cis" bzw. "trans" bezeichnet.

Die Isomeren können nach üblichen Verfahren wie z.B. Kristallisation oder Chromatographie getrennt werden. Für die erfindungsgemäßen Anwendungen als fungizide, akarizide, insektizide oder antimykotische Wirkstoffe sind die "trans"-Isomeren bevorzugt. Sofern bei der Synthese Isomerengemische anfallen, ist eine Trennung jedoch nicht unbedingt erforderlich, da sich die einzelnen Isomeren teilweise während der erfindungsgemäßen, praktischen Anwendung (etwa als Fungizide unter Freilandbedingungen) ineinander umwandeln können, z.B. unter Licht-, Säure-oder Baseneinwirkung, speziell auch in vivo.

Sowohl die einzelnen Isomeren als auch ihre Mischungen werden von der Erfindung umfaßt und sind biologisch wirksam.

Bestandteil der Erfindung sind unter anderen insbesondere Verbindungen der Formel 1, in der X Sauerstoff oder Schwefel bedeutet und m = 0 oder m = 1 ist.

Weiterhin sind Bestandteil der Erfindung unter anderen insbesondere solche Verbindungen der Formel 1, in der $R^1$ geradkettiges oder verzweigtes $C_1$-$C_4$-Alkyl bedeutet.

Besonders bevorzugt sind Verbindungen der Formel 1 in denen die Gruppe $R^1$-$X_m$- für Methoxy, Methyl oder Ethyl steht.

Weiterhin sind Bestandteil der Erfindung unter anderen solche Verbindungen der Formel 1, in der Y für die Gruppen $OR^4$ oder $NR^7R^8$ steht, und $R^4$, $R^7$ und $R^8$ die eingangs angegebenen Bedeutungen haben, wobei diejenigen Verbindungen bevorzugt sind, in denen $R^4$, $R^7$ bzw. $R^8$ gleich oder verschieden geradkettiges oder verzweigtes $C_1$-$C_4$-Alkyl bedeuten. Besonders bevorzugt sind Verbindungen der Formel 1 in der Y für Methoxy steht. Diese zeigen überraschenderweise eine besonders niedrige Säugetiertoxizität.

Ein weiterer Bestandteil der Erfindung sind unter anderen solche Verbindungen der Formel 1, in der U, V und W gleich oder verschieden Fluor, Chlor, Brom, Jod, Cyano, Nitro, Trifluormethyl, Methyl, Methoxy oder bevorzugt Wasserstoff bedeuten.

Weiterhin sind Bestandteil der Erfindung unter anderen solche Verbindungen der Formel 1, in der Z für die Gruppe $OR^{12}$ oder $SR^{13}$ steht und $R^{12}$ und $R^{13}$ die oben angegebene Bedeutung haben können, jedoch bevorzugt für gegebenenfalls substituiertes Aryl oder gegebenenfalls substituiertes fünf- oder sechsgliedriges Heteroaryl mit 1 bis 3 Heteroatomen (N, O, S) stehen.

Weiterhin sind Bestandteil der Erfindung unter anderen insbesondere Verbindungen der Formel 1a,

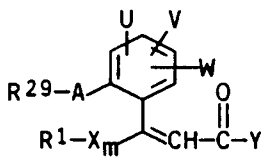

1a

in der U, V, W, $R^1$, $X_m$ und Y die oben angegebenen Bedeutungen haben können und -A- für die Gruppierungen -C≡C-, $-CR^{30}=CR^{31}-$, $-CHR^{30}-CHR^{31}-$, $-O-CHR^{31}-$, -O-CO-, $-NR^{30}CO-$, $-S-CHR^{31}-$, $-N=CR^{31}-$, $-COO-CHR^{31}-$, $-O-N=CR^{31}-$, $-R^{30}C=N-O-CHR^{31}-$, $-CR^{30}=N-$, -N=N- oder $-CHR^{31}-$ steht und in der $R^{29}$ gegebenenfalls substituiertes Alkyl, gegebenenfalls substituiertes Alkenyl, gegebenenfalls substituiertes Alkinyl, gegebenenfalls substituiertes Aralkyl, gegebenenfalls substituiertes Heteroarylalkyl, gegebenenfalls substituertes Aryloxyalkyl, gegebenenfalls substituiertes Heteroaryloxyalkyl oder Heteroarylthioalkyl sowie bevorzugt gegebenenfalls substituiertes Aryl oder gegebenenfalls substituiertes Heteroaryl bedeutet und $R^{30}$ und $R^{31}$ gleich oder verschieden geradkettiges oder verzweigtes $C_1$-$C_4$-Alkyl oder bevorzugt Wasserstoff bedeuten.

Sofern eine der für -A- genannten Gruppierungen eine -C = C-, -C = N-, -N = C-oder -N = N-Doppelbindung enthält, sind diejenigen Isomeren bevorzugt, in denen $R^{29}$ relativ zum Phenylring des Zimtsäurerestes trans-ständig ist.

Weiterhin sind Bestandteil der Erfindung insbesondere solche Verbindungen der Formel 1a, in der $R^{29}$ fünf- oder sechsgliedriges, gegebenenfalls ein- oder mehrfach mit Halogen, $C_1$-$C_{12}$-Alkyl, $C_1$-$C_{12}$-Halogenalkyl, $C_1$-$C_{12}$-Alkenyl, $C_1$-$C_{12}$-Alkinyl, gegebenenfalls substituiertem Aryl, gegebenenfalls substituiertem Heteroaryl, gegebenenfalls substituiertem Aralkyl, $C_1$-$C_{12}$- Alkoxy, $C_1$-$C_{12}$-Haloalkoxy, $C_1$-$C_{12}$-Alkenyloxy, $C_1$-$C_{12}$-Alkinyloxy, gegebenenfalls substituiertem Aryloxy, Formyl, $C_1$-$C_{12}$-Acyl, Cyano, Trifluormethyl, Nitro oder mit der Gruppe $-CR^{32}=N-OR^{33}$ substituiertes und/oder gegebenenfalls mit einem Benzolring anelliertes Aryl oder Heteroaryl bedeutet, wobei $R^{32}$ für Wasserstoff oder $C_1$-$C_4$-Alkyl und $R^{33}$ für Wasserstoff, $C_1$-$C_8$-Alkyl, $C_2$-$C_8$-Alkenyl oder $C_2$-$C_8$-Alkinyl steht.

Besonders bevorzugte Verbindungen der Formel 1 a sind solche, in der $R^{29}$ gegebenenfalls ein oder mehrfach mit Halogen, $C_1$-$C_{12}$-Alkyl, $C_1$-$C_{12}$-Alkenyl, gegebenenfalls substituiertem Aryl, gegebenenfalls substituiertem Heteroaryl, gegebenenfalls substituiertem Aralkyl, $C_1$-$C_{12}$-Alkoxy, $C_1$-$C_{12}$-Halogenalkoxy, $C_1$-$C_{12}$-Alkenyloxy, $C_1$-$C_{12}$-Alkinyloxy, gegebenenfalls substituiertem Aryloxy, Formyl, $C_1$-$C_{12}$-Acyl, Cyano, Trifluormethyl, Nitro oder mit der Gruppe $-CR^{32}=NOR^{33}$ substituiertes und/oder gegebenenfalls mit einem Benzolring anelliertes Phenyl, Furyl, Thienyl, Pyrrolyl, Isoxazolyl, Isothiazolyl, Oxazolyl, Thiazolyl, 1,2,4-Oxadiazolyl, 1,2,4-Thiazdiazolyl, 1,3,4-Oxadiazolyl, 1,3,4-Thiadiazolyl, Imidazolyl, Pyrazolyl, 1,2,4-Triazolyl, 1,3,4-Triazolyl, Pyridinyl, Pyrimidinyl, Pyridazinyl, Pyrazinyl, 1,3,5-Triazinyl oder 1,2,4-Triazinyl bedeutet.

Weiterhin sind Bestandteil der Erfindung Verbindungen der Formel 1 a, in der U, V und W gleich oder verschieden Fluor, Chlor, Brom, Jod, Cyano, Nitro, Trifluormethyl, Methyl oder Methoxy oder bevorzugt Wasserstoff bedeuten.

Bestandteil der Erfindung sind weiterhin bevorzugt Verbindungen der Formel 1a, in der $R^1$-$X_m$- für Methoxy, Methyl oder Ethyl steht.

Bestandteil der Erfindung sind weiterhin Verbindungen der Formel 1a, in der Y für die Gruppen $OR^4$ oder $NR^7R^8$ steht und $R^4$, $R^7$ und $R^8$ gleich oder verschieden geradkettiges oder verzweigtes $C_1$-$C_4$-Alkyl bedeuten.

Besonders bevorzugt sind dabei diejenigen, in denen Y Methoxy bedeutet.

Weiterhin sind Bestandteil der Erfindung Verbindungen der Formel 1 b,

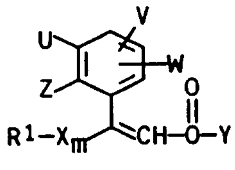

1b

in der Z und U in benachbarten Positionen des Phenylrings stehen und zusammen mit den Kohlenstoffatomen, deren Substituenten sie sind, einen gegebenenfalls substituierten fünf- oder sechsgliedrigen aromatischen Ring bilden, der gegebenenfalls ein bis drei Heteroatome (N, O oder S) enthalten kann und in der die Symbole $R^1$, $X_m$, Y, V und W die oben angegebenen Bedeutungen haben können.

Bevorzugt sind Verbindungen der Formel 1b, in der die Gruppe

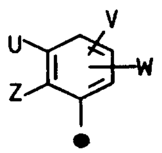

für folgende anellierte Ringsysteme steht:

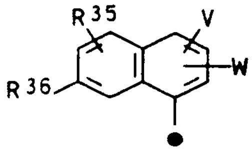
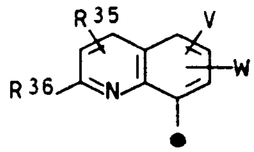
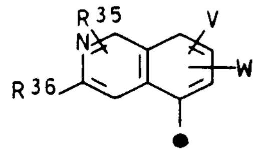
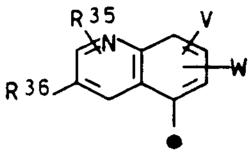
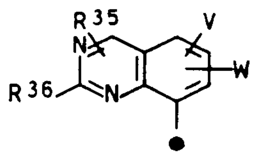
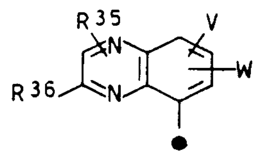
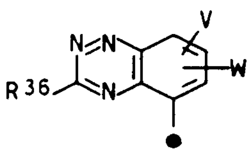
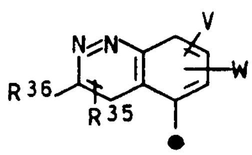
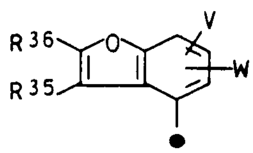
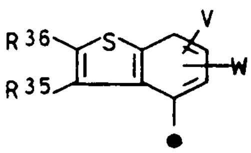
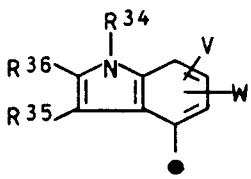
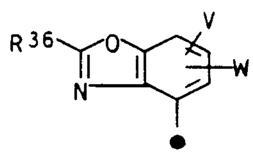
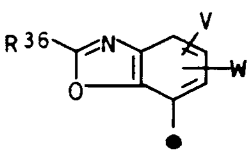
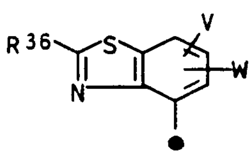
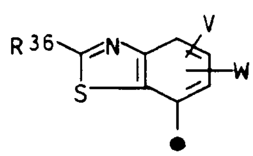
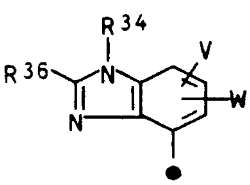
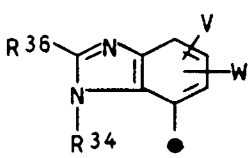

wobei $R^{36}$ die oben für Z genannten Bedeutungen hat, $R^{34}$ Wasserstoff oder $C_1$-$C_4$-Alkyl bedeutet und $R^{35}$ Wasserstoff, Methyl, Methoxy, Trifluormethyl, Fluor, Chlor, Brom, Cyan oder Nitro bedeutet.

Besonders bevorzugt sind hier wiederum solche Verbindungen der Formel 1b, in denen $R^{36}$ diejenigen Bedeutungen hat, die oben bei Z als besonders bevorzugt genannt sind oder Trialkylsilylethinyl bedeutet.

Beispiele für erfindungsgemäße Verbindungen der Formel 1 sind in der folgenden Tabelle 1 angegeben.

Beispiele für erfindungsgemäße Verbindungen der Formel 1a enthält die anschließende Tabelle 1a.

Beispiele für erfindungsgemäße Verbindungen der Formel 1b sind in Tabelle 1b aufgelistet.

Hinweise zur Kennzeichnung der jeweiligen Isomeren in der Spalte "Isomer" der Tabellen als "trans" bzw. "cis" siehe oben (Seite 7). Die strukturelle Zuordnung ist für die der Verbindung Nr. 1.1 der Tabelle 1 entsprechende freie Carbonsäure (mit Y = OH anstelle von Methoxy), die ihrerseits durch Verseifung aus der Verbindung 1.1 der Tabelle 1 hervorging, sowie für die Verbindung Nr. 1a.310 der Tabelle 1a durch

Röntgenstrukturanalyse abgesichert. Die Isomerenzuordnung läßt sich demnach leicht $^{1}$H-NMR-spektroskopisch treffen. In den trans-Isomeren erscheint das olefinische Proton in $\alpha$-Position zur Gruppe COY gegenüber dem entsprechenden Proton im entsprechenden cis-Isomeren nach tieferem Feld verschoben.

Die Angaben zum $^{1}$H-NMR-Spektrum in den folgenden Tabellen beziehen sich auf das olefinische Proton in $\alpha$-Position zur Gruppe COY; die Messungen erfolgten - sofern nicht anders vermerkt - in $CDCl_3$ als Lösungsmittel.

Tabelle 1

Verbindungen der Formel 1

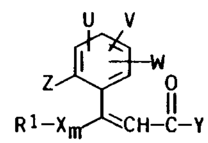

1

| Verb.Nr. | Z- | U,V,W | $R^1-X_m-$ | -Y | Isomer | Fp. ; (°C) | $^1H$-NMR (ppm) |
|---|---|---|---|---|---|---|---|
| 1.1 | Methyl | H,H,H | Methoxy | Methoxy | trans | Öl; | 5,34 |
| 1.2 | Methyl | H,H,H | Methoxy | n-Butoxy | trans | | |
| 1.3 | Chlormethyl | H,H,H | Methoxy | Methoxy | trans | | |
| 1.4 | Brommethyl | H,H,H | Methoxy | Methoxy | trans | 55 - 57; | 5,41 |
| 1.5 | Formyl | H,H,H | Methoxy | Methoxy | trans | 56 - 58; | 5,49 |
| 1.6 | HO-N=CH- | H,H,H | Methoxy | Methoxy | trans | | |
| 1.7 | Methyl | H,H,H | Methoxy | Ethoxy | trans | | |
| 1.8 | Methyl | H,H,H | Methoxy | N-Propoxy | trans | | |
| 1.9 | Methyl | H,H,H | Methoxy | Iso-Propoxy | trans | | |
| 1.10 | Hydroxy | H,H,H | Methoxy | Methoxy | trans | | |
| 1.11 | Hydroxymethyl | H,H,H | Methoxy | Methoxy | trans | | |
| 1.12 | HS- | H,H,H | Methoxy | Methoxy | trans | | |
| 1.13 | Cl | H,H,H | Methoxy | Methoxy | trans | Öl; | 5,40 |
| 1.14 | Br | H,H,H | Methoxy | Methoxy | trans | Öl; | 5,37 |
| 1.15 | N≡C- | H,H,H | Methoxy | Methoxy | trans | | |
| 1.16 | Ethyl | H,H,H | Methoxy | Methoxy | trans | | |
| 1.17 | Ethenyl | H,H,H | Methoxy | Methoxy | trans | | |

Tabelle 1 (Fortsetzung)

| Verb.Nr. | Z- | U,V,W | $R^1-X_m-$ | -Y | Isomer | Fp. ; (°C) | $^1$H-NMR (ppm) |
|---|---|---|---|---|---|---|---|
| 1.18 | Ethinyl | H,H,H | Methoxy | Methoxy | trans | | |
| 1.19 | Allyl | H,H,H | Methoxy | Methoxy | trans | | |
| 1.20 | Propargyl | H,H,H | Methoxy | Methoxy | trans | | |
| 1.21 | Allyloxy | H,H,H | Methoxy | Methoxy | trans | | |
| 1.22 | Acetyl | H,H,H | Methoxy | Methoxy | trans | | |
| 1.23 | Amino | H,H,H | Methoxy | Methoxy | trans | | |
| 1.24 | HO-CO- | H,H,H | Methoxy | Methoxy | trans | | |
| 1.25 | Undec-1-en-1-yl | H,H,H | Methoxy | Methoxy | trans | | |
| 1.26 | 4,8-Dimethyl-nona-1,3,7-<br>-trien-1-yl | H,H,H | Methoxy | Methoxy | trans | | |
| 1.27 | 3-Phenyl-isoxazol-5-yl | H,H,H | Methoxy | Methoxy | trans | | |
| 1.28 | 3-Heptyl-isoxazol-5-yl | H,H,H | Methoxy | Methoxy | trans | | |
| 1.29 | 3-(1-Ethylpentyl)-isoxazol-5-yl | H,H,H | Methoxy | Methoxy | trans | | |
| 1.30 | Phenyl | H,H,H | Methoxy | Methoxy | trans | | |
| 1.31 | 2-Phenoxy-ethoxy | H,H,H | Methoxy | Methoxy | trans | | |
| 1.32 | 3-(2,6-Difluorphenoxy)-<br>-prop-1-oxy | H,H,H | Methoxy | Methoxy | trans | | |
| 1.33 | 4-(2-Cyanophenoxy)-but-1-oxy | H,H,H | Methoxy | Methoxy | trans | | |
| 1.34 | 4-(3-Nitrophenoxy)-but-2-<br>-en-2-oxy | H,H,H | Methoxy | Methoxy | trans | | |
| 1.35 | 5-(2-Methylphenoxy)-pent-1-<br>-en-1-yl | H,H,H | Methoxy | Methoxy | trans | | |
| 1.36 | 4-Methylphenyl-sulfonyloxy-methyl | H,H,H | Methoxy | Methoxy | trans | | |
| 1.37 | Methyl-sulfonyloxy-methyl | H,H,H | Methoxy | Methoxy | trans | | |

Tabelle 1 (Fortsetzung)

| Verb.Nr. | Z- | U,V,W | $R^1-X_m-$ | -Y | Isomer | Fp. ; (°C) | $^1H$-NMR (ppm) |
|---|---|---|---|---|---|---|---|
| 1.38 | Methyl | H,H,H | Methyl | Methoxy | trans | öl; | 6,01 |
| 1.39 | Methoxymethyl | H,H,H | Methyl | Methoxy | trans | öl; | 6.01 |
| 1.40 | Brommethyl | H,H,H | Methyl | Methoxy | trans | öl; | 6,08 |
| 1.41 | Methyl | H,H,H | Ethyl | Methoxy | trans | | |
| 1.42 | HO-N=CH- | H,H,H | Methyl | Methoxy | trans | | |
| 1.43 | Br | H,H,H | Methyl | Methoxy | trans | | |
| 1.44 | Methoxymethyl | H,H,H | iso-Propyl | Methoxy | trans | | |
| 1.45 | Brommethyl | H,H,H | iso-Propyl | Methoxy | trans | | |
| 1.46 | Methoxymethyl | H,H,H | n-Hexyl | Methoxy | trans | | |
| 1.47 | Methyl | H,H,H | Ethoxy | Methoxy | trans | öl; | 5,32 |
| 1.48 | Methyl | H,H,H | Ethoxy | Methoxy | cis | öl; | 5,07 |
| 1.49 | Brommethyl | H,H,H | Ethoxy | Methoxy | trans | | |
| 1.50 | Methoxymethyl | H,H,H | Ethoxy | Methoxy | trans | | |
| 1.51 | Methyl | H,H,H | Ethoxy | Ethoxy | trans | öl; | 5,32 |
| 1.52 | Methyl | H,H,H | Ethoxy | n-Propoxy | trans | | |
| 1.53 | Methyl | H,H,H | n-Propoxy | Methoxy | trans | | |
| 1.54 | Formyl | H,H,H | Ethoxy | Methoxy | trans | | |
| 1.55 | 2,5-Dimethylbenzyloxy | H,H,H | Methoxy | Methoxy | trans | | |
| 1.56 | 2-Chlorbenzyloxy | H,H,H | Methoxy | Methoxy | trans | | |
| 1.57 | 3-Trifluormethylbenzyloxy | H,H,H | Methoxy | Methoxy | trans | | |
| 1.58 | 4-Brombenzyloxy | H,H,H | Methoxy | Methoxy | trans | | |
| 1.59 | 4-tert.-Butylbenzyloxy | H,H,H | Methoxy | Methoxy | trans | | |
| 1.60 | 4-Phenylbenzyloxy | H,H,H | Methoxy | Methoxy | trans | | |
| 1.61 | 3-Phenoxybenzyloxy | H,H,H | Methoxy | Methoxy | trans | | |

Tabelle 1 (Fortsetzung)

| Verb.Nr. | Z- | U,V,W | $R^1$-$X_m$- | -Y | Isomer | Fp. ; $^1$H-NMR (°C) (ppm) |
|---|---|---|---|---|---|---|
| 1.62 | 2,4-Dichlorbenzyloxy | H,H,H | Methoxy | Methoxy | trans | |
| 1.63 | 4-Nitrobenzyloxy | H,H,H | Methoxy | Methoxy | trans | |
| 1.64 | 3-Cyanobenzyloxy | H,H,H | Methoxy | Methoxy | trans | |
| 1.65 | 2-Methyl-3-(4-tert.-butyl-phenyl)-propyl-benzoyloxy | H,H,H | Methoxy | Methoxy | trans | |
| 1.66 | 4-Phenyl-benzoyloxy | H,H,H | Methoxy | Methoxy | trans | |
| 1.67 | 4-tert.-Butylbenzoyloxy | H,H,H | Methoxy | Methoxy | trans | |
| 1.68 | Benzoylamino | H,H,H | Methoxy | Methoxy | trans | |
| 1.69 | 4-Phenylbenzoylamino | H,H,H | Methoxy | Methoxy | trans | |
| 1.70 | 4-tert.-Butylbenzoylamino | H,H,H | Methoxy | Methoxy | trans | |
| 1.71 | 2-(4-Fluorophenyl)-thiazol-4--ylmethyloxy | H,H,H | Methoxy | Methoxy | trans | 105 - 108; 5,44 |
| 1.72 | 4-Phenylbenzyloxy | 4-Methyl | Methoxy | Methoxy | trans | |
| 1.73 | 4-Phenylbenzyloxy | 4,6-Di-iso-Propyl | Methoxy | Methoxy | trans | |
| 1.74 | 4-Phenylbenzyloxy | 4,6-Dimethyl | Methoxy | Methoxy | trans | |
| 1.75 | 4-Phenylbenzyloxy | 4-Brom | Methoxy | Methoxy | trans | |
| 1.76 | 4-Phenylbenzyloxy | 4-Jod | Methoxy | Methoxy | trans | |
| 1.77 | 4-Phenylbenzyloxy | 4-Trifluormethyl | Methoxy | Methoxy | trans | |
| 1.78 | 4-Phenylbenzyloxy | 4-iso-Propyl | Methoxy | Methoxy | trans | |
| 1.79 | 4-Phenylbenzyloxy | 4,6-Dichlor | Methoxy | Methoxy | trans | |
| 1.80 | 4-Phenylbenzyloxy | 5-Methoxy | Methoxy | Methoxy | trans | |
| 1.81 | 4-Phenylbenzyloxy | 3,5-Dimethyl | Methoxy | Methoxy | trans | |
| 1.82 | 4-Phenylbenzyloxy | 3-Methoxy | Methoxy | Methoxy | trans | |
| 1.83 | 4-Phenylbenzyloxy | 4,5-Dimethyl | Methoxy | Methoxy | trans | |

Tabelle 1 (Fortsetzung)

| Verb.Nr. | Z- | U,V,W | $R^1$-$X_m$- | -Y | Isomer | Fp. ; (°C) | $^1$H-NMR (ppm) |
|---|---|---|---|---|---|---|---|
| 1.84 | 4-Phenylbenzyloxy | 4-Methoxy | Methoxy | Methoxy | trans | | |
| 1.85 | 4-Phenylbenzyloxy | 6-Methyl-5-benzyloxy | Methoxy | Methoxy | trans | | |
| 1.86 | 4-Phenylbenzyloxy | 4-Fluor | Methoxy | Methoxy | trans | | |
| 1.87 | 4-Phenylbenzyloxy | 4-Ethyl | Methoxy | Methoxy | trans | | |
| 1.88 | 4-Phenylbenzyloxy | 5-Benzyloxy | Methoxy | Methoxy | trans | | |
| 1.89 | Phenoxy | H,H,H | Methoxy | Methoxy | trans | | |
| 1.90 | 3-Methylphenoxy | H,H,H | Methoxy | Methoxy | trans | öl; | 5,28 |
| 1.91 | 3-Methylphenoxy | H,H,H | Methoxy | Methoxy | cis | öl; | 5,21 |
| 1.92 | 2-Chlorphenoxy | H,H,H | Methoxy | Methoxy | trans | | |
| 1.93 | 4-Acetylphenoxy | H,H,H | Methoxy | Methoxy | trans | | |
| 1.94 | 3-Phenoxy-phenoxy | H,H,H | Methoxy | Methoxy | trans | | |
| 1.95 | 3-Methoxy-phenoxy | H,H,H | Methoxy | Methoxy | trans | öl; | 5,28 |
| 1.96 | 4-(4-Nitrobenzyloxy)phenoxy | H,H,H | Methoxy | Methoxy | trans | | |
| 1.97 | 2-Naphthyloxy | H,H,H | Methoxy | Methoxy | trans | | |
| 1.98 | 3-(Pyridin-2-yl)phenoxy | H,H,H | Methoxy | Methoxy | trans | | |
| 1.99 | 4-Phenoxymethyl-phenoxy | H,H,H | Methoxy | Methoxy | trans | | |
| 1.100 | 3-(Oxazol-2-yl)-phenoxy | H,H,H | Methoxy | Methoxy | trans | | |
| 1.101 | 4-(1,2,4-Triazol-1-yl)-phenoxy | H,H,H | Methoxy | Methoxy | trans | | |
| 1.102 | 2-Chlor-pyridin-4-yloxy | H,H,H | Methoxy | Methoxy | trans | | |
| 1.103 | Pyridin-2-yloxy | H,H,H | Methoxy | Methoxy | trans | | |
| 1.104 | 4-Methylpyridin-2-yloxy | H,H,H | Methoxy | Methoxy | trans | | |
| 1.105 | 4-Phenoxypyrimidin-2-yloxy | H,H,H | Methoxy | Methoxy | trans | | |
| 1.106 | 6-Phenoxy-pyrimidin-4-yloxy | H,H,H | Methoxy | Methoxy | trans | | |

Tabelle 1 (Fortsetzung)

| Verb.Nr. | Z- | U,V,W | $R^1$-$X_m$- | -Y | Isomer | Fp. ; (°C) | $^1$H-NMR (ppm) |
|---|---|---|---|---|---|---|---|
| 1.107 | Chinolin-4-yloxy | H,H,H | Methoxy | Methoxy | trans | | |
| 1.108 | 5-Phenyl-1,3,4-oxadiazol-2-yloxy | H,H,H | Methoxy | Methoxy | trans | | |
| 1.109 | 2-Brom-pyridin-3-yloxy | H,H,H | Methoxy | Methoxy | trans | | |
| 1.110 | Pyridin-3-yloxy | H,H,H | Methoxy | Methoxy | trans | | |
| 1.111 | 2-Chlor-pyridin-3-yloxy | H,H,H | Methoxy | Methoxy | trans | | |
| 1.112 | 4-Methyl-chinolin-2-yloxy | H,H,H | Methoxy | Methoxy | trans | | |
| 1.113 | 2-Methyl-chinolin-4-yloxy | H,H,H | Methoxy | Methoxy | trans | | |
| 1.114 | 5-Chlor-pyridin-3-yloxy | H,H,H | Methoxy | Methoxy | trans | | |
| 1.115 | 6-Methyl-2-nitro-pyridin-3-yloxy | H,H,H | Methoxy | Methoxy | trans | | |
| 1.116 | 4-Chlor-6-(2,6-difluorphenoxy)--1,3,5-triazin-2-yloxy | H,H,H | Methoxy | Methoxy | trans | | |
| 1.117 | 6-Phenylthio-pyridin-2-yloxy | H,H,H | Methoxy | Methoxy | trans | | |
| 1.118 | Phenylthio | H,H,H | Methoxy | Methoxy | trans | | |
| 1.119 | 2-Methyl-phenylthio | H,H,H | Methoxy | Methoxy | trans | | |
| 1.120 | 3-Phenoxy-phenylthio | H,H,H | Methoxy | Methoxy | trans | | |
| 1.121 | 1-Naphthylthio | H,H,H | Methoxy | Methoxy | trans | | |
| 1.122 | Pyridin-2-ylthio | H,H,H | Methoxy | Methoxy | trans | | |
| 1.123 | Pyridin-4-ylthio | H,H,H | Methoxy | Methoxy | trans | | |
| 1.124 | Benzthiazol-2-yl-thio | H,H,H | Methoxy | Methoxy | trans | | |
| 1.125 | Benzoxazol-2-ylthio | H,H,H | Methoxy | Methoxy | trans | | |
| 1.126 | Benzimidazol-2-ylthio | H,H,H | Methoxy | Methoxy | trans | | |
| 1.127 | Chinolin-2-ylthio | H,H,H | Methoxy | Methoxy | trans | | |
| 1.128 | 5-$CF_3$-Benzthiazol-2-ylthio | H,H,H | Methoxy | Methoxy | trans | | |
| 1.129 | 6-Methyl-benzthiazol-2-ylthio | H,H,H | Methoxy | Methoxy | trans | | |

Tabelle 1 (Fortsetzung)

| Verb.Nr. | Z- | U,V,W | $R^1-X_m-$ | -Y | Isomer | Fp. ; (°C) | $^1H$-NMR (ppm) |
|---|---|---|---|---|---|---|---|
| 1.130 | 6-Cl-Benzthiazol-2-yl-thio | H,H,H | Methoxy | Methoxy | trans | | |
| 1.131 | 5-Cl-Benzthiazol-2-yl-thio | H,H,H | Methoxy | Methoxy | trans | | |
| 1.132 | 6-Ethoxy-Benzthiazol-2-ylthio | H,H,H | Methoxy | Methoxy | trans | | |
| 1.133 | 7-$CF_3$-Chinolin-4-ylthio | H,H,H | Methoxy | Methoxy | trans | | |
| 1.134 | Pyrimidin-2-ylthio | H,H,H | Methoxy | Methoxy | trans | | |
| 1.135 | 1-Phenyl-1,2,3,4-tetrazol-5-ylthio | H,H,H | Methoxy | Methoxy | trans | | |
| 1.136 | N-Phenylamino | H,H,H | Methoxy | Methoxy | trans | | |
| 1.137 | N-Methyl,N-phenylamino | H,H,H | Methoxy | Methoxy | trans | | |
| 1.138 | N-Methyl, N-(3-chlorphenyl)-amino | H,H,H | Methoxy | Methoxy | trans | | |
| 1.139 | N-(3-Phenoxyphenyl)-amino | H,H,H | Methoxy | Methoxy | trans | | |
| 1.140 | N-(2,5-Dimethylbenzyl)-amino | H,H,H | Methoxy | Methoxy | trans | | |
| 1.141 | N-Methyl, N-(2-chlorbenzyl)-amino | H,H,H | Methoxy | Methoxy | trans | | |
| 1.142 | N-Acetyl-N-phenylamino | H,H,H | Methoxy | Methoxy | trans | | |
| 1.143 | Phenylsulfinyl | H,H,H | Methoxy | Methoxy | trans | | |
| 1.144 | 2-Methyl-phenylsulfinyl | H,H,H | Methoxy | Methoxy | trans | | |
| 1.145 | Naphthylsulfinyl | H,H,H | Methoxy | Methoxy | trans | | |
| 1.146 | Phenylsulfonyl | H,H,H | Methoxy | Methoxy | trans | | |
| 1.147 | Naphthylsulfonyl | H,H,H | Methoxy | Methoxy | trans | | |
| 1.148 | Chinolin-2-ylsulfonyl | H,H,H | Methoxy | Methoxy | trans | | |
| 1.149 | Benzoyloxymethyl | H,H,H | Methoxy | Methoxy | trans | | |
| 1.150 | 4-Acetylamino-benzoyloxymethyl | H,H,H | Methoxy | Methoxy | trans | | |
| 1.151 | 2-Chlorbenzoyloxymethyl | H,H,H | Methoxy | Methoxy | trans | | |
| 1.152 | 3-Cyanobenzoyloxymethyl | H,H,H | Methoxy | Methoxy | trans | | |

Tabelle 1 (Fortsetzung)

| Verb.Nr. | Z- | U,V,W | $R^1-X_m-$ | -Y | Isomer | Fp. ; (°C) | $^1H$-NMR (ppm) |
|---|---|---|---|---|---|---|---|
| 1.153 | 3-Phenoxybenzoyloxymethyl | H,H,H | Methoxy | Methoxy | trans | | |
| 1.154 | 4-Phenoxybenzoyloxymethyl | H,H,H | Methoxy | Methoxy | trans | | |
| 1.155 | n-Hexanoyloxymethyl | H,H,H | Methoxy | Methoxy | trans | | |
| 1.156 | 2-Pyridin-carbonyloxymethyl | H,H,H | Methoxy | Methoxy | trans | | |
| 1.157 | 2-Pyrazinyl-carbonyloxymethyl | H,H,H | Methoxy | Methoxy | trans | | |
| 1.158 | 2-Pyrazinyl-carbonyloxymethyl | H,H,H | Methoxy | Methoxy | trans | | |
| 1.159 | 4-Pyridazinyl-carbonyloxymethyl | H,H,H | Methoxy | Methoxy | trans | | |
| 1.160 | Phenylacetyloxymethyl | H,H,H | Methoxy | Methoxy | trans | | |
| 1.161 | Phenoxyacetyloxymethyl | H,H,H | Methoxy | Methoxy | trans | | |
| 1.162 | 2-Phenoxypropionyloxymethyl | H,H,H | Methoxy | Methoxy | trans | | |
| 1.163 | Cyclohexylcarbonyloxymethyl | H,H,H | Methoxy | Methoxy | trans | | |
| 1.164 | 4-(4-Carboxyphenoxy)-phenyl-carbonyloxymethyl | H,H,H | Methoxy | Methoxy | trans | | |
| 1.165 | Methoxymethyl | H,H,H | Methyl | Methoxy | cis | öl, | 5,82 |
| 1.166 | Methyl | H,H,H | Methyl | Methoxy | cis | öl; | 5,78 |
| 1.167 | Brommethyl | H,H,H | Methyl | Methoxy | cis | öl; | 5,90 |
| 1.168 | Methyl | H,H,H | Methyl | $-NHCH_3$ | trans | Harz; | 5,95 |
| 1.169 | Methyl | H,H,H | Methyl | $-NHCH_3$ | cis | Harz, | 5,80 |
| 1.170 | Methoxymethyl | H,H,H | Methyl | Methoxy | trans | öl; | 6,01 |
| 1.171 | Brommmethyl | H,H,H | Methyl | Methoxy | trans | öl; | 6,08 |
| 1.172 | 4-Phenylbenzyloxy | 4,6-Difluor | Methoxy | Methoxy | trans | | |
| 1.173 | 4-Phenylbenzyloxy | 4-Chlor | Methoxy | Methoxy | trans | | |

Tabelle 1 (Fortsetzung)

| Verb.Nr. | Z- | U,V,W | $R^1$-$X_m$- | -Y | Isomer | Fp. ; (°C) | $^1$H-NMR (ppm) |
|---|---|---|---|---|---|---|---|
| 1.174 | 3-Phenylphenoxy | H,H,H | Methoxy | Methoxy | trans | Öl; | 5,28 |
| 1.175 | 3-Phenylphenoxy | H,H,H | Methoxy | Methoxy | cis | Öl; | 5,23 |
| 1.176 | 4-Benzylphenoxy | H,H,H | Methoxy | Methoxy | trans | Öl; | 5,28 |
| 1.177 | 4-Benzylphenoxy | H,H,H | Methoxy | Methoxy | cis | Öl; | 5,18 |
| 1.178 | 2-Methylphenoxy | H,H,H | Methoxy | Methoxy | trans | Öl; | 5,32 |
| 1.179 | 2-Methylphenoxy | H,H,H | Methoxy | Methoxy | cis | Öl; | 5,25 |
| 1.180 | 4-Phenyl-butadien-1-yl | H,H,H | Methoxy | Methoxy | trans | 133-135; | 5,43 |
| 1.181 | trans-3-(Biphenyl-4-yl)-2,2-dibrom-cycloprop-1-yl | H,H,H | Methoxy | Methoxy | trans | 33- 36; | 5,39 |
| 1.182 | trans-3-(Biphenyl-4-yl)-2,2-dichlor-cycloprop-1-yl | H,H,H | Methoxy | Methoxy | trans | 44- 48; | 5,38 |
| 1.183 | 3,6,10-Trimethyl-undeca-1,3,5,9-tetraen-1-yl | H,H,H | Methoxy | Methoxy | trans | Öl; | 5,41 |

Tabelle 1 a

Verbindungen der Formel Ia

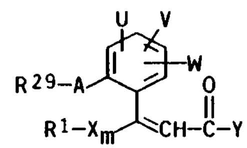

Ia

| Verb. Nr. | $R^{29}$- | -A- | U,V,W | $R^1$-$X_m$- | -Y | Isomer | Fp.; $^1$H-NMR (°C) (ppm) |
|---|---|---|---|---|---|---|---|
| Ia.1 | Phenyl | $-OCH_2-$ | H,H,H | Methoxy | Methoxy | trans | |
| Ia.2 | 2-Methylphenyl | $-OCH_2-$ | H,H,H | Methoxy | Methoxy | trans | Öl; 5,37 |
| Ia.3 | 3-Trifluoromethylphenyl | $-OCH_2-$ | H,H,H | Methoxy | Methoxy | trans | |
| Ia.4 | 4-Chlorphenyl | $-OCH_2-$ | H,H,H | Methoxy | Methoxy | trans | |
| Ia.5 | 2,4-Dichlorphenyl | $-OCH_2-$ | H,H,H | Methoxy | Methoxy | trans | |
| Ia.6 | 2,4,6-Trimethylphenyl | $-OCH_2-$ | H,H,H | Methoxy | Methoxy | trans | |
| Ia.7 | 3-Cyanophenyl | $-OCH_2-$ | H,H,H | Methoxy | Methoxy | trans | |
| Ia.8 | 2-Methyl- 4-acetylphenyl | $-OCH_2-$ | H,H,H | Methoxy | Methoxy | trans | |
| Ia.9 | 3-Formylphenyl | $-OCH_2-$ | H,H,H | Methoxy | Methoxy | trans | |
| Ia.10 | 3-Acetylphenyl | $-OCH_2-$ | H,H,H | Methoxy | Methoxy | trans | |
| Ia.11 | 2-Methyl-4-propionylphenyl | $-OCH_2-$ | H,H,H | Methoxy | Methoxy | trans | |
| Ia.12 | 2-Methyl-4-isobutyroyl-phenyl | $-OCH_2-$ | H,H,H | Methoxy | Methoxy | trans | |
| Ia.13 | 2-Methyl-4-butyroylphenyl | $-OCH_2-$ | H,H,H | Methoxy | Methoxy | trans | |
| Ia.14 | 2,5-Dimethyl-4-acetylphenyl | $-OCH_2-$ | H,H,H | Methoxy | Methoxy | trans | |
| Ia.15 | 2-Methyl-4-hexanoylphenyl | $-OCH_2-$ | H,H,H | Methoxy | Methoxy | trans | |
| Ia.16 | 2-Methyl-4-benzoylphenyl | $-OCH_2-$ | H,H,H | Methoxy | Methoxy | trans | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.17 | 3-(Phenylamino)phenyl | $-OCH_2-$ | H,H,H | Methoxy | Methoxy | trans | | |
| 1a.18 | 4-Fluorphenyl | $-OCH_2-$ | H,H,H | Methoxy | Methoxy | trans | | |
| 1a.19 | 2-Bromphenyl | $-OCH_2-$ | H,H,H | Methoxy | Methoxy | trans | | |
| 1a.20 | 4-Nitrophenyl | $-OCH_2-$ | H,H,H | Methoxy | Methoxy | trans | | |
| 1a.21 | 2-Methyl-4-Chlorphenyl | $-OCH_2-$ | H,H,H | Methoxy | Methoxy | trans | | |
| 1a.22 | 4-Cyanophenyl | $-OCH_2-$ | H,H,H | Methoxy | Methoxy | trans | | |
| 1a.23 | 4-(Acetylamino)phenyl | $-OCH_2-$ | H,H,H | Methoxy | Methoxy | trans | | |
| 1a.24 | 3-(Benzoylamino)phenyl | $-OCH_2-$ | H,H,H | Methoxy | Methoxy | trans | | |
| 1a.25 | 3-Benzyloxyphenyl | $-OCH_2-$ | H,H,H | Methoxy | Methoxy | trans | | |
| 1a.26 | 4-Benzyloxyphenyl | $-OCH_2-$ | H,H,H | Methoxy | Methoxy | trans | | |
| 1a.27 | 4-tert.-Butylphenyl | $-OCH_2-$ | H,H,H | Methoxy | Methoxy | trans | | |
| 1a.28 | 4-Cyclohexylpenyl | $-OCH_2-$ | H,H,H | Methoxy | Methoxy | trans | | |
| 1a.29 | 4-Phenylphenyl | $-OCH_2-$ | H,H,H | Methoxy | Methoxy | trans | | |
| 1a.30 | 2-Chlor-4-phenoxyphenyl | $-OCH_2-$ | H,H,H | Methoxy | Methoxy | trans | | |
| 1a.31 | 4-Phenoxyphenyl | $-OCH_2-$ | H,H,H | Methoxy | Methoxy | trans | öl; | 5,39 |
| 1a.32 | 4-(4-Trifluormethyl-phenoxy)-phenyl | $-OCH_2-$ | H,H,H | Methoxy | Methoxy | trans | | |
| 1a.33 | 3-Phenoxyphenyl | $-OCH_2-$ | H,H,H | Methoxy | Methoxy | trans | | |
| 1a.34 | 3-tert.-Butoxyphenyl | $-OCH_2-$ | H,H,H | Methoxy | Methoxy | trans | | |
| 1a.35 | 4-tert.-Butoxyphenyl | $-OCH_2-$ | H,H,H | Methoxy | Methoxy | trans | | |
| 1a.36 | 4-Ethoxyphenyl | $-OCH_2-$ | H,H,H | Methoxy | Methoxy | trans | | |
| 1a.37 | 4-n-Butoxyphenyl | $-OCH_2-$ | H,H,H | Methoxy | Methoxy | trans | öl; | 5.37 |
| 1a.38 | 4-n-Hexyloxyphenyl | $-OCH_2-$ | H,H,H | Methoxy | Methoxy | trans | | |
| 1a.39 | 4-(2-Ethylhexyloxy)-phenyl | $-OCH_2-$ | H,H,H | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.40 | 3-n-Decyloxyphenyl | $-OCH_2-$ | H,H,H | Methoxy | Methoxy | trans | | |
| 1a.41 | 3-(Phenylsulfonylamino)-phenyl | $-OCH_2-$ | H,H,H | Methoxy | Methoxy | trans | | |
| 1a.42 | 3-(Methylsulfinylamino)-phenyl | $-OCH_2-$ | H,H,H | Methoxy | Methoxy | trans | | |
| 1a.43 | 4-Trifluormethylsulfonylamino)-phenyl | $-OCH_2-$ | H,H,H | Methoxy | Methoxy | trans | | |
| 1a.44 | 4-(Phenylsulfinylamino)-phenyl | $-OCH_2-$ | H,H,H | Methoxy | Methoxy | trans | | |
| 1a.45 | 3-(Phenylaminosulfonyl)-phenyl | $-OCH_2-$ | H,H,H | Methoxy | Methoxy | trans | | |
| 1a.46 | 4-(Methylaminosulfonyl)-phenyl | $-OCH_2-$ | H,H,H | Methoxy | Methoxy | trans | | |
| 1a.47 | 3-(Phenoxycarbonyl)-phenyl | $-OCH_2-$ | H,H,H | Methoxy | Methoxy | trans | | |
| 1a.48 | 3-(Benzyloxycarbonyl)-phenyl | $-OCH_2-$ | H,H,H | Methoxy | Methoxy | trans | | |
| 1a.49 | 4-(Methoxycarbonyl)-phenyl | $-OCH_2-$ | H,H,H | Methoxy | Methoxy | trans | | |
| 1a.50 | 4-(2-Chlorbenzyloxycarbonyl)-phenyl | $-OCH_2-$ | H,H,H | Methoxy | Methoxy | trans | | |
| 1a.51 | 2-(Aminocarbonyl)-phenyl | $-OCH_2-$ | H,H,H | Methoxy | Methoxy | trans | | |
| 1a.52 | 2-(Aminothiocarbonyl)-phenyl | $-OCH_2-$ | H,H,H | Methoxy | Methoxy | trans | | |
| 1a.53 | 2-(Methylsulfinyloxy)-phenyl | $-OCH_2-$ | H,H,H | Methoxy | Methoxy | trans | | |
| 1a.54 | 3-[(4-Methylphenyl)-sulfonyloxy]-phenyl | $-OCH_2-$ | H,H,H | Methoxy | Methoxy | trans | | |
| 1a.55 | 4-(1,2,4-Triazol-1-yl)-phenyl | $-OCH_2-$ | H,H,H | Methoxy | Methoxy | trans | | |
| 1a.56 | 4'-Hydroxybiphenyl-4-yl | $-OCH_2-$ | H,H,H | Methoxy | Methoxy | trans | | |
| 1a.57 | 4-(4-Aminophenoxy)-phenyl | $-OCH_2-$ | H,H,H | Methoxy | Methoxy | trans | | |
| 1a.58 | 4-(5-Chlor-2-pyridyl)-phenyl | $-OCH_2-$ | H,H,H | Methoxy | Methoxy | trans | | |
| 1a.59 | 4-(5-Nitro-2-pyridyl)-phenyl | $-OCH_2-$ | H,H,H | Methoxy | Methoxy | trans | | |
| 1a.60 | $2-CH_3$, $4-[(CH_3)_2CH-CH_2-O-N=C(CH_3)]-C_6H_3-$ | $-OCH_2-$ | H,H,H | Methoxy | Methoxy | trans | öl; | 5,40 |
| 1a.61 | $2,5-(CH_3)_2-4-[CH_3-O-N=C(CH_3)]-C_6H_3-$ | $-OCH_2-$ | H,H,H | Methoxy | Methoxy | trans | öl; | 5,42 |
| 1a.62 | $3-(CH_3-O-N=CH)-C_6H_4-$ | $-OCH_2-$ | H,H,H | Methoxy | Methoxy | trans | öl; | 5,39 |
| 1a.63 | $3-(CH_2=CH-CH_2-O-N=CH)-C_6H_4$ | $-OCH_2-$ | H,H,H | Methoxy | Methoxy | trans | öl; | 5,39 |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.64 | 3-(2-Chlorbenzyloximinomethyl)-phenyl | $-OCH_2-$ | H,H,H | Methoxy | Methoxy | trans | Harz; | 5,36 |
| 1a.65 | 4-(Benzyloximinomethyl)-phenyl | $-OCH_2-$ | H,H,H | Methoxy | Methoxy | trans | | |
| 1a.66 | $2-CH_3-4-[Benzyl-O-N=C(CH_3)]-C_6H_3-$ | $-OCH_2-$ | H,H,H | Methoxy | Methoxy | trans | | |
| 1a.67 | $2-CH_3-4-[Isopentyl-O-N=C(CH_3)]-C_6H_3-$ | $-OCH_2-$ | H,H,H | Methoxy | Methoxy | trans | | |
| 1a.68 | $2-CH_3-4-[Geranyl-O-N=C(CH_3)]C_6H_3-$ | $-OCH_2-$ | H,H,H | Methoxy | Methoxy | trans | | |
| 1a.69 | $2-CH_3-4-[CH_3O-N=C(C_6H_5)]-C_6H_3-$ | $-OCH_2-$ | H,H,H | Methoxy | Methoxy | trans | | |
| 1a.70 | $2-CH_3-4-[CH_2=C(CH_3)-CH_2-O-N=C(C_2H_5)]-C_6H_3-$ | $-OCH_2-$ | H,H,H | Methoxy | Methoxy | trans | | |
| 1a.71 | $2-CH_3-4-[Cl-CH=CH-CH_2-O-N=C(CH_3)]-C_6H_3-$ | $-OCH_2-$ | H,H,H | Methoxy | Methoxy | trans | | |
| 1a.72 | $2-CH_3-4-[CH_3-O-N=C(CH_3)]-C_6H_3-$ | $-OCH_2-$ | H,H,H | Methoxy | Methoxy | trans | 58 - 60; | 5,38 |
| 1a.73 | $2-CH_3-4-[CH_3-O-N=C(CH_3)]-C_6H_3-$ | $-OCH_2-$ | H,H,H | Methoxy | n-Pentyloxy | trans | 154; | 5,32 |
| 1a.74 | $2-CH_3-4-[CH_3-O-N=C(CH_3)]-C_6H_3$ | $-OCH_2-$ | H,H,H | Methoxy | Ethoxy | trans | öl; | 5,37 |
| 1a.75 | $2-CH_3-4-[CH_3-O-N=C(CH_3)]-C_6H_3-$ | $-OCH_2-$ | H,H,H | Methoxy | n-Propoxy | trans | öl; | 5,38 |
| 1a.76 | $2-CH_3-4-[CH_3-O-N=C(CH_3)]-C_6H_3-$ | $-OCH_2-$ | H,H,H | Methoxy | Isopropoxy | trans | | |
| 1a.77 | $2-CH_3-4-[CH_3-O-N=C(CH_3)]-C_6H_3-$ | $-OCH_2-$ | H,H,H | Methoxy | n-Butoxy | trans | öl; | 5,37 |
| 1a.78 | $2-CH_3-4-[CH_3-O-N=C(CH_3)]-C_6H_3-$ | $-OCH_2-$ | H,H,H | Methoxy | tert.-Butoxy | trans | | |
| 1a.79 | $2-CH_3-4-[CH_3-O-N=C(CH_3)]-C_6H_3-$ | $-OCH_2-$ | H,H,H | Methoxy | n-Hexyloxy | trans | | |
| 1a.80 | $2-CH_3-4-[CH_3-O-N=C(CH_3)]-C_6H_3-$ | $-OCH_2-$ | H,H,H | Methoxy | Cyclohexyloxy | trans | | |
| 1a.81 | $2-CH_3-4-[CH_3-O-N=C(CH_3)]-C_6H_3-$ | $-OCH_2-$ | H,H,H | Methoxy | Cyclopropyl-methyloxy | trans | | |
| 1a.82 | $2-CH_3-4-[CH_3-O-N=C(CH_3)]-C_6H_3-$ | $-OCH_2-$ | H,H,H | Methoxy | $-ON=CH(CH_3)_2$ | trans | öl; | 5,47 |
| 1a.83 | $2-CH_3-4-[CH_3-O-N=C(CH_3)]-C_6H_3-$ | $-OCH_2-$ | H,H,H | Methoxy | Allyloxy | trans | öl; | 5,41 |
| 1a.84 | $2-CH_3-4-[CH_3-O-N=C(CH_3)]-C_6H_3-$ | $-OCH_2-$ | H,H,H | Methoxy | Propargyloxy | trans | öl; | 5,42 |
| 1a.85 | $2-CH_3-4-[CH_3-O-N=C(CH_3)]-C_6H_3-$ | $-OCH_2-$ | H,H,H | Methoxy | 2-Methoxy-ethoxy | trans | | |

Tabelle 1 a (Fortsetzung)

| Verb. Nr. | $R^{29}$- | -A- | U,V,W | $R^1-X_m$- | -Y | Isomer | Fp.; $^1H$-NMR (°C) (ppm) |
|---|---|---|---|---|---|---|---|
| 1a.86 | $2-CH_3-4-[CH_3-O-N=C(CH_3)]-C_6H_3-$ | $-OCH_2-$ | H,H,H | Methoxy | Methylthio | trans | öl; 5,72 |
| 1a.87 | $2-CH_3-4-[CH_3-O-N=C(CH_3)]-C_6H_3-$ | $-OCH_2-$ | H,H,H | Methoxy | Ethylthio | trans | |
| 1a.88 | $2-CH_3-4-[CH_3-O-N=C(CH_3)]-C_6H_3-$ | $-OCH_2-$ | H,H,H | Methoxy | Amino | trans | 97 - 99; 5,35 |
| 1a.89 | $2-CH_3-4-[CH_3-O-N=C(CH_3)]-C_6H_3-$ | $-OCH_2-$ | H,H,H | Methoxy | Methylamino | trans | öl; 5,35 |
| 1a.90 | $2-CH_3-4-[CH_3-O-N=C(CH_3)]-C_6H_3-$ | $-OCH_2-$ | H,H,H | Methoxy | Allylamino | trans | |
| 1a.91 | $2-CH_3-4-[CH_3-O-N=C(CH_3)]-C_6H_3$ | $-OCH_2-$ | H,H,H | Methoxy | Ethylamino | trans | 57 - 60; 5,37 |
| 1a.92 | $2-CH_3-4-[CH_3-O-N=C(CH_3)]-C_6H_3-$ | $-OCH_2-$ | H,H,H | Methoxy | n-Propylamino | trans | |
| 1a.93 | $2-CH_3-4-[CH_3-O-N=C(CH_3)]-C_6H_3-$ | $-OCH_2-$ | H,H,H | Methoxy | Isopropyl-amino | trans | |
| 1a.94 | $2-CH_3-4-[CH_3-O-N=C(CH_3)]-C_6H_3-$ | $-OCH_2-$ | H,H,H | Methoxy | n-Butlyamino | trans | |
| 1a.95 | $2-CH_3-4-[CH_3-O-N=C(CH_3)]-C_6H_3-$ | $-OCH_2-$ | H,H,H | Methoxy | iso-Butyl-amino | trans | |
| 1a.96 | $2-CH_3-4-[CH_3-O-N=C(CH_3)]-C_6H_3-$ | $-OCH_2-$ | H,H,H | Methoxy | tert.-Butyl-amino | trans | |
| 1a.97 | $2-CH_3-4-[CH_3-O-N=C(CH_3)]-C_6H_3-$ | $-OCH_2-$ | H,H,H | Methoxy | n-Hexylamino | trans | |
| 1a.98 | $2-CH_3-4-[CH_3-O-N=C(CH_3)]-C_6H_3-$ | $-OCH_2-$ | H,H,H | Methoxy | Dimethylamino | trans | öl; 5,51 |
| 1a.99 | $2-CH_3-4-[CH_3-O-N=C(CH_3)]-C_6H_3-$ | $-OCH_2-$ | H,H,H | Methoxy | Diethylamino | trans | |
| 1a.100 | $2-CH_3-4-[CH_3-O-N=C(CH_3)]-C_6H_3-$ | $-OCH_2-$ | H,H,H | Methoxy | N-Methyl-N-ethylamino | trans | |
| 1a.101 | $2-CH_3-4-[CH_3-O-N=C(CH_3)]-C_6H_3-$ | $-OCH_2-$ | H,H,H | Methoxy | N-Methyl-N-butylamino | trans | öl; 5,50; 5,58 (C-N-Rotamere) |
| 1a.102 | $2-CH_3-4-[CH_3-O-N=C(CH_3)]-C_6H_3-$ | $-OCH_2-$ | H,H,H | Methoxy | N-Methyl-N-cyclohexyalmino | trans | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.103 | 2-$CH_3$-4-[$CH_3$-O-N=C($CH_3$)]-$C_6H_3$- | -$OCH_2$- | H,H,H | Methoxy | Cyclopropyl-methylamino | trans | | |
| 1a.104 | 2-$CH_3$-4-[$CH_3$-O-N=C($CH_3$)]-$C_6H_3$- | -$OCH_2$- | H,H,H | Methoxy | -NHOH | trans | | |
| 1a.105 | 2-$CH_3$-4-[$CH_3$-O-N=C($CH_3$)]-$C_6H_3$- | -$OCH_2$- | H,H,H | Methoxy | -N($CH_3$)OH | trans | | |
| 1a.106 | 2-$CH_3$-4-[$CH_3$-O-N=C($CH_3$)]-$C_6H_3$- | -$OCH_2$- | H,H,H | Methoxy | -$NHOCH_3$ | trans | | |
| 1a.107 | 2-$CH_3$-4-[$CH_3$-O-N=C($CH_3$)]-$C_6H_3$- | -$OCH_2$- | H,H,H | Methoxy | -N($CH_3$)$OCH_3$ | trans | | |
| 1a.108 | 2-Methylphenyl | -$OCH_2$- | H,H,H | Methyl | Methoxy | trans | öl; | 5,98 |
| 1a.109 | 2-Methylphenyl | -$OCH_2$- | H,H,H | Methyl | Methoxy | cis | öl; | 5,85 |
| 1a.110 | 2-Methylphenyl | -$OCH_2$- | H,H,H | Ethyl | Methoxy | trans | öl; | 5,98 |
| 1a.111 | 2-Methyphenyl | -$OCH_2$- | H,H,H | Ethyl | Methoxy | cis | öl; | 5,80 |
| 1a.112 | 2-$CH_3$-4-[$CH_3$-O-N=C($CH_3$)]-$C_6H_3$- | -$OCH_2$- | H,H,H | Methyl | Methoxy | trans | öl; | 6,00 |
| 1a.113 | 2-$CH_3$-4-[$CH_3$-O-N=C($CH_3$)]-$C_6H_3$- | -$OCH_2$- | H,H,H | Methyl | Methoxy | cis | öl; | 5,85 |
| 1a.114 | 2-$CH_3$-4-[$CH_3$-O-N=C($CH_3$)]-$C_6H_3$- | -$OCH_2$- | H,H,H | Ethyl | Methoxy | trans | | |
| 1a.115 | 2-$CH_3$-4-[$CH_3$-O-N=C($CH_3$)]-$C_6H_3$- | -$OCH_2$- | H,H,H | Ethyl | Methoxy | cis | | |
| 1a.116 | 2-$CH_3$-4-[$CH_3$-O-N=C($CH_3$)]-$C_6H_3$- | -$OCH_2$- | H,H,H | n-Propyl | Methoxy | trans | | |
| 1a.117 | 2-$CH_3$-4-[$CH_3$-O-N=C($CH_3$)]-$C_6H_3$- | -$OCH_2$- | H,H,H | iso-Propyl | Methoxy | cis | | |
| 1a.118 | 2-$CH_3$-4-[$CH_3$-O-N=C($CH_3$)]-$C_6H_3$- | -$OCH_2$- | H,H,H | Ethoxy | Methoxy | trans | | |
| 1a.119 | 2-$CH_3$-4-[$CH_3$-O-N=C($CH_3$)]-$C_6H_3$- | -$OCH_2$- | H,H,H | n-Propoxy | Methoxy | trans | | |
| 1a.120 | 2-$CH_3$-4-[$CH_3$-O-N=C($CH_3$)]-$C_6H_3$- | -$OCH_2$- | H,H,H | i-Propoxy | Methoxy | trans | | |
| 1a.121 | 2-$CH_3$-4-[$CH_3$-O-N=C($CH_3$)]-$C_6H_3$ | -$OCH_2$- | H,H,H | n-Butoxy | Methoxy | trans | | |
| 1a.122 | 2-$CH_3$-4-[$CH_3$-O-N=C($CH_3$)]-$C_6H_3$- | -$OCH_2$- | H,H,H | n-Hexyloxy | Methoxy | trans | | |
| 1a.123 | 2-$CH_3$-4-[$CH_3$-O-N=C($CH_3$)]-$C_6H_3$- | -$OCH_2$- | H,H,H | Methylthio | Methoxy | trans | | |
| 1a.124 | 2-$CH_3$-4-[$CH_3$-O-N=C($CH_3$)]-$C_6H_3$- | -$OCH_2$- | H,H,H | Ethylthio | Methoxy | trans | | |

Tabelle 1 a (Fortsetzung)

| Verb. Nr. | $R^{29}$- | -A- | U,V,W | $R^1-X_m$- | -Y | Isomer | Fp.; (°C) | $^1$H-NMR (ppm) |
|---|---|---|---|---|---|---|---|---|
| 1a.125 | $2-CH_3-4-[CH_3-O-N=C(CH_3)]-C_6H_3-$ | $-OCH_2-$ | H,H,H | n-Propylthio | Methoxy | trans | | |
| 1a.126 | $2-CH_3-4-[CH_3-O-N=C(CH_3)]-C_6H_3-$ | $-OCH_2-$ | H,H,H | Methylamino | Methoxy | trans | | |
| 1a.127 | $2-CH_3-4-[CH_3-O-N=C(CH_3)]-C_6H_3-$ | $-OCH_2-$ | H,H,H | Diethylamino | Methoxy | trans | | |
| 1a.128 | $2-CH_3-4-[CH_3-O-N=C(CH_3)]-C_6H_3-$ | $-OCH_2-$ | H,H,H | Methoxyamino | Methoxy | trans | | |
| 1a.129 | $2-CH_3-4-[CH_3-O-N=C(CH_3)]-C_6H_3-$ | $-OCH_2-$ | H,H,H | Methyl | Cl | trans | | |
| 1a.130 | $2-CH_3-4-[CH_3-O-N=C(CH_3)]-C_6H_3-$ | $-OCH_2-$ | H,H,H | Methyl | iso-Butoxy | trans | | |
| 1a.131 | $2-CH_3-4-[CH_3-O-N=C(CH_3)]-C_6H_3-$ | $-OCH_2-$ | H,H,H | Methyl | Methylthio | trans | | |
| 1a.132 | $2-CH_3-4-[CH_3-O-N=C(CH_3)]-C_6H_3-$ | $-OCH_2-$ | H,H,H | Methyl | Amino | trans | | |
| 1a.133 | $2-CH_3-4-[CH_3-O-N=C(CH_3)]-C_6H_3-$ | $-OCH_2-$ | H,H,H | Methyl | Methylamino | trans | | |
| 1a.134 | $2-CH_3-4-[CH_3-O-N=C(CH_3)]-C_6H_3$ | $-OCH_2-$ | H,H,H | Methyl | Dimethylamino | trans | | |
| 1a.135 | $2-CH_3-4-[CH_3-O-N=C(CH_3)]-C_6H_3-$ | $-OCH_2-$ | H,H,H | Methyl | $-N(CH_3)OCH_3$ | trans | | |
| 1a.136 | $2-CH_3-4-[CH_3-O-N=C(CH_3)]-C_6H_3-$ | $-OCH_2-$ | H,H,H | Methyl | $-NHOCH_3$ | trans | | |
| 1a.137 | $2-CH_3-4-[CH_3-O-N=C(CH_3)]-C_6H_3$ | $-OCH_2-$ | H,H,H | Methyl | n-Propoxy | trans | | |
| 1a.138 | $2-CH_3-4-[CH_3-O-N=C(CH_3)]-C_6H_3-$ | $-OCH_2-$ | H,H,H | Methyl | i-Propoxy | trans | | |
| 1a.139 | $2-CH_3-4-[CH_3-O-N=C(CH_3)]-C_6H_3-$ | $-OCH_2-$ | H,H,H | Methyl | n-Butoxy | trans | | |
| 1a.140 | $2-CH_3-4-[CH_3-O-N=C(CH_3)]-C_6H_3-$ | $-OCH_2-$ | H,H,H | Methyl | sec.-Butoxy | trans | | |
| 1a.141 | $2-CH_3-4-[CH_3-O-N=C(CH_3)]-C_6H_3-$ | $-OCH_2-$ | H,H,H | Methyl | n-Hexyloxy | trans | | |
| 1a.142 | $2-CH_3-4-[CH_3-O-N=C(CH_3)]-C_6H_3-$ | $-OCH_2-$ | H,H,H | Methyl | Diethylamino | trans | | |
| 1a.143 | $2-CH_3-4-[CH_3-O-N=C(CH_3)]-C_6H_3-$ | $-OCH_2-$ | H,H,H | Methyl | i-Propylamino | trans | | |
| 1a.144 | $2-CH_3-4-[CH_3-O-N=C(CH_3)]-C_6H_3-$ | $-OCH_2-$ | 4-Ethoxy | Methoxy | Methoxy | trans | | |
| 1a.145 | $2-CH_3-4-[CH_3-O-N=C(CH_3)]-C_6H_3-$ | $-OCH_2-$ | 4-Methoxy | Methoxy | Methoxy | trans | | |
| 1a.146 | $2-CH_3-4-[CH_3-O-N=C(CH_3)]-C_6H_3-$ | $-OCH_2-$ | 4-t-Butyl | Methoxy | Methoxy | trans | | |
| 1a.147 | $2-CH_3-4-[CH_3-O-N=C(CH_3)-]C_6H_3-$ | $-OCH_2-$ | 4-Fluor | Methoxy | Methoxy | trans | | |
| 1a.148 | $2-CH_3-4-[CH_3-O-N=C(CH_3)]-C_6H_3-$ | $-OCH_2-$ | 6-Methyl | Methoxy | Methoxy | trans | | |

Tabelle 1 a (Fortsetzung)

| Verb. Nr. | $R^{29}$- | -A- | U,V,W | $R^1-X_m-$ | -Y | Isomer | Fp.; (°C) $^1$H-NMR (ppm) |
|---|---|---|---|---|---|---|---|
| 1a.149 | 2-$CH_3$-4-[$CH_3$-O-N=C($CH_3$)]-$C_6H_3$- | -$OCH_2$- | 5-CN | Methoxy | Methoxy | trans | |
| 1a.150 | 2-$CH_3$-4-[$CH_3$-O-N=C($CH_3$)]-$C_6H_3$- | -$OCH_2$- | 5-$NO_2$ | Methoxy | Methoxy | trans | |
| 1a.151 | 2-$CH_3$-4-[$CH_3$-O-N=C($CH_3$)]-$C_6H_3$- | -$OCH_2$- | 5-F | Methoxy | Methoxy | trans | |
| 1a.152 | 2-$CH_3$-4-[$CH_3$-O-N=C($CH_3$)]-$C_6H_3$- | -$OCH_2$- | 5-Br | Methoxy | Methoxy | trans | |
| 1a.153 | 2-$CH_3$-4-[$CH_3$-O-N=C($CH_3$)]-$C_6H_3$- | -$OCH_2$- | 5-J | Methoxy | Methoxy | trans | |
| 1a.154 | Phenyl | -$SCH_2$- | H,H,H | Methoxy | Methoxy | trans | |
| 1a.155 | 2-Methyl-, 4-tert.-butylphenyl | -$SCH_2$- | H,H,H | Methoxy | Methoxy | trans | |
| 1a.156 | Benzyl | -$SCH_2$- | H,H,H | Methoxy | Methoxy | trans | |
| 1a.157 | 4-Phenylbenzyl | -$SCH_2$- | H,H,H | Methoxy | Methoxy | trans | |
| 1a.158 | 2-Phenylethyl | -$SCH_2$- | H,H,H | Methoxy | Methoxy | trans | |
| 1a.159 | 4-Phenoxybutyl | -$SCH_2$- | H,H,H | Methoxy | Methoxy | trans | |
| 1a.160 | n-Octyl | -$SCH_2$- | H,H,H | Methoxy | Methoxy | trans | |
| 1a.161 | Geranyl | -$SCH_2$- | H,H,H | Methoxy | Methoxy | trans | |
| 1a.162 | 4-Phenyl-thiazol-2-yl | -$SCH_2$- | H,H,H | Methoxy | Methoxy | trans | Harz; 5,36 |
| 1a.163 | Pyridin-2-yl | -$SCH_2$- | H,H,H | Methoxy | Methoxy | trans | |
| 1a.164 | Pyridin-4-yl | -$SCH_2$- | H,H,H | Methoxy | Methoxy | trans | |
| 1a.165 | 2,3,5,6-Tetrachlorpyridin-4-yl | -$SCH_2$- | H,H,H | Methoxy | Methoxy | trans | |
| 1a.166 | Benzoxazol-2-yl | -$SCH_2$- | H,H,H | Methoxy | Methoxy | trans | öl; 5,38 |
| 1a.167 | Benzothiazol-2-yl | -$SCH_2$- | H,H,H | Methoxy | Methoxy | trans | öl; 5,38 |
| 1a.168 | 5-Chlor-benzothiazol-2-yl | -$SCH_2$- | H,H,H | Methoxy | Methoxy | trans | öl; 5,40 |
| 1a.169 | 6-Chlor-benzothiazol-2-yl | -$SCH_2$- | H,H,H | Methoxy | Methoxy | trans | öl; 5,40 |
| 1a.170 | 5-$CF_3$-Benzothiazol-2-yl | -$SCH_2$- | H,H,H | Methoxy | Methoxy | trans | 85 - 88; 5,41 |
| 1a.171 | 6-OEt-benzothiazol-2-yl | -$SCH_2$- | H,H,H | Methoxy | Methoxy | trans | öl; 5,39 |
| 1a.172 | 5-$CF_3$-Pyridin-2-yl | -$SCH_2$- | H,H,H | Methoxy | Methoxy | trans | öl; 5,40 |

Tabelle 1 a (Fortsetzung)

| Verb. Nr. | $R^{29}$- | -A- | U,V,W | $R^1$-$X_m$- | -Y | Isomer | Fp.; (°C) $^1$H-NMR (ppm) |
|---|---|---|---|---|---|---|---|
| 1a.173 | 3-Cyano-, 4-$CF_3$-, 6-(2-thienyl)-pyridin-2-yl | -$SCH_2$- | H,H,H | Methoxy | Methoxy | trans | 178; 5,42 |
| 1a.174 | Benzimidazol-2-yl | -$SCH_2$- | H,H,H | Methoxy | Methoxy | trans | Öl; 5,46 |
| 1a.175 | 4-Methylpyrimidin-2-yl | -$SCH_2$- | H,H,H | Methoxy | Methoxy | trans | |
| 1a.176 | Chinolin-2-yl | -$SCH_2$- | H,H,H | Methoxy | Methoxy | trans | Öl; 5,37 |
| 1a.177 | Chinolin-8-yl | -$SCH_2$- | H,H,H | Methoxy | Methoxy | trans | |
| 1a.178 | 7-Trifluoromethyl-chinolin-4-yl | -$SCH_2$- | H,H,H | Methoxy | Methoxy | trans | |
| 1a.179 | 3-Phenyl-1,2,4-triazol-5-yl | -$SCH_2$- | H,H,H | Methoxy | Methoxy | trans | |
| 1a.180 | 5-Phenyl-1,3,4-oxadiazol-2-yl | -$SCH_2$- | H,H,H | Methoxy | Methoxy | trans | Öl; 5,40 |
| 1a.181 | 4-Phenylthiazol-2-yl | -$OCH_2$- | H,H,H | Methoxy | Methoxy | trans | Öl; 5,38 |
| 1a.182 | 5-Methyl-2-phenylthiazol-4-yl | -$OCH_2$- | H,H,H | Methoxy | Methoxy | trans | Öl; 5,36 |
| 1a.183 | 4-Chlor-pyrazol | -$OCH_2$- | H,H,H | Methoxy | Methoxy | trans | |
| 1a.184 | Benzotriazol-1-yl | -$OCH_2$- | H,H,H | Methoxy | Methoxy | trans | |
| 1a.185 | 2-Methyl-chinolin-8-yl | -$OCH_2$- | H,H,H | Methoxy | Methoxy | trans | |
| 1a.186 | Chinoxalin-2-yl | -$OCH_2$- | H,H,H | Methoxy | Methoxy | trans | |
| 1a.187 | Dibenzofuran-2-yl | -$OCH_2$- | H,H,H | Methoxy | Methoxy | trans | |
| 1a.188 | 5,6-Diphenyl-1,2,4-triazin-3-yl | -$OCH_2$- | H,H,H | Methoxy | Methoxy | trans | |
| 1a.189 | Pyridin-2-yl | -$OCH_2$- | H,H,H | Methoxy | Methoxy | trans | |
| 1a.190 | Pyridin-3-yl | -$OCH_2$- | H,H,H | Methoxy | Methoxy | trans | |
| 1a.191 | Pyridin-4-yl | -$OCH_2$- | H,H,H | Methoxy | Methoxy | trans | |
| 1a.192 | Pyrimidin-2-yl | -$OCH_2$- | H,H,H | Methoxy | Methoxy | trans | |
| 1a.193 | Pyrimidin-4-yl | -$OCH_2$- | H,H,H | Methoxy | Methoxy | trans | |
| 1a.194 | Pyrimidin-5-yl | -$OCH_2$- | H,H,H | Methoxy | Methoxy | trans | |
| 1a.195 | Thiazolo[5,4-b]pyridin-2-yl | -$SCH_2$- | H,H,H | Methoxy | Methoxy | trans | 86 - 89, 5,40 |

Tabelle 1 a (Fortsetzung)

| Verb. Nr. | $R^{29}$- | -A- | U,V,W | $R^1-X_m$- | -Y | Isomer | Fp.; $^1H$-NMR (°C) (ppm) |
|---|---|---|---|---|---|---|---|
| 1a.196 | 5-Chlor-thiazolo[5,4-b]-pyridin-2-yl | $-SCH_2-$ | H,H,H | Methoxy | Methoxy | trans | 116 - 117, 5,39 |
| 1a.197 | 6-Chlor-thiazolo[5,4-b]-pyridin-2-yl | $-SCH_2-$ | H,H,H | Methoxy | Methoxy | trans | Öl; 5,40 |
| 1a.198 | 1-Phenyl-1,2,3,4-tetrazol-5-yl | $-SCH_2-$ | H,H,H | Methoxy | Methoxy | trans | Öl; 5,37 |
| 1a.199 | 4-Phenyl-1,3,4-oxadiazol-2-yl | $-OCH_2-$ | H,H,H | Methoxy | Methoxy | trans | |
| 1a.200 | 1-Phenylpyrazol-4-yl | $-OCH_2-$ | H,H,H | Methoxy | Methoxy | trans | |
| 1a.201 | 1-(4-Cyanophenyl)-pyrazol-4-yl | $-OCH_2-$ | H,H,H | Methoxy | Methoxy | trans | |
| 1a.202 | 5-Phenyl-isoxazol-3-yl | $-OCH_2-$ | H,H,H | Methoxy | Methoxy | trans | |
| 1a.203 | 1-Methyl-3-trifluormethyl-pyrazol-5-yl | $-OCH_2-$ | H,H,H | Methoxy | Methoxy | trans | |
| 1a.204 | 2-Propyl-6-trifluoromethyl-pyrimidin-4-yl | $-OCH_2-$ | H,H,H | Methoxy | Methoxy | trans | |
| 1a.205 | 5-Chlor-6-methoxy-2-methythio-pyrimidin-4-yl | $-OCH_2-$ | H,H,H | Methoxy | Methoxy | trans | |
| 1a.206 | 5-Chlor-4-cyclohexyl-6-methyl-pyrimidin-2-yl | $-OCH_2-$ | H,H,H | Methoxy | Methoxy | trans | |
| 1a.207 | 3-Cyano-4,6-dimethyl-pyridin-2-yl | $-OCH_2-$ | H,H,H | Methoxy | Methoxy | trans | |
| 1a.208 | Phenyl | -C≡C- | H,H,H | Methoxy | Methoxy | trans | |
| 1a.209 | 2-Methyl-phenyl | -C≡C- | H,H,H | Methoxy | Methoxy | trans | |
| 1a.210 | 3-Methyl-phenyl | -C≡C- | H,H,H | Methoxy | Methoxy | trans | |
| 1a.211 | 4-Methyl-phenyl | -C≡C- | H,H,H | Methoxy | Methoxy | trans | |
| 1a.212 | n-Butyl | -C≡C- | H,H,H | Methoxy | Methoxy | trans | |
| 1a.213 | Benzyl | -C≡C- | H,H,H | Methoxy | Methoxy | trans | |
| 1a.214 | 2-Chlorbenzyl | -C≡C- | H,H,H | Methoxy | Methoxy | trans | |
| 1a.215 | 2-Phenylethyl | -C≡C- | H,H,H | Methoxy | Methoxy | trans | |
| 1a.216 | Pyridin-2-yl | -C≡C- | H,H,H | Methoxy | Methoxy | trans | |
| 1a.217 | Pyridin-4-yl | -C≡C- | H,H,H | Methoxy | Methoxy | trans | |

Tabelle 1 a (Fortsetzung)

| Verb. Nr. | $R^{29}$- | -A- | U,V,W | $R^1-X_m$- | -Y | Isomer | Fp.; $^1H$-NMR (°C) (ppm) |
|---|---|---|---|---|---|---|---|
| 1a.218 | Chinolin-2-yl | -C≡C- | H,H,H | Methoxy | Methoxy | trans | |
| 1a.219 | Isochinolin-4-yl | -C≡C- | H,H,H | Methoxy | Methoxy | trans | |
| 1a.220 | Benzoxazol-2-yl | -C≡C- | H,H,H | Methoxy | Methoxy | trans | |
| 1a.221 | Benzthiazol-2-yl | -C≡C- | H,H,H | Methoxy | Methoxy | trans | |
| 1a.223 | Benzimidazol-2-yl | -C≡C- | H,H,H | Methoxy | Methoxy | trans | |
| 1a.224 | Thien-2-yl | -C≡C- | H,H,H | Methoxy | Methoxy | trans | |
| 1a.225 | Furan-2-yl | -C≡C- | H,H,H | Methoxy | Methoxy | trans | |
| 1a.226 | 4-(4-Acetylphenyl)-phenyl | -C≡C- | H,H,H | Methoxy | Methoxy | trans | |
| 1a.229 | 3-Phenoxy-phenyl | -C≡C- | H,H,H | Methoxy | Methoxy | trans | |
| 1a.230 | Biphenyl-4-yl | -C≡C- | H,H,H | Methoxy | Methoxy | trans | |
| 1a.231 | Biphenyl-3-yl | -C≡C- | H,H,H | Methoxy | Methoxy | trans | |
| 1a.232 | tert.-Butyl | -C≡C- | H,H,H | Methoxy | Methoxy | trans | |
| 1a.233 | Phenyl | (E)-HC=CH- | H,H,H | Methoxy | Methoxy | trans | öl; 5,44 |
| 1a.234 | Phenyl | (Z)-HC=CH- | H,H,H | Methoxy | Methoxy | trans | öl; 5,32 |
| 1a.235 | 2-Chlorphenyl | -HC=CH- | H,H,H | Methoxy | Methoxy | trans | |
| 1a.236 | 3-Chlorphenyl | -HC=CH- | H,H,H | Methoxy | Methoxy | trans | |
| 1a.237 | 4-Chlorphenyl | -HC=CH- | H,H,H | Methoxy | Methoxy | trans | |
| 1a.238 | 2-Nitrophenyl | (E)-HC=CH- | H,H,H | Methoxy | Methoxy | trans | 65 - 69; 5,45 |
| 1a.239 | 2-Nitrophenyl | (Z)-HC=CH- | H,H,H | Methoxy | Methoxy | trans | öl; 5,36 |
| 1a.240 | 3-Nitrophenyl | (E)-HC=CH- | H,H,H | Methoxy | Methoxy | trans | 128 - 132; 5,48 |
| 1a.241 | 3-Nitrophenyl | (Z)-HC=CH- | H,H,H | Methoxy | Methoxy | trans | 62 - 65; 5,34 |
| 1a.242 | 4-Nitrophenyl | -HC=CH- | H,H,H | Methoxy | Methoxy | trans | |
| 1a.243 | 2-Trifluormethylphenyl | (E)-HC=CH- | H,H,H | Methoxy | Methoxy | trans | öl; 5,44 |
| 1a.244 | 2-Trifluormethylphenyl | (Z)-HC=CH- | H,H,H | Methoxy | Methoxy | trans | öl; 5,37 |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.245 | 3-Trifluormethylphenyl | (E)-HC=CH- | H,H,H | Methoxy | Methoxy | trans | öl; | 5,46 |
| 1a.246 | 3-Trifluormethylphenyl | (Z)-HC=CH- | H,H,H | Methoxy | Methoxy | trans | öl; | 5, 33 |
| 1a.247 | 2-Methylphenyl | (E)-HC=CH- | H,H,H | Methoxy | Methoxy | trans | öl; | 5,42 |
| 1a.248 | 2-Methylphenyl | (Z)-HC=CH- | H,H,H | Methoxy | Methoxy | trans | öl; | 5,36 |
| 1a.249 | 3-Methylphenyl | -HC=CH- | H,H,H | Methoxy | Methoxy | trans | | |
| 1a.250 | 4-Methylphenyl | (E)-HC=CH- | H,H,H | Methoxy | Methoxy | trans | öl; | 5,43 |
| 1a.251 | 4-Methylphenyl | (Z)-HC=CH- | H,H,H | Methoxy | Methoxy | trans | öl; | 5,31 |
| 1a.252 | 2,4-Difluorphenyl | -HC=CH- | H,H,H | Methoxy | Methoxy | trans | | |
| 1a.253 | 2,4,6-Trichlorphenyl | -HC=CH- | H,H,H | Methoxy | Methoxy | trans | | |
| 1a.254 | 4-tert.-Butylphenyl | -HC=CH- | H,H,H | Methoxy | Methoxy | trans | | |
| 1a.255 | 3-Cyano-4-dimethylamino-2-fluorphenyl | -HC=CH- | H,H,H | Methoxy | Methoxy | trans | | |
| 1a.256 | 4-Cyanophenyl | -HC=CH- | H,H,H | Methoxy | Methoxy | trans | | |
| 1a.257 | 3-Benzoylphenyl | -HC=CH- | H,H,H | Methoxy | Methoxy | trans | | |
| 1a.258 | 4-Benzoylphenyl | -HC=CH- | H,H,H | Methoxy | Methoxy | trans | | |
| 1a.259 | 2-Cyclohexylphenyl | -HC=CH- | H,H,H | Methoxy | Methoxy | trans | | |
| 1a.260 | 3-Cyclohexylphenyl | -HC=CH- | H,H,H | Methoxy | Methoxy | trans | | |
| 1a.261 | 4-Cyclohexylphenyl | -HC=CH- | H,H,H | Methoxy | Methoxy | trans | | |
| 1a.262 | 2-Benzyloxyphenyl | -HC=CH- | H,H,H | Methoxy | Methoxy | trans | | |
| 1a.263 | 3-Benzyloxyphenyl | (E)-HC=CH- | H,H,H | Methoxy | Methoxy | trans | öl; | 5,43 |
| 1a.264 | 4-Benzyloxyphenyl | (E)-HC=CH- | H,H,H | Methoxy | Methoxy | trans | 118 - 120; | 5,43 |
| 1a.265 | 4-Benzyloxyphenyl | (Z)-HC=CH- | H,H,H | Methoxy | Methoxy | trans | | |
| 1a.266 | 2-Phenylphenyl | -HC=CH- | H,H,H | Methoxy | Methoxy | trans | | |
| 1a.267 | 3-Phenylphenyl | -HC=CH- | H,H,H | Methoxy | Methoxy | trans | | |
| 1a.268 | 4-Phenylphenyl | (E)-HC=CH- | H,H,H | Methoxy | Methoxy | trans | 110 - 112; | 5,44 |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.269 | 4-Phenylphenyl | (Z)-HC=CH- | H,H,H | Methoxy | Methoxy | trans | öl; | 5,33 |
| 1a.270 | 2-Phenoxyphenyl | -HC=CH- | H,H,H | Methoxy | Methoxy | trans | | |
| 1a.271 | 3-Phenoxyphenyl | (E)-HC=CH- | H,H,H | Methoxy | Methoxy | trans | öl; | 5,41 |
| 1a.272 | 3-Phenoxyphenyl | (Z)-HC=CH- | H,H,H | Methoxy | Methoxy | trans | öl; | 5,31 |
| 1a.273 | 4-Phenoxyphenyl | (E)-HC=CH- | H,H,H | Methoxy | Methoxy | trans | öl; | 5,44 |
| 1a.274 | 4-Phenoxyphenyl | (Z)-HC=CH- | H,H,H | Methoxy | Methoxy | trans | | |
| 1a.275 | 4-Pyrrolidin-1-yl-phenyl | -HC=CH- | H,H,H | Methoxy | Methoxy | trans | | |
| 1a.276 | 4-Methoxycarbonylphenyl | -HC=CH- | H,H,H | Methoxy | Methoxy | trans | | |
| 1a.277 | 3-Brom-5-methylphenyl | -HC=CH- | H,H,H | Methoxy | Methoxy | trans | | |
| 1a.278 | 4-[$(CH_3)_2$-CH-$CH_3$-O-N=C($CH_3$)-$C_6H_4$ | -HC=CH- | H,H,H | Methoxy | Methoxy | trans | | |
| 1a.279 | 3-(2-Pyrimidinyl)-phenyl | -HC=CH- | H,H,H | Methoxy | Methoxy | trans | | |
| 1a.280 | 4-(4-Thiazolyl)-phenyl | -HC=CH- | H,H,H | Methoxy | Methoxy | trans | | |
| 1a.281 | 5-[1-Methyl-3-trifluormethyl- | -HC=CH- | H,H,H | Methoxy | Methoxy | trans | | |
| | -pyrazol-5-yl)-thiophen-2-yl | -HC=CH- | H,H,H | Methoxy | Methoxy | trans | | |
| 1a.282 | 3-(5-n-Propyl-isoxazol-3-yl)-phenyl | -HC=CH- | H,H,H | Methoxy | Methoxy | trans | | |
| 1a.283 | Naphth-1-yl | -HC=CH- | H,H,H | Methoxy | Methoxy | trans | | |
| 1a.284 | Naphth-2-yl | -HC=CH- | H,H,H | Methoxy | Methoxy | trans | | |
| 1a.285 | Phenanthren-3-yl | -HC=CH- | H,H,H | Methoxy | Methoxy | trans | | |
| 1a.286 | Anthracen-2-yl | -HC=CH- | H,H,H | Methoxy | Methoxy | trans | | |
| 1a.287 | Pyrrol-2-yl | -HC=CH- | H,H,H | Methoxy | Methoxy | trans | | |
| 1a.288 | Pyrrol-3-yl | (E)-HC=CH- | H,H,H | Methoxy | Methoxy | trans | öl; | 5,42 |
| 1a.289 | Pyrrol-3-yl | (Z)-HC=CH- | H,H,H | Methoxy | Methoxy | trans | | |
| 1a.290 | Furan-2-yl | (E)-HC=CH- | H,H,H | Methoxy | Methoxy | trans | öl; | 5,44 |
| 1a.291 | Furan-2-yl | (Z)-HC=CH- | H,H,H | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.292 | Furan-3-yl | (E)-HC=CH- | H,H,H | Methoxy | Methoxy | trans | öl; | 5,43 |
| 1a.293 | Furan-3-yl | (Z)-HC=CH- | H,H,H | Methoxy | Methoxy | trans | öl; | 5,30 |
| 1a.294 | Thiophen-2-yl | (E)-HC=CH- | H,H,H | Methoxy | Methoxy | trans | öl; | 5,44 |
| 1a.295 | Thiophen-2-yl | (Z)-HC=CH- | H,H,H | Methoxy | Methoxy | trans | | |
| 1a.296 | Thiophen-3-yl | (E)-HC=CH- | H,H,H | Methoxy | Methoxy | trans | öl; | 5,43 |
| 1a.297 | Thiophen-3-yl | (Z)-HC=CH- | H,H,H | Methoxy | Methoxy | trans | öl; | 5,31 |
| 1a.298 | 2-Methyl-thiazol-4-yl | (E)-HC=CH- | H,H,H | Methoxy | Methoxy | trans | öl; | 5,44 |
| 1a.299 | 2-Methyl-thiazol-4-yl | (Z)-HC=CH- | H,H,H | Methoxy | Methoxy | trans | öl; | 5,30 |
| 1a.300 | 3-(4-Chlorphenyl)-isoxazol-5-yl | (E)-HC=CH- | H,H,H | Methoxy | Methoxy | trans | 128 - 130; | 5,48 |
| 1a.301 | 3-(4-Chlorphenyl)-isoxazol-5-yl | (Z)-HC=CH- | H,H,H | Methoxy | Methoxy | trans | | |
| 1a.302 | 2-(4-Chlorphenyl)-oxazol-4-yl | (E)-HC=CH- | H,H,H | Methoxy | Methoxy | trans | 132 - 136; | 5,48 |
| 1a.303 | 2-(4-Chlorphenyl)-oxazol-4-yl | (Z)-HC=CH- | H,H,H | Methoxy | Methoxy | trans | öl; | 5,28 |
| 1a.304 | 1-(4-Chlorphenyl)-pyrrol-3-yl | (E)-HC=CH- | H,H,H | Methoxy | Methoxy | trans | 132 - 135; | 5,46 |
| 1a.305 | 1-(4-Chlorphenyl)-pyrrol-3-yl | (Z)-HC=CH- | H,H,H | Methoxy | Methoxy | trans | 133 - 136; | 5,28 |
| 1a.306 | 2-Phenyl-thiazol-4-yl | (E)-HC=CH- | H,H,H | Methoxy | Methoxy | trans | öl; | 5,46 |
| 1a.307 | 2-Phenyl-thiazol-4-yl | (Z)-HC=CH- | H,H,H | Methoxy | Methoxy | trans | 102 - 105; | 5,29 |
| 1a.308 | 2-Phenyl-1,3,4-thiadiazol-5-yl | (E)-HC=CH- | H,H,H | Methoxy | Methoxy | trans | 129 - 131; | 5,49 |
| 1a.309 | 2-Phenyl-1,3,4-thiadiazol-5-yl | (Z)-HC=CH- | H,H,H | Methoxy | Methoxy | trans | | |
| 1a.310 | 3-(4-Fluorphenyl)-1,2,4-oxadiazol-5-yl | (E)-HC=CH- | H,H,H | Methoxy | Methoxy | trans | 154 - 156; | 5,51 |
| 1a.311 | 3-(4-Fluorphenyl)-1,2,4-oxadiazol-5-yl | (Z)-HC=CH- | H,H,H | Methoxy | Methoxy | trans | | |
| 1a.312 | 2-(4-Methylphenyl)-thiazol-4-yl | (E)-HC=CH- | H,H,H | Methoxy | Methoxy | trans | 147 - 150; | 5,46 |
| 1a.313 | 2-(4-Methylphenyl)-thiazol-4-yl | (Z)-HC=CH- | H,H,H | Methoxy | Methoxy | trans | | |
| 1a.314 | 2-(4-Chlorphenyl)-thiazol-4-yl | (E)-HC=CH- | H,H,H | Methoxy | Methoxy | trans | 145 - 147; | 5,47 |
| 1a.315 | 2-(4-Chlorphenyl)-thiazol-4-yl | (Z)-HC=CH- | H,H,H | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) $^1$H-NMR (ppm) |
|---|---|---|---|---|---|---|---|
| 1a.316 | 1-(3-Chlorphenyl)-pyrazol-4-yl | (E)-HC=CH- | H,H,H | Methoxy | Methoxy | trans | 124 - 128; 5,47 |
| 1a.317 | 1-(3-Chlorphenyl)-pyrazol-4-yl | (Z)-HC=CH- | H,H,H | Methoxy | Methoxy | trans | öl; 5,29 |
| 1a.318 | 1-(4-Methoxyphenyl)-pyrazol-4-yl | (E)-HC=CH- | H,H,H | Methoxy | Methoxy | trans | 121 - 123; 5,46 |
| 1a.319 | 1-(4-Methoxyphenyl)-pyrazol-4-yl | (Z)-HC=CH- | H,H,H | Methoxy | Methoxy | trans | 108 - 110; 5,28 |
| 1a.320 | 1-(4-Chlorphenyl)-pyrazol-4-yl | (E)-HC=CH- | H,H,H | Methoxy | Methoxy | trans | 140 - 142; 5,45 |
| 1a.321 | 1-(4-Chlorphenyl)-pyrazol-4-yl | (Z)-HC=CH- | H,H,H | Methoxy | Methoxy | trans | 142 - 145; 5,28 |
| 1a.322 | 5-(4-Chlorphenyl)-4-methyl-isoxazol-3-yl | (E)-HC=CH- | H,H,H | Methoxy | Methoxy | trans | 116 - 118; 5,46 |
| 1a.323 | 5-(4-Chlorphenyl)-4-methyl-isoxazol-3-yl | (Z)-HC=CH- | H,H,H | Methoxy | Methoxy | trans | öl; 5,36 |
| 1a.324 | 3-Isobutylisoxazol-5-yl | (E)-HC=CH- | H,H,H | Methoxy | Methoxy | trans | öl; 5,43 |
| 1a.325 | 3-Isobutylisoxazol-5-yl | (Z)-HC=CH- | H,H,H | Methoxy | Methoxy | trans | |
| 1a.326 | Isochinolin-6-yl | -HC=CH- | H,H,H | Methoxy | Methoxy | trans | |
| 1a.327 | Benzimidazol-2-yl | -HC=CH- | H,H,H | Methoxy | Methoxy | trans | |
| 1a.328 | 2-(4-Thiazolyl)-thiazol-4-yl | -HC=CH- | H,H,H | Methoxy | Methoxy | trans | |
| 1a.329 | Tetrahydropyran-3-yl | (E)-HC=CH- | H,H,H | Methoxy | Methoxy | trans | öl; 5,37 |
| 1a.330 | Tetrahydropyran-3-yl | (Z)-HC=CH- | H,H,H | Methoxy | Methoxy | trans | |
| 1a.331 | 2-Phenyl-tetrahydropyran-4-yl | (E)-HC=CH- | H,H,H | Methoxy | Methoxy | trans | öl; 5,33 |
| 1a.332 | 2-Phenyl-tetrahydropyran- | (Z)-HC=CH- | H,H,H | Methoxy | Methoxy | trans | |
| 1a.333 | 2-(3-Tetrahydropyranyl)-tetrahydro-pyran-4-yl | (E)-HC=CH- | H,H,H | Methoxy | Methoxy | trans | öl; 5,38 |
| 1a.334 | 2(3-Tetrahydropyranyl)-tetrahydro-pyran-4-yl | (Z)-HC=CH- | H,H,H | Methoxy | Methoxy | trans | |
| 1a.335 | Benzyl | -HC=CH- | H,H,H | Methoxy | Methoxy | trans | |
| 1a.336 | 4-Phenoxy-but-1-yl | -HC=CH- | H,H,H | Methoxy | Methoxy | trans | |
| 1a.337 | n-Nonyl | (E)-HC=CH- | H,H,H | Methoxy | Methoxy | trans | öl; 5,38 |
| 1a.338 | Phenylethinyl | (E)-HC=CH- | H,H,H | Methoxy | Methoxy | trans | öl; 5,42 |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.339 | Phenyl | $-CH_2-CH_2-$ | H,H,H | Methoxy | Methoxy | trans | | |
| 1a.340 | 2-Trifluorphenyl | $-CH_2-CH_2-$ | H,H,H | Methoxy | Methoxy | trans | | |
| 1a.341 | 3-Methylphenyl | $-CH_2-CH_2-$ | H,H,H | Methoxy | Methoxy | trans | | |
| 1a.342 | 4-Benzoylphenyl | $-CH_2-CH_2-$ | H,H,H | Methoxy | Methoxy | trans | | |
| 1a.343 | 2,4,6-Trichlorphenyl | $-CH_2-CH_2-$ | H,H,H | Methoxy | Methoxy | trans | | |
| 1a.344 | 4-Phenylphenyl | $-CH_2-CH_2-$ | H,H,H | Methoxy | Methoxy | trans | Öl; | 5,40 |
| 1a.345 | 4-[$(CH_3)_2$-CH-$CH_2$-O-N=C($CH_3$)]-$C_6H_4$ | $-CH_2-CH_2-$ | H,H,H | Methoxy | Methoxy | trans | | |
| 1a.346 | 3-(2-Pyrimidinyl)-phenyl | $-CH_2-CH_2-$ | H,H,H | Methoxy | Methoxy | trans | | |
| 1a.347 | 2-(4-Fluorphenyl)-thiazol-4-yl | $-CH_2-CH_2-$ | H,H,H | Methoxy | Methoxy | trans | 157-159; | 5,38 |
| 1a.348 | 3-(4-Fluorphenyl)-1,2,4-oxadiazol-5-yl | $-CH_2-CH_2-$ | H,H,H | Methoxy | Methoxy | trans | | |
| 1a.349 | Isochinolin-6-yl | $-CH_2-CH_2-$ | H,H,H | Methoxy | Methoxy | trans | | |
| 1a.350 | 2-(4-Thiazolyl)-thiazol-4-yl | $-CH_2-CH_2-$ | H,H,H | Methoxy | Methoxy | trans | | |
| 1a.351 | 4-Phenoxyphenyl | $-CH_2-CH_2-$ | H,H,H | Methoxy | Methoxy | trans | | |
| 1a.352 | 2-Phenyl-thiazol-4-yl | $-CH_2-CH_2-$ | H,H,H | Methoxy | Methoxy | trans | | |
| 1a.353 | 3-Cyclohexylphenyl | $-CH_2-CH_2-$ | H,H,H | Methoxy | Methoxy | trans | | |
| 1a.354 | Phenyl | $-C(CH_3)=N-O-CH_2-$ | H,H,H | Methoxy | Methoxy | trans | Öl; | 5,36 |
| 1a.355 | 3-Bromphenyl | $-C(CH_3)=N-O-CH_2-$ | H,H,H | Methoxy | Methoxy | trans | Öl; | 5,38 |
| 1a.356 | 4-Chlorphenyl | $-C(CH_3)=N-O-CH_2-$ | H,H,H | Methoxy | Methoxy | trans | Öl; | 5,36 |
| 1a.357 | 4-Chlorphenyl | $-C(H)=N-O-CH_2-$ | H,H,H | Methoxy | Methoxy | trans | | |
| 1a.358 | 4-Chlorphenyl | $-C(Ph)=N-O-CH_2-$ | H,H,H | Methoxy | Methoxy | trans | | |
| 1a.359 | 4-Phenylphenyl | $-C(CH_3)=N-O-CH_2-$ | H,H,H | Methoxy | Methoxy | trans | | |
| 1a.360 | Naphth-2-yl | $-C(CH_3)=N-O-CH_2-$ | H,H,H | Methoxy | Methoxy | trans | Öl; | 5,35 |
| 1a.361 | 2-Methyl-4-chlorphenyl | $-C(CH_3)=N-O-CH_2-$ | H,H,H | Methoxy | Methoxy | trans | | |
| 1a.362 | 2-Methyl-2,5-dimethoxyphenyl | $-C(CH_3)=N-O-CH_2-$ | H,H,H | Methoxy | Methoxy | trans | | |

Tabelle 1 a (Fortsetzung)

| Verb. Nr. | $R^{29}$- | -A- | U,V,W | $R^1-X_m$- | -Y | Isomer | Fp.; (°C) | $^1H$-NMR (ppm) |
|---|---|---|---|---|---|---|---|---|
| 1a.363 | Furan-2-yl | $-C(CH_3)=N-O-CH_2-$ | H,H,H | Methoxy | Methoxy | trans | öl; | 5,35 |
| 1a.364 | Pyridin-3-yl | $-C(CH_3)=N-O-CH_2-$ | H,H,H | Methoxy | Methoxy | trans | | |
| 1a.365 | Pyrazin-2-yl | $-C(CH_3)=N-O-CH_2-$ | H,H,H | Methoxy | Methoxy | trans | | |
| 1a.366 | 1-Methyl-pyrrol-2-yl | $-C(CH_3)=N-O-CH_2-$ | H,H,H | Methoxy | Methoxy | trans | öl; | 5,35 |
| 1a.367 | Thiazol-2-yl | $-C(CH_3)=N-O-CH_2-$ | H,H,H | Methoxy | Methoxy | trans | öl; | 5,37 |
| 1a.368 | 4-n-Hexylphenyl | $-C(CH_3)=N-O-CH_2-$ | H,H,H | Methoxy | Methoxy | trans | | |
| 1a.369 | Phenyl | -N=CH- | H,H,H | Methoxy | Methoxy | trans | | |
| 1a.370 | 2,4-Dimethylphenyl | -N=CH- | H,H,H | Methoxy | Methoxy | trans | | |
| 1a.371 | 4-Phenoxyphenyl | -N=CH- | H,H,H | Methoxy | Methoxy | trans | | |
| 1a.372 | 4-tert.-Butylphenyl | -N=CH- | H,H,H | Methoxy | Methoxy | trans | | |
| 1a.373 | Diphenyl-4-yl | -N=CH- | H,H,H | Methoxy | Methoxy | trans | | |
| 1a.374 | 4,6-Dimethylpyrimidin-2-yl | -N=CH- | H,H,H | Methoxy | Methoxy | trans | | |
| 1a.375 | Isochinolin-5-yl | -N=CH- | H,H,H | Methoxy | Methoxy | trans | | |
| 1a.376 | 3-Pyridyl | -N=CH- | H,H,H | Methoxy | Methoxy | trans | | |
| 1a.377 | Phenyl | -N=N- | H,H,H | Methoxy | Methoxy | trans | | |
| 1a.378 | Phenyl | -ON=CH- | H,H,H | Methoxy | Methoxy | trans | | |
| 1a.379 | Geranyl | -ON=CH- | H,H,H | Methoxy | Methoxy | trans | | |
| 1a.380 | Benzyl | -ON=CH- | H,H,H | Methoxy | Methoxy | trans | | |
| 1a.381 | 3-Chlorbenzyl | -ON=CH- | H,H,H | Methoxy | Methoxy | trans | | |
| 1a.382 | 2,4-Dimethylbenzyl | -ON=CH- | H,H,H | Methoxy | Methoxy | trans | | |
| 1a.383 | 4-Phenylbenzyl | -ON=CH- | H,H,H | Methoxy | Methoxy | trans | | |
| 1a.384 | 1-Naphthylmethyl | -ON=CH- | H,H,H | Methoxy | Methoxy | trans | | |
| 1a.385 | 2-(1-Naphthyl)-ethyl | -ON=CH- | H,H,H | Methoxy | Methoxy | trans | | |
| 1a.386 | 3-(4-Chlorphenyl)-allyl | -ON=CH- | H,H,H | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.387 | 2-(4-Fluorphenyl)-oxazol-4-yl-methyl | -ON=CH- | H,H,H | Methoxy | Methoxy | trans | | |
| 1a.388 | 2-(2-Fluorphenoxy)-ethyl | -ON=CH- | H,H,H | Methoxy | Methoxy | trans | | |
| 1a.389 | 3-Phenyl-benzyl | -O-N=CH- | H,H,H | Methoxy | Methoxy | trans | | |
| 1a.390 | 4-(3-Phenoxyphenyl)-butyl | -O-N=CH- | H,H,H | Methoxy | Methoxy | trans | | |
| 1a.391 | 6-Chlor-pyridin-3-yl-methyl | -O-N=CH- | H,H,H | Methoxy | Methoxy | trans | | |
| 1a.392 | 4-(4-Fluorphenyl)-3-methoximino-butyl | -O-N=CH- | H,H,H | Methoxy | Methoxy | trans | | |
| 1a.393 | 2-(4-Fluorophenyl)-thiazol-4-yl | (Z)-CH=CH- | H,H,H | Methoxy | Methoxy | trans | Öl; | 5,39 |
| 1a.394 | 2-(4-Fluorophenyl)-thiazol-4-yl | (E)-CH=CH- | H,H,H | Methoxy | Methoxy | trans | 148-150; | 5,48 |
| 1a.395 | 2-(4-Fluorophenyl)-thiazol-4-yl | (E)-CH=CH- | H,H,H | Methoxy | n-Butoxy | trans | 155-158; | 5,40 |
| 1a.396 | 2-(4-Fluorophenyl)-thiazol-4-yl | (E)-CH=CH- | H,H,H | Methoxy | Ethoxy | trans | | |
| 1a.397 | 2-(4-Fluorophenyl)-thiazol-4-yl | (E)-CH=CH- | H,H,H | Methoxy | n-Propoxy | trans | | |
| 1a.398 | 2-(4-Fluorophenyl)-thiazol-4-yl | (E)-CH=CH- | H,H,H | Methoxy | iso-Butoxy | trans | | |
| 1a.399 | 2-(4-Fluorophenyl)-thiazol-4-yl | (E)-CH=CH- | H,H,H | Methoxy | sec-Butoxy | trans | | |
| 1a.400 | 2-(4-Fluorophenyl)-thiazol-4-yl | (E)-CH=CH- | H,H,H | Methoxy | n-Pentyloxy | trans | | |
| 1a.401 | 2-(4-Fluorophenyl)-thiazol-4-yl | (E)-CH=CH- | H,H,H | Methoxy | Cyclopentyloxy | trans | | |
| 1a.402 | 2-(4-Fluorophenyl)-thiazol-4-yl | (E)-CH=CH- | H,H,H | Methoxy | Allyloxy | trans | | |
| 1a.403 | 2-(4-Fluorophenyl)-thiazol-4-yl | (E)-CH=CH- | H,H,H | Methoxy | 2-Chlorallyloxy | trans | | |
| 1a.404 | 2-(4-Fluorophenyl)-thiazol-4-yl | (E)-CH=CH- | H,H,H | Methoxy | Propargyloxy | trans | | |
| 1a.405 | 2-(4-Fluorophenyl)-thiazol-4-yl | (E)-CH=CH- | H,H,H | Methoxy | Methylthio | trans | | |
| 1a.406 | 2-(4-Fluorophenyl)-thiazol-4-yl | (E)-CH=CH- | H,H,H | Methoxy | Dimethylamino | trans | | |
| 1a.407 | 2-(4-Fluorophenyl)-thiazol-4-yl | (E)-CH=CH- | H,H,H | Methoxy | Ethylamino | trans | | |
| 1a.408 | 2-(4-Fluorophenyl)-thiazol-4-yl | (E)-CH=CH- | H,H,H | Methoxy | N-Methyl-N--ethylamino | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.409 | 2-(4-Fluorophenyl)-thiazol-4-yl | (E)-CH=CH- | H,H,H | Methoxy | Diethylamino | trans | | |
| 1a.410 | 2-(4-Fluorophenyl)-thiazol-4-yl | (E)-CH=CH- | H,H,H | Methoxy | Isopropylamino | trans | | |
| 1a.411 | 2-(4-Fluorophenyl)-thiazol-4-yl | (E)-CH=CH- | H,H,H | Methoxy | Amino | trans | | |
| 1a.412 | 2-(4-Fluorophenyl)-thiazol-4-yl | (E)-CH=CH- | H,H,H | Methoxy | Methylamino | trans | | |
| 1a.413 | 2-(4-Fluorophenyl)-thiazol-4-yl | (E)-CH=CH- | H,H,H | Methoxy | N-Methyl-N-iso-propylamino | trans | | |
| 1a.414 | 2-(4-Fluorophenyl)-thiazol-4-yl | (E)-CH=CH- | H,H,H | Methoxy | Allylamino | trans | | |
| 1a.415 | 2-(4-Fluorophenyl)-thiazol-4-yl | (E)-CH=CH- | H,H,H | Methoxy | -NHOH | trans | | |
| 1a.416 | 2-(4-Fluorophenyl)-thiazol-4-yl | (E)-CH=CH- | H,H,H | Methoxy | $-N(CH_3)OH$ | trans | | |
| 1a.417 | 2-(4-Fluorophenyl)-thiazol-4-yl | (E)-CH=CH- | H,H,H | Methoxy | $-NHOCH_3$ | trans | | |
| 1a.418 | 2-(4-Fluorophenyl)-thiazol-4-yl | (E)-CH=CH- | H,H,H | Methoxy | $-N(CH_3)OCH_3$ | trans | | |
| 1a.419 | 2-(4-Fluorophenyl)-thiazol-4-yl | (E)-CH=CH- | H,H,H | Methoxy | $-N(C_2H_5)OH$ | trans | | |
| 1a.420 | 2-(4-Fluorophenyl)-thiazol-4-yl | (Z)-CH=CH- | H,H,H | Methyl | Methoxy | trans | | |
| 1a.421 | 2-(4-Fluorophenyl)-thiazol-4-yl | (Z)-CH=CH- | H,H,H | Methyl | Methoxy | cis | | |
| 1a.422 | 2-(4-Fluorophenyl)-thiazol-4-yl | (E)-CH=CH- | H,H,H | Methyl | Methoxy | trans | | |
| 1a.423 | 2-(4-Fluorophenyl)-thiazol-4-yl | (E)-CH=CH- | H,H,H | Methyl | Methoxy | cis | | |
| 1a.424 | 2-(4-Fluorophenyl)-thiazol-4-yl | (E)-CH=CH- | H,H,H | Ethyl | Methoxy | trans | | |
| 1a.425 | 2-(4-Fluorophenyl)-thiazol-4-yl | (E)-CH=CH- | H,H,H | Ethyl | Methoxy | cis | | |
| 1a.426 | 2-(4-Fluorophenyl)-thiazol-4-yl | (E)-CH=CH- | H,H,H | n-Propyl | Methoxy | trans | | |
| 1a.427 | 2-(4-Fluorophenyl)-thiazol-4-yl | (E)-CH=CH- | H,H,H | iso-Propyl | Methoxy | cis | | |
| 1a.428 | 2-(4-Fluorophenyl)-thiazol-4-yl | (E)-CH=CH- | H,H,H | Ethoxy | Methoxy | trans | | |
| 1a.429 | 2-(4-Fluorophenyl)-thiazol-4-yl | (E)-CH=CH- | H,H,H | n-Propyloxy | Methoxy | trans | | |
| 1a.430 | 2-(4-Fluorophenyl)-thiazol-4-yl | (E)-CH=CH- | H,H,H | i-Propyloxy | Methoxy | trans | | |
| 1a.431 | 2-(4-Fluorophenyl)-thiazol-4-yl | (E)-CH=CH- | H,H,H | n-Butyloxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.432 | 2-(4-Fluorophenyl)-thiazol-4-yl | (E)-CH=CH- | H,H,H | n-Hexyloxy | Methoxy | trans | | |
| 1a.433 | 2-(4-Fluorophenyl)-thiazol-4-yl | (E)-CH=CH- | H,H,H | Methylthio | Methoxy | trans | | |
| 1a.434 | 2-(4-Fluorophenyl)-thiazol-4-yl | (E)-CH=CH- | H,H,H | Ethylthio | Methoxy | trans | | |
| 1a.435 | 2-(4-Fluorophenyl)-thiazol-4-yl | (E)-CH=CH- | H,H,H | n-Propylthio | Methoxy | trans | | |
| 1a.436 | 2-(4-Fluorophenyl)-thiazol-4-yl | (E)-CH=CH- | H,H,H | Methylamino | Methoxy | trans | | |
| 1a.437 | 2-(4-Fluorophenyl)-thiazol-4-yl | (E)-CH=CH- | H,H,H | Dimethylamino | Methoxy | trans | | |
| 1a.438 | 2-(4-Fluorophenyl)-thiazol-4-yl | (E)-CH=CH- | H,H,H | Methoxyamino | Methoxy | trans | | |
| 1a.439 | 2-(4-Fluorophenyl)-thiazol-4-yl | (E)-CH=CH- | H,H,H | Cl | Methoxy | trans | | |
| 1a.440 | 2-(4-Fluorophenyl)-thiazol-4-yl | (E)-CH=CH- | H,H,H | Methyl | sec.-Butyloxy | trans | | |
| 1a.441 | 2-(4-Fluorophenyl)-thiazol-4-yl | (E)-CH=CH- | H,H,H | Methyl | Methylthio | trans | | |
| 1a.442 | 2-(4-Fluorophenyl)-thiazol-4-yl | (E)-CH=CH- | H,H,H | Methyl | Amino | trans | | |
| 1a.443 | 2-(4-Fluorophenyl)-thiazol-4-yl | (E)-CH=CH- | H,H,H | Methyl | Methylamino | trans | | |
| 1a.444 | 2-(4-Fluorophenyl)-thiazol-4-yl | (E)-CH=CH- | H,H,H | Methyl | Dimethylamino | trans | | |
| 1a.445 | 2-(4-Fluorophenyl)-thiazol-4-yl | (E)-CH=CH- | H,H,H | Methyl | $-N(CH_3)OCH_3$ | trans | | |
| 1a.446 | 2-(4-Fluorophenyl)-thiazol-4-yl | (E)-CH=CH- | H,H,H | Methyl | $-NHOCH_3$ | trans | | |
| 1a.447 | 2-(4-Fluorophenyl)-thiazol-4-yl | (E)-CH=CH- | H,H,H | Methyl | n-Propoxy | trans | | |
| 1a.448 | 2-(4-Fluorophenyl)-thiazol-4-yl | (E)-CH=CH- | H,H,H | Methyl | i-Propoxy | trans | | |
| 1a.449 | 2-(4-Fluorophenyl)-thiazol-4-yl | (E)-CH=CH- | 4-Ethoxy | Methoxy | Methoxy | trans | | |
| 1a.450 | 2-(4-Flurophenyl)-thiazol-4-yl | (E)-CH=CH- | 4-Methoxy | Methoxy | Methoxy | trans | | |
| 1a.451 | 2-(4-Fluorophenyl)-thiazol-4-yl | (E)-CH=CH- | 4-t-Butyl | Methoxy | Methoxy | trans | | |
| 1a.452 | 2-(4-Fluorophenyl)-thiazol-4-yl | (E)-CH=CH- | 4-Fluor | Methoxy | Methoxy | trans | | |
| 1a.453 | 2-(4-Fluorophenyl)-thiazol-4-yl | (E)-CH=CH- | 6-Methyl | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.454 | Biphenyl-4-yl | (Z)-CH=CH- | H,H,H | Methyl | Methoxy | trans | | |
| 1a.455 | Biphenyl-4-yl | (Z)-CH=CH- | H,H,H | Methyl | Methoxy | cis | | |
| 1a.456 | Biphenyl-4-yl | (E)-CH=CH- | H,H,H | Methyl | Methoxy | trans | | |
| 1a.457 | Biphenyl-4-yl | (E)-CH=CH- | H,H,H | Methyl | Methoxy | cis | | |
| 1a.458 | Biphenyl-4-yl | (E)-CH=CH- | H,H,H | Ethyl | Methoxy | trans | | |
| 1a.459 | Biphenyl-4-yl | (E)-CH=CH- | H,H,H | Ethyl | Methoxy | cis | | |
| 1a.460 | Biphenyl-4-yl | (E)-CH=CH- | H,H,H | n-Propyl | Methoxy | trans | | |
| 1a.461 | Biphenyl-4-yl | (E)-CH=CH- | H,H,H | iso-Propyl | Methoxy | trans | | |
| 1a.462 | Biphenyl-4-yl | (E)-CH=CH- | H,H,H | Ethoxy | Methoxy | trans | | |
| 1a.463 | Biphenyl-4-yl | (E)-CH=CH- | H,H,H | n-Propyloxy | Methoxy | trans | | |
| 1a.464 | Biphenyl-4-yl | (E)-CH=CH- | H,H,H | i-Propyloxy | Methoxy | trans | | |
| 1a.465 | Biphenyl-4-yl | (E)-CH=CH- | H,H,H | n-Butyloxy | Methoxy | trans | | |
| 1a.466 | Biphenyl-4-yl | (E)-CH=CH- | H,H,H | n-Hexyloxy | Methoxy | trans | | |
| 1a.467 | Biphenyl-4-yl | (E)-CH=CH- | H,H,H | Methylthio | Methoxy | trans | | |
| 1a.468 | Biphenyl-4-yl | (E)-CH=CH- | H,H,H | Ethylthio | Methoxy | trans | | |
| 1a.469 | Biphenyl-4-yl | (E)-CH=CH- | H,H,H | n-Propylthio | Methoxy | trans | | |
| 1a.470 | Biphenyl-4-yl | (E)-CH=CH- | H,H,H | Methylamino | Methoxy | trans | | |
| 1a.471 | Biphenyl-4-yl | (E)-CH=CH- | H,H,H | Dimethylamino | Methoxy | trans | | |
| 1a.472 | Biphenyl-4-yl | (E)-CH=CH- | H,H,H | Methoxyamino | Methoxy | trans | | |
| 1a.473 | Biphenyl-4-yl | (E)-CH=CH- | H,H,H | Cl | Methoxy | trans | | |
| 1a.474 | Biphenyl-4-yl | (E)-CH=CH- | H,H,H | Methyl | iso-Butoxy | trans | | |
| 1a.475 | Biphenyl-4-yl | (E)-CH=CH- | H,H,H | Methyl | Methylthio | trans | | |
| 1a.476 | Biphenyl-4-yl | (E)-CH=CH- | H,H,H | Methyl | Amino | trans | | |
| 1a.477 | Biphenyl-4-yl | (E)-CH=CH- | H,H,H | Methyl | Methylamino | trans | | |

Tabelle 1 a (Fortsetzung)

| Verb. Nr. | $R^{29}$- | -A- | U,V,W | $R^1-X_m$- | -Y | Isomer | Fp.; (°C) | $^1$H-NMR (ppm) |
|---|---|---|---|---|---|---|---|---|
| Ia.478 | Biphenyl-4-yl | (E)-CH=CH- | H,H,H | Methyl | Dimethylamino | trans | | |
| Ia.479 | Biphenyl-4-yl | (E)-CH=CH- | H,H,H | Methyl | $-N(CH_3)OCH_3$ | trans | | |
| Ia.480 | Biphenyl-4-yl | (E)-CH=CH- | H,H,H | Methyl | $-NHOCH_3$ | trans | | |
| Ia.481 | Biphenyl-4-yl | (E)-CH=CH- | H,H,H | Methyl | n-Propoxy | trans | | |
| Ia.482 | Biphenyl-4-yl | (E)-CH=CH- | H,H,H | Methyl | i-Propoxy | trans | | |
| Ia.488 | Biphenyl-4-yl | (E)-CH=CH- | 4-Ethoxy | Methoxy | Methoxy | trans | | |
| Ia.489 | Biphenyl-4-yl | (E)-CH=CH- | 4-Methoxy | Methoxy | Methoxy | trans | | |
| Ia.490 | Biphenyl-4-yl | (E)-CH=CH- | 4-t-Butyl | Methoxy | Methoxy | trans | | |
| Ia.491 | Biphenyl-4-yl | (E)-CH=CH- | 4-Fluor | Methoxy | Methoxy | trans | | |
| Ia.492 | Biphenyl-4-yl | (E)-CH=CH- | 6-Methyl | Methoxy | Methoxy | trans | | |
| Ia.493 | Phenyl | -O-CO- | H,H,H | Methoxy | Methoxy | trans | | |
| Ia.494 | 2-Chlorphenyl | -O-CO- | H,H,H | Methoxy | Methoxy | trans | | |
| Ia.495 | 3-Fluorphenyl | -O-CO- | H,H,H | Methoxy | Methoxy | trans | | |
| Ia.496 | Diphenyl-4-yl | -O-CO- | H,H,H | Methoxy | Methoxy | trans | | |
| Ia.497 | 2-Methylbenzyl | -O-CO- | H,H,H | Methoxy | Methoxy | trans | | |
| Ia.498 | 3-Phenoxyphenyl | -O-CO- | H,H,H | Methoxy | Methoxy | trans | | |
| Ia.499 | tert.-Butyl | -O-CO- | H,H,H | Methoxy | Methoxy | trans | | |
| Ia.500 | Methyl | -O-CO- | H,H,H | Methoxy | Methoxy | trans | | |
| Ia.501 | Phenyl | -NH-CO- | H,H,H | Methoxy | Methoxy | trans | | |
| Ia.502 | Cyclohexyl | -NH-CO- | H,H,H | Methoxy | Methoxy | trans | | |
| Ia.503 | 3-Pyridyl | -NH-CO- | H,H,H | Methoxy | Methoxy | trans | | |
| Ia.504 | Benzyl | $-N(CH_3)-CO-$ | H,H,H | Methoxy | Methoxy | trans | | |
| Ia.505 | Phenyl | $-N(CH_3)-CO-$ | H,H,H | Methoxy | Methoxy | trans | | |
| Ia.506 | 2-Pyridyl | $-N(CH_3)-CO-$ | H,H,H | Methoxy | Methoxy | trans | | |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.507 | Phenyl | $-CH_2-$ | H,H,H | Methoxy | Methoxy | trans | | |
| 1a.508 | Phenyl | -HC=N- | H,H,H | Methoxy | Methoxy | trans | | |
| 1a.509 | 3-Trifluormethylphenyl | -HC=N- | H,H,H | Methoxy | Methoxy | trans | | |
| 1a.510 | 2-Methylphenyl | -HC=N- | H,H,H | Methoxy | Methoxy | trans | | |
| 1a.511 | 4-Chlorphenyl | -HC=N- | H,H,H | Methoxy | Methoxy | trans | | |
| 1a.512 | Biphenyl-4-yl | -HC=N- | H,H,H | Methoxy | Methoxy | trans | | |
| 1a.513 | 2-Pyridyl | $-(CH_3)C=N-$ | H,H,H | Methoxy | Methoxy | trans | | |
| 1a.514 | Phenyl | $-(CH_3)C=N-$ | H,H,H | Methoxy | Methoxy | trans | | |
| 1a.516 | 4-Trifluormethylphenyl | $-(CH_3)C=N-$ | H,H,H | Methoxy | Methoxy | trans | | |
| 1a.517 | 4-Nitrophenyl | $-(CH_3)C=N-$ | H,H,H | Methoxy | Methoxy | trans | | |
| 1a.518 | 3-Pyridyl | $-(CH_3)C=N-$ | H,H,H | Methoxy | Methoxy | trans | | |
| 1a.519 | 4-Trifluormethylphenyl | (E)-HC=CH- | H,H,H | Methoxy | Methoxy | trans | Öl; | 5,46 |
| 1a.520 | 4-Trifluormethylphenyl | (Z)-HC=CH- | H,H,H | Methoxy | Methoxy | trans | Öl; | 5,32 |
| 1a.521 | 4,5-Diphenyl-oxazol-2-yl | $-SCH_2-$ | H,H,H | Methoxy | Methoxy | trans | Öl; | 5,39 |
| 1a.522 | 4-Carboxyethyl-imidazol-2-yl | $-SCH_2-$ | H,H,H | Methoxy | Methoxy | trans | Öl; | 5,48 |
| 1a.523 | 5-Methyl-1,3,4-thiadiazol-2-yl | $-SCH_2-$ | H,H,H | Methoxy | Methoxy | trans | Öl; | 5,39 |
| 1a.524 | 1-Methyl-1,3,4-triazol-2-yl | $-SCH_2-$ | H,H,H | Methoxy | Methoxy | trans | Öl; | 5,37 |
| 1a.525 | 5-(4-Chlorphenyl)-1,3,4-oxa-diazol-2-yl | $-SCH_2-$ | H,H,H | Methoxy | Methoxy | trans | Öl; | 5,41 |
| 1a.526 | 5-Methylamino-1,3,4-thia-diazol-2-yl | $-SCH_2-$ | H,H,H | Methoxy | Methoxy | trans | Öl; | 5,38 |

Tabelle 1 a (Fortsetzung)

| Verb. Nr. | $R^{29}$- | -A- | U,V,W | $R^1$-$X_m$- | -Y | Isomer | Fp.; (°C) | $^1$H-NMR (ppm) |
|---|---|---|---|---|---|---|---|---|
| 1a.527 | 5-Benzylmercapto-1,3,4-thiadiazol-2-yl | $-SCH_2-$ | H,H,H | Methoxy | Methoxy | trans | öl; | 5,38 |
| 1a.528 | 5-(2-Chlorbenzyl)mercapto-1,3,4-thiadiazol-2-yl | $-SCH_2-$ | H,H,H | Methoxy | Methoxy | trans | öl; | 5,38 |
| 1a.529 | 5-(3-Chlorbenzyl)mercapto-1,3,4-thiadiazol-2-yl | $-SCH_2-$ | H,H,H | Methoxy | Methoxy | trans | öl; | 5,37 |
| 1a.530 | 5-(3-Chlorbenzyl)mercapto-1,3,4-thiadiazol-2-yl | $-SCH_2-$ | H,H,H | Methoxy | Methoxy | trans | öl; | 5,38 |
| 1a.531 | 5-(2,4-Dichlorbenzyl)mercapto-1,3,4-thiadiazol-2-yl | $-SCH_2-$ | H,H,H | Methoxy | Methoxy | trans | öl; | 5,38 |
| 1a.532 | 5-(3,4-Dichlorbenzyl)mercapto-1,3,4-thiadiazol-2-yl | $-SCH_2-$ | H,H,H | Methoxy | Methoxy | trans | öl; | 5,38 |
| 1a.533 | 5-(2,6-Dichlorbenzyl)mercapto-1,3,4-thiadiazol-2-yl | $-SCH_2-$ | H,H,H | Methoxy | Methoxy | trans | öl; | 5,40 |
| 1a.534 | 5-(2-Fluor,6-Chlorbenzyl)mercapto-1,3,4-thiadiazol-2-yl | $-SCH_2-$ | H,H,H | Methoxy | Methoxy | trans | öl; | 5,40 |
| 1a.535 | 5-(2-Naphthylmethyl)mercapto-1,3,4-thiadiazol-2-yl | $-SCH_2-$ | H,H,H | Methoxy | Methoxy | trans | öl; | 5,35 |
| 1a.536 | 5-(3-Phenoxybenzyl)mercapto-1,3,4-thiadiazol-2-yl | $-SCH_2-$ | H,H,H | Methoxy | Methoxy | trans | öl; | 5,38 |
| 1a.537 | 5-(4-Phenylbenzyl)mercapto-1,3,4-thiadiazol-2-yl | $-SCH_2-$ | H,H,H | Methoxy | Methoxy | trans | öl; | 5,39 |
| 1a.538 | 5-[4-(2-Chlor,4-$CF_3$-Phenoxy)benzyl]-mercapto-1,3,4-thiadiazol-2-yl | $-SCH_2-$ | H,H,H | Methoxy | Methoxy | trans | öl; | 5,40 |

Tabelle 1 a (Fortsetzung)

| Verb. Nr. | $R^{29}$- | -A- | U,V,W | $R^1$-$X_m$- | -Y | Isomer | Fp.; (°C) $^1$H-NMR (ppm) |
|---|---|---|---|---|---|---|---|
| Ia.539 | 3-(6-Methylpyrid-2-yl)-isoxazol-5-yl | (E)-HC=CH- | H,H,H | Methoxy | Methoxy | trans | 103-105; 5,49 |
| Ia.540 | 3-(4-Chlorphenyl)-1,2,4-oxadiazol-5-yl | (E)-HC=CH- | H,H,H | Methoxy | Methoxy | trans | 159-160; 5,51 |
| Ia.541 | 3-Isobutyl-5-methyl-4,5-dihydroisoxazol-5-yl | (E)-HC=CH- | H,H,H | Methoxy | Methoxy | trans | öl; 5,38 |
| Ia.542 | 3-Chlorphenyl | $-C(CH_3)=N-O-CH_2$ | H,H,H | Methoxy | Methoxy | trans | öl; 5,37 |
| Ia.543 | 4-Methylphenyl | $-C(CH_3)=N-O-CH_2$ | H,H,H | Methoxy | Methoxy | trans | öl; 5,36 |
| Ia.544 | 4-Methoxyphenyl | $-C(CH_3)=N-O-CH_2$ | H,H,H | Methoxy | Methoxy | trans | öl; 5,37 |
| Ia.545 | Benzoyl | $-C(CH_3)=N-O-CH_2$ | H,H,H | Methoxy | Methoxy | trans | öl; 5,35 |
| Ia.546 | 3-Cyanophenyl | $-C(CH_3)=N-O-CH_2$ | H,H,H | Methoxy | Methoxy | trans | öl; 5,39 |
| Ia.547 | Indan-1-yl | $-C(CH_3)=N-O-CH_2$ | H,H,H | Methoxy | Methoxy | trans | öl; 5,35 |
| Ia.548 | Thiophen-3-yl | $-C(CH_3)=N-O-CH_2$ | H,H,H | Methoxy | Methoxy | trans | öl; 5,33 |
| Ia.549 | 3-Nitrophenyl | $-C(CH_3)=N-O-CH_2$ | H,H,H | Methoxy | Methoxy | trans | öl; 5,40 |
| Ia.550 | Cyclohexyl | $-C(CH_3)=N-O-CH_2$ | H,H,H | Methoxy | Methoxy | trans | öl; 5,34 |
| Ia.551 | Thiophen-2-yl | $-C(CH_3)=N-O-CH_2$ | H,H,H | Methoxy | Methoxy | trans | öl; 5,34 |
| Ia.552 | Diphenylmethyl | $-C(CH_3)=N-O-CH_2$ | H,H,H | Methoxy | Methoxy | trans | öl; 5,33 |
| Ia.553 | 2-Methylfuran-5-yl | $-C(CH_3)=N-O-CH_2$ | H,H,H | Methoxy | Methoxy | trans | öl; 5,33 |
| Ia.554 | 3-Acetylphenyl | $-C(CH_3)=N-O-CH_2$ | H,H,H | Methoxy | Methoxy | trans | öl; 5,39 |
| Ia.555 | 3-[(Methoxycarbonyl)amino]-phenyl | $-C(CH_3)=N-O-CH_2$ | H,H,H | Methoxy | Methoxy | trans | öl; 5,37 |
| Ia.556 | 3-[([N-Methoxy-N-methylamino]-carbonyl)amino]-phenyl | $-C(CH_3)=N-O-CH_2$ | H,H,H | Methoxy | Methoxy | trans | öl; 5,38 |

Tabelle 1 a (Fortsetzung)

| Verb. Nr. | $R^{29}$- | -A- | U,V,W | $R^1$-$X_m$- | -Y | Isomer | Fp.; (°C) $^1$H-NMR (ppm) |
|---|---|---|---|---|---|---|---|
| 1a.557 | 2-(2-Methylthiazol-4-yl)-thiazol-4-yl | (E)-HC=CH- | H,H,H | Methoxy | Methoxy | trans | 163-165; 5,49 |
| 1a.558 | 2-Phenyl-eth-1-yl | (E)-HC=CH- | H,H,H | Methoxy | Methoxy | trans | Öl; 5,35 |
| 1a.559 | Phenyl | $-CO-O-CH_2$ | H,H,H | Methoxy | Methoxy | trans | Öl; 5,35 |
| 1a.560 | Benzyl | $-CO-O-CH_2$ | H,H,H | Methoxy | Methoxy | trans | Öl; 5,35 |
| 1a.561 | 2-Phenylethen-1-yl | $-CO-O-CH_2$ | H,H,H | Methoxy | Methoxy | trans | Öl; 5,38 |
| 1a.562 | trans-2,2-Dimethyl-3-(2,2-dibromethen-1-yl)-cycloprop-1-yl | $-CO-O-CH_2$ | H,H,H | Methoxy | Methoxy | trans | Öl; 5,37 |
| 1a.563 | 1-Methyl-cycloprop-1-yl | $-CO-O-CH_2$ | H,H,H | Methoxy | Methoxy | trans | Öl; 5,36 |
| 1a.564 | 4-(4-Nitrophenoxy)-phenyl | $-CO-O-CH_2$ | H,H,H | Methoxy | Methoxy | trans | Öl; 5,37 |
| 1a.565 | 1-(4-Chlorphenyl)-cycloprop-1-yl | $-CO-O-CH_2$ | H,H,H | Methoxy | Methoxy | trans | Öl; 5,31 |
| 1a.566 | 4-Benzoylphenyl | $-CO-O-CH_2$ | H,H,H | Methoxy | Methoxy | trans | Öl; 5,40 |
| 1a.567 | 4-(4-Methylphenoxy)-phenyl | $-CO-O-CH_2$ | H,H,H | Methoxy | Methoxy | trans | Öl; 5,35 |
| 1a.568 | 3-(3-Chlorpyridazin-6-yloxy)-phenyl | $-CO-O-CH_2$ | H,H,H | Methoxy | Methoxy | trans | Öl; 5,35 |
| 1a.569 | 2-(4-tert.-Butylphenyl)-ethen-1-yl | $-CO-O-CH_2$ | H,H,H | Methoxy | Methoxy | trans | Öl; 5,39 |
| 1a.570 | Biphenyl-4-yl | $-CO-O-CH_2$ | H,H,H | Methoxy | Methoxy | trans | Öl; 5,38 |

Tabelle 1b: Verbindungen der Formel 1b

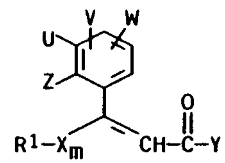

1b

| Verb. Nr. | 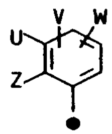 | $R^1-X_m-$ | -Y | Isomer | Fp (°C); $^1H$-NMR (ppm) |
|---|---|---|---|---|---|
| 1b.1 | 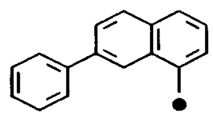 | Methoxy | Methoxy | trans | 62- 64; 5,61 |
| 1b.2 | 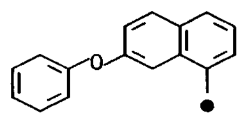 | Methoxy | Methoxy | trans | |
| 1b.3 | 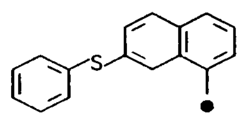 | Methoxy | Methoxy | trans | |
| 1b.4 | 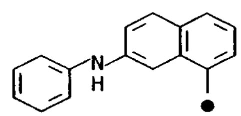 | Methoxy | Methoxy | trans | |

| Verb. Nr. | 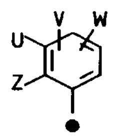 | $R^1-X_m-$ | -Y | Isomer | Fp (°C); $^1H$-NMR (ppm) |
|---|---|---|---|---|---|
| 1b.5 | 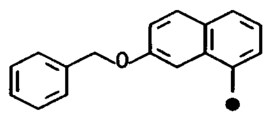 | Methoxy | Methoxy | trans | |
| 1b.6 | 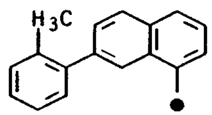 | Methoxy | Methoxy | trans | |
| 1b.7 | 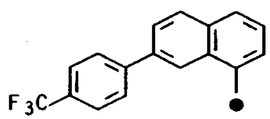 | Methoxy | Methoxy | trans | |
| 1b.8 | 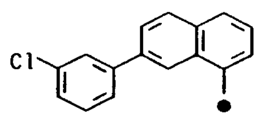 | Methoxy | Methoxy | trans | |
| 1b.9 | 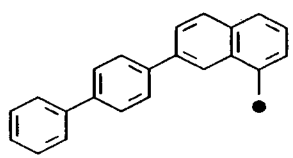 | Methoxy | Methoxy | trans | 96- 98; 5,61 |

| Verb. Nr. | 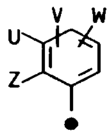 | $R^1$-$X_m$- | -Y | Isomer | Fp (°C); $^1$H-NMR (ppm) |
|---|---|---|---|---|---|
| 1b.10 | 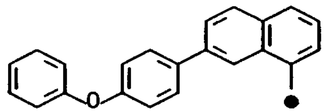 | Methoxy | Methoxy | trans | öl; 5,60 |
| 1b.11 | 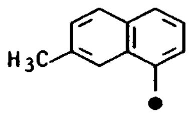 | Methoxy | Methoxy | trans | öl; 5,58 |
| 1b.12 | 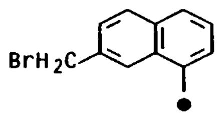 | Methoxy | Methoxy | trans | |
| 1b.13 | 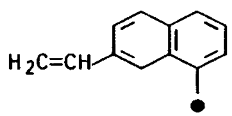 | Methoxy | Methoxy | trans | |
| 1b.14 | 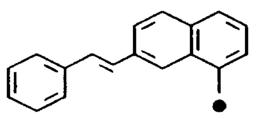 | Methoxy | Methoxy | trans | |
| 1b.15 | 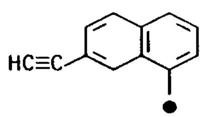 | Methoxy | Methoxy | trans | 95- 97; 5,58 |

| Verb. Nr. | 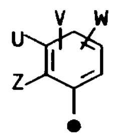 | $R^1-X_m-$ | -Y | Isomer | Fp (°C); $^1H$-NMR (ppm) |
|---|---|---|---|---|---|
| 1b.16 | 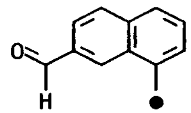 | Methoxy | Methoxy | trans | |
| 1b.17 | 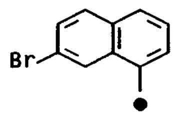 | Methoxy | Methoxy | trans | 111-113; 5,58 |
| 1b.18 | 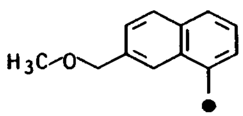 | Methoxy | Methoxy | trans | |
| 1b.19 | 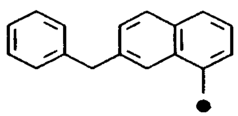 | Methoxy | Methoxy | trans | |
| 1b.20 | 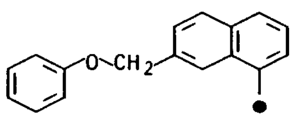 | Methoxy | Methoxy | trans | Öl; 5,56 |
| 1b.21 | 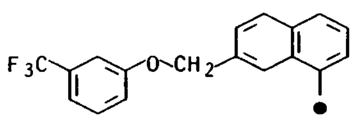 | Methoxy | Methoxy | trans | |

| Verb. Nr. | 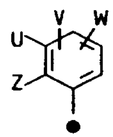 | $R^1-X_m-$ | -Y | Isomer | Fp (°C); $^1H$-NMR (ppm) |
|---|---|---|---|---|---|
| 1b.22 | 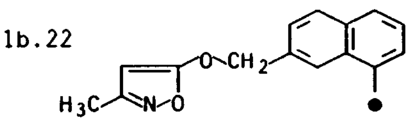 | Methoxy | Methoxy | trans | |
| 1b.23 | 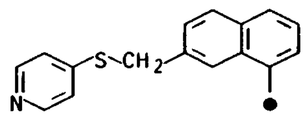 | Methoxy | Methoxy | trans | |
| 1b.24 | 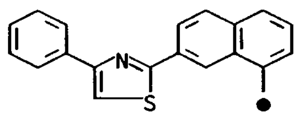 | Methoxy | Methoxy | trans | |
| 1b.25 | 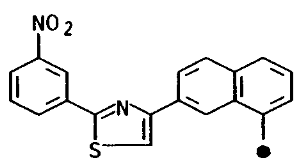 | Methoxy | Methoxy | trans | |
| 1b.26 | 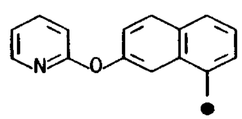 | Methoxy | Methoxy | trans | |

| Verb. Nr. | 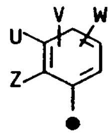 | $R^1-X_m-$ | -Y | Isomer | Fp (°C); $^1H$-NMR (ppm) |
|---|---|---|---|---|---|
| 1b.27 | 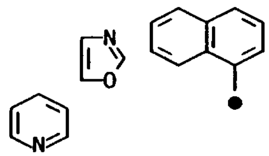 | Methoxy | Methoxy | trans | |
| 1b.28 | 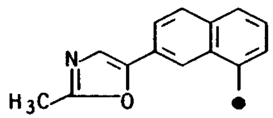 | Methoxy | Methoxy | trans | |
| 1b.29 | 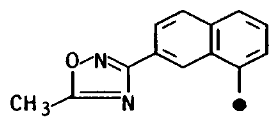 | Methoxy | Methoxy | trans | |
| 1b.30 | 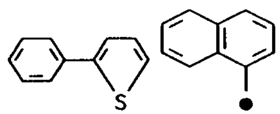 | Methoxy | Methoxy | trans | |
| 1b.31 | 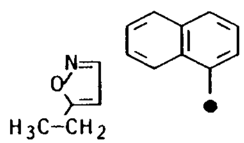 | Methoxy | Methoxy | trans | |

| Verb. Nr. | 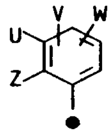 | $R^1-X_m-$ | -Y | Isomer | Fp (°C); $^1H$-NMR (ppm) |
|---|---|---|---|---|---|
| 1b.32 | 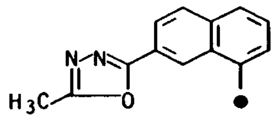 | Methoxy | Methoxy | trans | |
| 1b.33 | 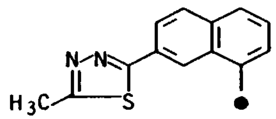 | Methoxy | Methoxy | trans | |
| 1b.34 | 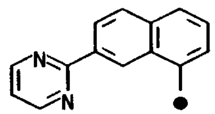 | Methoxy | Methoxy | trans | |
| 1b.35 | 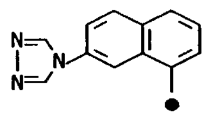 | Methoxy | Methoxy | trans | |
| 1b.36 | 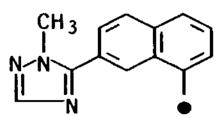 | Methoxy | Methoxy | trans | |
| 1b.37 | 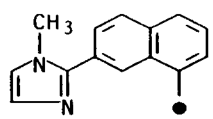 | Methoxy | Methoxy | trans | |

| Verb. Nr. | 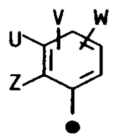 | $R^1-X_m-$ | $-Y$ | Isomer | Fp (°C); $^1H$-NMR (ppm) |
|---|---|---|---|---|---|
| 1b.38 | 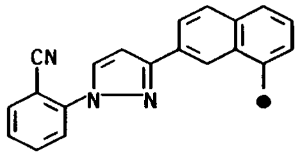 | Methoxy | Methoxy | trans | |
| 1b.39 | 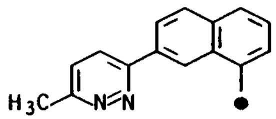 | Methoxy | Methoxy | trans | |
| 1b.40 | 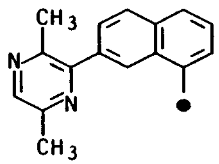 | Methoxy | Methoxy | trans | |
| 1b.41 | 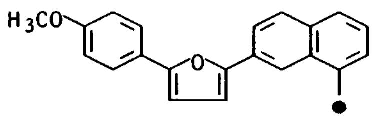 | Methoxy | Methoxy | trans | |
| 1b.42 | 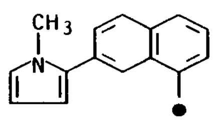 | Methoxy | Methoxy | trans | |

| Verb. Nr. | 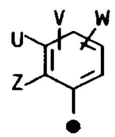 | $R^1-X_m-$ | −Y | Isomer | Fp (°C); $^1H$-NMR (ppm) |
|---|---|---|---|---|---|
| 1b.43 | 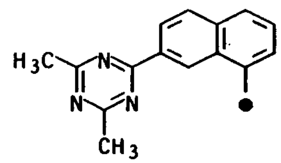 | Methoxy | Methoxy | trans | |
| 1b.44 | 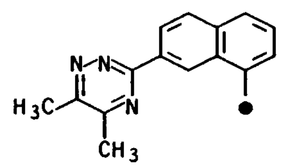 | Methoxy | Methoxy | trans | |
| 1b.45 | 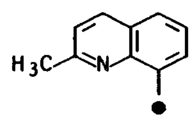 | Methoxy | Methoxy | trans | |
| 1b.46 | 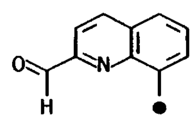 | Methoxy | Methoxy | trans | |
| 1b.47 | 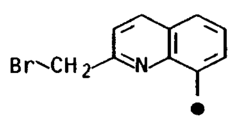 | Methoxy | Methoxy | trans | |

| Verb. Nr. | 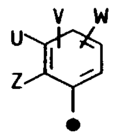 | $R^1-X_m-$ | $-Y$ | Isomer | Fp (°C); $^1H$-NMR (ppm) |
|---|---|---|---|---|---|
| 1b.48 | 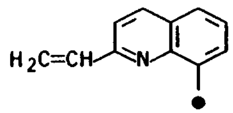 | Methoxy | Methoxy | trans | |
| 1b.49 | 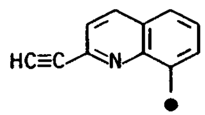 | Methoxy | Methoxy | trans | |
| 1b.50 | 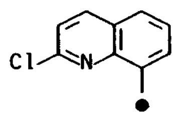 | Methoxy | Methoxy | trans | |
| 1b.51 | 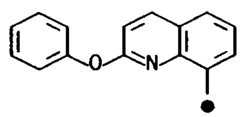 | Methoxy | Methoxy | trans | |
| 1b.52 | 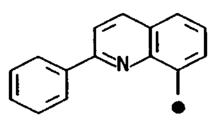 | Methoxy | Methoxy | trans | |
| 1b.53 | 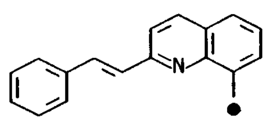 | Methoxy | Methoxy | trans | |

| Verb. Nr. | 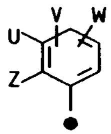 | $R^1-X_m-$ | $-Y$ | Isomer | Fp (°C); $^1H$-NMR (ppm) |
|---|---|---|---|---|---|
| 1b.54 | 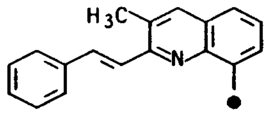 | Methoxy | Methoxy | trans | |
| 1b.55 | 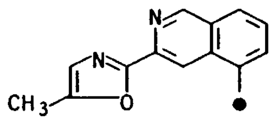 | Methoxy | Methoxy | trans | |
| 1b.56 | 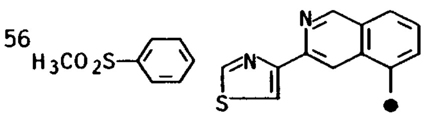 | Methoxy | Methoxy | trans | |
| 1b.57 | 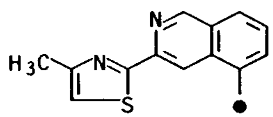 | Methoxy | Methoxy | trans | |
| 1b.58 | 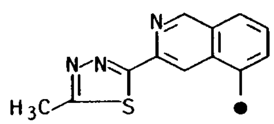 | Methoxy | Methoxy | trans | |
| 1b.59 | 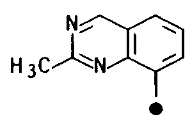 | Methoxy | Methoxy | trans | |

| Verb. Nr. | 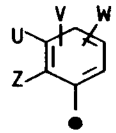 | $R^1-X_m-$ | $-Y$ | Isomer | Fp (°C); $^1H$-NMR (ppm) |
|---|---|---|---|---|---|
| 1b.60 | 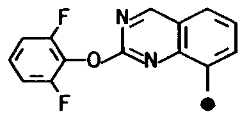 | Methoxy | Methoxy | trans | |
| 1b.61 | 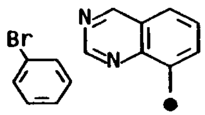 | Methoxy | Methoxy | trans | |
| 1b.62 | 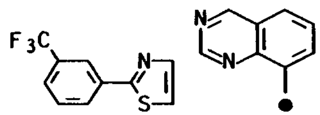 | Methoxy | Methoxy | trans | |
| 1b.63 | 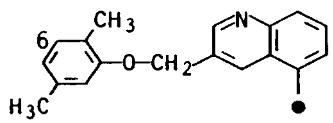 | Methoxy | Methoxy | trans | |
| 1b.64 | 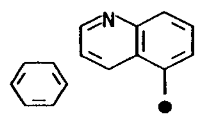 | Methoxy | Methoxy | trans | |
| 1b.65 | 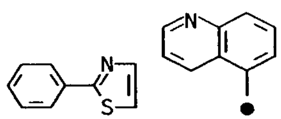 | Methoxy | Methoxy | trans | |

| Verb. Nr. | 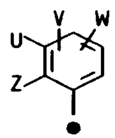 | $R^1-X_m-$ | -Y | Isomer | Fp (°C); $^1H$-NMR (ppm) |
|---|---|---|---|---|---|
| 1b.66 | 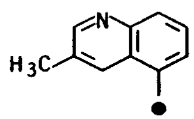 | Methoxy | Methoxy | trans | |
| 1b.67 | 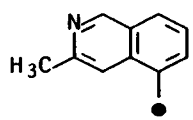 | Methoxy | Methoxy | trans | |
| 1b.68 | 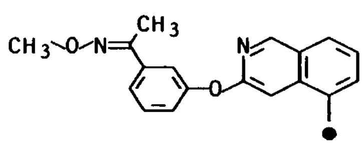 | Methoxy | Methoxy | trans | |
| 1b.69 | 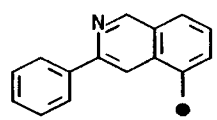 | Methoxy | Methoxy | trans | |
| 1b.70 | 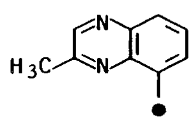 | Methoxy | Methoxy | trans | |

| Verb. Nr. | 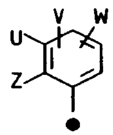 | $R^1-X_m-$ | $-Y$ | Isomer | Fp (°C); $^1H$-NMR (ppm) |
|---|---|---|---|---|---|
| 1b.71 | 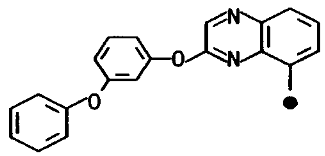 | Methoxy | Methoxy | trans | |
| 1b.72 | 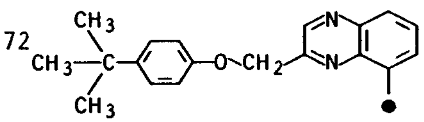 | Methoxy | Methoxy | trans | |
| 1b.73 | 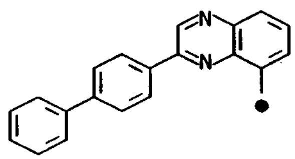 | Methoxy | Methoxy | trans | |
| 1b.74 | 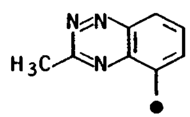 | Methoxy | Methoxy | trans | |
| 1b.75 | 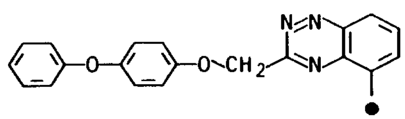 | Methoxy | Methoxy | trans | |

| Verb. Nr. | 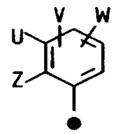 | $R^1-X_m-$ | $-Y$ | Isomer | Fp (°C); $^1H$-NMR (ppm) |
|---|---|---|---|---|---|
| 1b.76 | 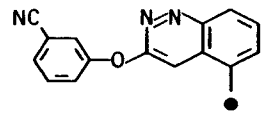 | Methoxy | Methoxy | trans | |
| 1b.77 | 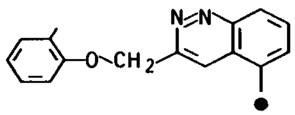 | Methoxy | Methoxy | trans | |
| 1b.78 | 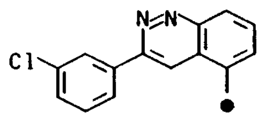 | Methoxy | Methoxy | trans | |
| 1b.79 | 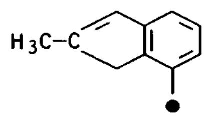 | Methoxy | Methoxy | trans | |
| 1b.80 | 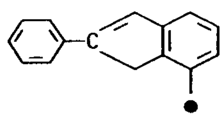 | Methoxy | Methoxy | trans | |
| 1b.81 | 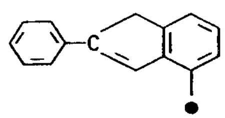 | Methoxy | Methoxy | trans | |

| Verb. Nr. | 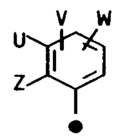 | $R^1-X_m-$ | -Y | Isomer | Fp (°C); $^1H$-NMR (ppm) |
|---|---|---|---|---|---|
| 1b.82 | 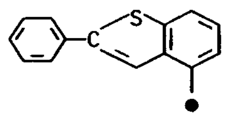 | Methoxy | Methoxy | trans | |
| 1b.83 | 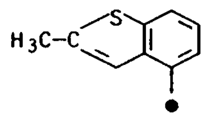 | Methoxy | Methoxy | trans | |
| 1b.84 | 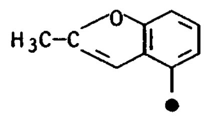 | Methoxy | Methoxy | trans | |
| 1b.85 | 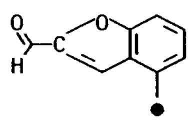 | Methoxy | Methoxy | trans | |
| 1b.86 | 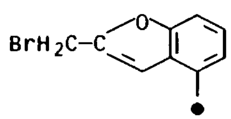 | Methoxy | Methoxy | trans | |
| 1b.87 | 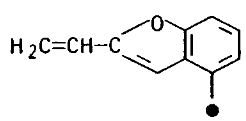 | Methoxy | Methoxy | trans | |

| Verb. Nr. | 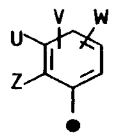 | $R^1-X_m-$ | $-Y$ | Isomer | Fp (°C); $^1H$-NMR (ppm) |
|---|---|---|---|---|---|
| 1b.88 | 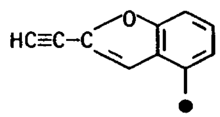 | Methoxy | Methoxy | trans | |
| 1b.89 | 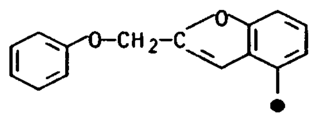 | Methoxy | Methoxy | trans | |
| 1b.90 | 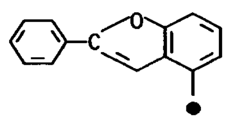 | Methoxy | Methoxy | trans | |
| 1b.91 | 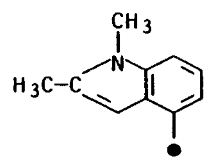 | Methoxy | Methoxy | trans | |
| 1b.92 | 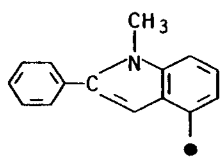 | Methoxy | Methoxy | trans | |

| Verb. Nr. | 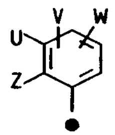 | $R^1-X_m-$ | $-Y$ | Isomer | Fp (°C); $^1H$-NMR (ppm) |
|---|---|---|---|---|---|
| 1b.93 | 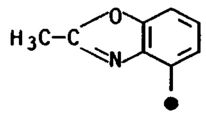 | Methoxy | Methoxy | trans | |
| 1b.94 | 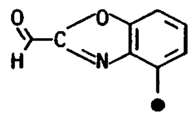 | Methoxy | Methoxy | trans | |
| 1b.95 | 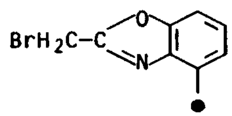 | Methoxy | Methoxy | trans | |
| 1b.96 | 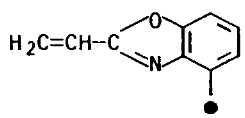 | Methoxy | Methoxy | trans | |
| 1b.97 | 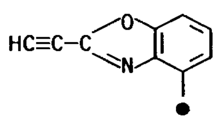 | Methoxy | Methoxy | trans | |
| 1b.98 | 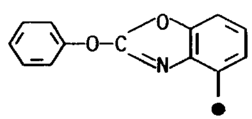 | Methoxy | Methoxy | trans | |

| Verb. Nr. | 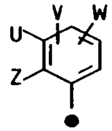 | $R^1-X_m-$ | -Y | Isomer | Fp (°C); $^1H$-NMR (ppm) |
|---|---|---|---|---|---|
| 1b.99 | 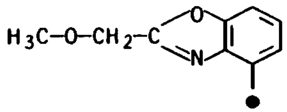 | Methoxy | Methoxy | trans | |
| 1b.100 | 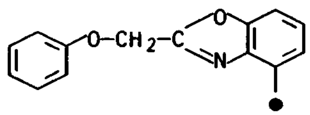 | Methoxy | Methoxy | trans | |
| 1b.101 | 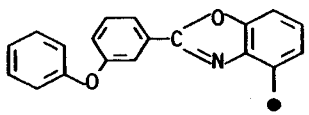 | Methoxy | Methoxy | trans | |
| 1b.102 | 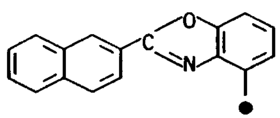 | Methoxy | Methoxy | trans | |
| 1b.103 | 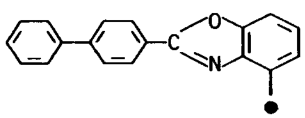 | Methoxy | Methoxy | trans | |
| 1b.104 | 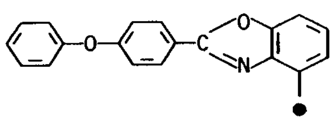 | Methoxy | Methoxy | trans | |

| Verb. Nr. | 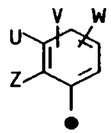 | $R^1-X_m-$ | -Y | Isomer | Fp (°C); $^1H$-NMR (ppm) |
|---|---|---|---|---|---|
| 1b.105 | 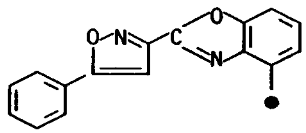 | Methoxy | Methoxy | trans | |
| 1b.106 | 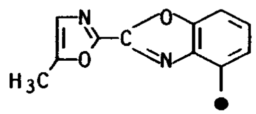 | Methoxy | Methoxy | trans | |
| 1b.107 | 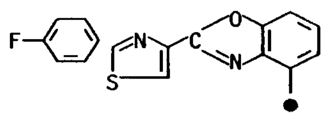 | Methoxy | Methoxy | trans | |
| 1b.108 | 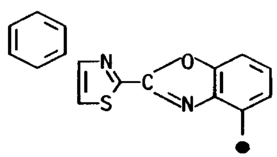 | Methoxy | Methoxy | trans | |
| 1b.109 | 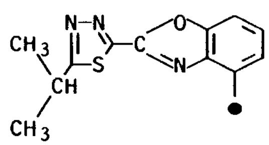 | Methoxy | Methoxy | trans | |

| Verb. Nr. | 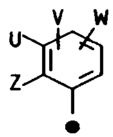 | $R^1-X_m-$ | -Y | Isomer | Fp (°C); $^1H$-NMR (ppm) |
|---|---|---|---|---|---|
| 1b.110 | 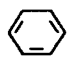 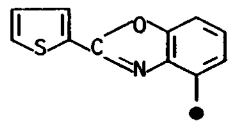 | Methoxy | Methoxy | trans | |
| 1b.111 | 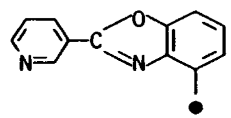 | Methoxy | Methoxy | trans | |
| 1b.112 | 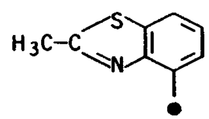 | Methoxy | Methoxy | trans | |
| 1b.113 | 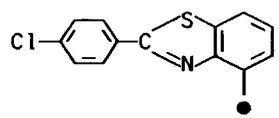 | Methoxy | Methoxy | trans | |
| 1b.114 | 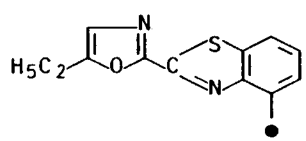 | Methoxy | Methoxy | trans | |

| Verb. Nr. | 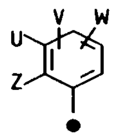 | $R^1-X_m-$ | $-Y$ | Isomer | Fp (°C); $^1H$-NMR (ppm) |
|---|---|---|---|---|---|
| 1b.115 | 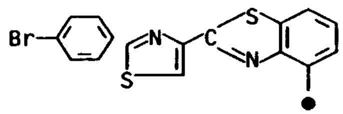 | Methoxy | Methoxy | trans | |
| 1b.116 | 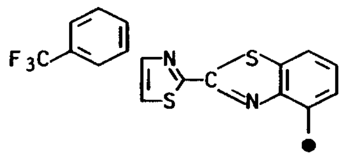 | Methoxy | Methoxy | trans | |
| 1b.117 | 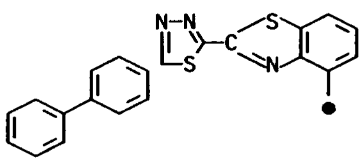 | Methoxy | Methoxy | trans | |
| 1b.118 | 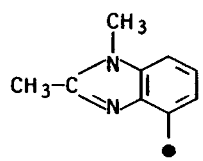 | Methoxy | Methoxy | trans | |

| Verb. Nr. | 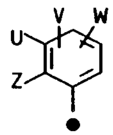 | $R^1-X_m-$ | -Y | Isomer | Fp (°C); $^1H$-NMR (ppm) |
|---|---|---|---|---|---|
| 1b.119 | 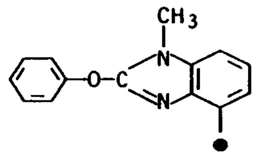 | Methoxy | Methoxy | trans | |
| 1b.120 | 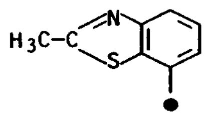 | Methoxy | Methoxy | trans | |
| 1b.121 | 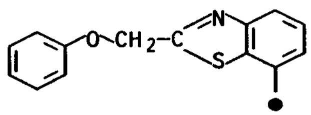 | Methoxy | Methoxy | trans | |
| 1b.122 | 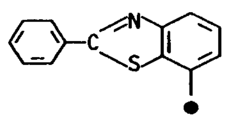 | Methoxy | Methoxy | trans | |
| 1b.123 | 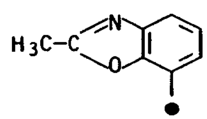 | Methoxy | Methoxy | trans | |
| 1b.124 | 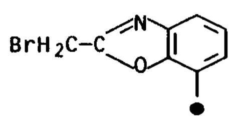 | Methoxy | Methoxy | trans | |

| Verb. Nr. | 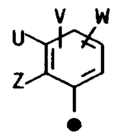 | $R^1-X_m-$ | -Y | Isomer | Fp (°C); $^1H$-NMR (ppm) |
|---|---|---|---|---|---|
| 1b.125 | 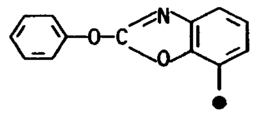 | Methoxy | Methoxy | trans | |
| 1b.126 | 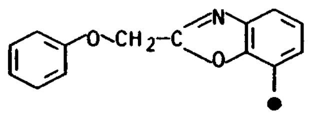 | Methoxy | Methoxy | trans | |
| 1b.127 | 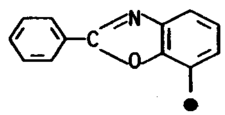 | Methoxy | Methoxy | trans | |
| 1b.128 | 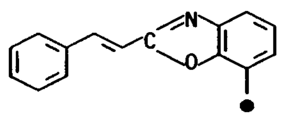 | Methoxy | Methoxy | trans | |
| 1b.129 | 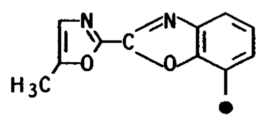 | Methoxy | Methoxy | trans | |
| 1b.130 | 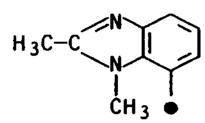 | Methoxy | Methoxy | trans | |

| Verb. Nr. | 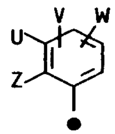 | $R^1-X_m-$ | $-Y$ | Isomer | Fp (°C); $^1H$-NMR (ppm) |
|---|---|---|---|---|---|
| 1b.131 | 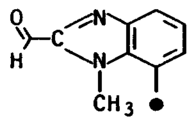 | Methoxy | Methoxy | trans | |
| 1b.132 | 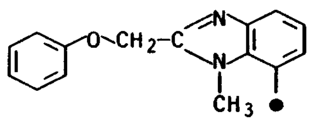 | Methoxy | Methoxy | trans | |
| 1b.133 | 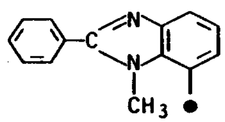 | Methoxy | Methoxy | trans | |
| 1b.134 | 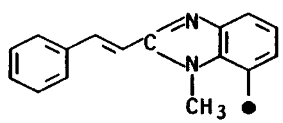 | Methoxy | Methoxy | trans | |
| 1b.135 | 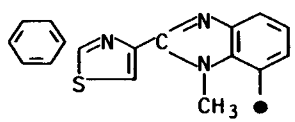 | Methoxy | Methoxy | trans | |
| 1b.136 | 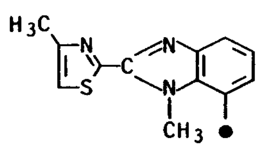 | Methoxy | Methoxy | trans | |

| Verb. Nr. | 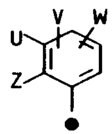 | $R^1-X_m-$ | -Y | Isomer | Fp (°C); $^1H$-NMR (ppm) |
|---|---|---|---|---|---|
| 1b.137 | 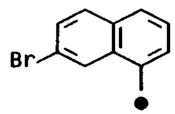 | Methoxy | Methoxy | cis | 96- 97; 5,25 |
| 1b.138 | 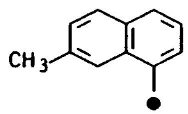 | Methoxy | Methoxy | cis | öl; 5,23 |
| 1b.139 | 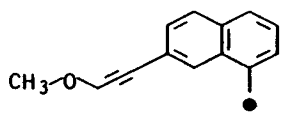 | Methoxy | Methoxy | trans | öl; 5,58 |
| 1b.140 | 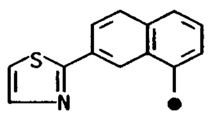 | Methoxy | Methoxy | trans | öl; 5,62 |
| 1b.141 | 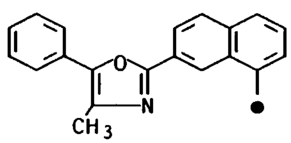 | Methoxy | Methoxy | trans | öl; 5,66 |

| Verb. Nr. | 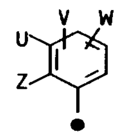 | $R^1-X_m-$ | -Y | Isomer | Fp (°C); $^1H$-NMR (ppm) |
|---|---|---|---|---|---|
| Ib.142 | 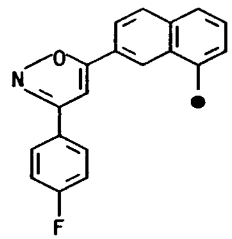 | Methoxy | Methoxy | trans | 155-158; 5,67 |
| Ib.143 | 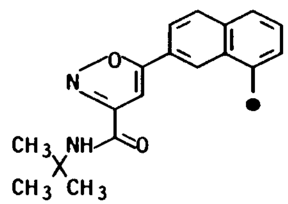 | Methoxy | Methoxy | trans | 190-192; 5,63 |
| Ib.144 | 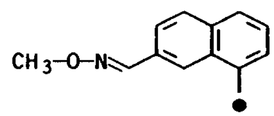 | Methoxy | Methoxy | trans | Öl; 5,60 |
| Ib.145 | 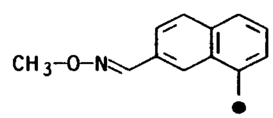 | Methoxy | Methoxy | cis | Öl; 5,25 |
| Ib.146 | 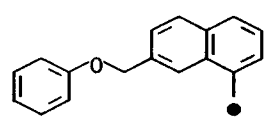 | Methoxy | Methoxy | cis | Öl; 5,24 |

| Verb. Nr. | 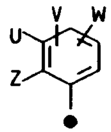 | $R^1-X_m-$ | -Y | Isomer | Fp (°C); $^1H$-NMR (ppm) |
|---|---|---|---|---|---|
| 1b.147 | 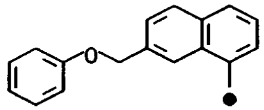 | Methoxy | Methoxy | trans | öl; 5,61 |
| 1b.148 | 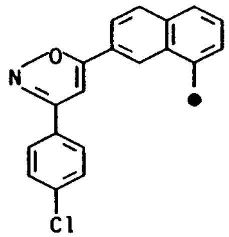 | Methoxy | Methoxy | trans | 184-187; 5,62 |
| 1b.149 | 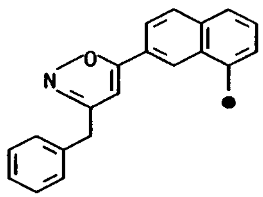 | Methoxy | Methoxy | trans | öl; 5,62 |
| 1b.150 | 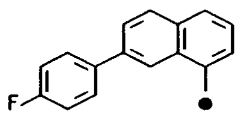 | Methoxy | Methoxy | cis | 65- 67; 5,61 |

| Verb. Nr. | 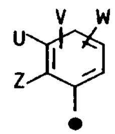 | $R^1-X_m-$ | -Y | Isomer | Fp (°C); $^1H$-NMR (ppm) |
|---|---|---|---|---|---|
| Ib.151 | 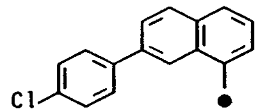 | Methoxy | Methoxy | cis | öl; 5,60 |
| Ib.152 | 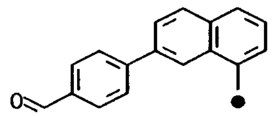 | Methoxy | Methoxy | cis | 102-105; 5,62 |
| Ib.153 | 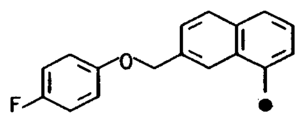 | Methoxy | Methoxy | trans | 93- 95; 5,58 |
| Ib.154 | 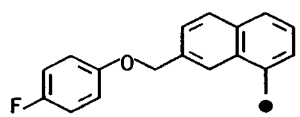 | Methoxy | Methoxy | cis | öl; 5,24 |
| Ib.155 | 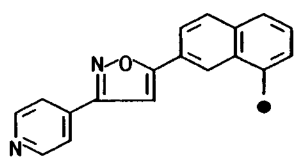 | Methoxy | Methoxy | trans | öl; 5,68 |
| Ib.156 | 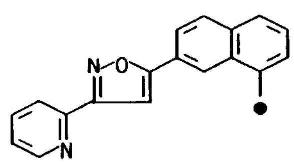 | Methoxy | Methoxy | trans | 114-116; 5,67 |

| Verb. Nr. | 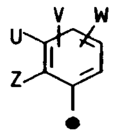 | $R^1-X_m-$ | -Y | Isomer | Fp (°C); $^1H$-NMR (ppm) |
|---|---|---|---|---|---|
| 1b.157 | 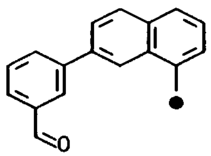 | Methoxy | Methoxy | trans | öl; 5,63 |
| 1b.158 | 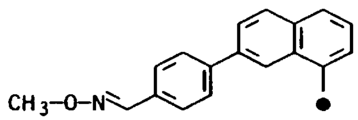 | Methoxy | Methoxy | trans | öl; 5,61 |
| 1b.159 | 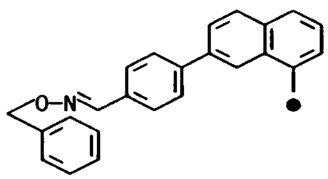 | Methoxy | Methoxy | trans | öl; 5,60 |
| 1b.160 | 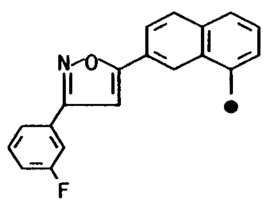 | Methoxy | Methoxy | trans | 154-155; 5,67 |

| Verb. Nr. | 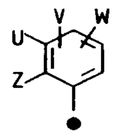 | $R^1-X_m-$ | -Y | Isomer | Fp (°C); $^1H$-NMR (ppm) |
|---|---|---|---|---|---|
| 1b.161 | 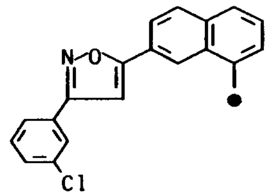 | Methoxy | Methoxy | trans | 144-146; 5,67 |
| 1b.162 | 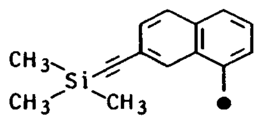 | Methoxy | Methoxy | trans | öl; 5,56 |
| 1b.163 | 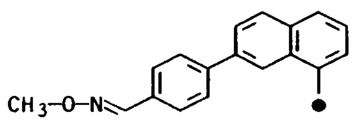 | Methoxy | Amino | trans | 181; 5,61 |
| 1b.164 | 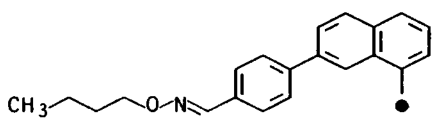 | Methoxy | Amino | trans | öl; 5,58 |
| 1b.165 | 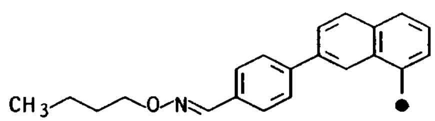 | Methoxy | Amino | cis | öl; 5,25 |

| Verb. Nr. | 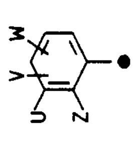 | $R^1-X_m-$ | -Y | Isomer | Fp (°C); $^1H$-NMR (ppm) |
|---|---|---|---|---|---|
| 1b.166 | 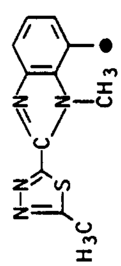 | Methoxy | Methoxy | trans | |

Es ist bekannt, daß Inhibitoren der mitochondrialen Atmung von Pilzen als Fungizide eingesetzt werden können, z.B. Antimycin (vgl. U.S. Rieske in: M. Erecinska, D.F. Wilson (Hrsg.), "Inhibitors of Mitochondrial Function", Pergamon Press, Oxford, 1981, S. 110). Der praktischen Anwendung von Antimycin stehen jedoch verschiedene Nachteile im Wege, z.B. die schwierige und aufwendige Herstellung und die nicht ausreichende Stabilität unter den Bedingungen der Anwendung im Freiland.

Die erfindungsgemäßen Zimtsäurederivate hemmen die mitochondriale Atmung von Pilzen (siehe Anwendungsbeispiel 1) und können als Fungizide eingesetzt werden.

Die mitochondriale Atmung ist für den Stoffwechsel essentiell. In den Mitochondrien wird mit Hilfe der Atmung Energie in Form von Adenosintriphosphat (ATP) gespeichert. Die Hemmung der Atmung durch mitochondriale Atmungshemmer führt letztlich zur Hemmung der ATP-Bildung. Dadurch kommt es z.B. in Pilzen, die mit den erfindungsgemäßen, $\beta$-substituierten Zimtsäurederivaten behandelt wurden, zum Erliegen der ATP- bzw. Energieabhängigen Stoffwechselprozesse und somit zum Stillstand des Wachstums bzw. zum Absterben.

Darüberhinaus können die erfindungsgemäßen Verbindungen auch als Tier- bzw. Humanantimykotika eingesetzt werden, da auch die mitochondriale Atmung von tierpathogenen bzw. humanpathogenen Pilzen gehemmt wird. Einige der Verbindungen zeigen auch Wirkung gegen Insekten und Spinmilben, da auch bei diesen Organismen die mitochondriale Atmung gehemmt wird (siehe Anwendungsbeispiel 1).

Es sind andererseits aus der Deutschen Offenlegungsschrift 33 06 996 gewisse $\beta$-Arylsubstituierte Zimtsäureamide mit fungizider Wirkung bekannt geworden, z.B. das $\beta$-Phenyl-zimtsäuremorpholid. Diese Verbindungen hemmen jedoch die mitochondriale Atmung von Pilzen praktisch nicht (siehe Anwendungsbeispiel 1) und sind hinsichtlich ihrer fungiziden Wirkung bei verschiedenen Pilzen unbefriedigend.

Demgegenüber zeichnen sich die erfindungsgemäßen Verbindungen durch ein breites Wirkungsspektrum sowohl bei Phycomyceten als auch bei Ascomyceten, Basidiomyceten und Deuteromyceten aus.

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen der Verbindung Nr. 1.38 und 10 Gew.-Teilen N-Methyl-$\alpha$-pyrrolidon, die zur Anwendung in Form kleinster Tropfen geeignet ist;

II. eine Mischung aus 20 Gew.-Teilen der Verbindung Nr. 1a.108, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 1a.2, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 1.1, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 1a.108, 3 Gew.-Teilen des Natriumsalzes der Diisobutylnaphtalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 1a.2 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 1.1, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 1a.2, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 1b.2, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Phycomyceten, Ascomyceten, Basidiomyceten und Deuteromyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,

Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,

2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Die neuen Verbindungen sind auch geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Überraschenderweise zeigen die neuen Verbindungen neben einer sehr guten antimykotischen In-vitro-Wirksamkeit eine gute, therapeutisch nutzbare In-vivo-Wirksamkeit, insbesondere gegen Dermatophyten, aber auch gegen Candida. Sie besitzen auch antibakterielle Wirksamkeiten. Die Wirkstoffe stellen somit eine wertvolle Bereicherung der Pharmazie dar.

Die Wirkung gegen Dermatophyten, Bakterien und Protozoen kann nach Methoden, wie sie beispielsweise in P. Klein, Bakteriologische Grundlagen der chemotherapeutischen Laboratoriumspraxis, Springer-Verlag, Berlin, 1957, beschrieben wird, aufgezeigt werden. Die Wirkung gegenüber Hefen können im Pseudomycel- bzw. Mycelphasentest mit Candida albicans nachgewiesen werden (vgl. DE-OS 30 20 093).

Im Modell der Meerschweinchen-Trichophytie (Trichophyton mentagrophytes), vgl. Heffter-Heubner: Handbuch der exp. Pharmakologie, Vol. XVI/II A, sind die neuen Verbindungen bei äußerlicher Anwendung auch rezidivfrei gut wirksam.

Die Wirkung der Prüfsubstanzen bei topischer Anwendung im Modell der experimentellen Candida albicans-Vaginitis war ebenfalls gut.

Die Verbindungen sind auch oral wirksam. Im Modell der experimentellen generalisierten Candidose der Maus bzw. im Modell der experimentellen Vaginitis mit Candida albicans an der Ratte konnten mit den Prüfsubstanzen in niedrigen therapeutischen Dosen nach oraler Gabe gute Ausheilungen der Infektionen erreicht werden.

Die Verbindungen sind daher besonders zur äußerlichen, aber auch oralen Behandlung von Pilzinfektionen an Mensch und Tier geeignet. Als Indikationsgebiete an Mensch und Tier sind beispielsweise zu nennen: Dermatomykosen, insbesondere verursacht durch Dermatophyten, wie Spezies der Gattungen Epidermatophyton, Microsporum oder Trichophyton, Hefen, wie Spezies der Gattungen Candida und Schimmelpilze, wie Spezies der Gattungen Aspergillus, Mucor oder Absidia.

Die Verbindungen können allein oder zusammen mit anderen bekannten Wirkstoffen, insbesondere Antibiotika, verwendet werden.

Die Herstellung der chemotherapeutischen Mittel oder Zubereitungen, die mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H. Sucker et al., Pharmazeutische Technologie, Thieme-Verlag, Stuttgart, 1978).

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nicht-wäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,01 bis 90 Gew.% der Gesamtmischung vorhanden.

Im allgemeinen können bei oraler Verabfolgung sowohl in der Human- als auch in der Veterinärmedizin der oder die Wirkstoffe in Mengen von etwa 1,0 bis etwa 50,0, vorzugsweise 2 bis 10 mg/kg Körpergewicht pro Tag, vorzugsweise 2 bis 10 mg/kg Körpergewicht pro Tag, vorzugsweise in Form mehrerer Einzelgaben zur Erzielung der gewünschten Ergebnisse verabreicht werden. Es kann jedoch erforderlich sein, von den genannten Dosierungen abzuweichen, und zwar in Abhängigkeit von der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß.

Beispiele für pharmazeutische Zubereitungen:

Beispiel A

Tablette mit 250 mg Wirkstoff

Zusammensetzung für 1000 Tabletten:

| | |
|---|---|
| Wirkstoff | 250 g |
| Kartoffelstärke | 100 g |
| Milchzucker | 50 g |
| Gelatinelösung 4 % | 45 g |
| Talkum | 10 g |

Herstellung:

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel B

Creme aus 1 % Wirkstoff

| | |
|---|---|
| Wirkstoff | 1,0 g |
| Glycerinmonostearat | 10,0 g |
| Cetylalkohol | 4,0 g |
| Polyethylenglykol-400-stearat | 10,0 g |
| Polyethylenglykol-sorbitanmonostearat | 10,0 g |
| Propylenglykol | 6,0 g |
| p-Hydroxybenzoesäuremethylester | 0,2 g |
| Entmineralisiertes Wasser | ad 100,0 g |

Herstellung:

Der feinst gepulverte Wirkstoff wird mit Propylenglykol suspendiert und die Suspension in die auf 65 °C erwärmte Schmelze aus Glycerinmonostearat, Cetylalkohol, Polyethylenglykol-400-stearat und Polyethylenglykolsorbitanmonostearat gerührt. In diese Mischung wird die 70 °C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel C

Puder mit 1 % Wirkstoff

| | |
|---|---|
| Wirkstoff | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 75,5 g |

Herstellung:

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

Anwendungsbeispiel 1

Wirkung der Verbindungen als Hemmstoffe der mitochondrialen Atmung

Um die Wirkung der erfindungsgemäßen Verbindungen auf die mitochondriale Atmung zu bestimmen, müssen zunächst die Mitochondrien aus den zu untersuchenden Organismen isoliert werden. Hierzu wird im Falle des Hefepilzes Saccharomyces cerevisiae eine 30- bis 40 %ige Zellsuspension des Pilzes in einer wäßrigen Lösung von 0,6 M Mannit + 0,01 M Tris-[hydroxymethyl]aminomethan + 0,002 M Ethylendiamintetraessigsäure-dinatriumsalz (pH 6,8) mit einer Glaskugelmühle aufgeschlossen und der Extrakt durch differentielle Zentrifugation bei 1000 x g und 12000 x g fraktioniert. Die Mitochondrien befinden sich im Niederschlag der 12000 x g-Zentrifugation.

Analog können auch Mitochondrien aus anderen Pilzen isoliert werden, z.B. aus dem phytopathogenen Pilz Drechslera sorokiniana (synonym Helminthosphorium sativum), aus dem humanpathogenen Dermatophyten Trichophyton mentagrophytes sowie aus anderen Organismen, z.B. aus der Stubenfliege (musca domestica) als Insekt oder aus der Spinnmilbe Tetranychus urticae.

Die Wirkung der Verbindungen auf die Atmungskette wird bestimmt, indem zu einer Suspension von Mitochondrien in einer wäßrigen Lösung von 0,01 M Tris-[hydroxymethyl]aminomethan + 0,65 M Sorbit + 0,01 M $KH_2PO_4$ + 0,01 M KCl + 0,0004 M Ethylendiamintetraessigsäure-dinatriumsalz + 0,3 % Rinderserumalbumin + 0,0007 M KCN + 6 x $10^{-6}$ Ubichinon-50 + 0,01 M Na-Succinat + 0,15 % Cytochrom c (pH 7,5) eine Lösung der zu untersuchenden Verbindung in Dimethylsulfoxid gegeben wird und die Geschwindigkeit der Reduktion des Cytochrom c photometrisch anhand der Extinktionszunahme bei 546 nm bestimmt wird. Als Kontrolle dient ein Ansatz mit der entsprechenden Menge reinem DMSO. Der Hemmwert für die jeweils angegebene Wirkstoffkonzentration wird dann wie folgt berechnet.

$$\% \text{ Hemmung} = 100 \cdot \frac{\left(\frac{\Delta E}{\Delta t}\right)_o - \left(\frac{\Delta E}{\Delta t}\right)_x}{\left(\frac{\Delta E}{\Delta t}\right)_o}$$

wobei ΔE die Extinktionsänderung, Δt die Zeitänderung,

$(\frac{\Delta E}{\Delta t})_O$ die Umsatzgeschwindigkeit der Kontrolle und

$(\frac{\Delta E}{\Delta t})_x$ die Umsatzgeschwindigkeit der Probe x bedeutet.

Die folgende Tabelle zeigt für eine Reihe von erfindungsgemäßen Verbindungen deren Wirkung als Atmungshemmer auf die Mitochondrien des Hefepilzes Saccharomyces cerevisiae, des phytopathogenen Pilzes Drechslera sorokiniana sowie der Stubenfliege musca domestica. Die angegebenen Hemmwerte

wurden jeweils bei einer Konzentration der Testsubstanz von 1,8 • $10^{-5}$ Mol/l bestimmt. Zum Vergleich wurde der entsprechende Wert für das β-Phenylzimtsäuremorpholid (A) - bekannt aus DE 33 06 996 - angegeben.

Tabelle zu Anwendungsbeispiel 1

Hemmung der mitochondrialen Atmung durch β-substituierte Zimtsäurederivate

| | % Atmungshemmung bei $1,8 \cdot 10^{-5}$ Mol/l Testsubstanz | | |
|---|---|---|---|
| Verbindung Nr. | Saccharomyces cerevisiae | Drechslera sorokiniana | Musca domestica |
| 1.1 | 23 | | |
| 1.71 | 30 | | 18 |
| 1.90 | 85 | | |
| 1.91 | 22 | | |
| 1.167 | 99 | | |
| 1a.2 | 88 | | 21 |
| 1a.37 | 98 | | |
| 1a.60 | 97 | 99 | 93 |
| 1a.61 | 98 | | 97 |
| 1a.62 | 87 | | 74 |
| 1a.63 | 93 | | 85 |
| 1a.64 | 94 | 98 | 93 |
| 1a.72 | 98 | 100 | 94 |
| 1a.74 | 99 | | |
| 1a.75 | 70 | | |
| 1a.77 | 63 | | |
| 1a.82 | 94 | | |
| 1a.83 | 78 | | |
| 1a.84 | 98 | | |
| 1a.86 | 97 | | |
| 1a.88 | 99 | | |
| 1a.89 | 99 | | |
| 1a.91 | 99 | | |
| 1a.98 | 100 | | |
| 1a.101 | 69 | | |
| 1a.108 | 98 | | |
| 1a.109 | 31 | | |
| 1a.110 | | | 12 |
| 1a.112 | 99 | | |
| 1a.113 | 80 | | |

Tabelle zu Anwendungsbeispiel 1 (Fortsetzung)

| Verbindung Nr. | % Atmungshemmung bei $1{,}8\cdot10^{-5}$ Mol/l Testsubstanz<br>Saccharomyces cerevisiae | Drechslera sorokiniana | Musca domestica |
|---|---|---|---|
| 1a.162 | 76 | | |
| 1a.181 | 99 | | |
| 1a.182 | 98 | | |
| 1a.233 | 88 | | 89 |
| 1a.238 | 99 | 74 | 87 |
| 1a.239 | 44 | | 16 |
| 1a.240 | 94 | | 84 |
| 1a.241 | 20 | | 9 |
| 1a.243 | 99 | 94 | 89 |
| 1a.244 | 65 | | 50 |
| 1a.245 | 98 | | 91 |
| 1a.246 | 84 | | 81 |
| 1a.247 | 99 | | |
| 1a.248 | 95 | | |
| 1a.250 | 99 | | |
| 1a.251 | 54 | | |
| 1a.264 | 98 | | |
| 1a.268 | 95 | 97 | 95 |
| 1a.269 | 96 | 81 | 76 |
| 1a.271 | 92 | | 94 |
| 1a.272 | 51 | | 78 |
| 1a.273 | 95 | 96 | 95 |
| 1a.288 | 50 | | 22 |
| 1a.290 | 98 | | 86 |
| 1a.292 | 87 | 79 | 61 |
| 1a.293 | 65 | | 27 |
| 1a.294 | 98 | | 95 |
| 1a.295 | 94 | | 76 |
| 1a.297 | 70 | | 22 |
| 1a.300 | 98 | | 93 |
| 1a.302 | 97 | | 96 |
| 1a.303 | 98 | | 86 |
| 1a.304 | 97 | | 96 |
| 1a.305 | 30 | | 39 |
| 1a.306 | 98 | | 96 |
| 1a.307 | 58 | | 71 |
| 1a.308 | 99 | | |
| 1a.310 | 99 | | |

Tabelle zu Anwendungsbeispiel 1 (Fortsetzung)

| Verbindung Nr. | % Atmungshemmung bei $1{,}8 \cdot 10^{-5}$ Mol/l Testsubstanz Saccharomyces cerevisiae | Drechslera sorokiniana | Musca domestica |
|---|---|---|---|
| 1a.312 | 99 | | |
| 1a.314 | 99 | | |
| 1a.316 | 98 | | |
| 1a.317 | 29 | | |
| 1a.318 | 98 | | |
| 1a.319 | 11 | | |
| 1a.320 | 98 | | |
| 1a.321 | 19 | | |
| 1a.322 | 99 | | |
| 1a.323 | 99 | | |
| 1a.324 | 99 | | |
| 1a.329 | 9 | | |
| 1a.331 | 79 | | |
| 1a.333 | 20 | | |
| 1a.358 | 99 | | |
| 1a.394 | 96 | | 99 |
| 1a.519 | 97 | | 89 |
| β-Phenylzimtsäure-morpholid (Vergleichsverbindung A) | 2 | 0 | 5 |

Anwendungsbeispiel 2

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis der Versuche zeigt, daß die neuen Verbindungen Nr. 1a.31, 1a.61, 1a.62, 1a.63, 1a.72, 1a.110, 1a.182, 1a.238, 1a.310, 1a.312, 1a.318, 1a.321, 1a.323, 1a.356 und 1a.394 bei der Anwendung als 250 ppm Wirkstoff enthaltende wäßrige Dispersion eine bessere fungizide Wirkung zeigen (10 % Pilzbefall) als der bekannte Wirkstoff A (35 % Pilzbefall).

Anwendungsbeispiel 3

Wirksamkeit gegen Weizenmehltau

Blätter von in Töpfen gewachsenen Weizensämlingen der Sorte "Frühgold" wurden mit wäßriger Spritzbrühe, die 80 % Wirkstoff und 20 % Emulgiermittel in der Trockensubstanz enthielt, besprüht und 24

Stunden nach dem Antrocknen des Spritzbelages mit Oidien (Sporen) des Weizenmehltaus (Erysiphe graminis var. tritici) bestäubt. Der Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis der Versuche zeigt, daß die neuen Verbindungen Nr. 1a.2, 1a.60, 1a.61, 1a.63, 1a.72, 1a.239, 1a.245, 1a.246, 1a.248, 1a.290, 1a.292, 1a.294, 1a.304 und 1a.356 bei der Anwendung als 250 ppm Wirkstoff enthaltende wäßrige Dispersion eine bessere fungizide Wirkung zeigen (15 % Pilzbefall) als der bekannte Wirkstoff A (45 % Pilzbefall).

Anwendungsbeispiel 4

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis der Versuche zeigt, daß die neuen Verbindungen Nr. 1a.72, 1a.181, 1a.248, 1a.302, 1a.314 und 1a.356 bei der Anwendung als 250 ppm Wirkstoff enthaltende wäßrige Dispersion eine bessere fungizide Wirkung zeigen (15 % Pilzbefall) als der bekannte Wirkstoff A (60 % Pilzbefall).

Die Verbindungen der Formel 1, in der $R^1$, U, V, W, $X_m$, Y und Z die oben angegebene Bedeutung haben, können nach den im folgenden Teil beschriebenen Synthesewegen und Verfahren hergestellt werden.

Die Verbindungen der allgemeinen Formel 3, in der U, V, W, Y und Z die oben angegebene Bedeutung haben, sind dabei wichtige Zwischenprodukte und Gegenstand der Erfindung.

Insbesondere umfaßt die Erfindung Verbindungen der Formel 3, in der U, V und W Wasserstoff bedeuten und Y für Methoxy steht, mit Ausnahme der Verbindungen,

- in denen Z Hydroxy, Methoxy, Phenoxy, Benzyloxy, n-Tetradecyloxy, 2-Butyloctyloxy, n-Hexadecylthio, Nitro, Amino, N-Formylamino, N-(Undecylsulfonyl)amino, Dimethylaminosulfonyl, Isohexadecenyloxy, Octadecyloxy, Thien-2-yl und 3,5-Dimethyl-thien-2-yl bedeutet,

sowie ausgenommen Verbindungen, in denen Z für eine Gruppe -$Z^a$-$Z^b$ steht, wobei $Z^a$ und $Z^b$ die folgende Bedeutung haben:

$Z^a$ Methylenoxy, Oxymethylen, Ethylen, Ethenylen, Thiomethylen oder Sauerstoff, und
$Z^b$ ggf. subst. Phenyl oder Naphthyl.

Verbindungen 3, in denen Z für Hydroxy, Methoxy, Phenoxy, Benzyloxy, n-Tetradecyloxy, 2-Butyloctyloxy, n-Hexadecylthio, Nitro, Amino, N-Formylamino, N-(Undecylsulfonyl)amino, Dimethylaminosulfonyl, Isohexadecenyloxy, Octadecyloxy, Thien-2-yl und 3,5-Dimethyl-thien-2-yl steht, sind beispielsweise aus den folgenden Druckschriften bekannt: DE-A 21 08 234, Chem. Abstr. 115, 28568h und Acta Chem. Scand. B40, 303 (1986).

Verbindungen 3, in denen Z für eine Gruppe -$Z^a$-$Z^b$ steht, wobei $Z^a$ und $Z^b$ die folgende Bedeutung haben:

$Z^a$ Methylenoxy, Oxymethylen, Ethylen, Ethenylen, Thiomethylen oder Sauerstoff, und
$Z^b$ ggf. subst. Phenyl oder Naphthyl,

werden in der EP-A 407 891 beschrieben.

Die Möglichkeiten zur Darstellung dieser Zwischenprodukte der Formel 3 sind im Schema 1 angegeben. Ihr Einsatz zur Synthese von Endprodukten der Formel 1 wird anschließend erläutert (siehe z.B. Schemata 2 und 3).

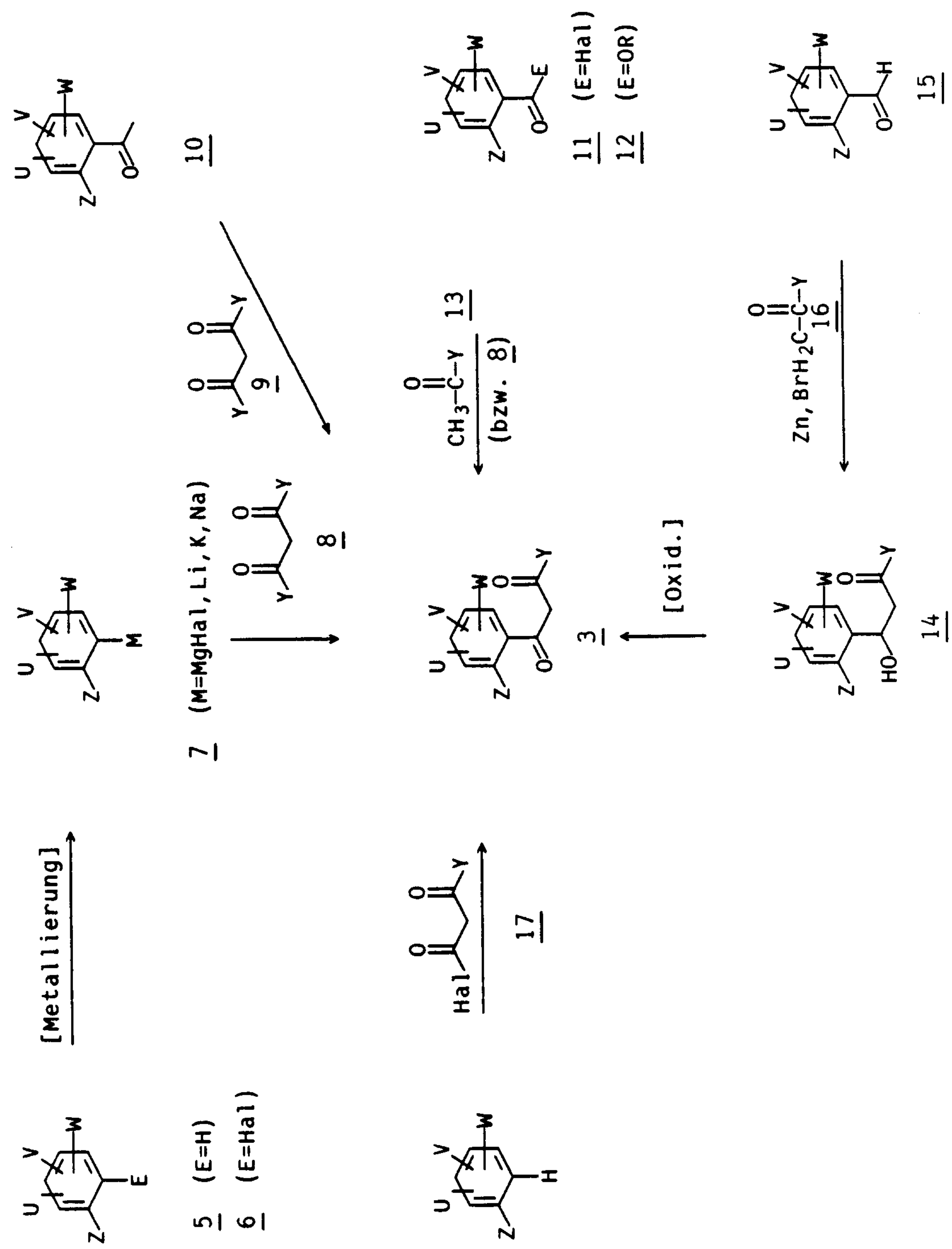


Schema 1

Die Zwischenprodukte der allgemeinen Formel 3 erhält man beispielsweise durch Umsetzung der entsprechenden Metallorganyl-Verbindungen 7, die ihrerseits durch Metallierung, bzw. Metall-Halogenaustausch aus den entsprechenden Vorstufen 5 bzw. 6 nach an sich bekannten Verfahren (vgl. Advanced Org. Chem., 3rd. Ed., 545f, 1985) zugänglich sind, mit den Malonsäure-Derivaten 8 in an sich bekannter Weise (vgl. z.B. Houben-Weyl, Bd. VII/2 a, 595 f).

Ausgehend von den Acetophenon-Derivaten 10 lassen sich die Zwischenprodukte der allgemeinen Formel 3 im Sinne einer Kondensationsreaktion mit den Kohlensäurederivaten 9 in Gegenwart einer Base, wie z.B. Natriumhydrid in an sich bekannter Weise darstellen (vgl. z.B. Org.Synth. 47, 20, 1967).

Ausgehend von den Benzoesäurederivaten 11 bzw. 12 erhält man die β-Ketosäurederivate 3 durch Umsetzung mit einem Essigsäurederivat 13 in Gegenwart einer Base, wie z.B. Natriumhydrid, Natriummethanolat oder Kalium-tert.-butanolat, in an sich bekannter Weise (vgl. z.B. Org.Synth. 23, 35, 1943).

Die Oxidation der Benzylalkohole 14 in an sich bekannter Weise (vgl. Organikum 16. Aufl. S. 358, 1986) liefert ebenfalls die Zwischenprodukte 3. Die Benzylalkohole 14 selbst lassen sich aus den entsprechenden Aldehyden 15 nach literaturbekannten Verfahren, z.B. im Sinne einer Reformatsky-Synthese, darstellen (vgl. z.B. J.Org.Chem., 35, 3966, 1970).

Ausgehend von den Benzolderivaten der allgemeinen Formel 5 lassen sich die Zwischenprodukte 3 auch im Sinne einer Friedel-Crafts-Acylierung, gegebenenfalls in Gegenwart eines Katalysators, wie z.B. $AlCl_3$, $FeCl_3$, $ZnCl_2$, in an sich bekannter Weise darstellen (vgl. z.B. Organikum 16. Auflage, S. 323f; 1986).

Die als Vorprodukte einsetzbaren Acetophenone 10, Benzoesäurederivate 11 bzw. 12 und Benzaldehyde 15 sind entweder bekannt oder können analog zu bekannten Methoden, wie z.B. aus den metallierten Zwischenstufen der Formel 7 durch Umsetzung mit einem Elektrophil, z.B. mit einem Essigsäurederivat (→ 10), einem Kohlensäurederivat (→ 12) oder einem Ameisensäurederivat (→ 15) dargestellt werden.

Die Verbindungen der allgemeinen Formel 1, in der $R^1$, U, V, W, Y und Z die oben angegebene Bedeutung haben und in der X Sauerstoff und m = 1 bedeutet, können z.B. nach Schema 2 dargestellt werden.

Schema 2

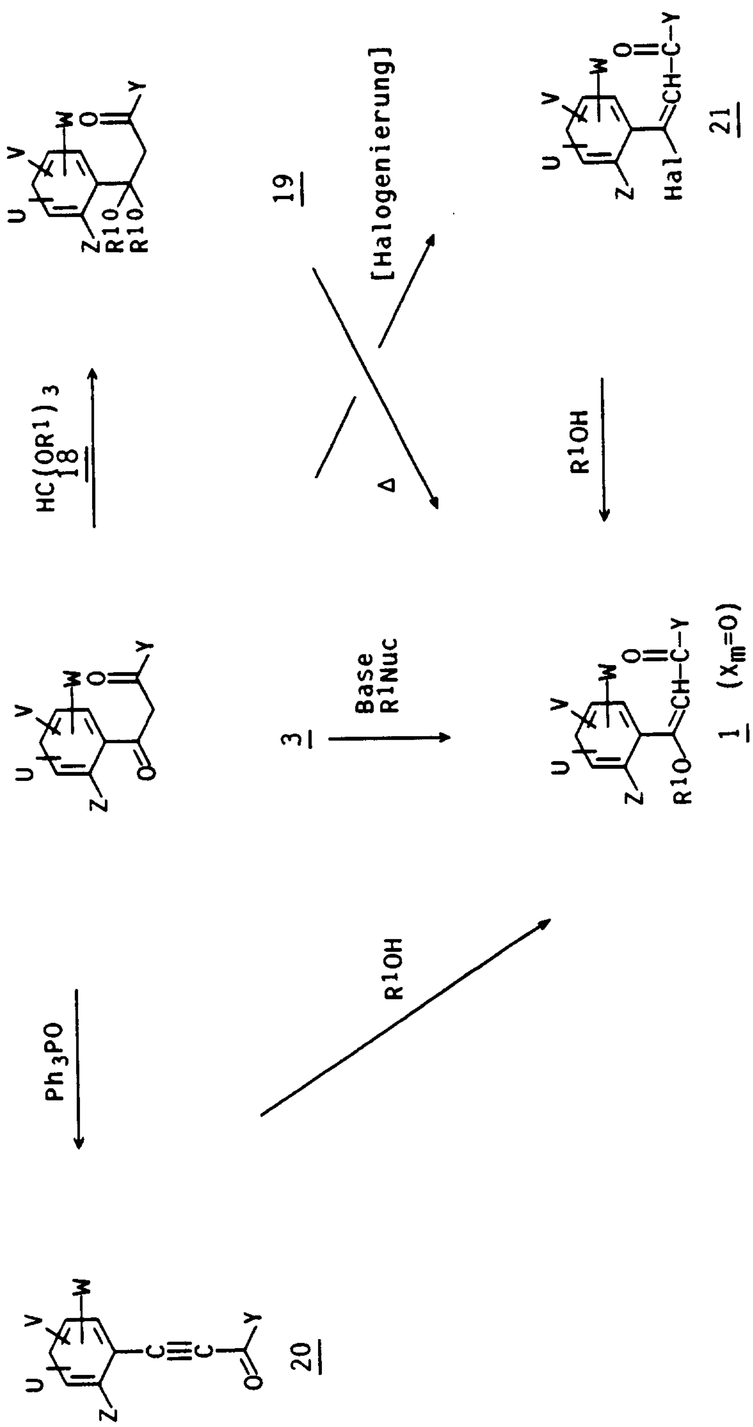


Die Umsetzung der $\beta$-Ketosäurederivate 3, insbesondere der entsprechenden $\beta$-Ketoester, in an sich bekannter Weise mit einem Alkylierungsmittel $R^1$Nuc, wie z.B. mit einem Dialkylsulfat, wie Dimethylsulfat oder mit einem Alkylhalogenid wie z.B. Methylchlorid oder Butylchlorid, in einem Lösungs-oder Verdünnungsmittel, wie z.B. Dimethylsulfoxid oder Hexamethylphosphorsäuretriamid, gegebenenfalls in Gegenwart einer Base, wie z.B. Natriumhydrid oder Kalium-tert.-butylat, führt zu Verbindungen der Formel 1 in denen $X_m$ Sauerstoff bedeutet (vgl. z.B. Synthesis 1, 1970).

Alternativ kann man auch so vorgehen, daß man β-Ketosäurederivate der Formel 3 in an sich bekannter Weise mit einem Ortho-Ameisensäureester 18, gegebenenfalls in Gegenwart einer Säure, wie z.B. Salzsäure, Schwefelsäure oder p-Toluolsulfonsäure, umsetzt. Die dabei gebildeten Ketale 19 können entweder isoliert und dann durch Erhitzen in die Produkte 1 überführt werden, oder aber direkt ohne Isolierung in die Produkte 1 überführt werden (vgl. Houben-Weyl, Bd. 6/3, S. 112, 1965).

Ausgehend von den Acetylenderivaten 20 lassen sich die neuen Verbindungen der Formel 1 durch Addition eines Alkohols $R^1OH$, gegebenenfalls in Gegenwart eines Katalysators, wie z.B. HCl oder KOH, darstellen (vgl. z.B. Houben-Weyl, Bd. 6/3, S. 114, 1965).

Die Acetylenderivate 20 selbst können nach an sich literaturbekannten Verfahren, z.B. aus den β-Ketosäurederivaten 3 z.B. durch Reaktion mit Triphenylphosphinoxid erhalten werden (vgl. z.B. Synthesis, 217, 1989).

Zudem lassen sich die neuen Verbindungen der Formel 1 aus den Vinylhalogeniden 21 durch nucleophile Substitution mit einem Alkohol $R^1OH$, gegebenenfalls in Gegenwart einer Base, wie z.B. Natriumhydrid darstellen (vgl. z.B. J. Org. Chem. 50, 4664, 1985).

Die Vinylhalogenide 21 selbst sind beispielsweise durch Halogenierung der β-Ketoesäurederivate 3 in an sich bekannter Weise zugänglich (vgl. z.B. Org. Synth. 6, 505).

Schema 3 erläutert die Synthese von Verbindungen der Formel 1, in der $R^1$, U, V, W, Y und Z die oben angegebene Bedeutung haben und in der $X_m$ für Schwefel steht (entspricht Formel 22 in Schema 3).

Schema 3

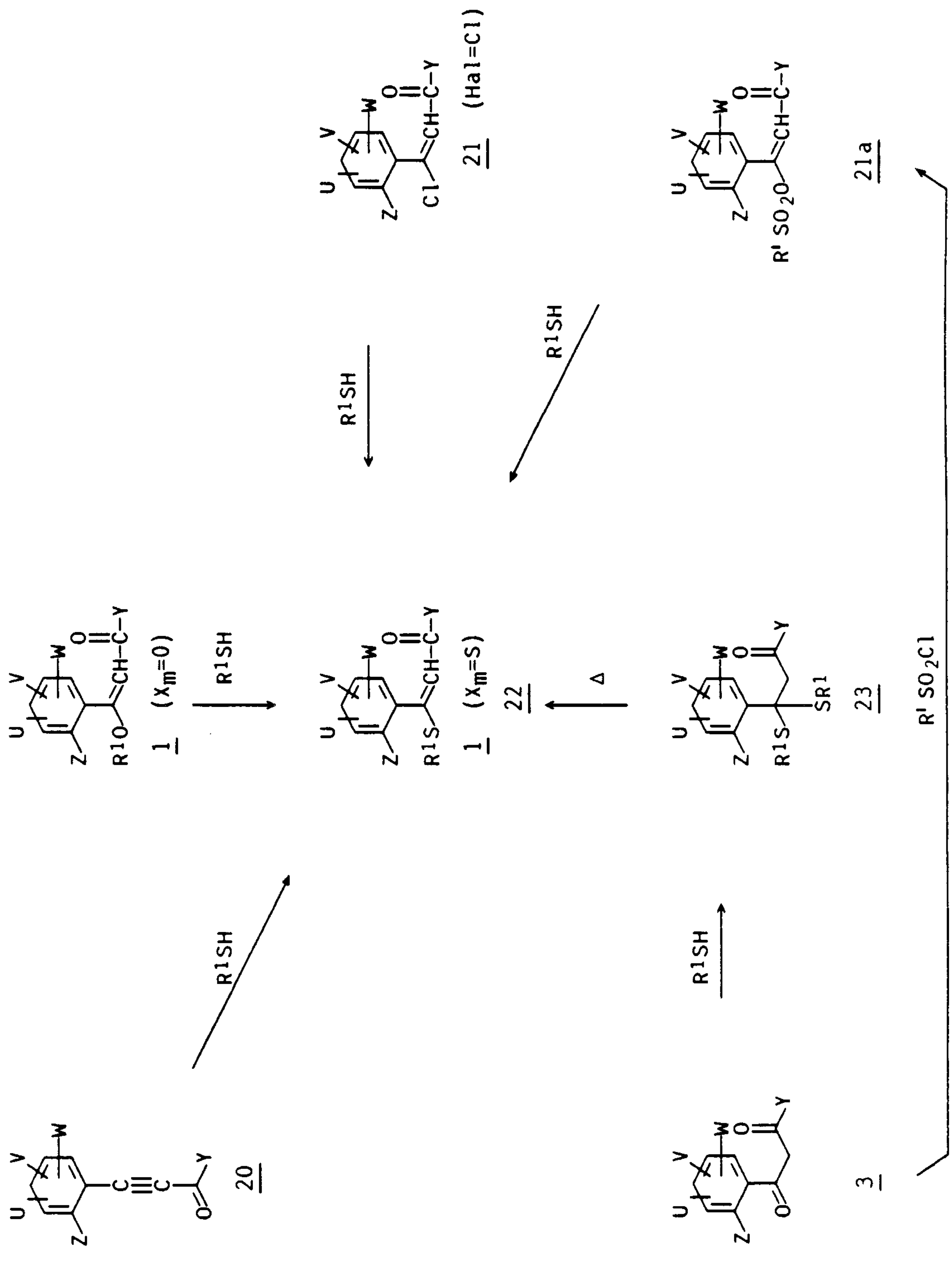


Ausgehend von den Acetylenderivaten 20 lassen sich die neuen Verbindungen der Formel 22 durch Addition eines Thiols $R^1SH$ gegebenenfalls in Gegenwart einer Base, wie z.B. KOH, Pyridin, oder einer Säure, wie z.B. HCl, darstellen (vgl. z.B. E. Larsson, J. Prakt. Chem. 325(2), S. 328, 1983; Synthesis 12, S. 1070, 1986; Houben-Weyl Bd. 9, S. 133, 1955).

Verbindungen der Formel 22 lassen sich ebenfalls dadurch herstellen, daß man $\beta$-Ketosäurederivate, insbesondere $\beta$-Ketoester 3 in an sich bekannter Weise gegebenenfalls in Gegenwart einer Säure, wie z.B. Salzsäure, Schwefelsäure, p-Toluolsulfonsäure oder einer Lewis-Säure, wie z.B. $ZnCl_2$ oder $BF_3$, mit

Thiolen $R^1SH$ umsetzt. Die dabei gebildeten Thioketale 23 können entweder direkt, oder nach Isolierung, durch Erhitzen in die Produkte 22 überführt werden. (vgl. z.B. Angew. Chem. 77, S. 1140, 1965; J. Org. Chem. 40, S. 812, 1975).

Ausgehend von Verbindungen der Formel 21 oder 21a lassen sich die Verbindungen der Formel 22 durch Umsetzung mit Thiolen $R^1SH$, gegebenenfalls in Gegenwart einer Base in an sich bekannter Weise darstellen. Verbindungen der Formel 21a lassen sich durch Umsetzung von Verbindungen der Formel 3 mit Sulfonsäurechloriden $R^1SO_2Cl$, wie z.B. Methansulfonsäurechlorid (R' = Methyl), Trifluormethansulfochlorid (R' = p-Methylphenyl), gegebenenfalls nach Zusatz von Basen (z.B. Triethylamin, Natriumhydrid) darstellen.

Verbindungen der allgemeinen Formel 22 lassen sich außerdem darstellen, indem man Verbindungen der allgemeinen Formel 1, in der X Sauerstoff und m = 1 bedeutet, mit Thiolen $R^1SH$ unter sauren oder alkalischen Bedingungen umsetzt.

Die Enamine der allgemeinen Formeln 24 bzw. 25 (entsprechend Formel 1 mit $X_m$ = $NR^2$ bzw. $NOR^3$), in denen $R^1$, $R^2$, $R^3$, U, V, W, Y und Z die oben angegebene Bedeutung haben, können z.B. nach Schema 4 dargestellt werden.

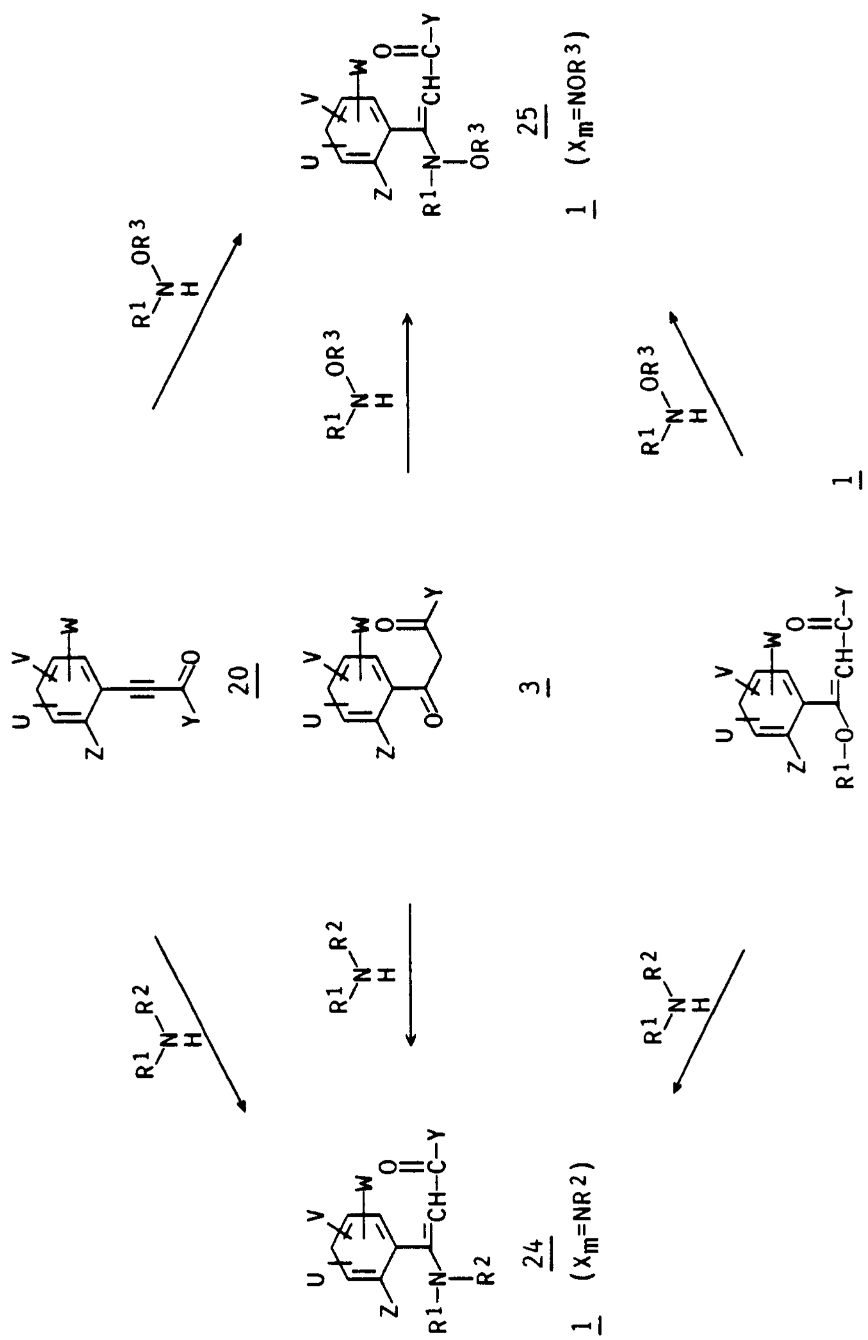

1 25 ($X_m$=NOR3)

20

3

1

1 24 ($X_m$=NR2)

Schema 4

Ausgehend von Verbindungen der allgemeinen Formel 20 lassen sich die neuen Verbindungen der allgemeinen Formel 24 in an sich bekannter Weise dadurch darstellen, daß man Amine der allgemeinen Formel $R^1R^2NH$ addiert (vgl. z.B. Houben-Weyl, Bd. 11/1, S. 299ff, 1957). Durch Addition von Alkoxyaminen R1R3ONH an Verbindungen der allgemeinen Formel 20 lassen sich analog die Verbindungen der allgemeinen Formel 25 darstellen.

Weiterhin können Enamine der allgemeinen Formel 24 dargestellt werden, indem man β-Ketosäurederivate, insbesondere β-Ketoester 3 mit primären oder sekundären Aminen $R^1R^2NH$ gegebenenfalls unter sauren oder basischen Bedingungen in an sich bekannter Weise umsetzt (vgl. z.B. Houben-Weyl, Bd. 11/1,

S. 172, 1957). Zum Beispiel können β-Ketoester 3 gegebenenfalls zunächst mit einer Base, wie z.B. Natriumhydrid oder Natriumhydroxid, in die entsprechenden Enolate überführt werden, die mit den Hydrochloriden von primären oder sekundären Aminen unter Freisetzung von Natriumchlorid zu Verbindungen der allgemeinen Formel 24 reagieren (vgl. z.B. Ann. Chim. [10] 18 (1932) 103; J. Am. Chem. Soc. 70, S. 3350 (1948)). Die Darstellung von Verbindungen der allgemeinen Formel 25 aus β-Ketosäurederivaten 3 erfolgt analog durch Umsetzung mit Alkoxyaminen $R^3ONHR^1$.

Weiterhin können Enamine 24 dargestellt werden, indem man Verbindungen der allgemeinen Formel 1, in der X = Sauerstoff und m = 1 bedeutet, mit primären oder sekundären Aminen $R^1R^2NH$ gegebenenfalls unter sauren oder basischen Bedingungen in an sich bekannter Weise umsetzt (vgl. z.B. Houben Weyl, Bd. 11/1, s. 198, (1957); J. Heterocycl. Chem. 21(6), s. 1753, (1984); Indian J. Chem. 27(11), s. 1044 (1988)).

Schema 5 verdeutlicht die Synthese von Verbindungen der Formel 1, in der $R^1$, U, V, W, Y und Z die oben angegebene Bedeutung haben und m = 0 ist (entsprechend Formel 26 in Schema 5).

Schema 5

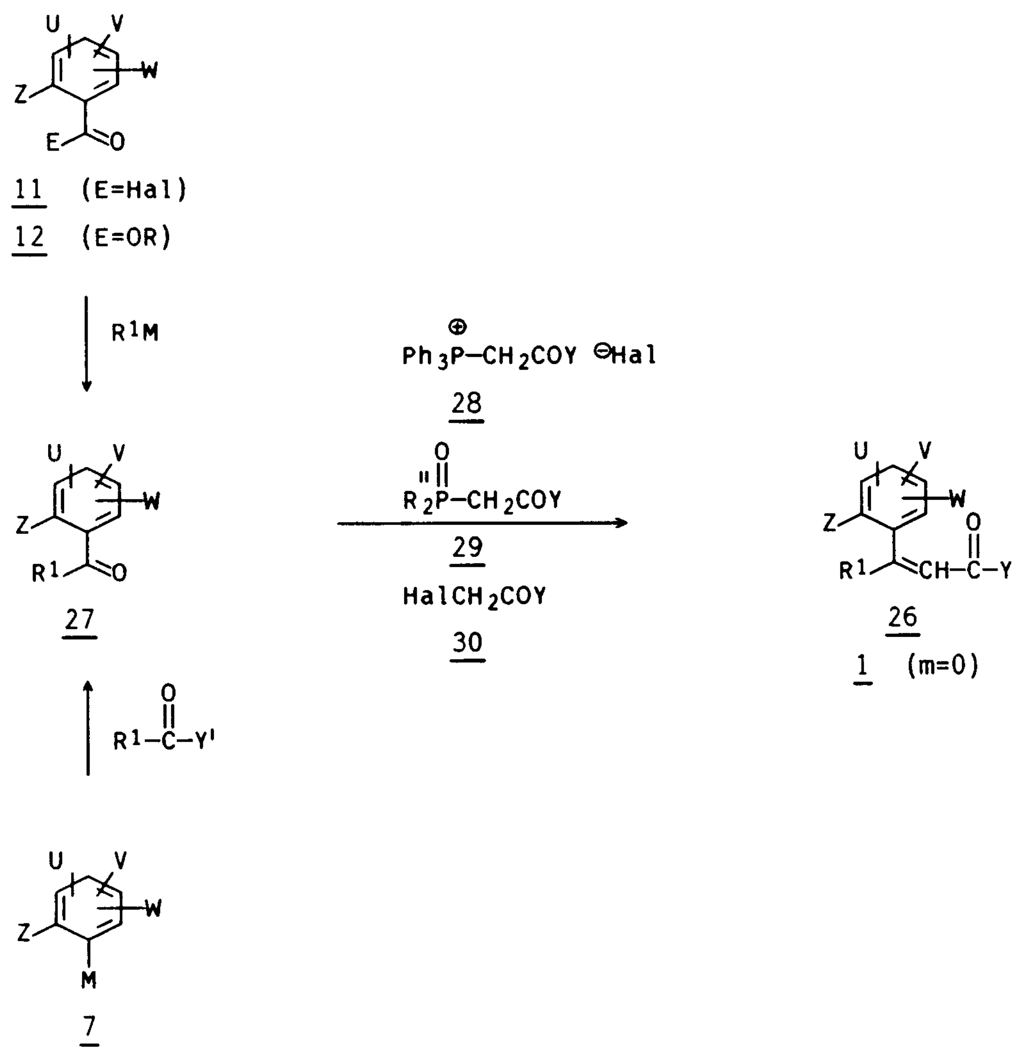


Ausgehend von den Arylketonen 27 erhält man die β-Alkylzimtsäure-Derivate 26 beispielsweise, indem man in an sich bekannter Weise (vgl. z.B. Tetrahedron 46, S. 5859, (1990)) mit einem Wittig-bzw. Wittig-Horner-Reagens der allgemeinen Formeln 28 bzw. 29 in einem inerten organischen Lösungsmittel in Gegenwart einer Base, wie z.B. Hatriumhydrid oder Kalium-t.-butylat, umsetzt. In der Formel 28 steht Hal für ein Halogenatom wie Chlor oder Brom. In der Formel 29 steht R" für Alkoxy wie z.B. Methoxy oder Ethoxy.

Alternativ können Verbindungen der allgemeinen Formel 26 dargestellt werden, indem man 27 in an sich bekannter Weise (vgl. z.B. Bull. Soc. Chim. Belg., 79 (1-2), S. 61-74 (1970)) mit α-Halogenessigsäurede-

rivaten der allgemeinen Formel 30 und Zink im Sinne einer Reformatsky-Reaktion umsetzt und anschließend dehydratisiert. Hal in Formel 30 steht für Chlor oder Brom.

Die Arylketone 27 sind entweder bekannt oder können in Analogie zu bekannten Methoden erhalten werden, wie zum Beispiel aus Verbindungen der allgemeinen Formel 7 durch Umsetzung mit Carbonsäurederivaten $R^1COY'$, wobei Y' für Halogen, OR oder $NR_2$ steht, oder aus Verbindungen der Formeln 11 bzw. 12 durch Umsetzung mit metallorganischen Verbindungen $R^1M$.

Verbindungen der Formel 1, in denen die Gruppe COY die verschiedenen in Frage kommenden Ester-, Thiolester- bzw. Amidgruppierungen repräsentiert, lassen sich leicht nach einer Reihe von Standardverfahren durch Umwandlung der Gruppe COY herstellen (s. z.B. Houben-Weyl, Methoden der Organischen Chemie, Band E 5 sowie die Herstellungsbeispiele 9-11). So lassen sich beispielsweise aus den Estern der Formeln 1, in der $Y = OR^4$ bedeutet, leicht die entsprechenden Carbonsäuren durch Hydrolyse nach gängigen Verfahren (s. z.B. Houben-Weyl, Band E5, S. 223-254, speziell S. 231-237) herstellen. Diese können anschließend in aktivierte Carbonsäurederivate, wie etwa die Säureimidazolide mit Y = Imidazol-1-yl oder die Säurechloride mit Y = Cl überführt werden (s. z.B. Houben-Weyl, Band VIII, S. 463ff). Aus diesen wiederum resultieren durch Umsetzung mit Alkoholen bzw. Oximen die entsprechenden Ester der Formel 1 mit $Y = OR^4$ bzw. Oximester mit $Y = O\text{-}N = CR^5R^6$ (s. z.B. Houben-Weyl, Band VIII, S. 543ff) bzw. durch Umsetzung mit Thiolen die entsprechenden Thiolester der Formel 1 mit $Y = SR^{11}$ (s. z.B. Houben-Weyl, Band IX, S. 753-755) bzw. durch Umsetzung mit Aminen die entsprechenden Amide der Formel 1 mit $Y = NR^7R^8$ bzw. $Y = N(OR^9)R^{10}$ (s. z.B. Houben-Weyl, Band VIII, S. 655ff).

Die neuen Verbindungen der allgemeinen Formel 1b, in der V, W, $X_m$, Y und $R^1$ die in Anspruch 1 angegebene Bedeutung haben, und Z und U in benachbarten Positionen des Phenylringes zusammen einen gegebenenfalls substituierten fünf- oder sechsgliedrigen aromatischen oder heteroaromatischen Ring bilden, werden nach den oben beschriebenen Verfahren aus den entsprechenden benzoanellierten Zwischenprodukten hergestellt z.B. analog zu den in dem Schemata 1 bis 5 angegebenen Synthesewegen. Die Darstellung der anellierten Zwischenprodukte wird im folgenden beschrieben (vgl. Schemata 6 bis 8).

Beispielsweise sind als Vorprodukte geeignete 1,7-disubstituierte Naphthalinderivate der Formel 10a bekannt oder lassen sich in an sich bekannter Weise darstellen. Beispielsweise erhält man aus 2-substituierten Naphthalinen durch Friedel-Crafts-Acylierung, gegebenenfalls in Gegenwart eines Katalysators, wie z.B. $AlCl_3$, $FeCl_3$, $ZnCl_2$ analog zu bekannten Methoden Verbindungen der Formel 10a (vgl. z.B. J.Chem.Soc.C, 1966, 518).

Schema 6

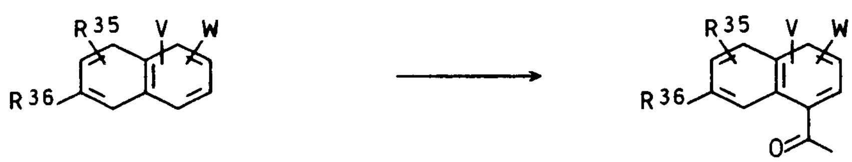

70 10a

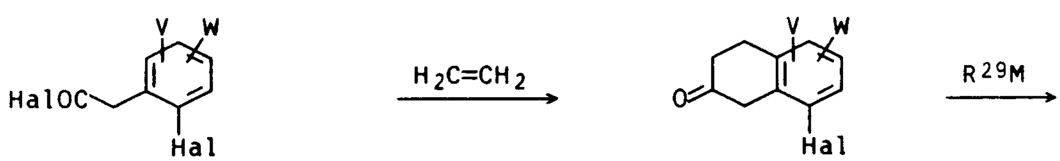


71 72

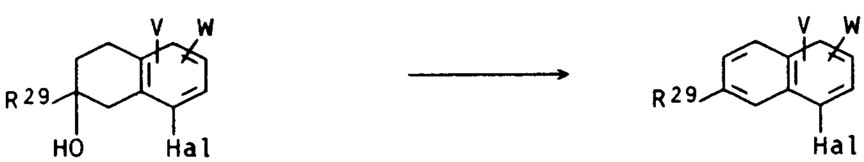

73 5a

V, W, Hal, $R^{29}$, $R^{35}$ und $R^{36}$ wie oben definiert.

Weiterhin erhält man Verbindungen der allgemeinen Formel 6a, in denen $R^{29}$ die oben angegebene Bedeutung hat, aus (2-Halogen-phenyl)essigsäurederivaten, beispielsweise den entsprechenden Säurechloriden, durch Umsetzung mit Ethylen in Gegenwart einer Lewis-Säure, beispielsweise $AlCl_3$, zum Keton 72. Die Umsetzung der Ketone mit metallorganischen Verbindungen $R^{29}M$ beispielsweise mit Lithiumorganischen Verbindungen oder Grignard-Verbindungen, liefert die Alkolhole 73, die mit Triphenylmethanol unter sauren Bedingungen zu den als Zwischenprodukte benötigten Naphthalinderivaten 6a aromatisiert werden (vgl. z.B. EP 393 941). Diese können nach den in Schema 1 erläuterten Wegen in die Endprodukte der Formel 1 bzw. 1b umgewandelt werden.

Die als Zwischenprodukte benötigten 2-substituierten 8-Chinolin-carbonsäurederivate der allgemeinen Formel 12b können für den Fall, daß $R^{36} = R^{29}$ ist, nach dem folgenden Schema 7 aus Aldehyden und o-Toluidinen erhalten werden:

Schema 7

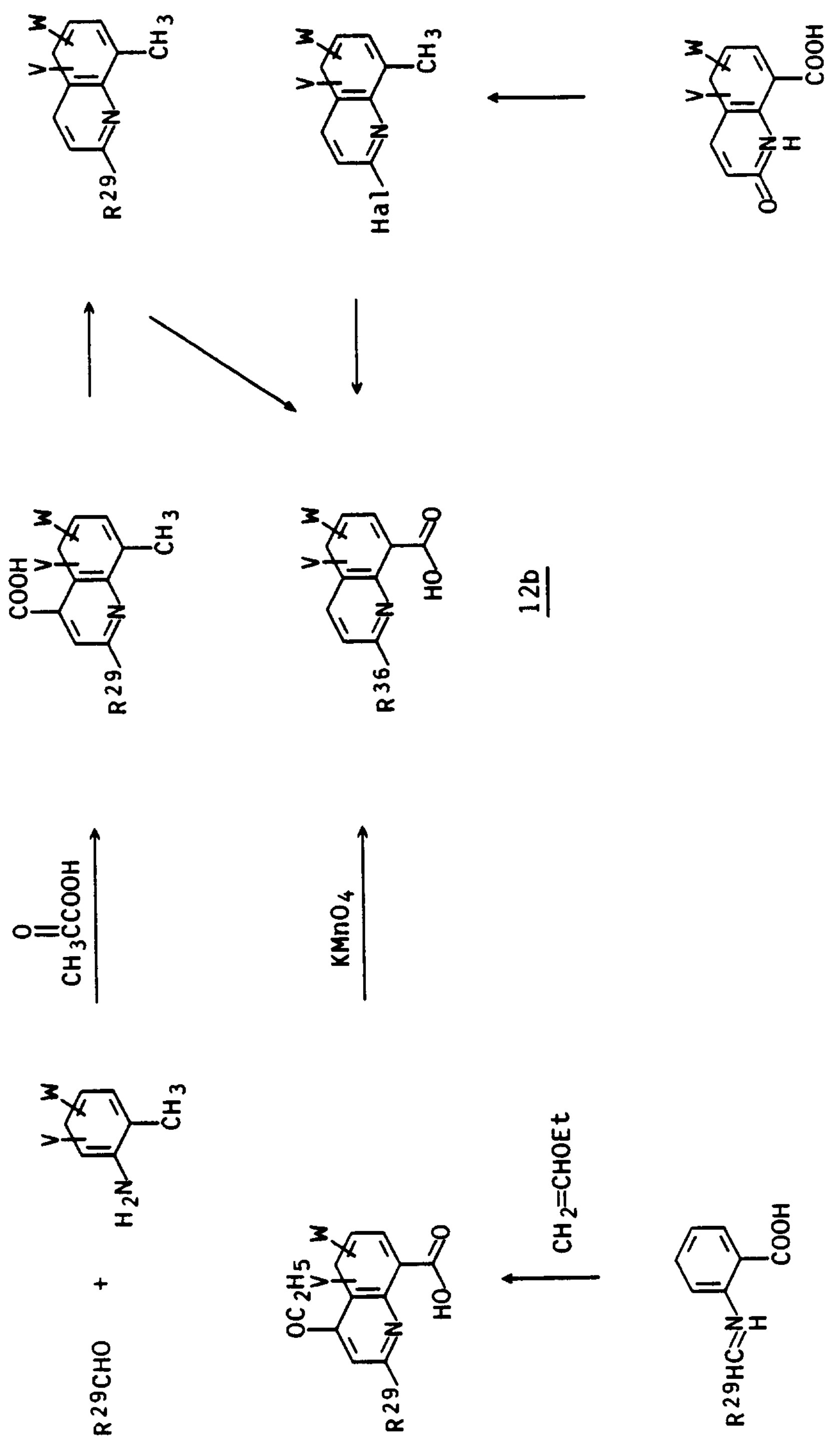


12b

V, W, R29, R36 wie oben definiert

Aldehyde reagieren mit o-Toluidinen im Sinne einer Doebner-Reaktion zu 2-substituierten 8-Methylchinolin-4-carbonsäuren, die durch eine Kupferkatalysierte Decarboxylierung in die entsprechenden Methylchinoline umgewandelt werden. Die Oxidation der Methylgruppe nach bekannten Methoden ergibt die gewünschten 8-Chinolincarbonsäuren 12b (vgl. z.B. J. Am. Chem. Soc. 68, 1589 (1946)).

Weiterhin erhält man für den Fall, daß $R^{36} = R^{29}$ ist, 8-Chinolin-carbonsäuren der Formel 12b aus Schiffschen Basen von Anthranilsäurederivaten durch Umsetzung mit Vinylethern in Gegenwart eines

sauren Katalysators, beispielsweise $H_2SO_4$ oder $BF_3$, und anschließende oxidative Aromatisierung der 4-Alkoxy-1,2,3,4-tetrahydrochinolin-8-carbonsäure, beispielsweise mit $KMnO_4$ (vgl. z.B. Izv. AN SSSR, Ser. kim., 1966, 120 (engl.)).

Für den Fall, daß $R^{36}$ für die Substituenten $OR^{12}$, $SR^{13}$ oder für $NR^{37}R^{38}$ steht, lassen sich die Verbindungen 12b aus den 2-Halogenchinolinen durch Umsetzung mit einen Alkohol ($R^{12}OH$), einem Mercaptan ($R^{13}SH$) oder einem Amin ($R^{37}R^{38}NH$), ggf. in Gegenwart einer Base, erhalten. Die 2-Halogenchinoline werden nach bekannten Verfahren (vgl. z.B. Houben-Weyl, Bd. V/3, 920) aus den 2-Chinolonen erhalten (vgl. z.B. Chem. Pharm. Bull. 34, 682 (1986)).

Aus den Verbindungen der Formel 12b werden dann durch Umwandlung der Carboxylatgruppe die entsprechenden Ester bzw. Säurehalogenide hergestellt, die in Analogie zu den Verbindungen der Formel 11 bzw. 12 entsprechend den in Schema 1 und 2 aufgezeigten Wegen in die Endprodukte der Formel 1 bzw. 1b überführt werden.

5-Chinolincarbonsäurederivate der Formel 12 c werden aus 3-Aminobenzoesäuren und $\beta$-Alkoxyacrylsäurehalogeniden nach folgendem Schema 8 erhalten:

Schema 8

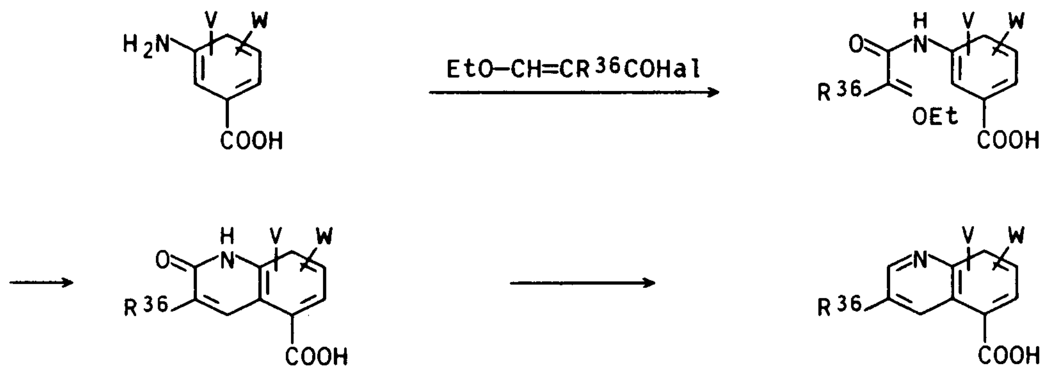


12c

Die Überführung der 2-Chinolone in die Chinoline erfolgt analog zu bekannten Methoden (vgl. z.B. J. Heterocyclic Chem. 24, 351 (1987)).

Weitere Chinolin-Zwischenprodukte können analog zu den in Chem. Heterocyclic Comp. Vol. 32, Chap. 2 beschriebenen Verfahren hergestellt werden.

Chinazoline sind aus o-Amido-benzaldehyden durch Kondensation mit Ammoniak im Sinne einer Bischler-Synthese erhältlich (vgl. z.B. J. Chem. Soc., 1965, 5360).

Cinnoline sind aus 2-Aminoacetophenonen durch Diazotierung und Cyclisierung zu 4-Hydroxycinnolinen im Sinne einer Borsche-Synthese (vgl. z.B. Liebigs Ann. Chem. 546, 293 (1941)) und anschließende Reduktion zugänglich (vgl. z.B. J. Chem. Soc., 1959, 2858).

Chinoxaline können aus o-Phenylendiaminen durch Umsetzung mit $\alpha$-Bromketonen und Oxidation zum aromatischen System erhalten werden (vgl. z.B. J. Chem. Eng. Data, 18, 102 (1973)).

1,2,4-Benzotriazine werden aus o-Nitrophenylhydraziden von Carbonsäuren nach bekannten Verfahren hergestellt (vgl. z.B. Chem. Ber. 22, 2801 (1889)).

3,5-Disubstituierte Isochinoline sind bekannt oder können nach bekannten Verfahren hergestellt werden (vgl. z.B. J. Org. Chem. 23, 435 (1958)).

2,4- und 2,7-disubstituierte Benzoxazole werden nach literaturbekannten Verfahren aus o-Aminophenolen durch Umsetzung mit einem Carbonsäurederivat, z.B. einem Carbonsäurechlorid, erhalten (vgl. z.B. J.med.Chem. 20, 169 (1977)). Die als Vorprodukte einsetzbaren o-Aminophenole sind bekannt oder lassen sich nach bekannten Methoden herstellen (vgl. z.B. Biochem. Prep., 6, 20 (1958)).

2,4-disubstituierte Indolderivate lassen sich aus 2-Halogenanilinen, beispielsweise 2-Bromanilinen, und Enaminen in Gegenwart eines Übergangsmetall-Katalysators, wie z.B. einer Pd-Verbindung, herstellen (vgl. z.B. J. Heterocycl. Chem. 24, 1555 (1987)).

2,4-disubstituierte Benzofuranderivate erhält man nach literaturbekannten Verfahren aus 2-Vinylfuranen und Acetylenderivaten beispielsweise Methylpropiolat, durch Cycloaddition und anschließende Oxidation (vgl. z.B. Aust. J. Chem. 26, 1059 (1973)).

2,4- und 2,7-disubstituierte Benzimidazole sind bekannt oder lassen sich in bekannter Weise aus o-Phenylendiaminen und Carbonsäurederivaten oder Carbonsäuren in Gegenwart eines Kondensationsmittels, beispielsweise Phosphoniumanhydrid, herstellen (vgl. z.B. J. Org. Chem. 54, 1144 (1989)). 2,4-disubstituierte Benzothiazole sind bekannt oder lassen sich nach bekannten Verfahren aus 2-Halogenthioaniliden herstellen (vgl. z.B. Synthesis 1976, 730).

In den folgenden Schemata 9 bis 21 wird der synthetische Aufbau der verschiedenen Seitenketten Z im einzelnen erläutert.

Verbindungen der Formel 33 lassen sich beispielsweise nach den in Schema 9 angegebenen Synthesewegen herstellen.

Schema 9

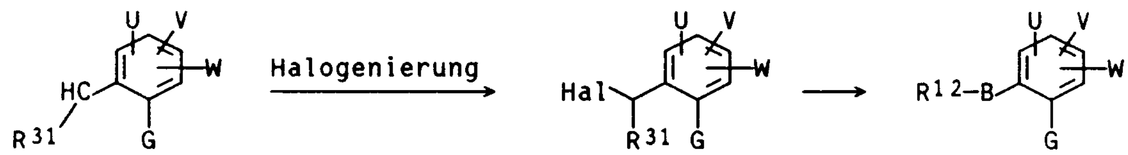


31 32 33

Hal: Cl, Br, I

B: $-O-CHR^{31}-$, $-S-CHR^{31}-$,

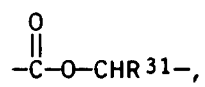

$-R^{30}C=N-O-CHR^{31}-$, $-R^{17}N-CHR^{31}-$

G:

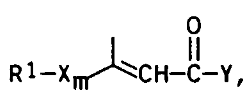

$-COOR^4$, $-COR^1$, -CHO, Hal, H

$R^1$, $R^4$, $R^{12}$, $R^{17}$, $R^{30}$, $R^{31}$, U, V, W, X, Y, m wie oben definiert.

Die Benzylhalogenide der Formel 32, bei denen $R^{31}$ bevorzugt Wasserstoff bedeutet, lassen sich nach an sich bekannten Methoden dadurch herstellen, daß man die Verbindungen 31, bei denen $R^{31}$ ebenfalls bevorzugt Wasserstoff bedeutet, mit einem Halogenierungsmittel wie zum Beispiel Chlor, Brom, Jod, N-Bromsuccinimid oder N-Chlorsuccinimid in einem inerten Lösungsmittel wie z.B. Tetrachlorkohlenstoff, Methylenchlorid oder Cyclohexan, gegebenenfalls unter Belichtung mit einer Lichtquelle (z.B. Hg-Dampflampe, 300 W) oder unter Zusatz von Radikalstartern wie z.B. Dibenzoylperoxid oder Azoisobutyronitril, umsetzt [siehe Angew. Chem. 71, 349 (1959)].

Die Verbindungen 32 mit Hal = Iodid lassen sich auch nach literaturbekannten Methoden aus den Chloriden oder Bromiden durch Umsetzung mit z.B. Natriumiodid in Aceton herstellen [s. J. Chem. Soc., Perkin Trans I, 416 (1976)].

Die Benzylhalogenide 32, bei denen $R^{31}$ bevorzugt Wasserstoff bedeutet, können nach Standardverfahren mit Nucleophilen, bevorzugt N-, O- oder S-Nucleophilen wie z.B. Carbonsäuren, Phenolen, Alkoholen, Mercaptanen, Aminen und Oximen zu den erfindungsgemäßen Verbindungen 33 umgesetzt werden, bei denen $R^{31}$ wiederum bevorzugt für Wasserstoff steht, indem man beispielsweise Verbindungen der Formel 32 mit den obengenannten Nucleophilen selbst oder den davon abgeleiteten Alkali-, Erdalkali-, Silber- oder Ammoniumsalzen in einem Lösungsmittel wie Aceton, Acetonitril, Toluol, DMF oder THF, gegebenenfalls unter Zusatz eines Katalysators z.B. 0,01 bis 10 Gew.-% Kaliumiodid zur Reaktion bringt [siehe Organikum, 17. Auflage, S. 172ff, VEB Berlin (1988); Org. Synth., Coll. Vol. 5, S. 1031ff, J. Wiley & Sons (1973)].

Die Verbindungen der allgemeinen Formeln 35, 38, 39 und 40 lassen sich beispielsweise wie in Schema 10 beschrieben herstellen.

Schema 10

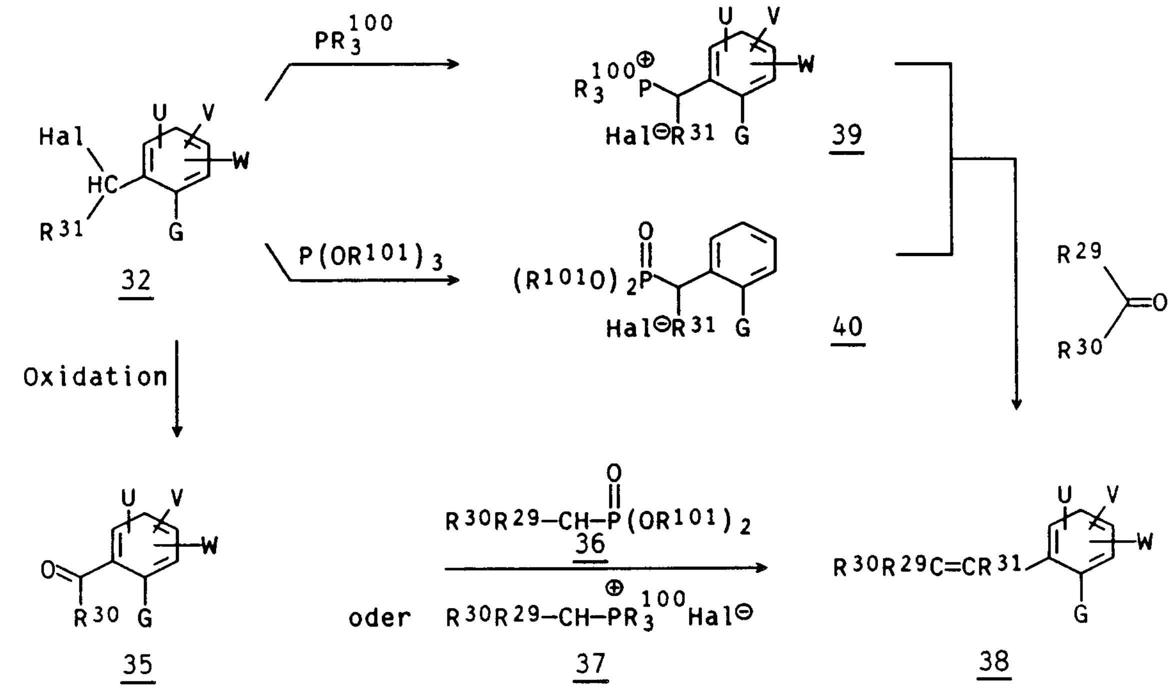


32, 39, 40, 35, 36, 37, 38

| | | |
|---|---|---|
| Hal | = | Cl, Br, I |
| $R^{100}$ | = | Phenyl |
| $R^{101}$ | = | $C_1$-$C_8$-Alkyl |

G, $R^{29}$, $R^{30}$, $R^{31}$, U, V, W wie oben definiert.

Die Benzylhalogenide der Formel 32, bei denen $R^{31}$ bevorzugt Wasserstoff bedeutet, können mit Oxidationsmitteln wie z.B. N-Methylmorpholin-N-oxidmonohydrat oder Dimethylsulfoxid in bekannter Weise zu den Oxoverbindungen 35 umgesetzt werden, wobei $R^{31}$ wiederum bevorzugt für Wasserstoff steht [siehe J. Org. Chem. 24, 1792 (1959)].

Zur Darstellung der Olefine 38 können die Oxoverbindungen 35 beispielsweise mit einem Phosphonsäureester der Formel 36 oder einem Phosphoniumsalz der Formel 37 umgesetzt werden. Die Durchführung der Reaktion erfolgt in bekannter Weise [siehe J.Am.Chem.Soc. 83, 1733 (1961); Angew.Chem. 71, 260ff (1959)].

Die Ausgangsstoffe werden üblicherweise in einem stöchiometrischen Verhältnis eingesetzt. Ein Überschuß bis zu 10 % (Gew.-%) an einem der beiden Reaktionsteilnehmer über die stöchiometrische Menge hinaus ist möglich. Die Umsetzung wird in einem inerten Lösungs- oder Verdünnungsmittel (z.B. Hexan, Toluol, Methylenchlorid, Dimethylsulfoxid, besonders bevorzugt Tetrahydrofuran, Dimethylformamid oder Diethylether; es können auch Gemische der genannten Lösungsmittel verwendet werden) in Gegenwart einer äquivalenten Menge einer Base (z.B. Natriumhydrid, Natriumamid, Kalium-tert.-butylat, Natriummethylat, Butyllithium, Lithiumdiisopropylamid, Kaliumhydroxid) durchgeführt.

Die Umsetzungen verlaufen gewöhnlich in einem Temperaturbereich von - 70 °C bis + 80 °C, vorzugsweise -70 °C bis + 60 °C.

Beide Isomere, E und Z, werden von der Erfindung erfaßt, insbesondere aber die E-Isomeren.

Die Phosphonate 36 bzw. Phosphoniumsalze 37 sind leicht zugänglich aus den Halogeniden $R^{30}R^{29}$-CH-Hal (Hal = Cl, Br, I) [siehe Houben-Weyl, 4. Aufl., Bd. XII/1, S. 79ff und S. 433ff (1963)].

Die Olefine 38 können alternativ auch durch Umsetzung der Carbonylverbindungen 41 mit den Phosphoniumsalzen 39 bzw. den Phosphonsäureestern 40 erhalten werden [siehe J. Am. Chem. Soc. 83, 1733 (1961); Angew. Chem. 71, 260ff (1959)].

Die Phosphoniumsalze 39 bzw. die Phosphonsäureester 40 erhält man beispielsweise durch Umsetzung der Halogenide 32 mit einem Phosphin der Formel $P(R^{100})_3$ bzw. einem Phosphit der Formel $P(OR^{101})_3$. Die Durchführung der Reaktion erfolgt in bekannter Weise [siehe Houben-Weyl, 4. Aufl., Bd. XII/1,

S. 79ff und S. 433ff (1963)].

Die Phosphonate der Formel 40 mit $R^{31}$ ≠ Wasserstoff können zum Beispiel auch durch Umsetzung der Phosphonate 40 mit $R^{31}=H$ mit Alkylhalogeniden $R^{31}$-Hal (Hal = Cl, Br, I) unter Verwendung einer Base hergestellt werden [siehe J. Am. Chem. Soc. 83, 1733 (1961)].

Die Hydrierung der Styrolderivate 38 mit Diimin oder auch mit $H_2$ in Gegenwart geeigneter Hydrierkatalysatoren wie z.B. Pd oder Pt in geeigneten organischen Lösungmitteln wie Tetrahydrofuran, Methanol oder Essigsäure liefert in an sich bekannter Weise die alkylierten Aromaten 42 [siehe Organikum, 17. Aufl., S. 288ff, VEB Berlin (1988)] (Schema 11).

Die Verbindungen der allgemeinen Formeln 43 bis 47 lassen sich beispielsweise wie in Schema 12 beschrieben darstellen.

Schema 11

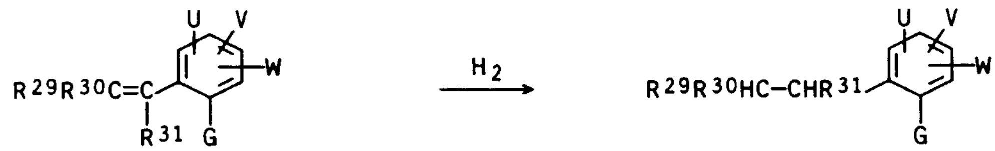


38 42

G, $R^{29}$, $R^{30}$, $R^{31}$, U, V, W wie oben definiert

Schema 12

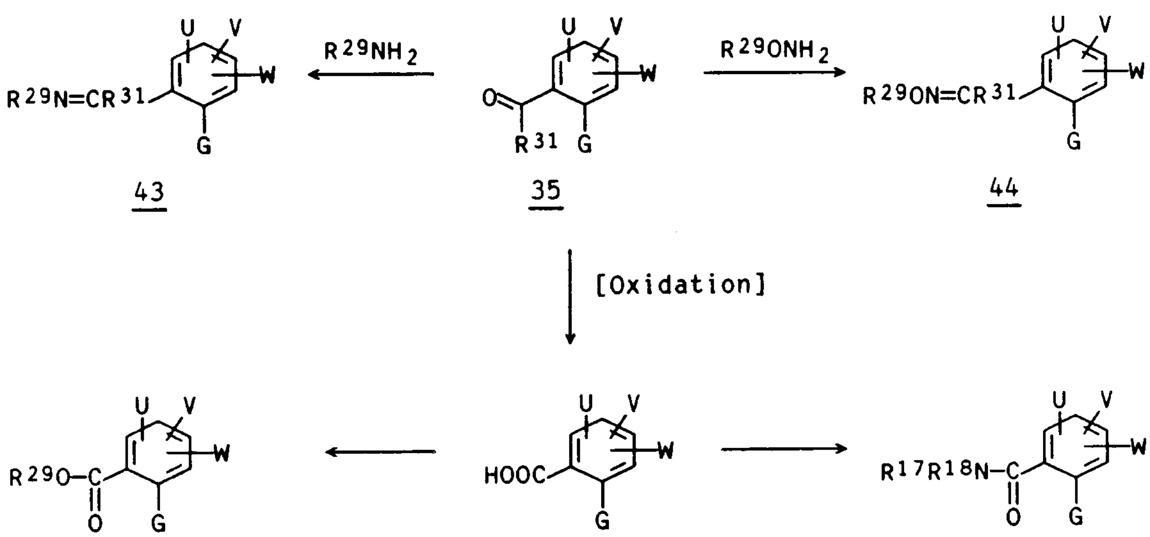


43 35 44

46 45 47

G, $R^{17}$, $R^{18}$, $R^{29}$, $R^{31}$, U, V, W wie oben definiert.

Die Umsetzung der Oxoverbindungen 35 mit primären Aminen $R^{29}$-$NH_2$ zu den Schiff'schen Basen 43 bzw. mit O-substituierten Hydroxylaminen $R^{29}O$-$NH_2$ zu den Oximethern 44 kann in an sich bekannter Weise in einem inerten Lösungs- oder Verdünnungsmittel wie z.B. Methanol, Ethanol, Toluol oder in einem Zweiphasensystem wie z.B. Toluol/Wasser durchgeführt werden. Gegebenenfalls erfolgt die Reaktion unter Zusatz einer geeigneten Base wie z.B. Triethylamin, Natriumcarbonat, Kaliumcarbonat, Kaliumhydroxid oder Pyridin [siehe Houben-Weyl, 4. Aufl., Bd. XI/2, S. 73ff (1958); Houben-Weyl, 4.Aufl., Bd. X/4, S. 55ff (1968)]. Beide Isomeren, E und Z, werden von der Erfindung erfaßt, insbesondere aber die E-Isomeren.

Durch die Oxidation der Aldehyde ($R^{31}=H$) 35 nach bekannten Verfahren können beispielsweise die Benzoesäuren 45 dargestellt werden [siehe J. March, Advanced Organic Chemistry, 3. Aufl., S. 629ff, Wiley-Interscience Publication (1985)].

Nach Standardmethoden der organischen Chemie sind daraus weitere Carbonsäurederivate wie z.B. die Ester 46 und Amide 47 erhältlich [siehe Organikum, 17. Aufl., S. 400ff, VEB Berlin (1988)].

Die Schemata 13 bis 15 illustrieren die Synthese von Verbindungen der Formel 1, in denen die Seitenkette Z für die Gruppen OH, SH und $NH_2$ steht (vgl. Formeln 49, 50 und 51). Diese Verbindungen sind als Zwischenprodukte für die Synthese anderer Verbindungen der Formel 1 (z.B. nach Schema 17) von besonderem Interesse und auch insofern Gegenstand der vorliegenden Erfindung.

Schema 13

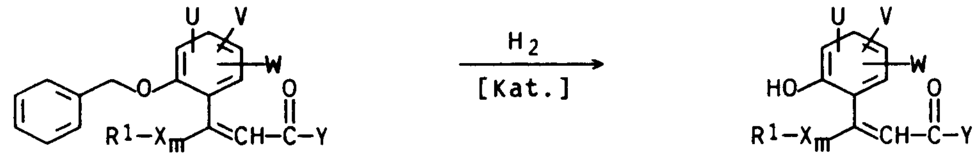


48 → 49

1 (Z=OH)

$R^1$, U, V, W, $X_m$, Y wie oben definiert.

Die ortho-substituierten Phenole 49 lassen sich - wie in Schema 13 beschrieben - in an sich bekannter Weise aus den Benzylethern 48 durch katalytische Hydrierung erhalten (vgl. z.B. Prot. Groups in org. Synth., S. 19, 1981). Die Benzylether 48 ihrerseits lassen sich z.B. auf dem in Schema 1 beschriebenen Weg aus den entsprechenden Acetophenonderivaten 10 (mit $Z = -O-CH_2Ph$) darstellen.

Die Thiophenole 50 lassen sich aus den Phenolen 49 z.B. durch Umsetzung mit Dimethylthiocarbamidsäurechlorid nach bekannten Methoden (vgl. Chem. Ber., 106, 24/9, 1973) herstellen (Schema 14).

Schema 14

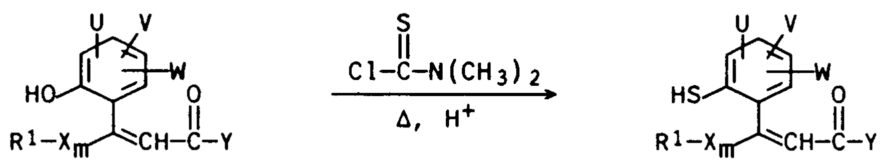


49 → 50

1 (Z=SH)

$R^1$, U, V, W, $X_m$, Y wie oben definiert.

Die Aniline 51 lassen sich z.B. durch katalytische Reduktion aus den entsprechenden Nitroaromaten 52 darstellen (vgl. z.B. Organikum 16. Aufl., S. 534f., 1986) (Schema 15).

Schema 15

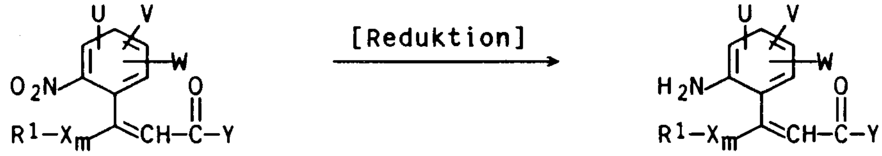


52 → 51

1 ($Z=NH_2$)

$R^1$, U, V, W, $X_m$, Y wie oben definiert.

Die neuen Verbindungen der allgemeinen Formel 54, in der Z $OR^{12}$, $SR^{13}$ bzw. $NR^{37}R^{38}$ bedeutet, lassen sich gemäß Schema 16 darstellen:

Schema 16

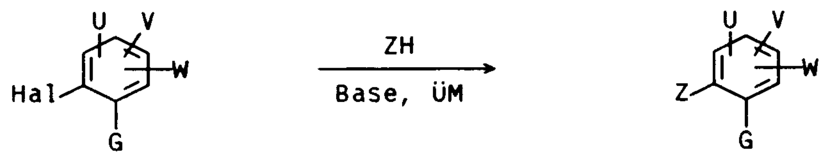


53 54

Hal: Brom, Chlor
Z: $OR^{12}$, $SR^{13}$, $NR^{37}R^{38}$
G:

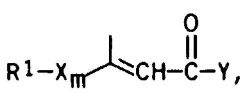

$CO_2R^4$, $COR^1$, CHO, H, Hal
$R^1$, $R^4$, $R^{12}$, $R^{13}$, $R^{37}$, $R^{38}$, X, Y, U, V, W und m wie oben definiert.

Die ortho-Halogenverbindungen 53, in denen Hal vorzugsweise Brom bedeutet, lassen sich in Gegenwart einer Base, wie z.B. $K_2CO_3$, NaH oder Kalium-t.-butylat, gegebenenfalls in Gegenwart eines Übergangsmetallkatalysators (ÜM), wie Kupferpulver oder Kupfer(I)chlorid, mit einem Alkohol ($R^{12}OH$), einem Mercaptan ($R^{13}SH$) oder einem Amin ($R^{37}R^{38}NH$), gegebenenfalls in Gegenwart eines Lösungs- oder Verdünnungsmittels, wie z.B. Aceton, DMF in an sich bekannter Weise , z.B. im Sinne einer Ullmann-Reaktion (vgl. z.B. Russ. Chem. Rev. 43, 679, 1974; J. Org. Chem. 29, 977, 1964) zu den neuen Verbindungen der allgemeinen Formel 54 umsetzen, in der Z für $OR^{12}$, $SR^{13}$ bzw. für $NR^{37}R^{38}$ steht.

Alternativ hierzu kann man zur Synthese von Verbindungen der Formel 54 auch gemäß Schema 17 verfahren:

Schema 17

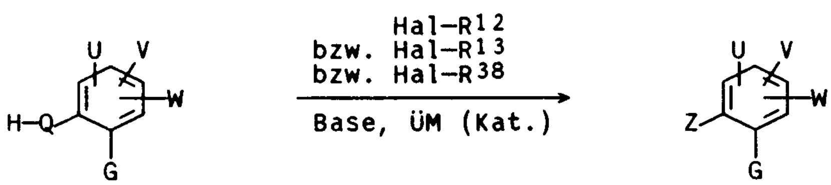


55 54

Q: O, S, $NR^{37}$
Hal: F, Cl, Br, J
G, Z, $R^{12}$, $R^{13}$, $R^{37}$, $R^{38}$, U, V, W wie in Schema 16 definiert.

Hiernach lassen sich die neuen Verbindungen der allgemeinen Formel 54 darstellen, indem man die Verbindungen der allgemeinen Formel 55 in Gegenwart einer Base, gegebenenfalls in Gegenwart eines Übergangsmetall-Katalysators [ÜM (Kat.)] mit einer Halogenverbindung, $R^{12}$-Hal, $R^{13}$-Hal bzw. $R^{38}$-Hal, vorzugsweise einer entsprechenden Bromverbindung, gegebenenfalls in Gegenwart eines Lösungs- oder Verdünnungsmittels, wie z.B. Aceton oder DMF, in an sich bekannter Weise , z.B. im Sinne einer Ullmann-Reaktion (vgl. z.B. Russ. Chem. Rev. 43, 679, 1974; J. Org. Chem. 29, 977, 1964) bzw. im Sinne einer nucleophilen Substitutionsreaktion (vgl. z.B. J. Chem. Soc., 381, 1942; J. Heterocycl. Chem. 15, 1513, 1978) umsetzt.

Die neuen Verbindungen der allgemeinen Formel 1, in der Z- eine acetylenische Seitenkette $R^{29}$-C≡C- bedeutet (siehe Formel 56), lassen sich z.B. gemäß Schema 18 darstellen.

Schema 18

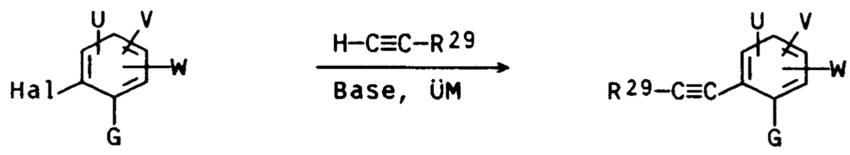


53 56

Hal: F, Cl, Br, J

G, U, V, W, $R^{29}$ wie in Schema 16 definiert.

Die ortho-Halogen-Verbindungen 53, vorzugsweise die o-Brom-Verbindungen 53 (Hal = Br), lassen sich in an sich bekannter Weise im Sinne einer Heck-Reaktion (vgl. z.B. J. Organomet. Chem. 93, 259, 1975) mit den terminalen Acetylenen, H-C≡C-$R^{29}$, gegebenenfalls in Gegenwart einer Base, wie z.B. $K_2CO_3$, NaH, $NHEt_2$ oder $NEt_3$, gegebenfalls in Gegenwart eines Lösungs- oder Verdünnungsmittels, wie z.B. DMF, Acetonitril, THF oder Toluol, in Gegenwart eines Übergangsmetallkatalysators, ÜM, beispielsweise einer Palladium- oder Nickelverbindung, wie z.B. $Pd(OAc)_2$, $NiCl_2$, $PdCl_2$ oder $Pd(PPh_3)_4$ zu den Acetylenen 56 umsetzen.

Neue Verbindungen der allgemeinen Formel 1, in der Z eine Heteroarylgruppe bedeutet (siehe Formel 58), lassen sich z.B. gemäß Schema 19 darstellen.

Schema 19

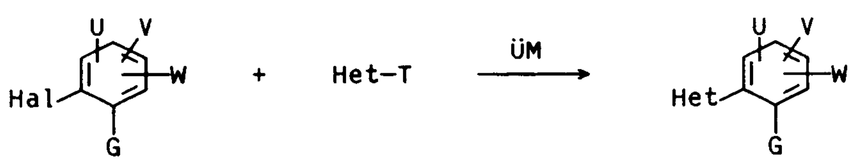


53 57 58

H: F, Cl, Br, J

T: $Sn(C_1\text{-}C_4\text{-Alkyl})_3$, $B(O\text{-}H)_2$

Het: Heteroaryl wie oben definiert

G, U, V, W: wie in Schema 16 definiert.

Danach lassen sich die ortho-Halogen-Verbindungen, vorzugsweise die ortho-Brom-Verbindungen 53 (Hal = Br) in an sich bekannter Weise mit den Heteroarylstannanen 57 (T = $Sn(C_1\text{-}C_4\text{-Alkyl})_3$) bzw. mit den Heteroaryl-Borverbindungen 57 (T = $B(O\text{-}H)_2$) in Gegenwart eines Übergangsmetall-Katalysators ÜM, wie z.B. $PdCl_2$, $Pd(OAc)_2$, $NiCl_2$, $Pd(PPh_3)_4$ zu den neuen Verbindungen der allgemeinen Formel 58 umsetzen (vgl. z.B. Tetrahedron Lett. 27, 4407, 1986; Synth. Commun. 19, 101, 1989).

Weiterhin lassen sich Verbindungen der Formel 1, in denen Z eine Fünfringheteroaromatische Gruppe bedeutet, in vielen Fällen dadurch synthetisieren, daß man Acetylene der Formel 56 als Dipolarophile in 1,3-dipolaren Cycloadditionen einsetzt [vgl. z.B. R. Huisgen, Angew. Chem. Intern. Ed., 2, 565, 633 (1963)].

Verbindungen der Formel 1, in der Z eine substituierte Iminogruppe bedeutet (entsprechend Formel 59), lassen sich gemäß Schema 20 herstellen.

Schema 20

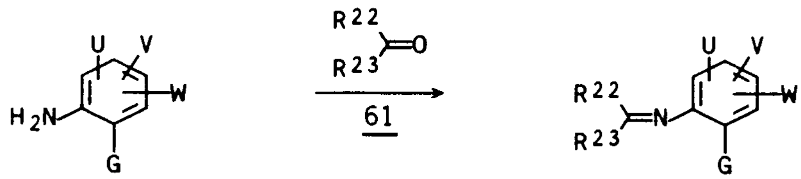


60 59

$R^{22}$, $R^{23}$ wie oben definiert
G, U, V, W wie in Schema 16 definiert.

Die Imine der allgemeinen Formel 59 lassen sich in an sich bekannter Weise [vgl. z.B. Houben-Weyl, Bd. 7/1, 453; Houben-Weyl, Bd. 11/2, 74] aus den Anilinderivaten 60 darstellen, indem man diese mit den Carbonylverbindungen 61, gegebenenfalls in Gegenwart einer Säure (z.B. HCl, $H_2SO_4$) oder in Gegenwart einer Base (z.B. $K_2CO_3$, $NEt_3$) umsetzt, bevorzugt unter gleichzeitiger Entfernung des entstehenden Reaktionswassers mittels Molekularsieb oder azeotroper Destillation.

Neue Verbindungen der allgemeinen Formel 63 und 64 lassen sich gemäß Schema 21 darstellen.

Schema 21

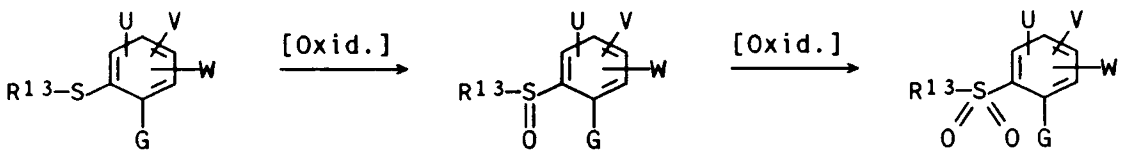


62 63 64

$R^{13}$, G, U, V, W wie in Schema 16 definiert.

Die Thioverbindungen 62 lassen sich in an sich bekannter Weise (vgl. z.B. Houben Weyl, Bd. 9, 219f) durch ein Oxidationsmittel, wie z.B. Sauerstoff, Wasserstoffperoxid oder m-Chlorperbenzoesäure, gegebenenfalls in Gegenwart eines Lösungs- oder Verdünnungsmittel, wie z.B. Toluol, in die Sulfoxide 63 bzw. in die Sulfone 64 umwandeln, die auf diese Weise selektiv erhalten werden oder als Gemisch anfallen, aus dem die Einzelkomponenten nach bekannten Verfahren, wie z.B. Destillation oder Chromatographie, isoliert werden können.

Die vorstehend beschriebenen Verfahren zum synthetischen Aufbau der Seitenketten Z können auch zur Herstellung von weiteren Verbindungen der Gruppe 1b aus gemäß Schema 6-8 synthetisierten Zwischenprodukten mit anelliertem Ringsystem verwendet werden.

Die Herstellung der erfindungsgemäßen Verbindungen wird durch folgende Herstellungsbeispiele erläutert:

Beispiel 1

Herstellung von (E)-β-Methoxy-2-methylzimtsäuremethylester (Verbindung Nr. 1.1 der Tabelle 1).

a) Zu einer Suspension von 48 g (2,0 mol) NaH und 180 g (2,0 mol) Dimethylcarbonat in 1,5 l Tetrahydrofuran wird bei 60 °C unter Stickstoffatmosphäre eine Lösung von 200 g (1,5 mol) 2-Methylacetophenon in 500 ml Tetrahydrofuran langsam zugetropft. Anschließend wird noch 2 h bei 60 °C nachgerührt.

Nach dem Abkühlen wird mit 100 ml Methanol versetzt, die Mischung auf Eiswasser gegeben und mit 10 %iger Salzsäure angesäuert. Nach dem Extrahieren mit Methyl-tert.-butylether werden die vereinigten organischen Phasen mit $NaHCO_3$-Lösung und Wasser gewaschen, über $Na_2SO_4$ getrocknet und eingeengt. Als Rückstand verbleiben 271 g (94 %) 2-Methylbenzoyl-essigsäure-methylester als rotbraunes Öl.

$^1H$-NMR ($CDCl_3$): Ketoform: $\delta$ = 2,56 (s, 3H); 3,73 (s, 3H); 3,97 (s, 2H); 7,13 - 7,63 (m, 4H); ppm. Gleichzeitig treten in untergeordnetem Maße (ca. 30 %) die Signale der zugehörigen Enolform auf: 2,45 (s, 3H); 3,77 (s, 3H); 5,28 (s, 1H); 7,13 - 7,63 (m, 4H); 12,45 (s, 1H) ppm.

b) Eine Mischung von 1280 g (6,7 mol) 2-Methylbenzoylessigsäuremethylester, 848 g (8,0 mol) Orthoameisensäuretrimethylester, 400 ml Methanol und 10 ml konz. $H_2SO_4$ wird 12 h bei Raumtemperatur gerührt. Anschließend wird mit 20 ml Pyridin neutralisiert, die Mischung eingeengt und der Rückstand fraktioniert destilliert (Kp. = 120-123 °C/0,6 Torr). Man erhält 1300 g (94 %) (E)-$\beta$-Methoxy-2-methylzimtsäuremethylester als hellgelbes Öl.

$^1H$-NMR ($CDCl_3$): $\delta$ = 2,23 (s, 3H); 3,52 (s, 3H); 3,76 (s, 3H); 5,34 (s, 1H); 7,13 - 7,31 (m, 4H) ppm.

Beispiel 2

Herstellung von (E)-$\beta$-Methoxy-2-brommethylzimtsäuremethylester (Verbindung Nr. 1.4 der Tabelle 1)

200 g (0,97 mol) (E)-$\beta$-Methoxy-2-methylzimtsäuremethylester und 190 g (1,07 mol) mit Methanol gewaschenes, trockenes N-Brom-succinimid in 2 l trockenem Tetrachlorkohlenstoff werden 75 min. mit einer 300 Watt UV-Lampe bestrahlt, wobei sich die Lösung auf Rückflußtemperatur erwärmt.

Anschließend wird das ausgefallene Succinimid abgesaugt und die organische Phase mit $NaHCO_3$-Lösung und Wasser gewaschen. Nach dem Trocknen über $Na_2SO_4$ wird das Lösungsmittel abdestilliert und der Rückstand aus Methyl-tert.-butylether/n-Hexan kristallisiert. Man erhält so 152 g (55 %) (E)-$\beta$-Methoxy-2-brommethylzimtsäuremethylester als farblose Kristalle mit einem Schmelzpunkt von 55 - 57 °C.

$^1H$-NMR ($CDCl_3$): $\delta$ = 3,54 (s, 3H); 3,82 (s, 3H); 4,44 (s, 2H); 5,41 (s, 1H); 7,16 - 7,47 (m, 4H) ppm.

Beispiel 3

Herstellung von (E)-2-Formyl-$\beta$-methoxyzimtsäuremethylester (Verbindung Nr. 1.5 der Tabelle 1).

100 g (0,35 mol) (E)-$\beta$-Methoxy-2-brommethylzimtsäuremethylester (s. Beispiel 2) und 142 g (1,05 mol) N-Methylmorpholin-N-oxidmonohydrat in 300 ml Dimethylsulfoxid werden 1 h bei 5 °C gerührt.

Man versetzt mit 400 ml gesättigter $NH_4Cl$-Lösung und extrahiert mit Essigsäureethylester. Die vereinigten organischen Phasen werden mit Wasser gewaschen, über $Na_2SO_4$ getrocknet und eingeengt. Man nimmt den Rückstand in Methyl-tert.-butylether/n-Hexan = 2:1 auf, filtriert über Kieselgel und engt ein.

Nach Behandeln des Rückstandes mit Methyl-tert.-butylether/n-Hexan erhält man 52 g (68 %) (E)-2-Formyl-$\beta$-methoxyzimtsäuremethylester als farblose Kristalle mit einem Schmelzpunkt von 56 - 58 °C.

$^1H$-NMR ($CDCl_3$): $\delta$ = 3,52 (s, 3H); 3,86 (s, 3H); 5,49 (s, 1H); 7,33 - 7,97 (m, 4H); 10,03 (s, 1H) ppm.

Beispiel 4

Herstellung von (E)- und (Z)-{2-2-[2-(4-Fluorphenyl)-thiazol-4-yl]--ethenyl}-(E)-$\beta$-methoxyzimtsäuremethylester (Verbindungen Nr. 1a.394 und Nr. 1a.393 der Tabelle 1a)

a) 20 g (88 mol) 4-Chlormethyl-2-(4-fluorphenyl)-thiazol und 23 g (88 mmol) Triphenylphosphin in 250 ml Tetrahydrofuran werden 24 h unter Rückfluß erhitzt. Nach Absaugen und Waschen mit Tetrahydrofuran erhält man 21 g (49 %) beigefarbene Kristalle (Fp.: > 220 °C) von 2-(4-Fluorphenyl)-thiazol-4-ylmethyl-triphenylphosphoniumchlorid.

$^1H$-NMR ($CDCl_3$): $\delta$ = 5,77 (d, 2H); 6,98 - 8,22 (m, 20H) ppm.

b) Zu einer Suspension von 0,65 g (27 mmol) NaH in 100 ml Tetrahydrofuran gibt man bei 60 °C eine Suspension von 10 g (20 mmol) 2-(4-Fluorphenyl)-thiazol-4-ylmethyl-triphenyl-phosphoniumchlorid und 4,0 g (18 mmol) (E)-2-Formyl-$\beta$-methoxyzimtsäuremethylester (s. Beispiel 3) in 50 ml Tetrahydrofuran und läßt 45 min unter Rückfluß rühren.

Nach dem Abkühlen wird mit $NH_4Cl$-Lösung versetzt und die organische Phase abgetrennt. Die wäßrige Phase wird nochmals mit Methyl-tert.-butylether extrahiert und die vereinigten organischen Phasen mit Wasser gewaschen, über $Na_2SO_4$ getrocknet und eingeengt. Der Rückstand wird über eine Kieselgel-Säule (Methyl-tert.-butylether/n-Hexan = 1:5) chromatographiert. Man erhält so 0,8 g (11 %) 2-{2-[2-(4-Fluorphenyl)-thiazol-4-yl]-(Z)-ethenyl}-E)-$\beta$-methoxyzimtsäuremethylester als gelbes Öl, das zuerst eluiert wird sowie 2,3 g (33 %) 2-{2-[2-(4-Flourphenyl)-thiazol-4-yl]-(E)-ethenyl-(E)-$\beta$-methoxyzimtsäuremethylester, das als zweites Produkt eluiert wird und in Form von beigefarbenen Kristallen anfällt.

(Z), (E)-Isomer: $^1$H-NMR ($CDCl_3$): δ = 3,50 (s; 3H); 3,74 (s, 3H); 5,39 (s, 1H); 6,67 und 6,74 (AB, 2H); 6,89 -7,94 (m, 9H) ppm.
(E), (E)-Isomer: Fp.: 148 - 150 °C
$^1$H-HMR ($CDCl_3$): δ = 3,51 (s; 3H); 3,86 (s, 3H); 5,48 (s, 1H); 7,04 - 7,99 (m; 11H) ppm.

Beispiel 5

Herstellung von (E)- und (Z)-2-[2-(4-Phenylphenyl)-ethenyl]-(E)-β-methoxyzimtsäuremethylester (Verbindungen Nr. 1a.268 und Nr. 1a.269 der Tabelle 1a).

a) 115 g (0,4 mol) (E)-2-Brommethyl-β-methoxy-zimtsäuremethylester und 105 g (0,4 mol) Triphenylphosphin in 700 ml Tetrahydrofuran werden 24 h unter Rückfluß erhitzt. Nach Absaugen und Waschen mit Tetrahydrofuran erhält man 181 g (82 % Ausbeute) (E)-2-(1-Methoxy-2-methoxycarbonylethenyl)-benzyl-triphenylphosphoniumbromid in Form farbloser Kristalle mit einem Schmelzpunkt von 189 - 192 °C.
$^1$H-NMR ($CDCl_3$): δ = 3,45 (s, 3H); 3,57 (s, 3H); 4,95 (d, 2H); 5,06 (s, 1H); 6,96 - 7,84 (m; 19H) ppm.
b) Zu einer Lösung von 2,1 g (19,6 mmol) Lithiumdiisopropylamid in 100 ml Tetrahydrofuran gibt man bei 0 °C eine Suspension von 3,3 g (18 mmol) 4-Phenylbenzaldehyd und 10 g (18 mmol) E-2-(1-Methoxy-2-methoxycarbonylethenyl)-benzyltriphenyl-phosphoniumbromid in 150 ml Tetrahydrofuran. Nach 3 h Rühren bei Raumtemperatur wird mit $NH_4Cl$-Lösung versetzt und die organische Phase abgetrennt. Die wäßrige Phase wird nochmals mit Methyl-tert.-butylether extrahiert und die vereinigten organischen Phasen mit Wasser gewaschen, über $Na_2SO_4$ getrocknet und eingeengt. Der Rückstand wird über eine Kieselgel-Säule (Methyl-tert.-butylether/n-Hexan = 1:5) chromatographiert. Man erhält so 0,6 g (9 %) 2-[2-(4-Phenylphenyl)-(Z)-ethenyl]-(E)-β-methoxyzimtsäuremethylester als gelbes Öl, das zuerst eluiert wird sowie 1,1 g (16 %) 2-[2-(4-Phenyl-phenyl)-(E)-ethenyl]-(E)-β-methoxyzimtsäuremethylester, der als zweites Produkt eluiert wird und in Form von gelben Kristallen anfällt.
(Z), (E)-Isomer : $^1$H-NMR ($CDCl_3$): δ = 3,52 (s, 3H); 3,74 (s, 3H); 5,33 (s, 1H); 6,51 und 6,58 (AB, 2H); 7,13 - 7,65 (m, 13H) ppm.
(E), (E)-Isomer: Fp.: 110 - 112 °C
$^1$H-NMR ($CDCl_3$): δ = 3,50 (s, 3H); 3,83 (s, 3H); 5,44 (s, 1H); 7,10 - 7,74 (m; 15H) ppm.

Beispiel 6

Herstellung von 2-[2-(4-Benzyloxyphenyl)-(E)-ethenyl]-(E)-β-methoxyzimtsäuremethylester (Verbindung Nr. 1a.264 der Tabelle 1a).

a) 20 g (70 mmol) (E)-2-Brommethyl-β-methoxyzimtsäuremethylester und 50 ml Trimethylphosphit in 100 ml Dimethylformamid werden nach Zusatz von 0,1 g Kaliumiodid znächst 1 h bei 40 °C, dann 1 h bei 100 °C gerührt. Das Lösungsmittel bzw. überschüssiges Trimethylphosphit wird abdestilliert und der Rückstand in Dichlormethan aufgenommen. Nach Waschen mit Wasser und Trocknen über $Na_2SO_4$ wird eingeengt und der Rückstand säulenchromatographisch an Kieselgel (zuerst Methyl-tert.-butylether,dann Methanol) gereinigt. Man erhält so 12 g (55 %) (E)-2-(1-Methoxy-2-methoxycarbonylethenyl)-benzylphosphonsäuredimethylester als braunes Öl.
$^1$H-NMR ($CDCl_3$): δ = 3,18 (d, 2H); 3,53 (s, 3H); 3,61 (d, 6H); 3,79 (s, 3H); 5,38 (s, 1H); 7,18 - 7,50 (m, 4H) ppm.
b) Zu 0,26 g (11 mmol) NaH in 20 ml Dimethylformamid wird eine Lösung von 3,0 g (10 mmol) (E)-2-(1-Methoxy-2-methoxy-carbonylethenyl)-benzylphosphonsäuredimethy lester und 2,1 g (10 mmol) 4-Benzyloxybenzaldehyd in 50 ml Dimethylformamid bei Raumtemperatur zugetropft. Es wird 3 h nachgerührt und mit $NH_4Cl$-Lösung versetzt. Nach dem Extrahieren mit Essigsäureethylester wird die organische Phase mit Wasser gewaschen, über $Na_2SO_4$ getrocknet und eingeengt. Nach Umkristallisation aus Diisopropylether/n-Hexan erhält man 1,9 g (48 %) hellgelbe Kristalle bestehend aus 2-[2-(4-Benzyloxyphenyl)-(E)-ethenyl]-(E)-β-methoxyzimtsäuremethylester vom Fp. 118 -120 °C.
$^1$H-NMR ($CDCl_3$): δ = 3,51 (s, 3H); 3,82 (s, 3H); 5,07 (s, 2H); 5,43 (s, 1H); 6,88 - 7,72 (m, 15H) ppm.

Beispiel 7

Herstellung von (E)-2-{2-[2-(4-Fluorphenyl)-thiazol-4-yl]-ethyl}-β-methoxyzimtsäuremethylester (Verbindung Nr. 1a.347 der Tabelle 1a).

3 g (7,6 mmol) 2-{2-[2-(4-Fluorphenyl)-thiazol-4-yl]-(Z)-ethenyl)-(E)-β-methoxyzimtsäuremethylester werden in 100 ml Methanol in Gegenwart von 0,2 g Pd/C (10 %) bei 0,05 bar Wasserstoffüberdruck und 20-25 °C hydriert, bis kein Wasserstoff mehr aufgenommen wird (1 h). Anschließend wird abfiltriert und eingeengt.

Nach Kristallisation aus Diisopropylether/n-Hexan erhält man 1,5 g (50 % (E)-2-{2-[2-(4-Fluorphenyl)-thiazol-4-yl]-ethyl}-β-methoxy-zimtsäuremethylester als orange Kristalle mit einem Schmelzpunkt von 157 - 159 °C.
$^1$H-NMR ($CDCl_3$): δ = 2,95 - 3,10 (m, 4H); 3,55 (s, 3H); 3,80 (s, 3H); 5,38 (s, 1H); 6,76 - 7,94 (m, 9H) ppm.

Beispiel 8

Herstellung von (E)-β-Methoxy-2-[2-methyl-4-(1-methoximinoeth-1-yl)-phenoxymethyl]-zimtsäuremethylester (Verbindung Nr. 1a.72 der Tabelle 1a).

a) Zur Lösung von 52,6 g (0,35 M) 2-Methyl-4-acetylphenol und 35,0 g (0,42 M) O-Methylhydroxylaminhydrochlorid in 150 ml Methanol gibt man 25 g trockene Molekularsiebperlen (3 Å läßt 24 Stdn. bei Raumtemperatur stehen. Nach Abfiltrieren des Molekularsiebes wird die Lösung eingeengt, der Rückstand in Dichlormethan aufgenommen, zweimal mit Wasser gewaschen, über $MgSO_4$ getrocknet und eingeengt. Man erhält 58,3 g (93 % Ausbeute) 2-Methyl-4-(1-methoximinoeth-1-yl)-phenol als weiße Festsubstanz vom Fp. 96 - 99 °C, die laut GC- und $^1$H-NMR-Kontrolle zu > 95 % einheitlich ist und deshalb offenbar aus dem thermodynamisch günstigeren Isomeren mit E-Konfiguration hinsichtlich der C = N- Doppelbindung besteht.
$^1$H-NMR ($CDCl_3$): δ = 2,19 (3H, s); 2,21 (3H, s); 3,96 (3H,s); 6,0 (O-H, s); 6,69 (1H, m); 7,30 (1H, m); 7,42 (1H, m) ppm.
b) Zu 12,5 g (0,07 M) 2-Methyl-4-(1-methoximinoeth-1-yl)-phenol in 50 ml Methanol gibt man 12,6 g einer 30 proz. Lösung von Natriummethanolat (0,07 M) in Methanol und engt die Lösung i. Vak. zur Trockene ein. Der Rückstand wird in 300 ml Dimethylformamid (DMF) gelöst und anschliessend mit 0,5 g Kaliumiodid sowie einer Lösung von 21,9 g (0,074 M) (E)-β-Methoxy-2-brommethylzimtsäuremethylester, (siehe Beispiel 2) in 100 ml DMF versetzt. Nach 8-stündigem Rühren bei Raumtemperatur wird die Mischung i. Vak. zur Trockene eingeengt, der Rückstand in Dichlormethan gelöst und die organische Phase zweimal mit Wasser extrahiert, getrocknet und eingeengt. Nach Anreiben mit Methanol erhält man 25,0 g (93 % Ausbeute) (E)-β-Methoxy-2-[2-methyl-4-(1-methoximinoeth-1-yl)-phenoxymethyl]-zimtsäuremethylester in Form fast farbloser Kristalle vom Fp. 58 - 60 °C. Nach GC- und $^1$H-NMR-Kontrolle ist das Produkt zu > 95 % einheitlich und besteht deshalb aus dem Isomeren mit E-Konfiguration hinsichtlich der C = N-Doppelbindung.
$^1$H-NMR ($CDCl_3$): δ = 2,17 (3H, s); 2,26 (3H, s); 3,55 (3H, s) 3,74 (3H, s); 3,94 (3H, s); 5,06 (2H, s); 5,38 (1H, s); 6,77 (1H, dd); 7,1 - 7,7 (6H, m) ppm.

Beispiel 9

Herstellung von(E)-β-Methoxy-2-[2-methyl-4-(1-methoximinoeth-1-yl)-phenoxymethyl]-zimtsäure (Verbindung Nr. 1a.73)

Die Mischung von 24,5 g (0,064 m) (E)-β-Methoxy-2-[2-methyl-4-(1-methoximinoeth-1-yl)-phenoxymethyl]-zimtsäuremethylester (siehe Beispiel 8), 4,6 g (0,07 M) 85 proz. Kaliumhydroxid, 10 ml Wasser und 90 ml Methanol wird 16 Stunden bei Rückflußtemperatur gerührt, sodann abgekühlt und in 1 l Eiswasser gegossen. Unter Rühren wird die zunächst klare Lösung mit wässriger Salzsäure tropfenweise bis pH 2 angesäuert, wobei sich die Carbonsäure kristallin abscheidet. Nach Absaugen und Trocknen bei 75 °C i. Vak. erhält man 17,2 g (73 % Ausbeute) (E)-β-Methoxy-2-[2-methyl-4-(1-methoximinoeth-1-yl)-phenoxymethyl]-zimtsäure vom Fp. 154 °C in Form weißer Kristalle. Laut GC und $^1$H-NMR blieb die weitgehend einheitliche Isomerenzusammensetzung bezüglich der C = N-Doppelbindung nach der Verseifung erhalten.
$^1$H-NMR ($CDCl_3$): δ = 2,14 (3H, s); 2,24 (3H, s); 3,73 (3H, s); 3,93 (3H, s); 5,04 (2H, s); 5,32 (1H, s); 6,74 (1H, dd); 7,1-7,6 (6H, m); 8-9 (COO-H, sehr breites Signal) ppm.

Beispiel 10

Herstellung von (E)-β-Methoxy-2-[2-methyl-4-(1-methoximinoeth-1-yl)-phenoxymethyl]-zimtsäureethylester (Verbindung Nr. 1a.74 der Tabelle 1a).

Die Mischung von 1,1 g (0,003 M) (E)-β-Methoxy-2-[2-methyl-4-(1-methoximinoeth-1-yl)-phenoxymethyl]-zimtsäure (Beispiel 9), 0,9 g Pyridin und 20 ml Methyl-tert.-butyl-ether wird unter Rühren zunächst bei 0°C mit 0,4 g (0,0033 M) Thionylchlorid versetzt und dann eine Stunde bei Raumtemperatur gerührt. Anschließend tropft man 5 g Ethanol zu und rührt eine weitere Stunde bei Raumtemperatur. Man extrahiert die Mischung anschließend mit Wasser, sodann mit verdünnter Salzsäure und schließlich mit Natriumcarbonatlösung, trocknet die organische Phase über $MgSO_4$ und engt i. Vak. zur Trockene ein. Man erhält 1,0 g (88 % Ausbeute) des o.a. Ethylesters in Form eines blaßgelben Öls. Auch hier blieb lt. GC- und NMR-Kontrolle die einheitliche E-Isomerenzusammensetzung bezüglich der C=N-Doppelbindung während der Reaktion erhalten.
$^1$H-NMR ($CDCl_3$): δ = 1,10 (3H, tr,); 2,19 (3H, s); 2,29 (3H, s); 3,76 3H, s); 3,97 (3H, s); 3,99 (2H, q); 5,08 2H, s), 5,37 (1H, s); 6,80 (1H, dd); 7,1 - 7,7 (6H, m) ppm.

Beispiel 11

Herstellung von (E)-β-Methoxy-2-[2-methyl-4-(1-methoximinoeth-1-yl)-phenoxymethyl]-zimtsäureamid (Verbindung Nr. 1a.88 der Tabelle 1a)

Die Mischung von 1,1 g (0,003 M) (E)-β-Methoxy-2-[2-methyl-4-(1-methoximinoeth-1-yl)-phenoxymethyl]-zimtsäure (Beispiel 9), 0,9 g Pyridin und 20 ml Methyl-tert.-butylether wird unter Rühren zunächst bei 0°C mit 0,4 g (0,0033 M) Thionylchlorid versetzt und dann eine Stunde bei Raumtemperatur gerührt. Anschließend leitet man gasförmiges Ammoniak im Überschuß ein und rührt nach Beendigung der Gaseinleitung noch eine Stunde bei Raumtemperatur. Man extrahiert die Mischung anschließend mit Wasser, sodann mit verdünnter Salzsäure und schließlich mit Natriumcarbonatlösung, trocknet die organische Phase über Magnesiumsulfat und engt i.Vak. zur Trockene ein. Man erhält 1,0 g (88 % Ausbeute) des o.a. Säureamids in Form farbloser Kristalle vom Fp. 97 - 99°C. Auch hier blieb lt. GC- und 1H-NMR-Kontrolle die einheitliche E-Isomerenzusammensetzung bezüglich der C=N-Doppelbindung während der Reaktion erhalten.
$^1$H-NMR ($CDCl_3$): δ = 2,20 (3H; s); 2,27 (3H, s); 3,75 (3H, s); 3,94 (3H, s); 5,0 (NH2, breites Signal); 5,12 (2H, s); 5,35 (1H, s); 6,83 (1H, dd); 7,2 - 7,7 (6H, m) ppm.

Beispiel 12

Herstellung von (E)-β-Methoxy-2-[4-phenyl-thiazol-2-yloxy-methyl]-zimtsäuremethylester (Verbindung Nr. 1a.181 der Tabelle 1a).

a) 54 g (0,35 mol) ω-Chloracetophenon werden mit 200 ml Ethanol und mit 26,6 g $NH_4SCN$ versetzt. Man erhitzt 15 min unter Rückfluß, saugt entstandenes $NH_4Cl$ ab und versetzt mit 175 ml konz. HCl/350 ml $H_2O$. Nach 2-stdg. Erhitzen unter Rückfluß wird auf RT abgekühlt, der gebildete Niederschlag bis pH 7 mit ges. $Na_2CO_3$-Lösung versetzt, 15 min gerührt und abfiltriert.

Man wäscht mit $H_2O$, trocknet i. Vak. und erhält so 40 g (67 % Ausbeute) 2-Hydroxy-4-phenylthiazol als gelben Feststoff vom Fp. 194 bis 197°C.
$^1$H-NMR (DMSO): 6,81 (s, 1H); 7,33 - 7,49 (m, 3H), 7,62 - 7,69 (m, 2H), 11,80 (br.s, 1H) ppm.
b) 1,2 g (0,007 M) 2-Hydroxy-4-phenylthiazol, 2,1 g (0,0077 M) Silbercarbonat und 2,1 g (0,0074 M) (E)-β-Methoxy-2-brom-methylzimtsäuremethylester (siehe Beispiel 2) werden in 15 ml DMF 8 Stunden bei 60°C gerührt. Nach Abfiltrieren des Niederschlags wird die DMF-Lösung in 100 ml Wasser gegossen und mit Methyl-tert.-butylether extrahiert. Nach dem Trocknen und Einengen der organische Phase bleibt ein braunes Öl zurück, das an Kieselgel mit Cyclohexan/Aceton (95:5) chromatographiert wird. Man isoliert als Hauptfraktion 1,5 g (56 % Ausbeute) (E)-β-Methoxy-2-[4-phenyl-thiazol-2-yloxy-methyl]-zimtsäuremethylester als gelbliches Öl.
$^1$H-NMR ($CDCl_3$): δ = 3,52 (3H, s), 3,74 (3H, s); 5,38 (1H, s); 5,53 (2H, s); 6,82 (1H, s); 7,2 - 7,9 (9H, m) ppm.
IR: 1128, 1156, 1195, 1231, 1268, 1374, 1522, 1530, 1624, 1714 $cm^{-1}$.

Beispiel 13

Herstellung von (E)-β-Methoxy-2-[2-(4-fluorophenyl)-thiazol-4-ylmethyloxy]-zimtsäuremethylester (Verbindung Nr. 1. 71 der Tabelle 1).

a) 11,2 g (0,05 M) 4-Chlormethyl-2-(4-fluorophenyl)-thiazol (siehe Beispiel 4), 5,4 g (0,04 M) 2-Hydroxyacetophenon, 7,5 g (0,055 M) Kaliumcarbonat und 0,5 g Kaliumiodid in 20 ml Ethanol werden 24 Stunden lang bei Rückflußtemperatur gerührt. Man filtriert, engt das Filtrat ein, nimmt den Rückstand in Dichlormethan auf, extrahiert die Lösung mit verdünnter Natriumhydroxidlösung und anschließend mit Wasser und trocknet die organische Phase über $MgSO_4$. Nach Abdestillieren des Lösungsmittels i. Vak. bleibt ein fester Rückstand, der nach Waschen mit Diisopropylether 9,7 g (59 % Ausbeute) 2-[2-(4-Fluorophenyl)-thiazol-4-ylmethyloxy]-acetophenon als gelbliche Kristalle vom Fp. 104 - 106 °C ergibt.
$^1$H-NMR ($CDCl_3$): δ = 2,67 (3H, s); 5,36 (2H, s); 6,9 - 8,1 (9H, m) ppm.
b) Zu 3,0 g (0,033 M) Dimethylcarbonat und 0,8 g (0,033 M) Natriumhydrid in 20 ml Tetrahydrofuran tropft man bei 60 °C unter Rühren und unter Stickstoff eine Lösung von 9,8 g (0,03 M) 2-[2-(4-Fluorophenyl)-thiazol-4-ylmethyloxy]-aceto-phenon in 80 ml Tetrahydrofuran und rührt nach Beendigung des Zutropfens noch 1 Stunde bei 60 °C. Nach dem Abkühlen gibt man zunächst 5 ml Methanol, sodann 500 ml Wasser zu, säuert mit verdünnter Salzsäure an und extrahiert mit Methyl-tert.-butylether.Die organische Phase wird mit Natriumhydrogencarbonatlösung gewaschen, getrocknet und eingeengt. Man erhält 10,8 g (93 % Ausbeute) ω-Methoxycarbonyl-2-[2-{4-fluoro-phenyl)-thiazol-4-ylmethyloxy]-acetophenon als ockerfarbiges Festprodukt vom Fp. 86 - 89 °C.
$^1$H-NMR ($CDCl_3$): δ = 3,62 (3H, s); 4,12 (2H, s); 5,31 (2H, s); 6,9 - 8,1 (9H, m) ppm.
c) Zur Lösung von 2,5 g (0,0065 M) ω-Methoxycarbonyl-2-[2-(4-fluorophenyl)-thiazol-4-ylmethyloxy]-acetophenon und 0,83 g (0,0078 M) Trimethylorthoformiat in 10 ml Methanol gibt man einen Tropfen konz. Schwefelsäure. Nach vierstündigem Erhitzen auf Rückflußtemperatur engt man die Lösung i. Vak. ein, nimmt den Rückstand in Toluol auf und wäscht die Toluolphase mit Natriumhydrogencarbonatlösung. Nach Trocknung und Einengen erhält man 1,9 g bräunliches Öl. Nach Chromatographie an Kieselgel mit Toluol/Aceton (98:2) isoliert man 1,0 g (38 % Ausbeute) (E)-β-Methoxy-2-[2-(4-fluorophenyl)-thiazol-4-ylmethyloxy]-zimtsäureme thylester als bräunliche Kristalle vom Fp. 105 - 108 °C.
$^1$H-NMR ($CDCl_3$): δ = 3,51 (3H, s); 3,81 (3H, s), 5,31 (2H, s), 5,44 (1H, s), 6,9 - 8,1 (9H, m) ppm.

Beispiel 14

Herstellung von 2-[1-(4-Chlorphenyl)-1-methyl]-iminooxymethyl-(E)-β-methoxyzimtsäuremethylester (Verbindung Nr. 1a.356 der Tabelle 1a.)

0,5 g (20 mmol) Natriumhydrid werden in 80 ml Dimethylformamid vorgelegt und bei Raumtemperatur 3,1 g (18 mmol) 4-Chloracetophenonoxim zugegeben.

Nach 30 min Rühren werden 5 g (18 mmol) (E)-2-Brommethyl-β-methoxyzimtsäuremethylester in 20 ml Dimethylformamid zugetropft. Anschließend wird 1 h nachgerührt. Nach Zugabe von $NH_4Cl$-Lösung wird mit Methyl-tert.-butylether extrahiert. Die vereinigten organischen Phasen werden mit Wasser gewaschen, über $Na_2SO_4$ getrocknet und eingeengt. Man nimmt in Methyl-tert.-butylether/n-Hexan = 2:1 auf, filtriert über Kieselgel und engt ein.

Es resultieren 5,8 g (86 %) 2-[1-(4-Chlorphenyl)-1-methyl]-imino-oxymethyl-(E)-β-methoxyzimtsäuremethylester als gelbes Öl.
$^1$H-NMR ($CDCl_3$): δ = 2,20 (s, 3H); 3,50 (s, 3H); 3,78 (s, 3H); 5,20 (s, 2H), 5,36 (s, 1H); 7,20 - 7,57 (m, 8H) ppm.

Beispiel 15

Herstellung von (E)- und (Z)-β-Methoxy-2-(3-methylphenoxy)-zimtsäuremethylester (Verbindungen Nr. 1.90 und 1.91 der Tabelle 1)

a) Eine Mischung aus 50 g (0,324 mol) 2-Chloracetophenon, 104,8 g (0,97 mol) m-Kresol, 45 g (0,326 mol) Kaliumcarbonat und 1 g Kupfer wird 16 Stunden auf 120 °C erhitzt. Man läßt abkühlen, gießt auf Wasser und extrahiert zweimal mit Methyl-tert.-Butylether (MTB-Ether). Die Etherphase wird fünfmal mit konz. Natronlauge und anschließend 2 mal mit Wasser gewaschen, getrocknet und eingeengt. Es verbleiben 67,2 g (92 % Ausbeute) 2-(3-Methyl-phenoxy)-acetophenon als gelbes Öl.

$^1$H-NMR ($CDCl_3$): δ = 2,35 (s, 3H); 2,65 (s, 3H); 6,79-7,84 (m, 8H) ppm.

b) Zu einer Suspension von 10,3 g (0,43 mol) Natriumhydrid in 230 ml THF gibt man 38,7 g (0,43 mol) Dimethylcarbonat und tropft anschließend bei 50 °C eine Lösung von 67,2 g (0,30 mol) 2-(3-Methyl-phenoxy)-acetophenon zu. Man rührt 3 Stunden bei 50 °C, läßt abkühlen, gibt 60 ml Methanol zu und rührt über Nacht bei Raumtemperatur. Es wird mit 10 proz. Salzsäure angesäuert (pH 2) und mit MTB-Ether extrahiert. Die organische Phase wird mit Wasser gewaschen, getrocknet und eingeengt. Es verbleiben 79,5 g (93 % Ausbeute) 2-(3-Methyl-phenoxy)-benzoylessigsäuremethylester als dunkles Öl.

$^1$H-NMR ($CDCl_3$): [Ketoform] δ = 2,34 (s, 3H), 3,60 (s, 3H), 4,05 (s, 2H); 6,80 - 7,98 (m, 8H) ppm.

c) Zu einer Suspension von 500 mg (21 mmol) Natriumhydrid in 20 ml Hexamethylphosphorsäuretriamid (HMPT) tropft man bei Raumtemperatur eine Lösung von 5 g (18 mmol) Methyl-2-[3-methylphenoxy]-benzoylacetat in 30 ml HMPT und rührt bis zum Ende der Gasentwicklung nach. Anschließend tropft man 2,5 g (20 mol) Dimethylsulfat zu, rührt 30 min nach und versetzt dann mit 30 ml konz. Ammoniaklösung. Man gießt auf Wasser und extrahiert mit MTB-Ether. Die organische Phase wird getrocknet und eingeengt. Chromatographie des Rückstandes an Kieselgel mit Hexan/MTB-Ether liefert in der ersten Produktfraktionen 1,2 g (22 %) (E)-β-Methoxy-2-(3-methylphenoxy)-zimtsäure-methylester (trans-Produkt) als gelbes Öl

$^1$H-NMR ($CDCl_3$): δ = 2,28 (s, 3H); 3,56 (s, 3H), 3,71 (s, 3H), 5,28 (s, 1H) 6,77 - 7,32 (m, 8H) ppm

und anschließend 0,5 g (9 %) (Z)-β-Methoxy-2-(3-methyl-phenoxy)-zimtsäuremthylester (cis-Produkt) als gelbes Öl

$^1$H-NMR ($CDCl_3$): δ = 2,33 (s, 3H); 3,70 (s, 3H), 3,75 (s, 3H), 5,21 (s, 1H) 6,77 - 7,36 (m, 8H) ppm.

Beispiel 16

Herstellung von (Z)- und (E)-β-Ethoxy-2-methylzimtsäuremethylester (Verb. Nr. 1.48 und 1.47 der Tabelle 1)

9,6 g (50 mmol) 2-Methylbenzoylessigsäuremethylester, 6 g Ethylbromid und 13,8 g Kaliumcarbonat werden für 14 Stunden unter Rückfluß gekocht. Das Reaktionsgemisch wird eingeengt, mit Methyl-t.-butylether aufgenommen und 3 x mit Wasser extrahiert. Die organische Phase wird über Natriumsulfat getrocknet und eingeengt. Das Rohprodukt wird an Kieselgel mit 1,2-Dichlorethan/Cyclohexan (2:1) chromatogrphiert. Man erhält 2,3 g (21 %) des (E)-Isomeren (Verb. Nr. 1.47) und 1,3 g (12 %) des (Z)-Isomeren (Verb. Nr. 1.48) jeweils in Form farbloser Öle.

$^1$H-NMR ($CDCl_3$):

(Z)-Isomer (Verb. Nr. 1.48): δ = 1,24 (3H, t), 2,35 (3H, s), 3,70 (3H, s), 3,73 (2H, q); 5,07 (1H, s), 7,1 - 7,3 (4H, m) ppm.

(E)-Isomer (Verb. Nr. 1.47): δ = 1,37 (3H, t); 2,25 (3H, s), 3,53 (3H, s); 3,97 (2H, q); 5,32 (1H, s); 7,1 - 7,3 (4H, m) ppm.

Beispiel 17

Herstellung von (E)-β-Ethoxy-2-methylzimtsäureethylester (Verb. Nr. 1.51 der Tabelle 1)

19,2 g (100 mmol) 2-Methylbenzoylessigsäuremethylester und 17,7 g Orthoameisensäuretriethylester werden in 40 ml Ethanol unter Zugabe von 0,4 ml konz. Schwefelsäure für 16 Stunden unter Rückfluß gerührt. Nach dem Abkühlen werden 2 ml Pyridin zugesetzt und anschließend wird eingeengt. Der Rückstand wird mit Methyl-t.-butylether aufgenommen und 2 x mit Wasser extrahiert. Die organische Phase wird über Natriumsulfat getrocknet und eingeengt. Das Rohprodukt enthält neben der Titelverbindung etwa 15 % des entsprechenden (E)-Methylesters (Verb. Nr. 1.47). Nach Destillation erhält man 16,5 g (75 %) der Titelverbindung als farbloses Öl.

$^1$H-NMR ($CDCl_3$): δ = 1,05 (3H, t); 1,37 (3H, t); 2,25 (3H, s); 3,97 (2H, q); 3,99 (2H, q); 5,32 (1H, s); 7,1 - 7,3 (4H, m) ppm.

Beispiel 18

Herstellung von (E)- und (Z)-β-Methyl-2-[(2-methylphenoxy)-methyl]-zimtsäuremethylester (Verb. Nr. 1a.109 und Nr. 1a.108 der Tabelle 1a)

a) 99,4 g (0,92 mol) ortho-Kresol und 102 g Kaliumcarbonat werden in 450 ml Ethanol gemischt. Bei 22 °C werden 202 g 2-Brom-benzylbromid, gelöst in 150 ml Ethanol, zugetropft. Es wird 8 h zum Rückfluß erhitzt. Es wird abgekühlt, dann filtriert. Das Filtrat wird eingeengt. Das Rohprodukt wird in Methyl-t.-butylether aufgenommen, mit Wasser gewaschen und über Natriumsulfat getrocknet. Nach Abdampfen des mittels und anschließender Destillation erhält man 163 g (73 %) 2-[(2-Methylphenoxy)-methyl]-brombenzol als farbloses Öl, das nach einiger Zeit erstarrt (Fp.: 44 - 45 °C).

b) Zu 27,7 g (0,1 mol) 2-[(2-Methylphenoxy)-methyl]-brombenzol, gelöst in 200 ml Diethylether, werden bei 0 °C 67 ml n-Butyl-lithium (1,6 molar in Hexan) getropft. Nach 30 Minuten werden 11 g N-Methoxy-N-methylessigsäureamid, gelöst in 40 ml Diethylether, bei 0 °C zugetropft. Es wird 3 Stunden bei Raumtemperatur nachgerührt. Es wird mit Wasser verdünnt, mit Salzsäure angesäuert und mit Diethylether extrahiert. Die vereinigten organischen Phasen werden mit Wasser gewaschen, über Natriumsulfat getrocknet und eingeengt. Das verbleibende Rohprodukt wird an Kieselgel mit Cyclohexan/Methyl-t.-butylether (10/1) chromatographiert. Man erhält 13,5 g (56 %) 2-[(2-Methylphenoxy)-methyl]-acetophenon in Form eines farblosen Öls.

c) Zu einer Mischung aus 2,8 g Natriumhydrid in 450 ml Tetrahydrofuran wird bei Raumtemperatur eine Lösung aus 20,4 g Phosphonoessigsäuretrimethylester in 60 ml Tetrahydrofuran getropft. Nach 20 Minuten wird eine Lösung aus 13,4 g (56 mmol) 2-[(2-Methylphenoxy)-methyl]-acetophenon in 45 ml Tetrahydrofuran zugetropft. Die Reaktionsmischung wird 12 Stunden unter Rückfluß gekocht. Nach dem Abkühlen wird mit Wasser verdünnt und mit Diethylether etrahiert. Die vereinigten organischen Phasen werden mit Wasser gewaschen, getrocknet und eingeengt. Das Rohprodukt wird an Kieselgel mit Cyclohexan/Methyl-t.-butylether (20:1) chromatographiert.

Man erhält 10,9 g (66 %) (E)-Produkt (Verb. Nr 1a.109) und 5,0 g (30 %) (Z)-Produkt (Verb. Nr. 1a.108 g) jeweils in Form farbloser Öle.

$^1$H-NMR ($CDCl_3$):

E-Isomer (Verb. Nr. 1a.109): δ = 2,21 (3H, s); 2,50 (3H, s); 3,70 (3H, s); 4,97 (2H, s); 5,85 (1H, s); 6,8 - 7,6 (8H, m) ppm.

Z-Isomer (Verb. Nr. 1a.108): δ = 2,18 (3H, s); 2,25 (3H, s); 3,55 (3H, s); 4,95 (2H, AB); 5,98 (1H, s); 6,8 - 7,6 (8H, m) ppm.

Beispiel 19

Herstellung von (E)- und (Z)-β-Methyl-2-methoxymethylzimtsäuremethylester (Verbindungen Nr. 1.165 und 1.170 der Tabelle 1)

Zu einer Mischung aus 1,3 g Natriumhydrid in 210 ml Tetrahydrofuran wird bei Raumtemperatur eine Lösung aus 9,8 g Phosphonoessigsäuretrimethylester in 30 ml Tetrahydrofuran getropft. Nach 20 Minuten wird eine Lösung aus 5,9 g (36 mmol) 2-Methoxymethylacetophenon [siehe: A.L. Maycock und G.A. Berchtold, J. Org. Chem. 35 (8), 2532 (1970)] in 30 ml Tetrahydrofuran zugetropft. Die Reaktionsmischung wird 12 h unter Rückfluß gekocht; nach dem Abkühlen wird mit Wasser verdünnt und mit Diethylether extrahiert. Die vereinigten organischen Phasen werden mit Wasser gewaschen, getrocknet und eingeengt. Das Rohprodukt (cis: trans β 3:2) wird an Kieselgel mit Cyclohexan/Methyl-t.-butylether (7:1) chromatographiert. Man erhält 4,5 g (57 %) (E)-Isomeres (Verb. Nr 1.165) und 2,9 g (37 %) (Z)-Isomeres (Verb. Nr. 1.170) jeweils als farbloses Öl.

$^1$H-NMR ($CDCl_3$):

E-Isomer (Verb. Nr. 1.165): δ = 2,48 (3H, s), 3,39 (3H, s); 3,77 (3H, s); 4,40 (2H, s); 5,82 (1H, s); 7,1 -7,5 (4H, m) ppm.

Z-Isomer (Verb. Nr. 1.170): δ = 2,17 (3H, s); 3,38 (3H, s); 3,51 (3H, s); 4,35 (2H, AB); 6,01(1H, s); 7,0 - 7,5 (4H, m) ppm.

Beispiel 20

Herstellung von (E)- und (Z)-β-Methyl-2-brommethylzimtsäure-methylester (Verb. Nr. 1.167 und Nr. 1.171 der Tabelle 1)

In einer Lösung aus 42,9 g (195 mmol) β-Methyl-2-methoxymethylzimtsäuremethylester (E:Z = 3:2) in 470 ml Methylenchlorid wird eine Lösung aus 48,7 g Bortribromid in 18 ml Methylenchlorid getropft. Es wird für 2 Stunden zum Rückfluß erhitzt. Nach dem Abkühlen werden 50 ml Methanol zugetropft. Es wird mit Wasser verdünnt und mit Dichlormethan extrahiert. Die vereinigten organischen Phasen werden über Natriumsulfat getrocknet und eingeengt.

Das Rohprodukt (50 g; 95 % Ausbeute) ist ein Isomerengemisch (E:Z = 3:2) der Titelverbindung, das sich chromatographisch trennen läßt.

$^1$H-NMR ($CDCl_3$):

(E)-Isomer: δ = 2,51 (3H, s); 3,77 (3H, s); 5,00 (2H, s); 5,90 (Verb.Nr. 1.167) (1H, s); 7,1 - 7,5 (4H, m) ppm.

(Z)-Isomer: δ = 2,25 (3H, s); 3,51 (3H, s); 5,00 (2H, s); 6,08 (Verb.Nr. 1.171) (1H, s); 7,0 - 7,5 (4H) ppm.

Beispiel 21

Herstellung von (E)- und (Z)-β-Methyl-2-2-methyl-4-[1-(E)-methoximinoethyl]-phenoxymethyl -zimtsäuremethylester (Verbindungen Nr. 1a.113 und Nr. 1a.112 der Tabelle 1a)

15 g (56 mmol) β-Methyl-2-brommethylzimtsäuremethylester ((E)/(Z)-Gemisch ~ 3:2), 10 g (56 mmol) 2-Methyl-4-[1-(E)-methoxyimino-ethyl]-phenol (s. Beispiel 8) und 11,6 g Kaliumcarbonat werden in 100 ml Dimethylformamid 30 Minuten bei 45 °C gerührt. Es wird eingeengt, mit Methyl-t.-butylether aufgenommen, mit Wasser gewaschen, über Natriumsulfat getrocknet und wieder eingeengt.

Das Rohprodukt wird an Kieselgel mit n-Hexan/Methyl-t.-butyl-ether (8:1) chromatographiert.

Man erhält 7,2 g (35 %) (E)-Isomeres (Verb. Nr. 1a.113) und 4,8 g (23 %) (Z)-Isomeres (Verb. Nr. 1a.112) jeweils als farbloses Öl.

$^1$H-NMR ($CDCl_3$):

(E)-Isomer (Verb. Nr. 1a.113): δ = 2,17 (3H, s); 2,25 (3H, s), 2,50 (3H, s); 3,72 (3H, s), 3,97 (3H, s) 5,03 (2H, s); 5,85 (1H, s); 6,8 - 7,6 (7H) ppm.

(Z)-Isomer (Verb. Nr. 1a.112): δ = 2,17 (3H, s); 2,18 (3H, s); 2,26 (3H, s); 3,53 (3H, s); 3,97 (3H, s); 4,98 (2H, AB); 6,00 (1H, s); 6,8 - 7,6 (7H) ppm.

Beispiel 22

Herstellung von (E)-3-Methoxy-3-[7-brom-1-naphthyl]-acrylsäuremethylester (Verbindung Nr. 1b.17 der Tabelle 1b)

a) Zu einer Suspension von 14 g (0,58 mol) NaH und 52 g (0,58 mol) Dimethylcarbonat in 500 ml Tetrahydrofuran wird bei 60 °C unter Argonatmosphäre eine Lösung von 105 g (0,42 mol) 1-Acetyl-7-bromnaphthalin in 500 ml Tetrahydrofuran langsam zugetropft. Anschließend wird noch 2 h bei 60 °C nachgerührt. Nach dem Abkühlen wird mit 30 ml Methanol versetzt, die Mischung auf Eiswasser gegeben und mit 10 %iger Salzsäure angesäuert. Nach dem Extrahieren mit Methyl-tert.-butylether werden die vereinigten organischen Phasen mit $NaHCO_3$-Lösung und Wasser gewaschen, über $Na_2SO_4$ getrocknet und eingeengt. Als Rückstand verbleiben 127 g (98 %) 7-Brom-1-naphthoyl-essigsäuremethylester als bräunliche Kristalle mit einem Schmelzpunkt von 82-83 °C.
$^1$H-NMR ($CDCl_3$): Ketoform: δ = 3,78 (s,3H); 4,12 (s,2H); 7,40-8,01 (m,5H); 9,17 (s,1H) ppm
Gleichzeitig treten in untergeordnetem Maße (ca. 30 %) die Signale der zugehörigen Enolform auf:
δ = 3,83 (s,3H); 5,48 (s,1H); 7,40-8,01 (m,5H); 8,51 (s,1H) ppm

b) Eine Mischung von 189 g (0,61 mol) 7-Brom-1-naphthoyl-essigsäuremethylester und 500 ml N,N'-Dimethylpropylenharnstoff wird unter Rühren mit 93 g (0,68 mol) Kaliumcarbonat versetzt und 2 h bei 40 °C gerührt. Anschließend wird auf 0-5 °C abgekühlt und eine Mischung aus 85 g (0,68 mol) Dimethylsulfat und 50 ml N,N'-Dimethylpropylenharnstoff zugetropft. Danach wird weitere 2 h bei 40 °C gerührt. Das Reaktionsgemisch wird mit Wasser und Methyl-tert.butylether versetzt. Die wäßrige Phase wird nochmals mit Methyl-tert.-butylether extrahiert und die vereinigten organischen Phasen über $Na_2SO_4$ getrocknet und eingeengt. Der Rückstand wird in 500 ml N,N'-Dimethylpropylenharnstoff aufgenommen und mit 2,7 g (0,11 mol) NaH versetzt. Die Mischung wird 15 h bei Raumtemperatur

gerührt. Anschließend wird mit 20 ml Methanol versetzt, mit Wasser verdünnt und mit Methyl-tert.-butylether extrahiert. Die organische Phase wird mit Wasser gewaschen, über $Na_2SO_4$ getrocknet und eingeengt. Der Rückstand wird aus Methanol umkristallisiert. Man erhält 102 g (52 %) (E)-3-Methoxy-3-[7-brom-1-naphthyl]-acrylsäuremethylester in Form von farblosen Kristallen vom Schmelzpunkt 111-113 °C.
$^1$H-NMR ($CDCl_3$): δ = 3,48 (s,3H); 3,88 (s,3H); 5,58 (s,1H); 7,44 (t,1H); 7,54 (m,2H); 7,72 (d,1H); 7,86 (d,1H); 7,90 (s,1H) ppm
Aus der Mutterlauge kann durch säulenchromatographische Reinigung über Kieselgel (Cyclohexan/Toluol 2:1 bis 1:10) der isomere (Z)-3-Methoxy-3-[7-brom-1-naphthyl]-acrylsäuremethylester in Form von farblosen Kristallen vom Schmelzpunkt 96-97 °C gewonnen werden.
$^1$H-NMR ($CDCl_3$): δ = 3,51 (s,3H); 3,78 (s,3H); 5,25 (s,1H); 7,50 (d, 2H); 7,61 (d,1H); 7,86 (d,1H); 7,90 (t,1H); 8,26 (s,1H) ppm

Beispiel 23

Herstellung von (E)-3-Methoxy-3-[7-phenyl-1-naphthyl]-acrylsäuremethylester (Verbindung Nr. 1b.1 der Tabelle 1b)

1,4 g (4,4 mmol) (E)-3-Methoxy-3-[7-brom-1-naphthyl]-acrylsäuremethylester, 0,36 g (0,3 mmol) Tetrakis(triphenylphosphin)palladium und 0,76 g (6,2 mmol) Phenylborsäure werden mit 75 ml Dimethoxyethan und 31 ml 20 %iger $Na_2CO_3$-Lösung versetzt und anschließend 8 h bei 80 °C gerührt. Die Mischung wird mit Methyl-tert.-butylether extrahiert und die organische Phase noch 2 x mit Wasser gewaschen, über $Na_2SO_4$ getrocknet und eingeengt. Der Rückstand wird säulenchromatographisch an Kieselgel (zuerst Cyclohexan, dann Toluol) gereinigt. Man erhält so 0,98 g (71 %) (E)-3-Methoxy-3-[7-phenyl-1-naphthyl]-acrylsäuremethylester in Form von gelblichen Kristallen mit einem Schmelzpunkt von 62-64 °C.
$^1$H-NMR ($CDCl_3$): δ = 3,46 (s,3H); 3,88 (s,3H); 5,61 (s,1H); 7,31-7,55 (m, 5H); 7,63 (d,2H); 7,71 (d,1H); 7,85-7,93 (m,3H) ppm

Beispiel 24

Herstellung von (E)-3-Methoxy-3-[7-methyl-1-naphthyl]-acrylsäuremethylester (Verbindung Nr. 1b.11 der Tabelle 1b)

a) Zu einer Suspension von 35 g (1,2 mol) NaH und 104 g (1,2 mol) Dimethylcarbonat in 750 ml Tetrahydrofuran wird bei 60 °C unter Stickstoffatmosphäre eine Lösung von 154 g (0,8 mol) 1-Acetyl-7-methylnaphthalin in 500 ml Tetrahydrofuran langsam zugetropft. Anschließend wird noch 2 h bei 60 °C nachgerührt. Nach dem Abkühlen wird mit 100 ml Methanol versetzt, die Mischung auf Eiswasser gegeben und mit 10 %iger Salzsäure angesäuert. Nach dem Extrahieren mit Methyl-tert.-butylether werden die vereinigten organischen Phasen mit $NaHCO_3$-Lösung und Wasser gewaschen, über $Na_2SO_4$ getrocknet und eingeengt. Als Rückstand verbleiben 177 g (88 %) 7-Methyl-1-naphthoyl-essigsäuremethylester als rotbraunes Öl.
$^1$H-HMR ($CDCl_3$): Ketoform: δ = 2,52 (s,3H); 3,73 (s,3H); 4,08 (s,2H); 7,29-7,46 (m,2H); 7,68-8,00 (m,3H); 8,59 (s,1H) ppm
Gleichzeitig treten in untergeordnetem Maße (ca. 30 %) die Signale der zugehörigen Enolform auf:
δ = 2,51 (s,3H); 3,81 (s,3H); 5,49 (s,1H); 7,29-8,00 (m,5H); 8,10 (s,1H) ppm
b) Eine Mischung von 48 g (0,2 mol) 7-Methyl-1-naphthoyl-essigsäuremethylester und 250 ml N,N'-Dimethylpropylenharnstoff wird unter Rühren mit 30 g (0,22 mol) Kaliumcarbonat versetzt und 2 h bei 40 °C gerührt. Anschließend wird auf 0-5 °C abgekühlt und eine Mischung aus 27,7 g (0,22 mol) Dimethylsulfat und 50 ml N,N' -Dimethylpropylenharnstoff zugetropft. Danach wird weitere 2 h bei 40 °C gerührt. Das Reaktionsgemisch wird mit Wasser und Methyl-tert.-butylether versetzt. Die wäßrige Phase wird nochmals mit Methyl-tert.-butylether extrahiert und die vereinigten organischen Phasen über $Na_2SO_4$ getrocknet und eingeengt. Der Rückstand wird in 200 ml N,N'-Dimethylpropylenharnstoff aufgenommen und mit 2,2 g (0,1 mol) NaH versetzt. Die Mischung wird 15 h bei Raumtemperatur gerührt. Anschließend wird mit 50 ml Methanol versetzt, mit Wasser verdünnt und mit Methyl-tert.-butylether extrahiert. Die organische Phase wird mit Wasser gewaschen, über $Na_2SO_4$ getrocknet und eingeengt. Der Rückstand wird säulenchromatographisch an Kieselgel (zuerst Cyclohexan, dann Toluol) gereinigt. Man erhält so 40,4 g (80 %) (E)-3-Methoxy-3-[7-methyl-1-naphthyl]-acrylsäuremethylester als braunes Öl.

$^1$H-NMR ($CDCl_3$): δ = 2,48 (s,3H); 3,45 (s,3H); 3,87 (s,3H); 5,58 (s,1H); 7,21-7,52 (m,4H); 7,72-7,90 (m,2H) ppm

Beispiel 25

Herstellung von (E)-3-Methoxy-3-[7-phenoxymethyl-1-naphthyl]-acrylsäuremethylester (Verbindung Nr. 1b.20 der Tabelle 1b)

a) 6,2 g (0,024 mol) (E)-3-Methoxy-3-[7-methyl-1-naphthyl]-acrylsäuremethylester und 7,1 g (0,025 mol) 1,3-Dibrom-5,5-dimethylhydantoin in 100 ml trockenem Tetrachlorkohlenstoff werden 75 min unter Bestrahlung mit einer Halogenlampe auf Rückflußtemperatur erhitzt. Anschließend wird abgesaugt und das Filtrat über eine Kieselgelschicht nochmals filtriert. Das Lösungsmittel wird abdestilliert und der Rückstand aus Methyl-tert.-butylether umkristallisiert. Man erhält so 6, g (67 %) (Z)-2-Brom-3-methoxy-3-[-brommethyl-1-naphthyl]-acrylsäuremethylester als farblose Kristalle mit einem Schmelzpunkt von 116-119 °C.
$^1$H-NMR ($CDCl_3$): δ = 3,40 (s,3H); 3,41 (s,3H), 4,63 (s,2H); 7,40 (d, 1H); 7,50-7,61 (m,2H); ,775 (s,1H), 7,88-7,99 (m,2H) ppm
b) Zu einer Lösung von 2,1 g (5 mmol) (Z)-2-Brom-3-methoxy-3-[-brommethyl-1-naphthyl]-acrylsäuremethylester in 30 ml Dimethylformamid (DMF) wird bei Raumtemperatur eine Lösung von 0,7 g (5,5 mmol) Kaliumphenolat in 25 ml DMF getropft und die Mischung 4 Stunden bei Raumtemperatur gerührt. Die Mischung wird i.Vak. zur Trockene eingeengt, der Rückstand in Essigester gelöst und die organische Phase zweimal mit Wasser extrahiert, getrocknet und eingeengt. Man erhält 1,85 g (87 %) (Z)-2-Brom-3-methoxy-3-[-phenoxymethyl-1-naphthyl]-acrylsäuremethylester als bräunliches Öl.
$^1$H-NMR ($CDCl_3$): δ = 3,39 (s,6H); 5,21 (s,2H); 6,91-7,32 (m,5H); 7,39 (d,1H); 7,52 (t,1H); 7,63 (d,1H); 7,80 (s,1H); 7,89-8,00 (m,2H) ppm
c) 1,64 g (3,8 mmol) (Z)-2-Brom-3-methoxy-3-[7-phenoxymethyl-1-naphthyl]-acrylsäuremethylester werden in 30 ml Toluol gelöst, mit 2,2 g (7,6 mmol) Tributylzinnhydrid sowie einigen mg α,α'-Azoisobutyronitril versetzt und 15 Stunden unter Rückfluß gerührt. Die Mischung wird anschließend eingeengt und der Rückstand säulenchromatographisch an Kieselgel (zuerst Cyclohexan, dann Toluol) gereinigt. Dabei werden zunächst die Zinnverbindungen eluiert. Die folgende Fraktion enthält 0,89 g (66 %) (E)-3-Methoxy-3-[7-phenoxymethyl-1-naphthyl]-acrylsäuremethylester.
$^1$H-NMR ($CDCl_3$): δ = 3,43 (s,3H); 3,82 (s,3H); 5,19 (s,2H); 5,56 (s, 1H); 6,90-7,56 (m,8H); 7,78 (s,1H); 7,84-7,91 (m,2H) ppm
Als zweites Produkt erhält man 0,14 g (10 %) des isomeren (Z)-3-Methoxy-3-[7-phenoxymethyl-1-naphthyl]-acrylsäuremethylesters.
$^1$H-NMR ($CDCl_3$): δ = 3,50 (s,3H); 3,76 (s,3H); 5,22 (s,2H); 5,24 (s,1H); 6,92-7,97 (m,10H), 8,08 (s,1H) ppm

**Patentansprüche**

**1.** β-substituierte Zimtsäurederivate der allgemeinen Formel 1,

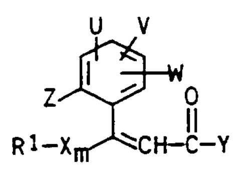

1

in der

R1 gegebenenfalls durch Halogen, $C_1$-$C_6$-Alkoxy, $C_1$-$C_6$-Alkylthio oder $C_3$-$C_6$-Cycloalkyl substituiertes $C_1$-$C_8$-Alkyl, $C_2$-$C_8$-Alkenyl, $C_2$-$C_8$-Alkinyl, $C_3$-$C_8$-Cycloalkyl oder $C_3$-$C_8$-Cycloalkenyl bedeutet, und in der $R^1$ zusätzlich Chlor oder Brom bedeutet, wenn m = 0 ist,

-X- für die Gruppierungen -O-, -S-,

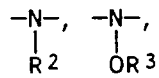

steht,

m — 0 oder 1 bedeutet,

-Y — für die Gruppen $-OR^4$, $-O-N=CR^5R^6$, $-NR^7R^8$, $-N(OR^9)R^{10}$ oder $-SR^{11}$ steht, wobei die Substituenten $R^2$ bis $R^{11}$ unabhängig voneinander gleich oder verschieden sind und gegebenenfalls durch Halogen, $C_1$-$C_6$-Alkoxy, $C_1$-$C_6$-Alkylthio oder $C_3$-$C_6$-Cycloalkyl substituiertes $C_1$-$C_8$-Alkyl, $C_2$-$C_8$-Alkenyl, $C_2$-$C_8$-Alkinyl, $C_3$-$C_8$-Cycloalkyl oder $C_3$-$C_8$-Cycloalkenyl bedeuten, und die Reste $R^2$, $R^3$ und $R^5$ bis $R^{11}$ zusätzlich Wasserstoff bedeuten, wenn $R^4$ nicht Ethyl oder t-Butyl bedeutet, und wenn m = 0 ist,

Z — Halogen, Nitro, Cyano, gegebenenfalls substituiertes Alkyl, gegebenenfalls substituiertes Cycloalkyl, gegebenenfalls substituiertes Aralkyl, gegebenenfalls substituiertes Aryloxyalkyl, gegebenenfalls substituiertes Arylthioalkyl, gegebenenfalls substituiertes Heteroarylalkyl, gegebenenfalls substituiertes Heteroaryloxyalkyl, gegebenenfalls substituiertes Heteroarylthioalkyl, gegebenenfalls substituiertes Alkenyl, gegebenenfalls substituiertes Aralkenyl, gegebenenfalls substituiertes Aryloxyalkenyl, gegebenenfalls substituiertes Arylthioalkenyl, gegebenenfalls substituiertes Heteroarylalkenyl, gegebenenfalls substituiertes Heteroaryloxyalkenyl, gegebenenfalls substituiertes Heteroarylthioalkenyl, gegebenenfalls substituiertes Alkinyl, gegebenenfalls substituiertes Arylalkinyl, gegebenenfalls substituiertes Heteroarylalkinyl, gegebenenfalls substituiertes Aryl, gegebenenfalls substituiertes Heteroaryl, gegebenenfalls substituiertes Arylazo, gegebenenfalls substituiertes Acylamino, $-OR^{12}$, $-SR^{13}$,$-SOR^{14}$, $-SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$, $-CH_2-OCOR^{39}$ oder $-NR^{37}R^{38}$ bedeutet,
wobei die Gruppierungen $R^{12}$ bis $R^{28}$, sowie $R^{38}$ und $R^{39}$ gleich oder verschieden sind und Wasserstoff, gegebenenfalls substituiertes Alkyl, gegebenenfalls substituiertes Alkenyl, gegebenenfalls substituiertes Alkinyl gegebenenfalls substituiertes Cycloalkyl, gegebenenfalls substituiertes Cycloalkylalkyl, gegebenenfalls substituiertes Aryl, gegebenenfalls substituiertes Heteroaryl, gegebenenfalls substituiertes Aralkyl, gegebenenfalls substituiertes Heteroarylalkyl, gegebenenfalls substituiertes Aryloxyalkyl, gegebenenfalls substituiertes Arylthioalkyl, gegebenenfalls substituiertes Heteroaryloxyalkyl oder gegebenenfalls substituiertes Heteroarylthioalkyl bedeuten und $R^{37}$ Wasserstoff oder $C_1$-$C_4$-Alkyl bedeutet, und $R^{19}$ nicht Wasserstoff und $R^{12}$ nicht gegebenenfalls substituiertes Alkyl bedeutet, wenn m = 0 ist
und in der

U,V, W — gleich oder verschieden sind und Wasserstoff bedeuten oder eine der für Z genannten Bedeutungen haben
oder in der
zwei der Gruppierungen Z, U, V oder W in benachbarten Positionen des Phenylrings gegebenenfalls zusammen mit den Kohlenstoffatomen, deren Substituenten sie sind, einen gegebenenfalls substituierten, an den Phenylring ankondensierten fünf- oder sechsgliedrigen, aromatischen oder aliphatischen Ring bilden, der ggf. ein bis drei Heteroatome (N, S, O) enthalten kann.

ausgenommen die Verbindungen

- 3-Methoxy-3-[2-methoxyphenyl]acrylsäure methylester,
- 3-Methoxy-3-[(1-hydroxy-3-methyl-6,8-dimethoxy)-naphth-2-yl]-acrylsäure methylester,
- 3-Chlor-3-[2-methyl-naphth-1-yl]-acrylsaure methylester,
- 3-Methyl-3-[2-methyl-naphth-1-yl]-acrylsäure methylester,
- 3-Methyl-3-[(2-isopropylcarbonyl-4-methoxycarbonyl)-phenyl]-acrylsäure methylester,

sowie Verbindungen, in denen Z für $NO_2$, $NH_2$ oder N-Pyrrollidon steht, wenn $R^1X_m$ Methyl bedeutet, U, V und W für Wasserstoff stehen und Y Alkylamino oder Cycloalkylamino bedeutet.

**2.** Verbindungen der Formel 1 gemäß Anspruch 1, in der X Sauerstoff oder Schwefel bedeutet.

**3.** Verbindungen gemäß Anspruch 1 der Formel 1a,

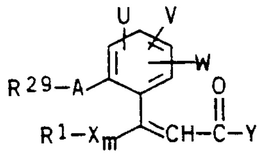

1a

in der U, V, W, R1, $X_m$ und Y die in Anspruch 1 angegebenen Bedeutungen haben und -A- für die Gruppierungen -C≡C-, $-CR^{30}=CR^{31}-$, $-CHR^{30}-CHR^{31}-$, $-O-CHR^{31}-$, -O-CO-, $-NR^{30}CO-$, $-S-CHR^{31}-$, $-N=CR^{31}-$, $-COO-CHR^{31}-$, $-O-N=CR^{31}-$, $-R^{30}C=N-O-CHR^{31}-$, $-CR^{30}=N-$, -N=N- oder $-CHR^{30}-$ steht und in der $R^{29}$ gegebenenfalls substituiertes Alkyl, gegebenenfalls substituiertes Alkenyl, gegebenenfalls substituiertes Alkinyl, gegebenenfalls substituiertes Aralkyl, gegebenenfalls substituiertes Heteroarylalkyl, gegebenenfalls substituiertes Aryloxyalkyl, gegebenenfalls substituiertes Heteroaryloxyalkyl oder Heteroarylthioalkyl oder gegebenenfalls substituiertes Aryl oder gegebenenfalls substituiertes Heteroaryl bedeutet und $R^{30}$ und $R^{31}$ gleich oder verschieden sind und geradkettiges oder verzweigtes $C_1$-$C_4$-Alkyl oder Wasserstoff bedeuten.

**4.** Verbindungen der Formel Ib

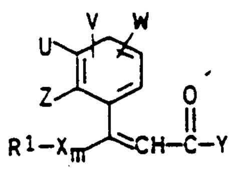

1b

in der die Gruppe

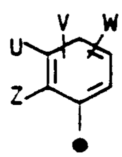

für die folgenden Gruppen steht:

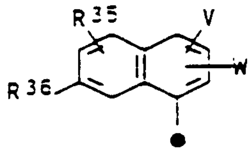
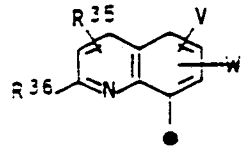
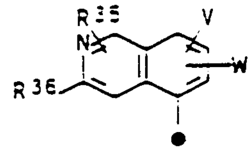

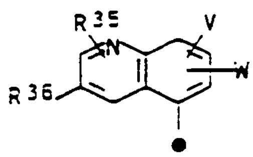
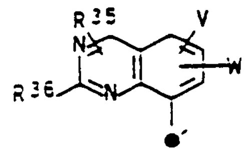
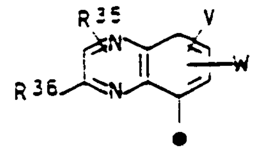

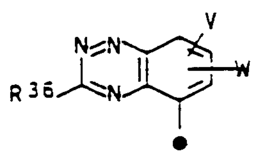
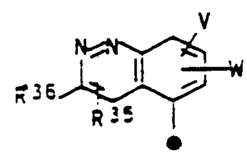
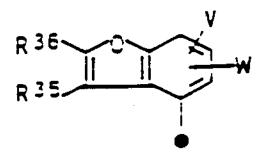

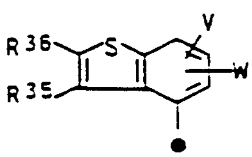
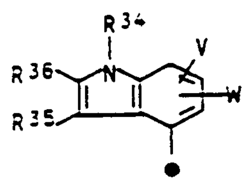
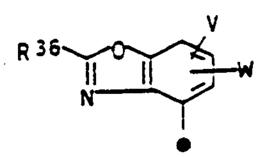

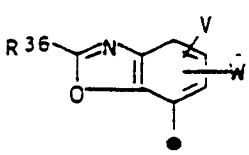
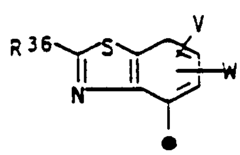
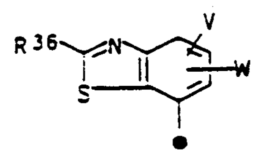

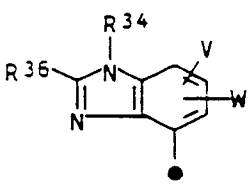
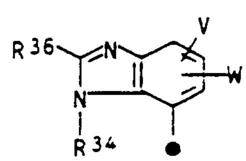

wobei

- $R^{34}$ Wasserstoff oder $C_1$-$C_4$-Alkyl bedeutet,
- $R^{35}$ Wasserstoff, Methyl, Methoxy, Trifluormethyl, Fluor, Chlor, Brom, Cyano oder Nitro bedeutet,
- $R^{36}$ gegebenenfalls einfach oder mehrfach durch Halogen, $C_1$-$C_{12}$-Alkyl, $C_1$-$C_{12}$-Alkenyl, gegebenenfalls substituiertem Aryl, gegebenenfalls substituiertem Heteroaryl, gegebenenfalls substituiertem Aralkyl, $C_1$-$C_{12}$-Alkoxy, $C_1$-$C_{12}$-Halogenalkoxy, $C_1$-$C_{12}$-Alkenyloxy, $C_1$-$C_{12}$-Alkinyloxy, gegebenenfalls substituiertem Aryloxy, Formyl, $C_1$-$C_{12}$-Acyl, Cyano, Trifluormethyl, Nitro oder mit der Gruppe $-CR^{32}=NOR^{33}$ substituiertes oder gegebenenfalls mit einem Benzolring anneliertes Phenyl, Furyl, Thienyl, Pyrrolyl, Isoxazolyl, Isothiazolyl, Oxazolyl, Thiazolyl, 1,2,4-Oxadiazolyl, 1,2,4-Thiadiazolyl, 1,3,4-Oxadiazolyl, 1,3,4-Thiadiazolyl, Imidazolyl, Pyrazolyl, 1,2,4-Triazolyl, 1,3,4-Triazolyl, Pyridinyl, Pyrimidinyl, Pyridazinyl, Pyrazinyl, 1,3,5-Triazolyl oder 1,2,4-Triazinyl bedeutet, und

in der $R^1$ $X_m$ und Y die in Anspruch 1 gegebene Bedeutung haben.

**5.** Mittel zur Behandlung von Pflanzen mit fungizider, insektizider oder akarizider Wirkung enthaltend eine wirksame Menge einer Verbindung der Formel 1 gemäß Anspruch 1 und einen inerten Trägerstoff.

**6.** Pharmazeutische Mittel mit antimycotischer Wirkung enthaltend eine wirksame Menge einer Verbindung der Formel 1 und einen inerten Trägerstoff.

**7.** Verbindungen der Formel 2, in der

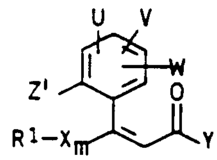

2

Z' für die Gruppen Cl, Br, CN, SH, $NH_2$, $CH_3$, $C_2H_5$, $CH_2Cl$, $CH_2Br$, $-CH_2OCH_3$, $-CH_2OH$, Acetyl, $-CH=CH_2$, $-C\equiv CH$, $-O-CH_2-CH=CH_2$ steht sowie zusätzlich CHO, OH oder COOH bedeutet, falls $m=1$ ist, und $R^1$, $X_m$, Y, U, V und W die in Anspruch 1 angegebene Bedeutung haben.

**8.** Verbindungen der Formel 2 gemäß Anspruch 7 in der X die im Anspruch 2 genannten Bedeutungen hat.

**9.** Verbindungen der Formel 3,

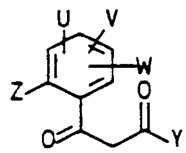

in der Z die in Anspruch 1 angegebene Bedeutung hat, U, V und W Wasserstoff bedeuten und Y für Methoxy steht, mit Ausnahme der Verbindungen,

- in denen Z Hydroxy, Methoxy, Phenoxy, Benzyloxy, n-Tetradecyloxy, 2-Butyloctyloxy, n-Hexadecylthio, Nitro, Amino, N-Formylamino, N-(Undecylsulfonyl)amino, Dimethylaminosulfonyl, Isohexadecenyloxy, Octadecyloxy, Thien-2-yl und 3,5-Dimethyl-thien-2-yl bedeutet,

sowie ausgenommen Verbindungen, in denen Z für eine Gruppe $-Z^a-Z^b$ steht, wobei $Z^a$ und $Z^b$ die folgende Bedeutung haben:

$Z^a$ Methylenoxy, Oxymethylen, Ethylen, Ethenylen, Thiomethylen oder Sauerstoff, und
$Z^b$ ggf. subst. Phenyl oder Naphthyl.

**10.** Verbindungen der Formel 3 gemäß Anspruch 9, in der Z für die Gruppe $OR^{12}$ oder $SR^{13}$ steht und $R^{12}$ und $R^{13}$ die in Anspruch 1 gegebene Bedeutung haben.

**11.** Verbindungen der Formel 3 gemäß Anspruch 10, in der $R^{12}$ und $R^{13}$ gegebenenfalls substituiertes Alkyl, gegebenenfalls substituiertes Aryl, oder gegebenenfalls substituiertes fünf- oder sechsgliedriges Heteroaryl mit 1 bis 3 Heteroatomen (N, O, S) bedeutet.

**12.** Verbindungen der Formel 4

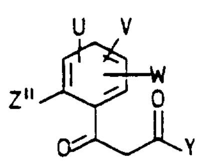

4

in der U, V und W Wasserstoff bedeuten, Y Methoxy bedeutet und Z'' $CH_3$, $C_2H_5$, $CH_2Cl$, $CH_2Br$,

$CH_2OCH_3$, $CH_2OH$, CHO, Acetyl, COOH, $-CH=CH_2$, $-C{\equiv}CH$ oder $-O-CH_2-CH=CH_2$ bedeutet.

**13.** Verfahren zur Herstellung von Verbindungen der Formel 1, in der U, V, W, Y, Z und $R^1$ die in Anspruch 1 angegebenen Bedeutungen haben und $X_m$ Sauerstoff bedeutet, dadurch gekennzeichnet, daß man Verbindungen der Formel 3 gemäß Anspruch 9, in der U, V, W, Y und Z die entsprechenden Bedeutungen haben, mit einem Orthoameisensäureester der Formel $(R^1O)_3CH$, in der $R^1$ die in Anspruch 1 angegebenen Bedeutungen hat, umsetzt.

**14.** Verfahren zur Herstellung von Verbindungen der Formel 1, in der U, V, W, Y, Z und $R^1$ die in Anspruch 1 angegebenen Bedeutungen haben und $X_m$ Sauerstoff bedeutet, dadurch gekennzeichnet, daß man Verbindungen der Formel 3 gemäß Anspruch 9, in der U, V, W, Y und Z die entsprechenden Bedeutungen haben, mit einem Alkylierungsmittel der Formel $R^1$-Nuc, in der $R^1$ die in Anspruch 1 angegebenen Bedeutungen hat und Nuc eine nucleofuge Abgangsgruppe wie Cl-, Br-, J-, Mesylat, Tosylat oder $R^{10}SO_2O$- bedeutet, gegebenenfalls in Gegenwart einer Base und eines - bevorzugt dipolar aprotischen - Verdünnungsmittels unter O-Alkylierung umsetzt.

**15.** Verfahren zur Herstellung von Verbindungen der Formel 1 gemäß Anspruch 1, in der U, V, W, Y, Z und $R^1$ die oben angegebene Bedeutung haben und $m=0$ ist, dadurch gekennzeichnet, daß man ein Keton der Formel 27, in der U, V, W, Z und $R^1$ die oben angegebenen Bedeutungen haben, mit einer Phosphoniumverbindung der Formel $(C_6H_5)_3P^{\oplus}-CH_2COY\ Hal^{\ominus}$, in der Y die oben angegebene Bedeutung hat und Hal Cl, Br oder J bedeutet, in Gegenwart einer Base und ggf. in Gegenwart eines Verdünnungsmittels im Sinne einer Wittig-Reaktion gemäß folgendem Reaktionsschema umsetzt.

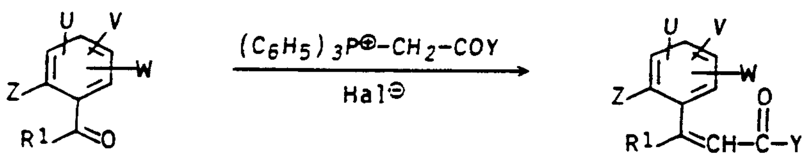


27 — 1 (m=0)

**16.** Verfahren gemäß Anspruch 15, dadurch gekennzeichnet, daß man anstelle der Phosphoniumverbindung einen Phosphonsäureester der Formel $(R'')_2PO-CH_2COY$, in der Y die oben angegebene Bedeutung hat und R'' für Niederalkoxy steht, im Sinne einer Wittig-Horner-Reaktion verwendet.

**17.** Verfahren gemäß Anspruch 15, dadurch gekennzeichnet, daß man anstelle der Phosphoniumverbindung ein Halogenessigsäurederivat der Formel $HalCH_2COY$, in der Hal Chlor oder Brom bedeutet und Y die oben angegebene Bedeutung hat, und Zink im Sinne einer Reformatsky-Reaktion verwendet und das Reaktionsprodukt anschließend dehydratisiert.

**18.** Verfahren zur Herstellung von Verbindungen der Formel 1a gemäß Anspruch 3, in der U, V, W, $X_m$, Y, $R^1$ und $R^{29}$ die oben angegebenen Bedeutungen haben und A für die Gruppe $-CR^{30}=CR^{31}$- steht, in der $R^{30}$ und $R^{31}$ Wasserstoff oder $C_1$-$C_4$-Alkyl bedeuten, dadurch gekennzeichnet, daß man eine Carbonylverbindung der Formel $R^{29}R^{30}C=O$ gegebenenfalls in Gegenwart einer Base und eines Lösungsmittels, mit einer Phosphoniumverbindung der Formel 39, in der Hal Cl, Br oder J bedeutet und die übrigen Symbole die oben angegebene Bedeutung haben, im Sinne einer Wittig-Reaktion oder mit einem entsprechenden Phosphonester der Formel 40, in der $R^{101}$ für $C_1$-$C_8$-Alkyl steht und die übrigen Symbole die oben angegebene Bedeutung haben, im Sinne einer Wittig-Horner-Reaktion umsetzt.

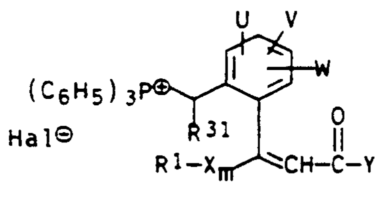

39

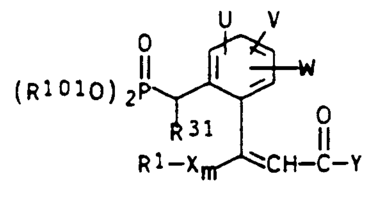

40

**19.** Verfahren zur Herstellung von Verbindungen der Formel 1a gemäß Anspruch 3, in der U, V, W, $X_m$, Y, $R^1$ und $R^{29}$ die oben angegebenen Bedeutungen haben und -A- für die Gruppe $-CR^{30}=CR^{31}-$ steht, in der $R^{30}$ und $R^{31}$ gleich oder verschieden sind und Wasserstoff oder $C_1$-$C_4$-Alkyl bedeuten, dadurch gekennzeichnet, daß man eine Carbonylverbindung der Formel 35, in der die einzelnen Symbole die oben angegebene Bedeutung haben gegebenenfalls in Gegenwart einer Base und eines Verdünnungsmittels mit einer Phosphoniumverbindung der Formel $R^{29}R^{30}CHP^{\oplus}(C_6H_5)_3$ $Hal^{\ominus}$, in der Hal Cl, Br oder J bedeutet und $R^{29}$ und $R^{30}$ die oben angegebene Bedeutung haben, im Sinne einer Wittig-Reaktion oder mit einem Phosphonester der Formel $R^{29}R^{30}CHPO(OR^{101})_2$, in der $R^{29}$ und $R^{30}$ die oben angegebenen Bedeutungen haben und $R^{101}$ $C_1$-$C_8$-Alkyl bedeutet, im Sinne einer Wittig-Horner-Reaktion umsetzt.

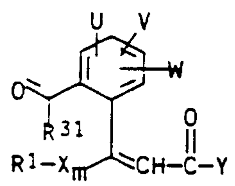

35

**20.** Verfahren zur Herstellung von Verbindungen der Formel 1a gemäß Anspruch 3, in der U, V, W, $X_m$, Y, $R^1$ und $R^{29}$ die oben angegebenen Bedeutungen haben und -A- die Gruppen $-O-CHR^{31}-$, $-S-CHR^{31}-$, $-COO-CHR^{31}-$, oder $-R^{30}C=N-O-CHR^{31}-$ bedeutet, dadurch gekennzeichnet, daß man das Halogenatom Hal in Benzylhalogeniden der Formel 32,

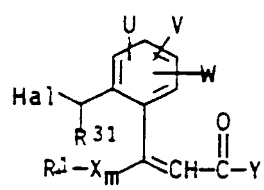

32

in der Hal Cl, Br, oder J bedeutet und die übrigen Symbole die oben angegebene Bedeutung haben, gegebenenfalls in Gegenwart einer Base und eines Verdünnungsmittels durch ein entsprechendes Nucleophil der Formel $R^{29}O$, $R^{29}S$, $R^{29}COO$ bzw. $R^{29}R^{30}C=N-O$, in denen $R^{29}$ und $R^{30}$ die oben angegebenen Bedeutungen haben, ersetzt.

**21.** Verfahren zur Herstellung von Verbindungen der Formel 1 gemäß Anspruch 1, in der Z für $OR^{12}$ oder $SR^{13}$ steht und in der U, V, W, $X_m$, Y, $R^1$, $R^{12}$ und $R^{13}$ die oben angegebenen Bedeutungen haben, dadurch gekennzeichnet, daß man eine Verbindung der Formel 1, in der Z OH oder SH bedeutet, mit einem Alkylierungsmittel, Arylierungsmittel bzw. Heteroarylierungsmittel der Formel $HalR^{12}$ oder $HalR^{13}$, in dem $R^{12}$ bzw. $R^{13}$ die oben angegebenen Bedeutungen haben, und Hal F, Cl, Br oder J bedeutet, gegebenenfalls in Gegenwart eines Verdünnungsmittels und bevorzugt in Gegenwart einer Base und gegebenenfalls in Gegenwart eines Übergangsmetallkatalysators umsetzt.

**22.** Verfahren zur Bekämpfung von Pilzen, dadurch gekennzeichnet, daß man die Pilze oder die Pflanzen, Saatgüter, Materialien oder den Boden mit einer fungizid wirksamen Menge einer Verbindung der Formel 1 gemäß Anspruch 1 behandelt.

**23.** Verfahren zur Bekämpfung von Insekten, dadurch gekennzeichnet, daß man die Insekten oder den Ort, wo sich Insekten aufhalten, mit einer insektizid wirksamen Menge einer Verbindung der Formel 1 gemäß Anspruch 1 behandelt.

## Claims

**1.** A $\beta$-substituted cinnamic acid derivative of the formula 1,

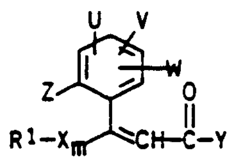

1

where

- $R^1$ is unsubstituted or halogen-, $C_1$-$C_6$-alkoxy-, $C_1$-$C_6$-alkylthio- or $C_3$-$C_6$-cycloalkyl-substituted $C_1$-$C_8$-alkyl, $C_2$-$C_8$-alkenyl, $C_2$-$C_8$-alkynyl, $C_3$-$C_8$-cycloalkyl or $C_3$-$C_8$-cycloalkenyl, and $R^1$ is additionally chlorine or bromine when m is 0,
- -X- is -O-, -S-,

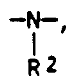, 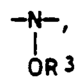,

- m is 0 or 1,
- -Y is $-OR^4$, $-O-N=CR^5R^6$, $-NR^7R^8$, $-N(OR^9)R^{10}$ or $-SR^{11}$,
  where the substituents $R^2$ to $R^{11}$ are, independently of one another, identical or different and are unsubstituted or halogen-, $C_1$-$C_6$-alkoxy-, $C_1$-$C_6$-alkylthio- or $C_3$-$C_6$-cycloalkyl-substituted $C_1$-$C_8$-alkyl, $C_2$-$C_8$-alkenyl, $C_2$-$C_8$-alkynyl, $C_3$-$C_8$-cycloalkyl or $C_3$-$C_8$-cycloalkenyl, and $R^2$, $R^3$ and $R^5$ to $R^{11}$ are additionally hydrogen when $R^4$ is not ethyl or t-butyl and when m is 0,
- Z is halogen, nitro, cyano, unsubstituted or substituted alkyl, unsubstituted or substituted cycloalkyl, unsubstituted or substituted aralkyl, unsubstituted or substituted aryloxyalkyl, unsubstituted or substituted arylthioalkyl, unsubstituted or substituted hetarylalkyl, unsubstituted or substituted hetaryloxyalkyl, unsubstituted or substituted hetarylthioalkyl, unsubstituted or substituted alkenyl, unsubstituted or substituted aralkenyl, unsubstituted or substituted aryloxyalkenyl, unsubstituted or substituted arylthioalkenyl, unsubstituted or substituted hetarylalkenyl, unsubstituted or substituted hetaryloxyalkenyl, unsubstituted or substituted hetarylthioalkenyl, unsubstituted or substituted alkynyl, unsubstituted or substituted arylalkynyl, unsubstituted or substituted hetarylalkynyl, unsubstituted or substituted aryl, unsubstituted or substituted hetaryl, unsubstituted or substituted arylazo, unsubstituted or substituted acylamino, $-OR^{12}$, $-SR^{13}$, $-SOR^{14}$, $-SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$, $-CH_2-OCOR^{39}$ or $-NR^{37}R^{38}$,
  where $R^{12}$ to $R^{28}$ and $R^{38}$ and $R^{39}$ are identical or different and are hydrogen, unsubstituted or substituted alkyl, unsubstituted or substituted alkenyl, unsubstituted or substituted alkynyl, unsubstituted or substituted cycloalkyl, unsubstituted or substituted cycloalkylalkyl, unsubstituted or substituted aryl, unsubstituted or substituted hetaryl, unsubstituted or substituted aralkyl, unsubstituted or substituted hetarylalkyl, unsubstituted or substituted aryloxyalkyl, unsubstituted or substituted arylthioalkyl, unsubstituted or substituted hetaryloxyalkyl or unsubstituted or substituted hetarylthioalkyl, and $R^{37}$ is hydrogen or $C_1$-$C_4$-alkyl, and $R^{19}$ is not hydrogen and $R^{12}$ is not unsubstituted or substituted alkyl when m is 0,

and where

U, V and W are identical or different and are hydrogen or have one of the meanings specified for Z,

or where

two of the groups Z, U, V or W in adjacent positions on the phenyl ring may, together with the carbons of which they are substituents, form an unsubstituted or substituted five- or six-membered aromatic or aliphatic ring which is fused onto the phenyl ring and may contain one to three hetero atoms (N, S, O), excepting the compounds

- methyl 3-methoxy-3-[2-methoxyphenyl]acrylate,
- methyl 3-methoxy-3-[(1-hydroxy-3-methyl-6,8-dimethoxy)-2-naphthyl]acrylate,
- methyl 3-chloro-3-[2-methyl-1-naphthyl]acrylate,
- methyl 3-methyl-3-[2-methyl-1-naphthyl]acrylate,
- methyl 3-methyl-3-[(2-isopropylcarboxyl-4-methoxycarbonyl)phenyl]acrylate,

and compounds where Z is $NO_2$, $NH_2$ or N-pyrrolidone when $R^1X_m$ is methyl, U, V and W are hydrogen, and Y is alkylamino or cycloalkylamino.

**2.** A compound of the formula 1 as claimed in claim 1, where X is oxygen or sulfur.

**3.** A compound as claimed in claim 1 of the formula 1a

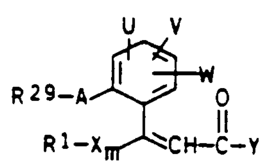

1a

where U, V, W, $R^1$, $X_m$ and Y have the meanings stated in claim 1, and -A- is -C≡C-, $-CR^{30}=CR^{31}-$, $-CHR^{30}-CHR^{31}-$, $-O-CHR^{31}-$, -O-CO-, $-NR^3 \circ CO-$, $-S-CHR^{31}-$, $-N=CR^{31}-$, $-COO-CHR^{31}-$, $-O-N=CR^{31}-$, $-R^{30}C=N-O-CHR^{31}-$, $-CR^{30}=N-$, -N=N-or $-CHR^{30}-$, and where $R^{29}$ is unsubstituted or substituted alkyl, unsubstituted or substituted alkenyl, unsubstituted or substituted alkynyl, unsubstituted or substituted aralkyl, unsubstituted or substituted hetarylalkyl, unsubstituted or substituted aryloxyalkyl, unsubstituted or substituted hetaryloxyalkyl or hetarylthioalkyl or unsubstituted or substituted aryl or unsubstituted or substituted hetaryl and $R^{30}$ and $R^{31}$ are identical or different and are straight-chain or branched $C_1$-$C_4$-alkyl or hydrogen.

**4.** A compound of the formula 1b

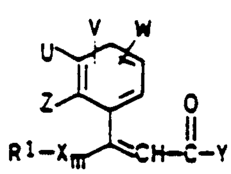

1b

where the group

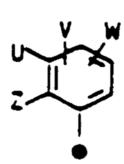

is one of the following groups:

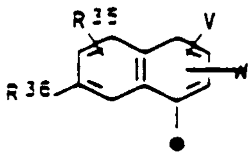
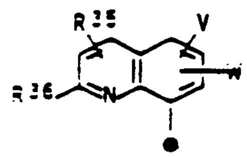
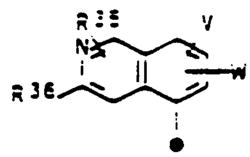
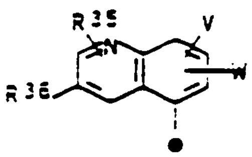
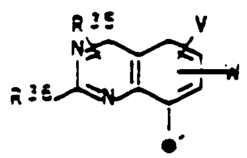
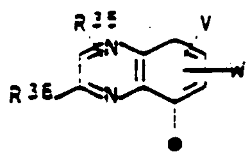
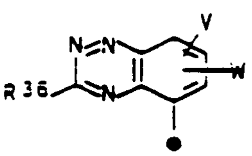
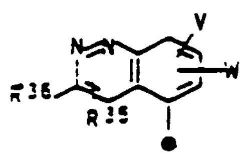
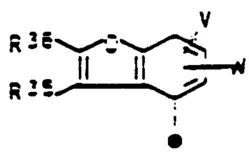
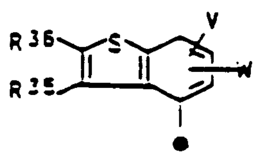
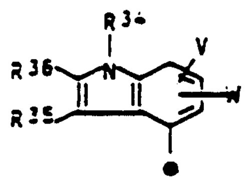
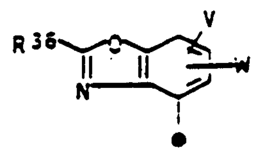
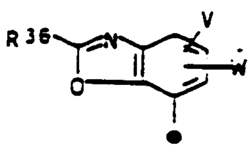
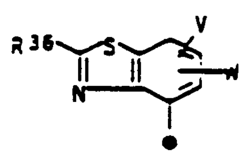
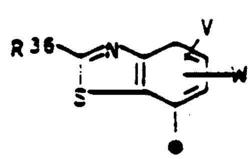
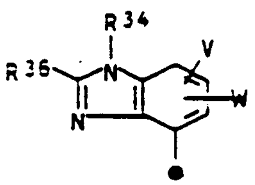
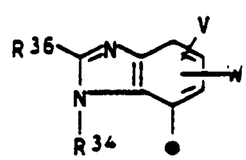

where

$R^{34}$ is hydrogen or $C_1$-$C_4$-alkyl,

$R^{35}$ is hydrogen, methyl, methoxy, trifluoromethyl, fluorine, chlorine, bromine, cyano or nitro,

$R^{36}$ is phenyl, furyl, thienyl, pyrrolyl, isoxazolyl, isothiazolyl, oxazolyl, thiazolyl, 1,2,4-oxadiazolyl, 1,2,4-thiadiazolyl, 1,3,4-oxadiazolyl, 1,3,4-thiadiazolyl, imidazolyl, pyrazolyl, 1,2,4-triazolyl, 1,3,4-triazolyl, pyridinyl, pyrimidinyl, pyridazinyl, pyrazinyl, 1,3,5-triazolyl or 1,2,4-triazinyl, each of which is unsubstituted or substituted one or more times by halogen, $C_1$-$C_{12}$-alkyl, $C_1$-$C_{12}$-alkenyl, unsubstituted or substituted aryl, unsubstituted or substituted hetaryl, unsubstituted or substituted aralkyl, $C_1$-$C_{12}$-alkoxy, $C_1$-$C_{12}$-haloalkoxy, $C_1$-$C_{12}$-alkenyloxy, $C_1$-$C_{12}$-alkynyloxy, unsubstituted or substituted aryloxy, formyl, $C_1$-$C_{12}$-acyl, cyano, trifluoromethyl, nitro or $-CR^{32}=NOR^{33}$ and may be fused to a benzene ring, and where $R^1$, $X_m$ and Y have the meanings given in claim 1.

**5.** An agent for the treatment of plants with fungicidal, insecticidal or acaricidal effect containing an effective amount of a compound of the formula 1 as claimed in claim 1 and an inert carrier.

**6.** A pharmaceutical agent with antimycotic effect containing an effective amount of a compound of the formula 1 and an inert carrier.

**7.** A compound of the formula 2

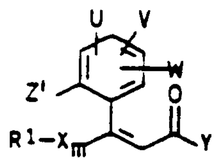

2

where Z' is Cl, Br, CN, SH, $NH_2$, $CH_3$, $C_2H_5$, $CH_2Cl$, $CH_2Br$, $-CH_2OCH_3$, $-CH_2OH$, acetyl, $-CH=CH_2$, $-C\equiv CH$, $-O-CH_2-CH=CH_2$,and is additionally CHO, OH or COOH when m is 1, and $R^1$, $X_m$, Y, U, V and W have the meanings stated in claim 1.

**8.** A compound of the formula 2 as claimed in claim 7, where X has the meanings specified in claim 2.

**9.** A compound of the formula 3

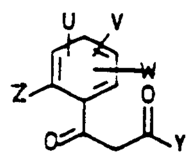

where Z has the meanings stated in claim 1, U, V and W are hydrogen, and Y is methoxy, with the exception of the compounds

- where Z is hydroxyl, methoxy, phenoxy, benzyloxy, n-tetradecyloxy, 2-butyloctyloxy, n-hexadecylthio, nitro, amino, N-formylamino, N-(undecylsulfonyl)amino, dimethylaminosulfonyl, isohexadecenyloxy, octadecyloxy, 2-thienyl and 3,5-dimethyl-2-thienyl, and excepting compounds where Z is $-Z^a-Z^b$, where $Z^a$ and $Z^b$ have the following meanings:
  $Z^a$ methyleneoxy, oxymethylene, ethylene, ethenylene, thiomethylene or oxygen, and
  $Z^b$ unsubstituted or substituted phenyl or naphthyl.

**10.** A compound of the formula 3 as claimed in claim 9, where Z is $OR^{12}$ or $SR^{13}$, and $R^{12}$ and $R^{13}$ have the meanings given in claim 1.

**11.** A compound of the formula 3 as claimed in claim 10, where $R^{12}$ and $R^{13}$ are unsubstituted or substituted alkyl, unsubstituted or substituted aryl, or unsubstituted or substituted five- or six-membered hetaryl with 1 to 3 hetero atoms (N, O, S).

**12.** A compound of the formula 4

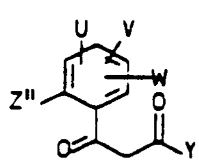

4

where U, V and W are hydrogen, Y is methoxy and Z'' is $CH_3$, $C_2H_5$, $CH_2Cl$, $CH_2Br$, $CH_2OCH_3$, $CH_2OH$, CHO, acetyl, COOH, $-CH=CH_2$, $-C\equiv CH$, or $-O-CH_2-CH=CH_2$.

**13.** A process for the preparation of a compound of the formula 1, where U, V, W, Y, Z and $R^1$ have the meanings stated in claim 1 and $X_m$ is oxygen, which comprises reacting compounds of the formula 3 as claimed in claim 9, where U, V, W, Y and Z have the appropriate meanings, with an orthoformic ester of the formula $(R^1O)_3CH$, where $R^1$ has the meanings stated in claim 1.

**14.** A process for the preparation of a compound of the formula 1, where U, V, W, Y, Z and $R^1$ have the meanings stated in claim 1 and $X_m$ is oxygen, which comprises subjecting compounds of the formula 3 as claimed in claim 9, where U, V, W, Y and Z have the appropriate meanings, to O-alkylation by reaction with an alkylating agent of the formula $R^1$-Nuc, where $R^1$ has the meanings stated in claim 1 and Nuc is a nucleofugic leaving group such as Cl-, Br-, I-, mesylate, tosylate or $R^{10}SO_2O$- in the presence or absence of a base and of a - preferably dipolar aprotic - diluent.

**15.** A process for preparing a compound of the formula 1 as claimed in claim 1, where U, V, W, Y, Z and $R^1$ have the meanings stated above, and m is 0, which comprises subjecting a ketone of the formula 27, where U, V, W, Z and $R^1$ have the meanings stated above, to a Wittig reaction with a phosphonium compound of the formula $(C_6H_5)_3P^{\oplus}$-$CH_2COY$ $Hal^{\ominus}$, where Y has the meaning stated above, and Hal is Cl, Br or I, in the presence of a base and in the presence or absence of a diluent, in accordance with the following reaction scheme.

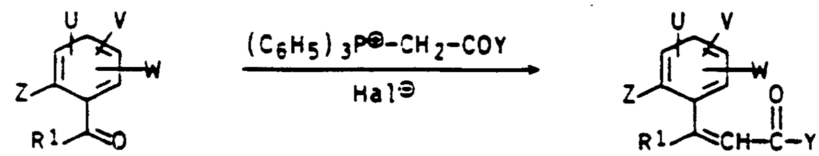


27 1 (m=0)

**16.** A process as claimed in claim 15, wherein a Wittig-Horner reaction is carried out using a phosphonic ester of the formula $(R'')_2PO$-$CH_2COY$, where Y has the meaning stated above, and R'' is lower alkoxy, in place of the phosphonium compound.

**17.** A process as claimed in claim 15, wherein a Reformatsky reaction is carried out using a halo-acetic acid derivative of the formula $HalCH_2COY$, where Hal is chlorine or bromine, and Y has the meaning stated above, in place of the phosphonium compound, and using zinc, and the reaction product is subsequently dehydrated.

**18.** A process for preparing a compound of the formula 1a as claimed in claim 3, where U, V, W, $X_m$, Y, $R^1$ and $R^{29}$ have the meanings stated above, and A is $CR^{30}=CR^{31}$- where $R^{30}$ and $R^{31}$ are hydrogen or $C_1$-$C_4$-alkyl, which comprises subjecting a carbonyl compound of the formula $R^{29}R^{30}C=O$, in the presence or absence of a base and of a solvent, to a Wittig reaction with a phosphonium compound of the formula 39 where Hal is Cl, Br or I and the other symbols have the meanings stated above, or to a Wittig-Horner reaction with a corresponding phosphonic ester of the formula 40, where $R^{101}$ is $C_1$-$C_8$-alkyl and the other symbols have the meanings stated above.

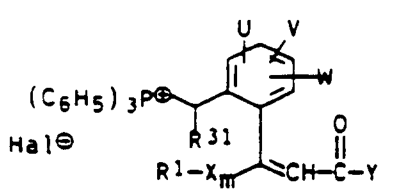

39

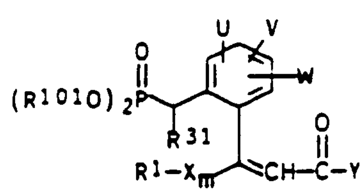

40

**19.** A process for preparing a compound of the formula 1a as claimed in claim 3, where U, V, W, $X_m$, Y, $R^1$ and $R^{29}$ have the meanings stated above, and -A- is $-CR^{30}=CR^{31}$- where $R^{30}$ and $R^{31}$ are identical or

different and are hydrogen or $C_1$-$C_4$-alkyl, which comprises subjecting a carbonyl compound of the formula 35, where the individual symbols have the meanings stated above, in the presence or absence of a base and of a diluent, to a Wittig reaction with a phosphonium compound of the formula $R^{29}R^{30}CHP^{\oplus}(C_6H_5)_3Hal^{\ominus}$, where Hal is Cl, Br or I and $R^{29}$ and $R^{30}$ have the meanings stated above, or to a Wittig-Horner reaction with a phosphonic ester of the formula $R^{29}R^{3\circ}CHPO(OR^{101})_2$, where $R^{29}$ and $R^{30}$ have the meanings stated above, and $R^{101}$ is $C_1$-$C_8$-alkyl.

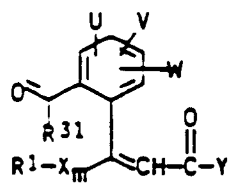

35

**20.** A process for preparing a compound of the formula 1a as claimed in claim 3, where U, V, W, $X_m$, Y, $R^1$ and $R^{29}$ have the meanings stated above, and -A- is -O-$CHR^{31}$-, -S-$CHR^{31}$-, -COO-$CHR^{31}$- or -$R^{30}C=N$-O-$CHR^{31}$-, which comprises replacing the halogen atom Hal in benzyl halides of the formula 32

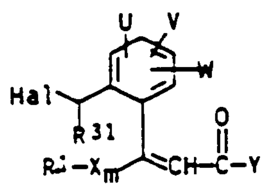

32

where Hal is Cl, Br or I and the other symbols have the meanings stated above, in the presence or absence of a base and of a diluent, by an appropriate nucleophile of the formula $R^{29}O$, $R^{29}S$, $R^{29}COO$ or $R^{29}R^{30}C=N$-O where $R^{29}$ and $R^{30}$ have the meanings stated above.

**21.** A process for preparing a compound of the formula 1 as claimed in claim 1, where Z is $OR^{12}$ or $SR^{13}$ and where U, V, W, $X_m$, Y, $R^1$, $R^{12}$ and $R^{13}$ have the meanings stated above, which comprises reacting a compound of the formula 1 where Z is OH or SH with an alkylating agent, arylating agent or hetarylating agent of the formula $HalR^{12}$ or $HalR^{13}$ where $R^{12}$ and $R^{13}$ have the meanings stated above, and Hal is F, Cl, Br or I, in the presence or absence of a diluent, and preferably in the presence of a base, and in the presence or absence of a transition metal catalyst.

**22.** A method for controlling fungi, which comprises treating the fungi or the plants, seeds, materials or the soil with a fungicidally effective amount of a compound of the formula 1 as claimed in claim 1.

**23.** A method for controlling insects, which comprises treating the insects or the place where the insects are present with an insecticidally effective amount of a compound of the formula 1 as claimed in claim 1.

**Revendications**

**1.** Dérivés de l'acide cinnamique substitués en bêta, répondant à la formule générale I

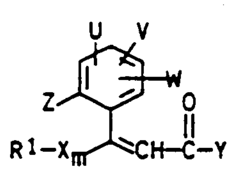

I

dans laquelle

$R^1$ représente un groupe alkyle en C1-C8, alcényle en C2-C8, alcynyle en C2-C8, cycloalkyle en C3-C8 ou cycloalcényle en C3-C8 éventuellement substitué par des halogènes, des groupes alcoxy en C1-C6, alkylthio en C1-C6 ou cycloalkyle en C3-C6, $R^1$ pouvant en outre représenter le chlore ou le brome lorsque m = 0,

-X- représente -O-, -S-,

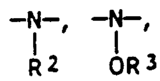

m est égal à 0 ou 1,

-Y représente l'un des groupes $-OR^4$, $-O-N=CR^5R^6$, $-NR^7R^8$, $-N(OR^9)R^{10}$ ou $-SR^{11}$, $R^2$ à $R^{11}$, ayant des significations identiques ou différentes, représentant chacun, indépendamment les uns des autres, un groupe alkyle en C1-C8, alcényle en C2-C8, alcynyle en C2-C8, cycloalkyle en C3-C8 ou cycloalcényle en C3-C8 éventuellement substitué par des halogènes, des groupes alcoxy en C1-C6, alkylthio en C1-C6 ou cycloalkyle en C3-C6, les symboles $R^2$, $R^3$ et $R^5$ à $R^{11}$ pouvant en outre représenter l'hydrogène lorsque $R^4$ a une signification autre que éthyle ou tert-butyle et lorsque m = 0,

Z représente un halogène, un groupe nitro, cyano, un groupe alkyle éventuellement substitué, cycloalkyle éventuellement substitué, aralkyle éventuellement substitué, aryloxyalkyle éventuellement substitué, arylthioalkyle éventuellement substitué, hétéroarylalkyle éventuellement substitué, hétéroaryloxyalkyle éventuellement substitué, hétéroarylthioalkyle éventuellement substitué, alcényle éventuellement substitué, aralcényle éventuellement substitué, aryloxyalcényle éventuellement substitué, arylthioalcényle éventuellement substitué, hétéroarylalcényle éventuellement substitué, hétéroaryloxyalcényle éventuellement substitué, hétéroarylthioalcényle éventuellement substitué, alcynyle éventuellement substitué, arylalcynyle éventuellement substitué, hétéroarylalcynyle éventuellement substitué, aryle éventuellement substitué, hétéroaryle éventuellement substitué, arylazo éventuellement substitué, acylamino éventuellement substitué, $-OR^{12}$, $-SR^{13}$, $-SOR^{14}$, $-SO_2R^{15}$, $-COOR^{16}$, $-CONR^{17}R^{18}$, $-COR^{19}$, $-CR^{20}=NR^{21}$, $-N=CR^{22}R^{23}$, $-CR^{24}=N-OR^{25}$, $-CR^{25}R^{26}-O-N=CR^{27}R^{28}$, $-CH_2-OCOR^{39}$ ou $-NR^{37}R^{38}$,

les symboles $R^{12}$ à $R^{28}$, $R^{38}$ et $R^{39}$, ayant des significations identiques ou différentes, représentant chacun l'hydrogène, un groupe alkyle éventuellement substitué, alcényle éventuellement substitué, alcynyle éventuellement substitué, cycloalkyle éventuellement substitué, cycloalkylalkyle éventuellement substitué, aryle éventuellement substitué, hétéroaryle éventuellement substitué, aralkyle éventuellement substitué, hétéroarylalkyle éventuellement substitué, aryloxyalkyle éventuellement substitué, arylthioalkyle éventuellement substitué, hétéroaryloxyalkyle éventuellement substitué ou hétéroarylthioalkyle éventuellement substitué, $R^{37}$ représente l'hydrogène ou un groupe alkyle en C1-C4 et $R^{19}$ ne peut représenter l'hydrogène ni $R^{12}$ un groupe alkyle éventuellement substitué lorsque m = 0,

U, V, W, ayant des significations identiques ou différentes, représentent chacun l'hydrogène ou ont l'une des significations indiquées pour Z,

ou bien

deux des groupes Z, U, V ou W, situés dans des positions voisines du cycle phényle, et le cas échéant avec les atomes de carbone auxquels ils sont reliés, forment ensemble un cycle aromatique ou aliphatique à 5 ou 6 chaînons condensé avec le noyau phényle et éventuellement substitué et qui peut contenir le cas échéant un à trois hétéroatomes (N, S, O), à l'exception des composés

- 3-méthoxy-3-(2-méthoxyphényl)acrylate de méthyle,
- 3-méthoxy-3-[(1-hydroxy-3-méthyl-6,8-diméthoxy)naphto-2-yl]acrylate de méthyle,
- 3-chloro-3-(2-méthylnaphto-1-yl)acrylate de méthyle,
- 3-méthyl-3-(2-méthylnaphto-1-yl)-acrylate de méthyle,
- 3-méthyl-3-[(2-isopropylcarbonyl-4-méthoxycarbonyl)phényl]acrylate de méthyle,

et des composés pour lesquels Z représente un groupe $NO_2$, $NH_2$ ou N-pyrrolidone lorsque $R^1X_m$ représente un groupe méthyle, U, V et W l'hydrogène et Y un groupe alkylamino ou cycloalkylamino.

**2.** Composés de formule 1 selon la revendication 1, dans laquelle X représente l'oxygène ou le soufre.

**3.** Composés selon la revendication 1 de formule 1a

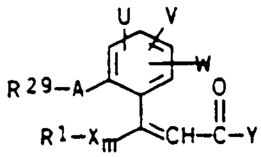

1a

dans laquelle U, V, W, $R^1$, $X_m$ et Y ont les significations indiquées dans la revendication 1, et -A- représente les groupements -C≡C-, $-CR^{30}=CR^{31}-$, $-CHR^{30}-CHR^{31}-$, $-O-CHR^{31}-$, -O-CO-, $-NR^{30}CO-$, $-S-CHR^{31}-$, $-N=CR^{31}-$, $-COO-CHR^{31}-$, $-O-N=CR^{31}-$, $-R^{30}C=N-O-CHR^{31}-$, $-CR^{30}=N-$, -N=N- ou $-CHR^{30}-$, et $R^{29}$ représente un groupe alkyle éventuellement substitué, alcényle éventuellement substitué, alcynyle éventuellement substitué, aralkyle éventuellement substitué, hétéroarylalkyle éventuellement substitué, aryloxyalkyle éventuellement substitué, hétéroaryloxyalkyle ou hétéroarylthioalkyle éventuellement substitué ou aryle éventuellement substitué ou aryle éventuellement substitué ou hétéroaryle éventuellement substitué et $R^{30}$ et $R^{31}$, ayant des significations identiques ou différentes, représentent chacun un groupe alkyle en C1-C4 à chaîne droite ou ramifiée ou l'hydrogène.

**4.** Composés de formule 1b

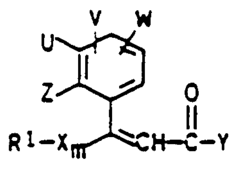

1b

dans laquelle le groupe

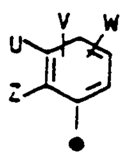

consiste en l'un des groupes suivants :

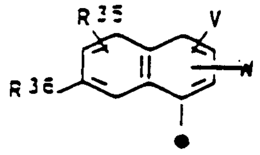
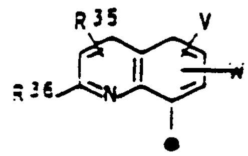
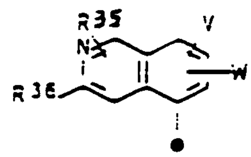
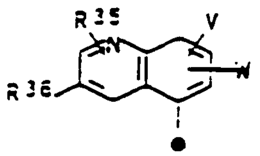
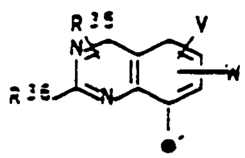
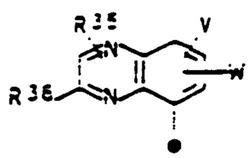
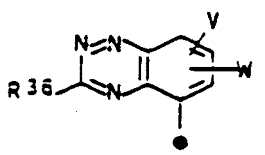
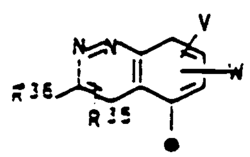
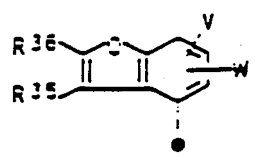
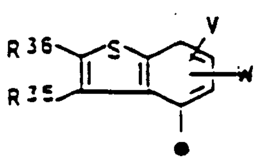
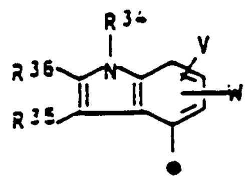
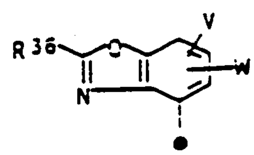
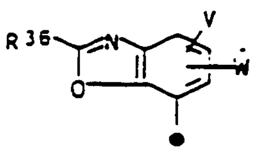
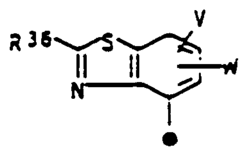
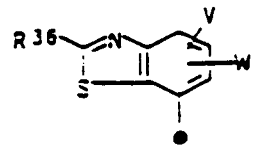
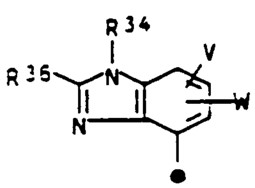
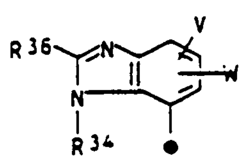

dans lesquels

$R^{34}$ représente l'hydrogène ou un groupe alkyle en C1-C4,

$R^{35}$ représente l'hydrogène, un groupe méthyle, methoxy, trifluorométhyle, le fluor, le chlore, le brome, un groupe cyano ou nitro,

$R^{36}$ représente un groupe phényle, furyle, thiényle, pyrrolyle, isoxazolyle, isothiazolyle, oxazolyle, thiazolyle, 1,2,4-oxadiazolyle, 1,2,4-thiadiazolyle, 1,3,4-oxadiazolyle, 1,3,4-thiadiazolyle, imidazolyle, pyrazolyle, 1,2,4-triazolyle, 1,3,4-triazolyle, pyridinyle, pyrimidinyle, pyridazinyle, pyrazinyle, 1,3,5-triazolyle ou 1,2,4-triazinyle portant éventuellement un ou plusieurs substituants choisis parmi les halogènes, les groupes alkyle en C1-C12, alcényle en C1-C12, aryle éventuellement substitué, hétéroaryle éventuellement substitué, aralkyle éventuellement substitué, alcoxy en C1-C12, halogénoalcoxy en C1-C12, alcényloxy en C1-C12, alcynyloxy en C1-C12, aryloxy éventuellement substitué, formyle, acyle en C1-C12, cyano, trifluorométhyle, nitro, ou substitué par le groupe $-CR^{32}=NOR^{33}$, et éventuellement condensé avec un noyau benzénique et $R^1$, $X_m$ et Y ont les significations indiquées dans la revendication 1.

**5.** Produit pour le traitement des plantes, possédant une activité fongicide, insecticide ou acaricide, qui contient une quantité efficace d'un composé de formule 1 selon la revendication 1 et un véhicule inerte.

**6.** Produit pharmaceutique à activité antimycotique, contenant une quantité efficace d'un composé de formule 1 et un véhicule inerte.

**7.** Composés de formule 2

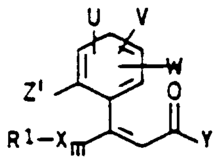

2

dans laquelle Z' représente Cl, Br, CN, SH, $NH_2$, $CH_3$, $C_2H_5$, $CH_2Cl$, $CH_2Br$, $-CH_2OCH_3$, $-CH_2OH$, un groupe acétyle, $-CH=CH_2$, $-C \equiv CH$, $-O-CH_2-CH=CH_2$, et peut en outre représenter CHO, OH ou COOH lorsque m = 1, et $R^1$, $X_m$, Y, U, V et W ont les significations indiquées dans la revendication 1.

**8.** Composés de formule 2 selon la revendication 7, dans laquelle X a les significations indiquées dans la revendication 2.

**9.** Composés de formule 3

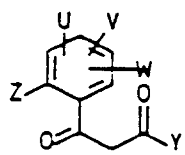

dans laquelle Z a les significations indiquées dans la revendication 1, U, V et W représentent l'hydrogène et Y représente un groupe méthoxy, à l'exception des composés :

- pour lesquels Z représente un groupe hydroxy, méthoxy, phénoxy, benzyloxy, n-tétradécyloxy, 2-butyloctyloxy, n-hexadécylthio, nitro, amino, N-formylamino, N-(undécylsulfonyl)amino, diméthylaminosulfonyle, isohexadécényloxy, octadécyloxy, thiéno-2-yle et 3,5-diméthylthiéno-2-yle,
  et des composés pour lesquels Z représente un groupe $-Z^a-Z^b$ dans lequel $Z^a$ et $Z^b$ ont les significations suivantes :
  $Z^a$ un groupe méthylèneoxy, oxyméthylène, éthylène, éthénylène, thiométhylène ou l'oxygène et
  $Z^b$ un groupe phényle ou naphtyle éventuellement substitué.

**10.** Composés de formule 3 selon la revendication 9, pour lesquels Z représente le groupe $OR^{12}$ ou $SR^{13}$, et $R^{12}$ et $R^{13}$ ont les significations indiquées dans la revendication 1.

**11.** Composés de formule 3 selon la revendication 10, dans laquelle $R^{12}$ et $R^{13}$ représentent des groupes alkyle éventuellement substitués, aryle éventuellement substitués, ou hétéroaryle à cinq ou six chaînons et un à trois hétéroatomes (N, O, S) éventuellement substitués.

**12.** Composés de formule 4

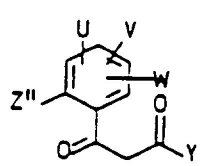

4

dans laquelle U, V et W représentent l'hydrogène, Y un groupe méthoxy et Z'' un groupe $CH_3$, $C_2H_5$,

$CH_2Cl$, $CH_2Br$, $CH_2OCH_3$, $CH_2OH$, CHO, acétyle, COOH, $-CH=CH_2$, $-C\equiv CH$ ou $-O-CH_2-CH=CH_2$.

**13.** Procédé de préparation des composés de formule 1 dans laquelle U, V, W, Y, Z et $R^1$ ont les significations indiquées dans la revendication 1 et $X_m$ représente l'oxygène, caractérisé en ce que l'on fait réagir des composés de formule 3 selon la revendication 9 dans laquelle U, V, W, Y et Z ont les significations correspondantes, avec un ester orthoformique de formule $(R^1O)_3CH$ dans laquelle $R^1$ a les significations indiquées dans la revendication 1.

**14.** Procédé de préparation des composés de formule 1 dans laquelle U, V, W, Y, Z et $R^1$ ont les significations indiquées dans la revendication 1 et Xm représente l'oxygène, caractérisé en ce que l'on fait réagir des composés de formule 3 selon la revendication 9, dans laquelle U, V, W, Y et Z ont les significations correspondantes, avec un agent alkylant de formule $R^1$-Nuc dans laquelle $R^1$ a les significations indiquées dans la revendication 1, et Nuc représente un substituant éliminable nucléofuge tel que Cl, Br, I, un groupe méthanesulfonate, toluènesulfonate et $R^{10}SO_2O$, éventuellement en présence d'une base et d'un diluant - de préférence aprotonique dipolaire -, par O-alkylation.

**15.** Procédé de préparation des composés de formule 1 de la revendication 1 dans laquelle U, V, W, X, Z et $R^1$ ont les significations indiquées ci-dessus et m = 0, caractérisé en ce que l'on fait réagir une cétone de formule 27 dans laquelle U, V, W, Z et $R^1$ ont les significations indiquées ci-dessus, avec un dérivé de phosphonium de formule $(C_6H_5)_3P^{\oplus}-CH_2COY\ Hal^{\ominus}$ dans laquelle Y a les significations indiquées ci-dessus et Hal représente le chlore, le brome ou l'iode, en présence d'une base et le cas échéant en présence d'un diluant, selon une réaction de Wittig, conformément à l'équation

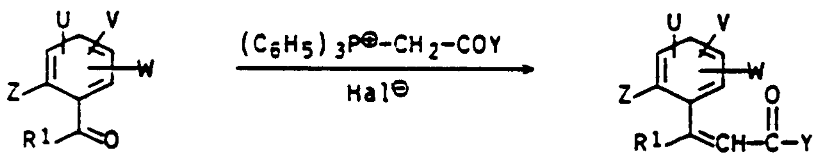


27 1 (m=0)

**16.** Procédé selon la revendication 15, caractérisé en ce que, à la place du dérivé de phosphonium, on utilise un ester phosphonique de formule $(R^{11})_2PO-CH_2COY$ dans laquelle Y a les significations indiquées ci-dessus et $R^{11}$ représente un groupe alcoxy inférieur, selon une réaction de Wittig-Horner.

**17.** Procédé selon la revendication 15, caractérisé en ce que, à la place du dérivé de phosphonium, on utilise un dérivé d'acide halogénoacétique de formule $HalCH_2COY$ dans laquelle Hal représente le chlore ou le brome et Y a les significations indiquées ci-dessus, et le zinc, selon une réaction de Reformatsky, après quoi on déshydrate le produit de réaction.

**18.** Procédé de préparation des composés de formule la selon la revendication 3, dans laquelle U, V, W, Xm, Y, $R^1$ et $R^{29}$ ont les significations indiquées ci-dessus et A représente le groupe $-CR^{30}=CR^{31}-$ dans lequel $R^{30}$ et $R^{31}$ représentent l'hydrogène ou des groupes alkyle en C1-C4, caractérisé en ce que l'on fait réagir un dérivé carbonylé de formule $R^{29}R^{30}C=O$, éventuellement en présence d'une base et d'un solvant, avec un dérivé de phosphonium de formule 39 dans laquelle Hal représente le chlore, le brome ou l'iode et les autres symboles ont les significations indiquées ci-dessus, dans le sens d'une réaction de Wittig, ou avec un ester phosphonique correspondant de formule 40 dans laquelle $R^{101}$ représente un groupe alkyle en C1-C6 et les autres symboles ont les significations indiquées ci-dessus, dans le sens d'une réaction de Wittig-Horner :

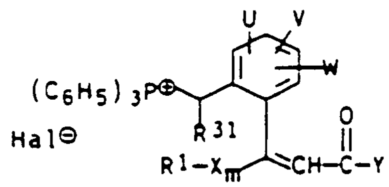

39

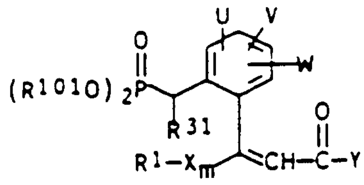

40

**19.** Procédé de préparation des composés de formule 1a de la revendication 3, dans laquelle U, V, W, Xm, Y, R1 et $R^{29}$ ont les significations indiquées ci-dessus et -A- représente le groupe $-CR^{30}-CR^{31}-$ dans lequel $R^{30}$ et $R^{31}$, ayant des significations identiques ou différentes, représentent chacun l'hydrogène ou un groupe alkyle en C1-C4, caractérisé en ce que l'on fait réagir un dérivé carbonylé de formule 35 dans laquelle les divers symboles ont les significations indiquées ci-dessus, le cas échéant en présence d'une base et d'un diluant, avec un dérivé de phosphonium de formule $R^{29}R^{30}CHP^{\oplus}(C_6H_5)_3$ $Hal^{\ominus}$ dans laquelle Hal représente Cl, Br ou I et $R^{29}$ et $R^{30}$ ont les significations indiquées ci-dessus, dans le sens d'une réaction de Wittig, ou avec un ester phosphonique de formule $R^{29}R^{30}CHPO(OR^{101})_2$ dans laquelle $R^{29}$ et $R^{30}$ ont les significations indiquées ci-dessus et $R^{101}$ représente un groupe alkyle en C1-C8, dans le sens d'une réaction de Wittig-Horner.

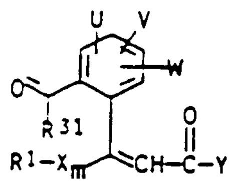

35

**20.** Procédé de préparation des composés de formule 1a de la revendication 3 dans laquelle U, V, W, Xm, Y, $R^1$ et $R^{29}$ ont les significations indiquées ci-dessus et -A- représente les groupes $-O-CHR^{31}-$, $-S-CHR^{31}-$, $-COO-CHR^{31}-$ ou $-R^{30}C=N-O-CHR^{31}-$, caractérisé en ce que, dans des halogénures de benzyle de formule 32

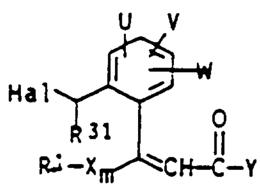

32

dans laquelle Hal représente le chlore, le brome ou l'iode et les autres symboles ont les significations indiquées ci-dessus, on remplace l'atome d'halogène Hal, éventuellement en présence d'une base et d'un diluant, par un groupe nucléophile correspondant de formule $R^{29}O$, $R^{29}S$, $R^{29}COO$ ou respectivement $R^{29}R^{30}C=N-O$, dans lesquels $R^{29}$ et $R^{30}$ ont les significations indiquées ci-dessus.

**21.** Procédé de préparation des composés de formule 1 de la revendication 1 dans laquelle Z représente $OR^{12}$ ou $SR^{13}$ et U, V, W, $X_m$, Y, $R^1$, $R^{12}$ et $R^{13}$ ont les significations indiquées ci-dessus, caractérisé en ce que l'on fait réagir un composé de formule 1 dans laquelle Z représente OH ou SH avec un agent alkylant, un agent arylant ou un agent hétéroarylant de formule $HalR^{12}$ ou $HalR^{13}$, $R^{12}$ et $R^{13}$ ayant les significations indiquées ci-dessus et Hal représentent le fluor, le chlore, le brome ou l'iode, éventuellement en présence d'un diluant et de préférence en présence d'une base et le cas échéant en présence d'un catalyseur à base d'un métal de transition.

**22.** Procédé pour combattre les mycètes, caractérisé en ce que l'on traite les mycètes ou les végétaux, semences, matières ou le sol par une quantité fongicide efficace d'un composé de formule 1 de la revendication 1.

**23.** Procédé pour combattre les insectes, caractérisé en ce que l'on traite les insectes ou leurs lieux de fréquentation par une quantité insecticide efficace d'un composé de formule 1 de la revendication 1.